# EUROPEAN PATENT APPLICATION

(11) **EP 1 939 294 A1**
(43) Date of publication of application: **02.07.2008**
(21) Application number: 07023008.1
(22) Date of filing: 07.08.2001
(51) Int. Cl.: C12N 15/31, C07K 14/20, C12N 15/62, C07K 19/00, C12N 15/63, A61K 48/00, A61K 39/02, G01N 33/53

(54) **Recombinant constructs of borrelia burgdorferi**

(30) Priority: 18.08.2000 US 226484 P; 19.09.2000 US 666017
(62) Divisional of application: 01959607.1
(71) Applicant: RESEARCH FOUNDATION OF STATE UNIVERSITY OF NEW YORK, Stony Brook, NY 11794-3369 (US); Brookhaven Sciences Associates, LLC, Upton, New York 11973-5000 (US)
(72) Inventor: Luft, Benjamin J., Riverhead, NY 11901 (US); Dunn, John J., Bellport, NY 11713-2417 (US)
(74) Representative: Kirkham, Nicholas Andrew

(57) **Abstract**

Chimeric nucleic acids, encoding chimeric Borrelia proteins comprising OspC or an antigenic fragment thereof and OspA or an antigenic fragment thereof, are disclosed. Chimeric proteins encoded by the nucleic acid sequences are also disclosed. The chimeric proteins are useful as vaccine immunogens against Lyme borreliosis, as well as for immunodiagnostic reagents.

## Description

### RELATED APPLICATIONS

This application is a continuation-in-part of U.S. Serial No. 09/666,017, filed September 19, 2000, which is itself a continuation-in-part of U.S. Serial No. 08/235,836, filed April 29, 1994, which is itself a continuation-in-part of U.S. Serial No. 08/148,191, filed November 1, 1993. This application also claims the benefit of U.S. Provisional Application No. 60/226,484, filed August 18, 2000. The teachings of all of these applications are incorporated herein by reference in their entirety.

### GOVERNMENT SUPPORT

The invention was supported, in whole or in part, by Grant 2R01AI37256-05A1 from the National Institute of Allergy and Infectious Diseases. The Government has certain rights in the invention.

### BACKGROUND OF THE INVENTION

Lyme disease (Lyme borreliosis) is the most common tick-borne infectious disease in North America and Europe, and has been found in Russia, Japan, China and Australia. Lyme disease begins at the site of a tick bite, producing a primary infection with spread of the organism to secondary sites occurring during the course of infection. The causative bacterial agent of this disease is the spirochete *Borrelia burgdorferi,* which was first isolated and cultivated in 1982 (Burgdorferi, W.A. et al., Science 216: 1317-1319 (1982); Steere, A.R. et al., N. Engl. J. Med. 308: 733-740 (1983)). With that discovery, a wide array of clinical syndromes, described in both the European and American literature since the early 20th century, could be attributed to infection by *B. burgdorferi* (Afzelius, A., Acta Derm. Venereol. 2: 120-125 (1921); Bannwarth, A., Arch. Psychiatr. Nervenkrankh. 117: 161-185 (1944); Garin, C. and A. Bujadouz, J Med. Lyon 71: 765-767 (1922); Herxheimer, K. and K. Hartmann, Arch. Dermatol. Syphilol. 61: 57-76, 255-300 (1902)).

Three pathogenic genospecies of *Borrelia, B. burgdorferi* sensu stricto *(B. burgdorferi* or *B.b.s.s.), B. afzelii* and *B. garinii* have been described (Baranton, G., et al., Int. J. Syst. Bacteriol. 42:378-383 (1992)). These are members of a species complex, *B. burgdorferi* sensu lato, which consists of at least 10 different genospecies (Piken, R.N., et al., J. Invest. Dermatol., 110:211-214 (1998); Postic, D., et al., Int. J. Syst. Bacteriol. 44:743-752 (1994); Valsangiacomo, C.T., et al., Int. J. Syst. Bacteriol. 47:1-10 (1997)). The three genospecies, *B. burgdorferi* sensu stricto, *B. afzelii* and *B. garinii,* all are thought to be pathogenic and all are found in Europe. However, in North America, *B. burgdorferi* sensu stricto is the only identified pathogenic genospecies. Each of these three genospecies is associated with distinct clinical manifestations (Van Dam, A. P. et al., Clin. Infect. Dis. 17:708-717 (1993)). This implies that differences in genospecies may play an important role in the wide array of clinical manifestations observed in Lyme Disease.

OspA is a basic lipoprotein of approximately 31 kd, which is encoded on a large linear plasmid along with OspB, a basic lipoprotein of approximately 34 kd (Szczepanski, A., and J.L. Benach, Microbiol. Rev. 55:21 (1991)). Analysis of isolates *of B. burgdorferi* obtained from North America and Europe has demonstrated that OspA has antigenic variability, and that several distinct groups can be serologically and genotypically defined (Wilske, B., et al., World J. Microbiol. 7: 130 (1991)). Other *Borrelia* proteins demonstrate similar antigenic variability. Surprisingly, the immune response to these outer surface proteins tends to occur late in the disease, if at all (Craft, J. E. et al., J. Clin Invest. 78: 934-939 (1986); Dattwyler, R.J. and B.J. Luft, Rheum. Clin. North Am. 15: 727-734 (1989)). Furthermore, patients acutely and chronically infected with *B. burgdorferi* respond variably to the different antigens, including OspA, OspB, OspC, OspD, p39, p41 and p93.

As an infected tick begins to feed on a mammal, the synthesis of another outer surface protein, outer surface protein C or OspC, is induced (Schwan, T.G., et al., Proc. Natl. Acad. Sci. 2:2909-2913 (1995)). Thus, in early infection, OspC is the major outer membrane protein expressed by the spirochete (Fung, B.P., et al., Infect. Immun. 62:3213-3221 (1994); Padula, S.J., et al., J. Clin. Microbiol., 32:1733-1738 (1994)). Even through OspC has been demonstrated to have limited surface exposure (Cox, D.L., et al., Proc. Natl. Acad. Sci., 93:7973-7978 (1996); Mathiesen, M. M., et al., Infect. Immun. 66:4073-4079 (1998)), OspC is a potent immunogen. Immunization with OspC is protective against tick-transmitted *Borrelia* infection (Gilmore Jr., R.D., Infect. Immun. 64:2234-2239 (1999)). However, because OspC is highly variable in its sequence, the protection is limited to the *Borrelia burgdorferi* strain expressing the same allele of OspC. Challenge with heterologous isolates, expressing other *ospC* alleles results in infection (Probert, W.S., et al., J. Infect. D., 175:400-405 (1997)). OspC is a very diverse genetic locus (Jauris-Heipke, S., et al., Med. Microbiol. Immunol. 182:37-50 (1993)) as evidenced by the fact that *Livey et al.* found thirty-four alleles of OspC in seventy-six *B. burgdorferi sensu lato* isolates (Livey, 1, et al., Mol. Microbiol. 18:257-269 (1995)).

Currently, Lyme Disease is treated with a range of antibiotics, e.g., tetracyclines, penicillin and cephalosporins. However, such treatment is not always successful in clearing the infection. Treatment is often delayed due to improper diagnosis with the deleterious effect that the infection proceeds to a chronic condition, where treatment with antibiotics is often not useful. One of the factors contributing to delayed treatment is the lack of effective diagnostic tools.

Vaccines against Lyme borreliosis have been attempted. Mice immunized with a recombinant form of OspA are protected from challenge with the same strain *of B. burgdorferi* from which the protein was obtained (Fikrig, E., et al., Science 250: 553-556 (1990)). Furthermore, passively transferred anti-OspA monoclonal antibodies (MAbs) have been shown to be protective in mice, and vaccination with a recombinant protein induced protective immunity against subsequent infection with the homologous strain of *B. burgdorferi* (Simon, M.M., et al., J. Infect. Dis. 164: 123 (1991)). In addition, there have been two independent trials of first generation vaccines for the prevention of Lyme disease that have studied the efficacy and safety of a vaccine based on recombinant outer surface protein A (OspA) (Sigal, L.H. et al., N. Engl. J. Med 339:216-222, 1998; Steere, A.C. et al., N. Engl. J. Med. 339:209-215, (1998)). However, a vaccine that consists of recombinant OspA may require frequent booster immunizations. An additional concern of OspA-based vaccines is the recent identification of a putative autoreactive OspA domain with a high degree of similarity to a region of human leukocyte function-associated antigen-1 (hLFA-1) (Gross, D.M. et al., Science, 281: 703-706 (1998)).

It has been noted that immunization with a single protein from a particular strain of *Borrelia* often does not confer resistance to that strain in all individuals (Fikrig, E. et al., J. Immunol. 7: 2256-1160 (1992)). There is considerable variation displayed in OspA, OspB and OspC, as well as p93, including the regions conferring antigenicity. Therefore, the degree and frequency of protection from vaccination with a protein from a single strain depend upon the response of the immune system to the particular variation, as well as the frequency of genetic variation in B. *burgdorferi.* In the case of vaccines directed against OspA, the vaccine is typically only effective against strains *of Borrelia* that express OspA that is homologous to OspA from which the vaccine was derived.

Another limitation of current OspA Lyme Disease vaccines is that they are directed against an antigen that is expressed predominantly in the tick vector. Indeed, recent reports have indicated that *Borrelia burgdorferi* in infected ticks alter their surface expression by increasing expression of OspC during ingestion of a blood meal (Schwan, T.G. et al., Proc. Natl. Acad. Sci. USA, 92: 2909-2913 (1995)). Thus, it seems that natural infection with *B. burgdorferi* does not elicit an antibody response to OspA, as it does against OspC.

Given the heterogeneity of antigenic determinants present in *Borrelia* proteins, a need exists for a vaccine and diagnostic tool which can provide immunogenicity to various strains and/or genospecies of *Borrelia burgdorferi,* as well as to more epitopes within a strain or genospecies. There is also a need for vaccines and diagnostic tools which detect antibody responses against immunoprotective targets that are expressed at different stages of the life cycle of *Borrelia burgdoferi.* This would allow for diagnosis and/or vaccination against all, or most forms, *of Borrelia* that cause systemic disease.

### SUMMARY OF THE INVENTION

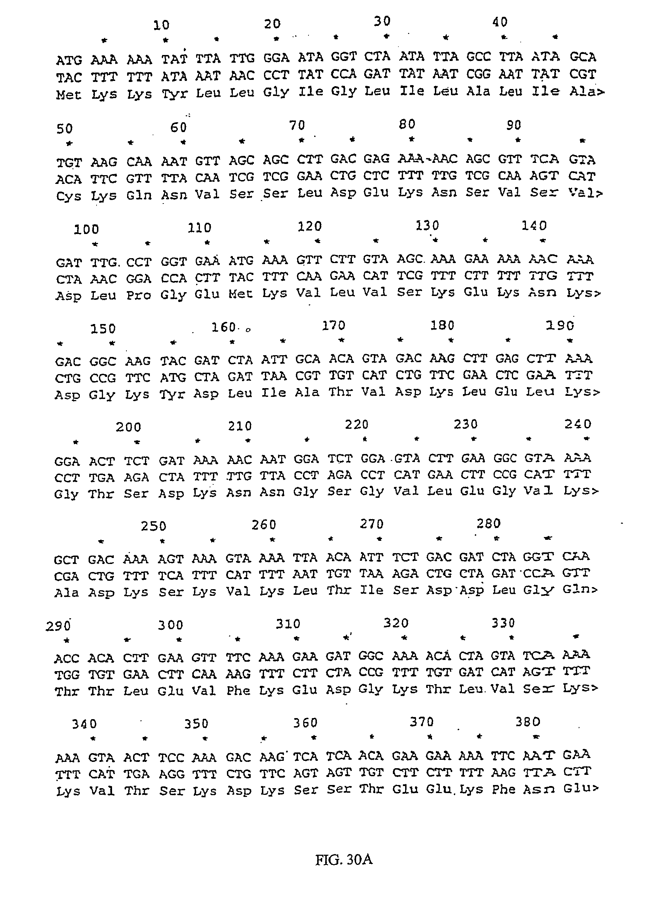

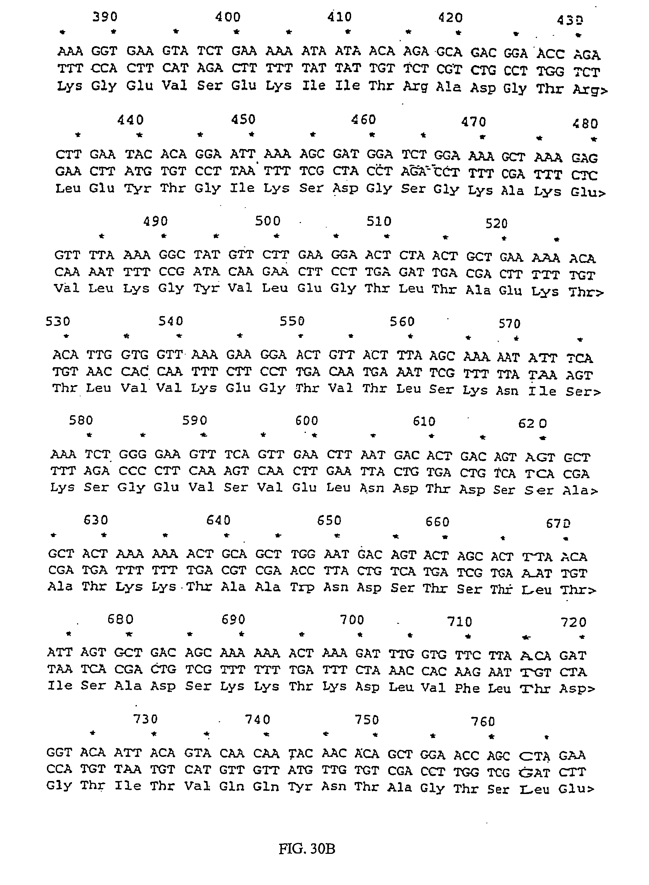

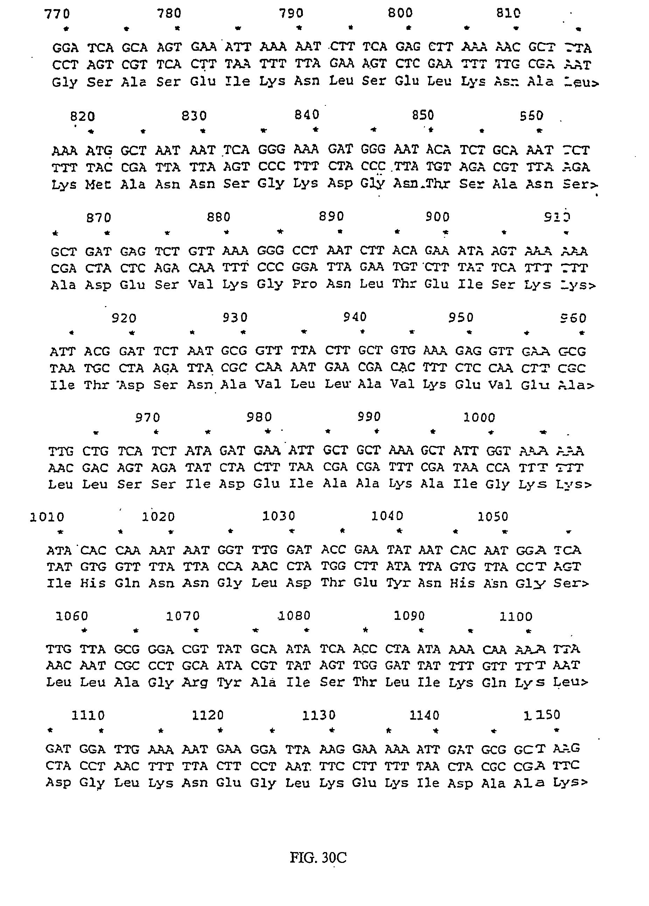

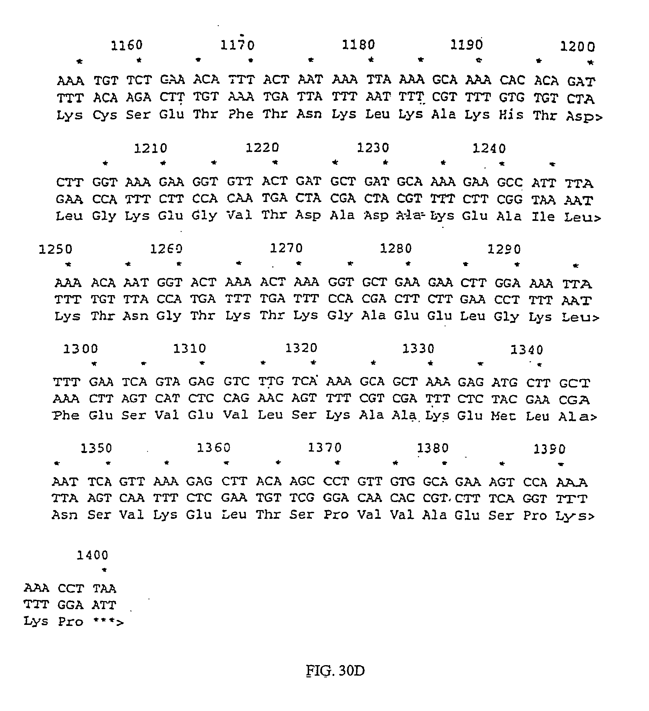

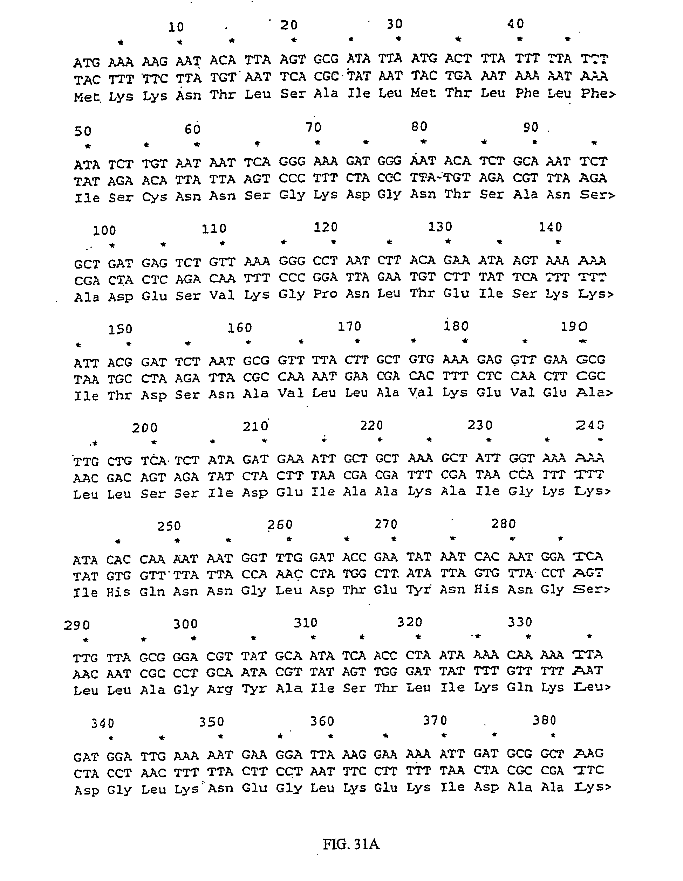

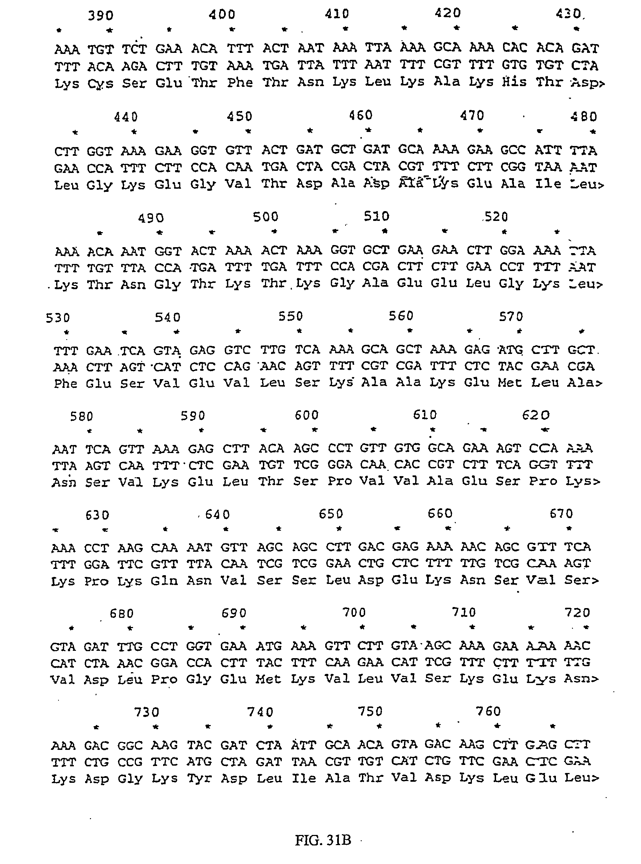

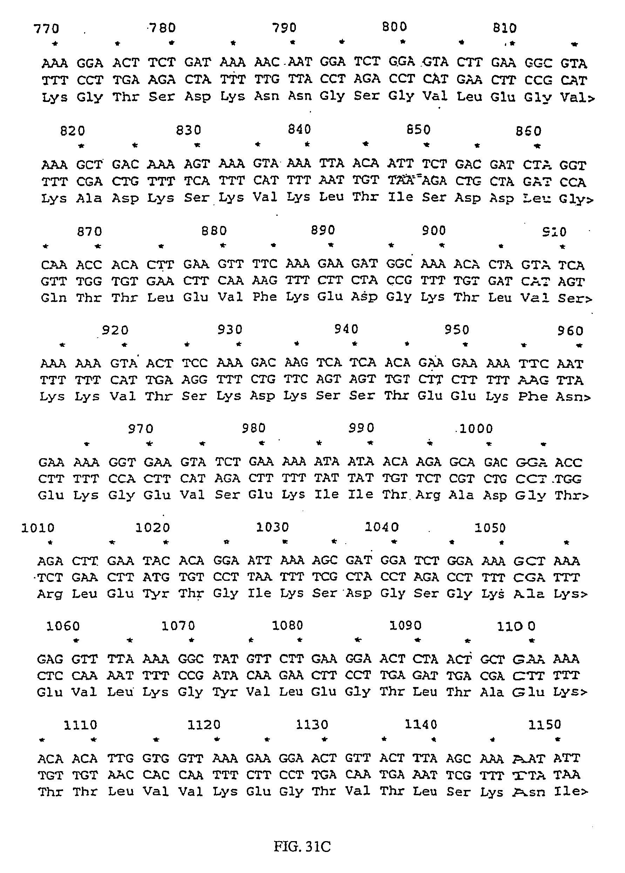

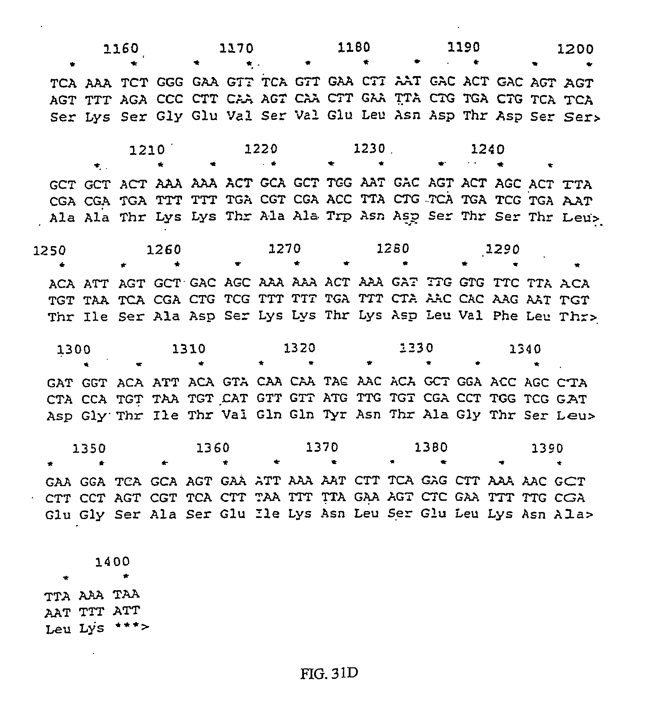

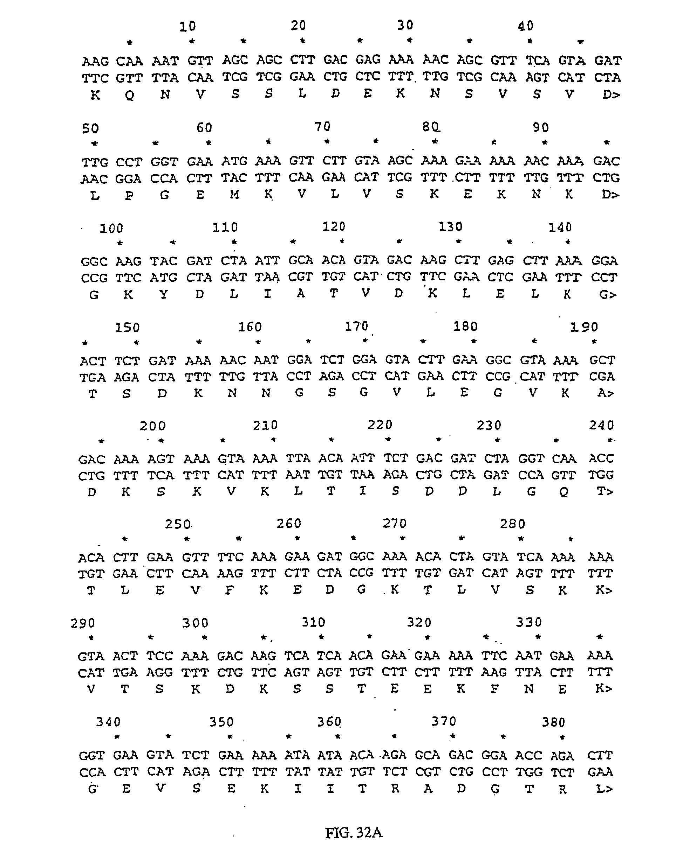

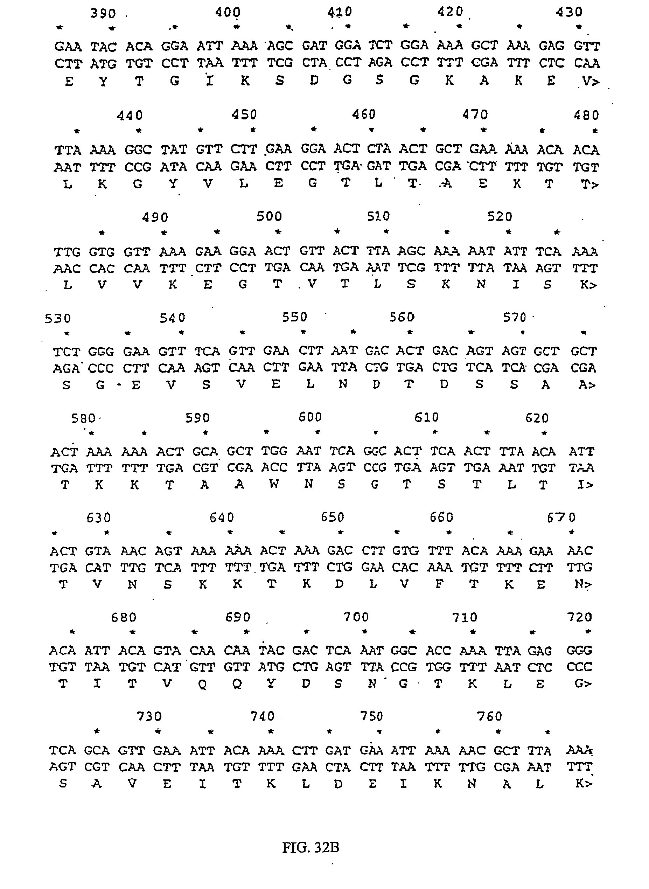

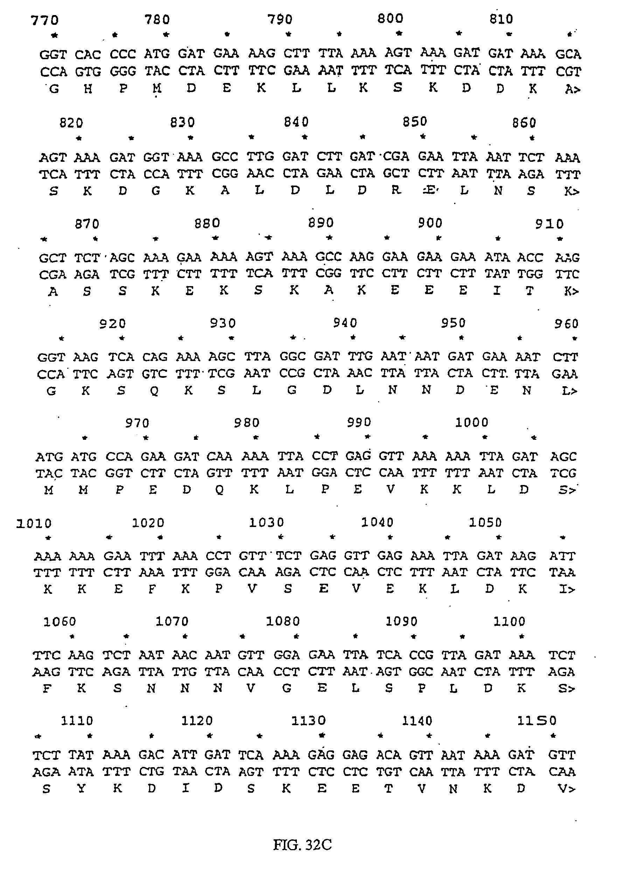

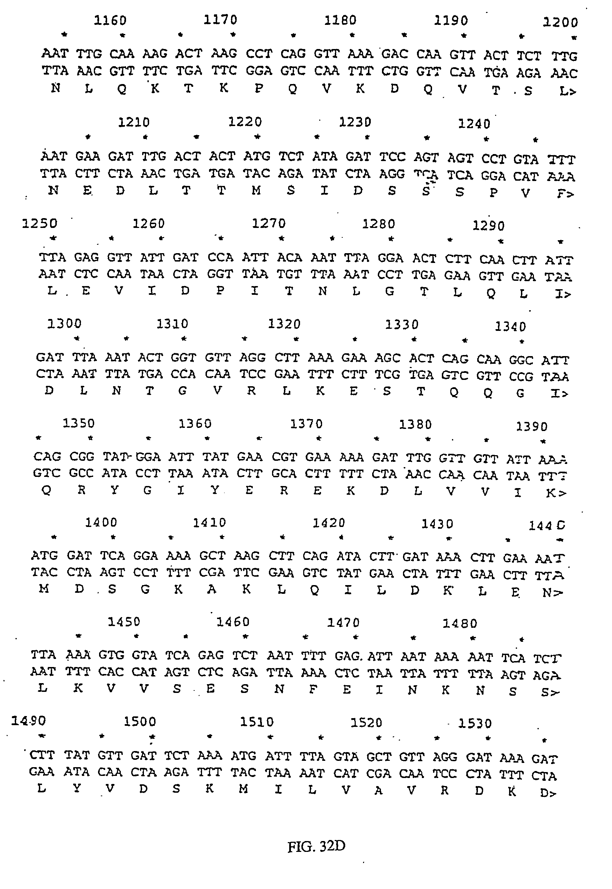

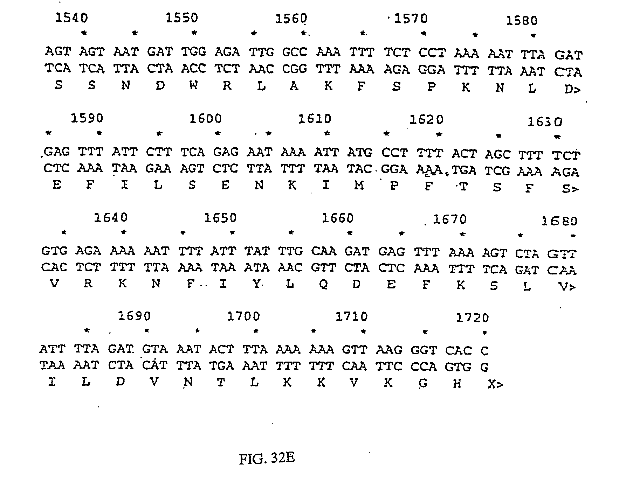

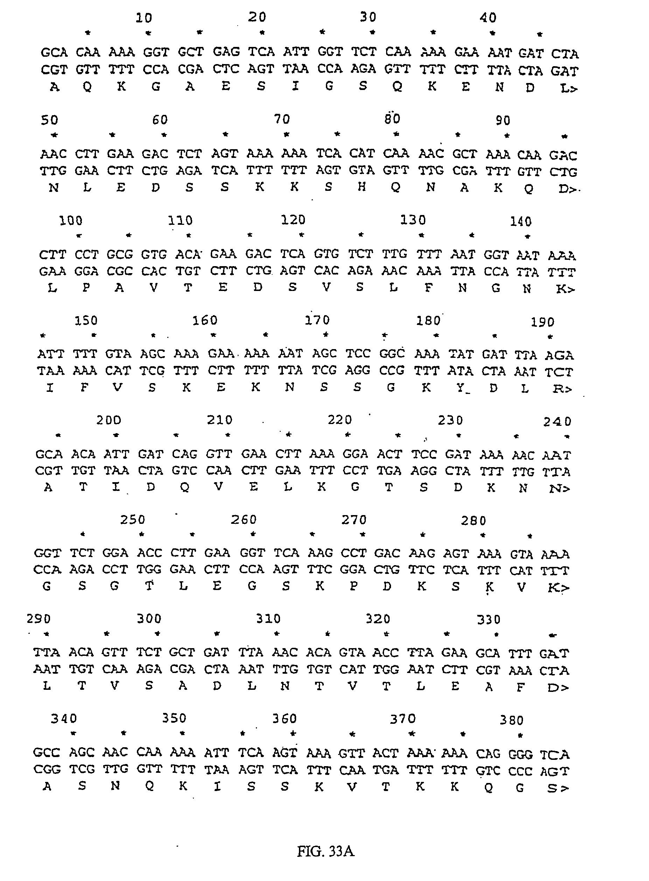

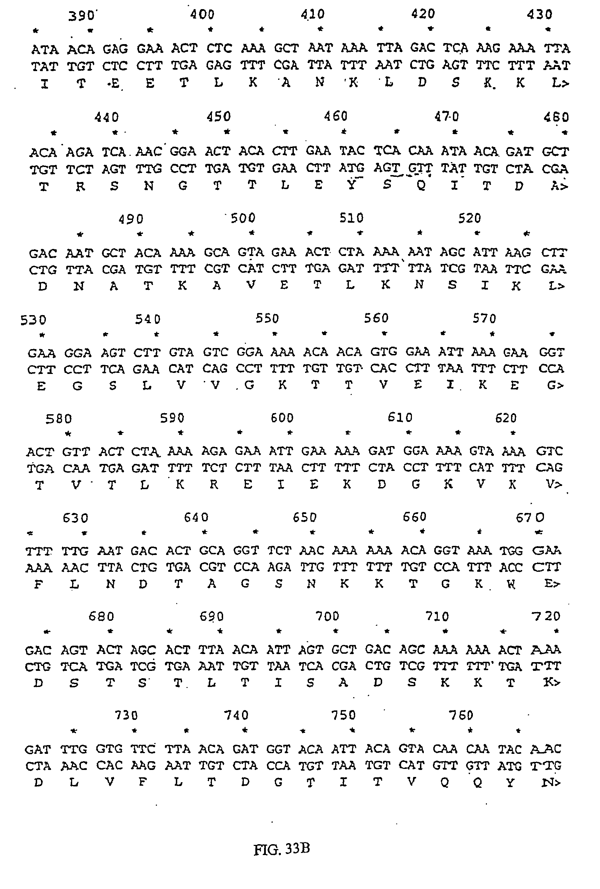

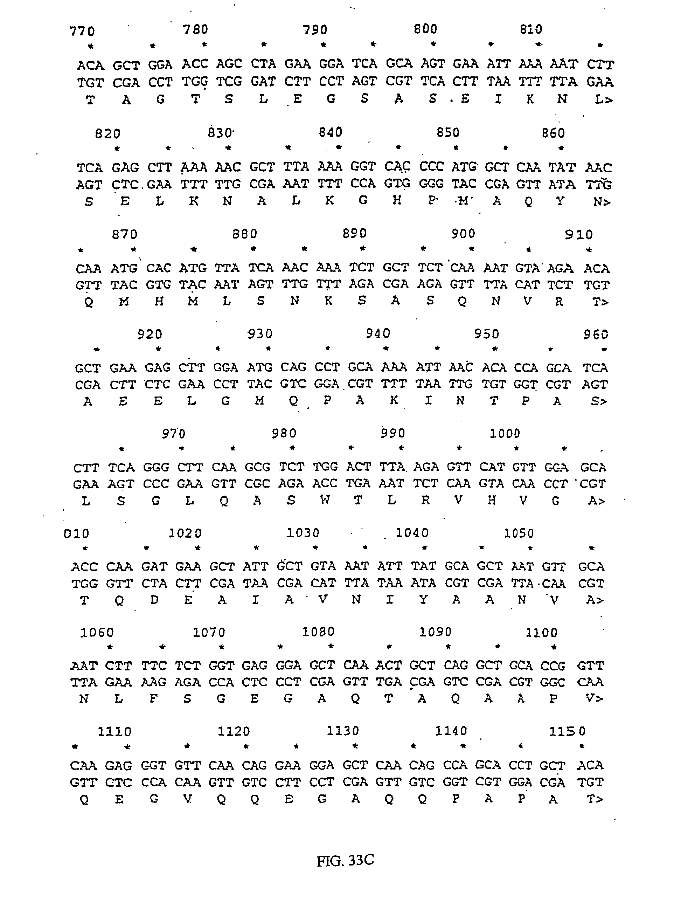

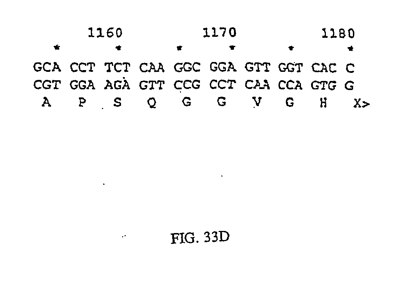

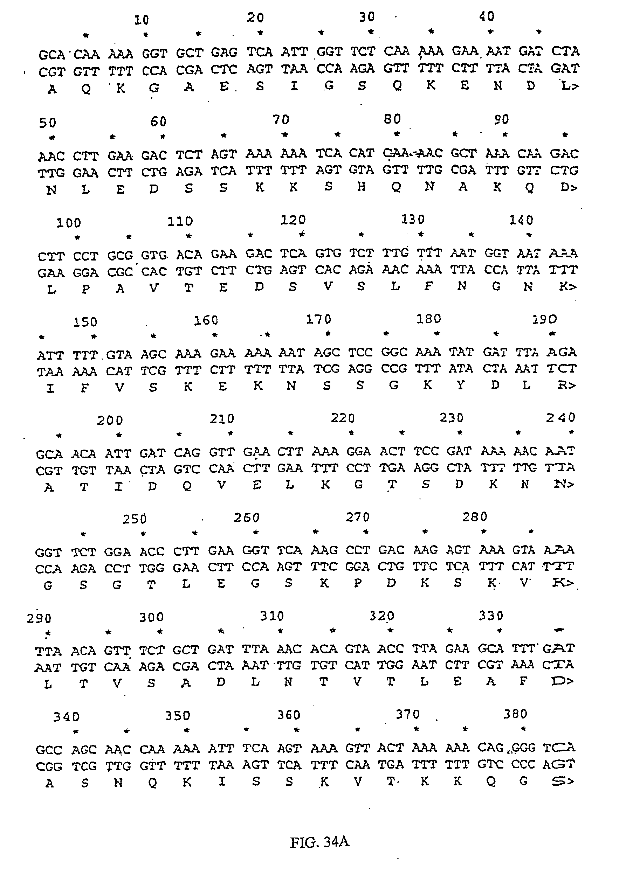

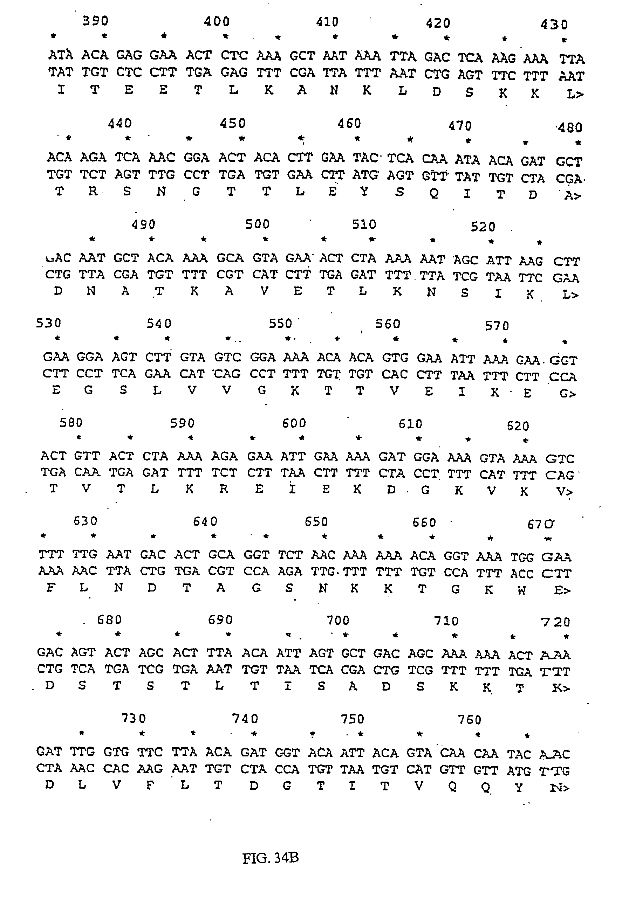

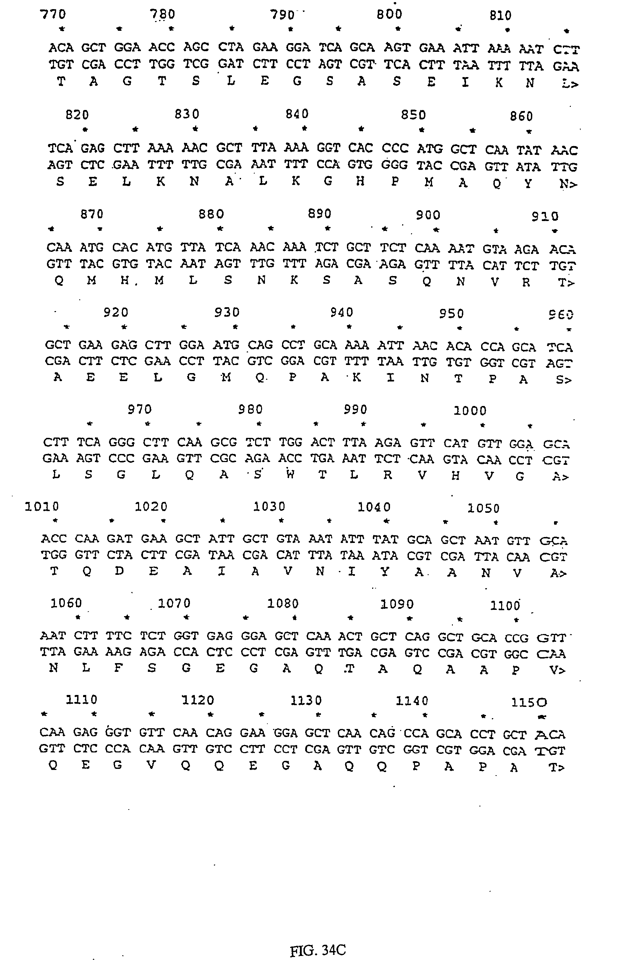

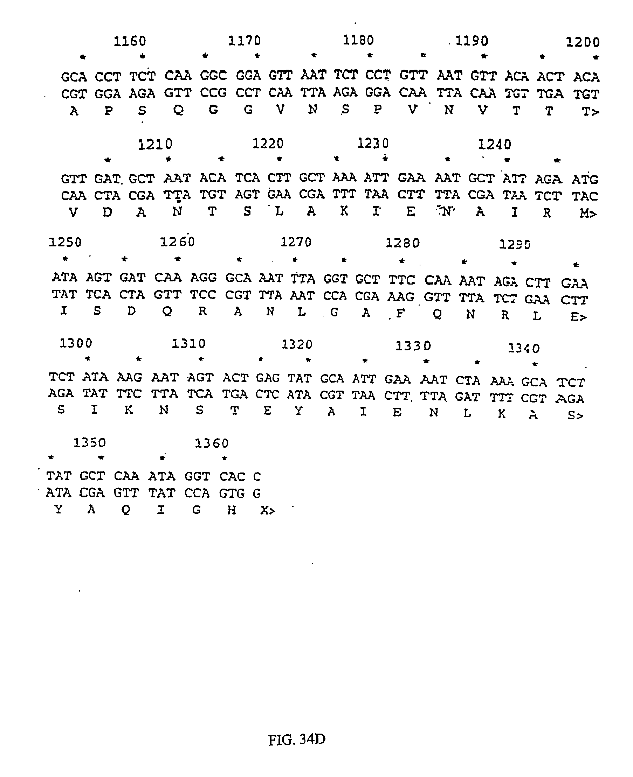

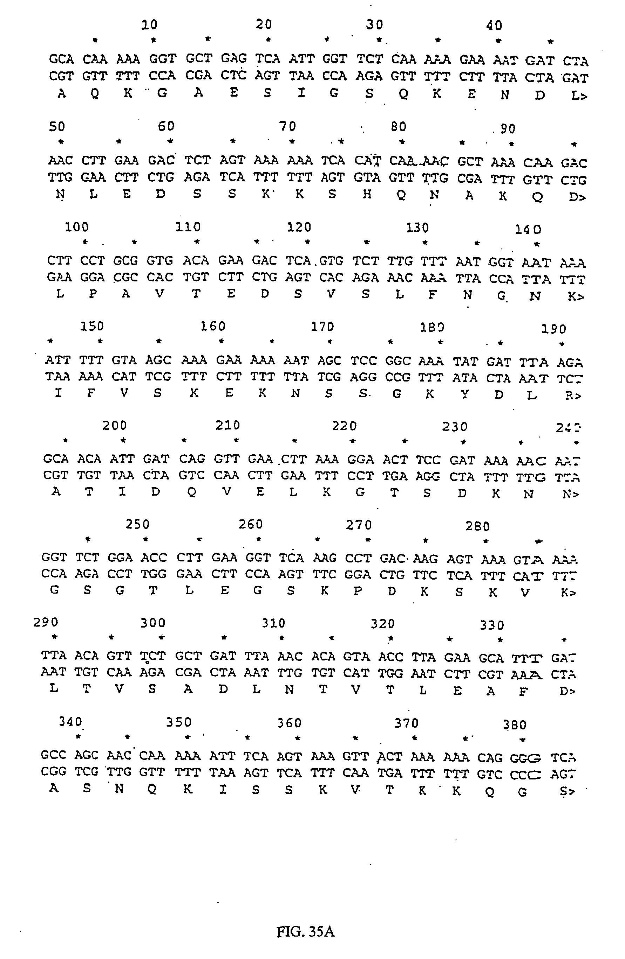

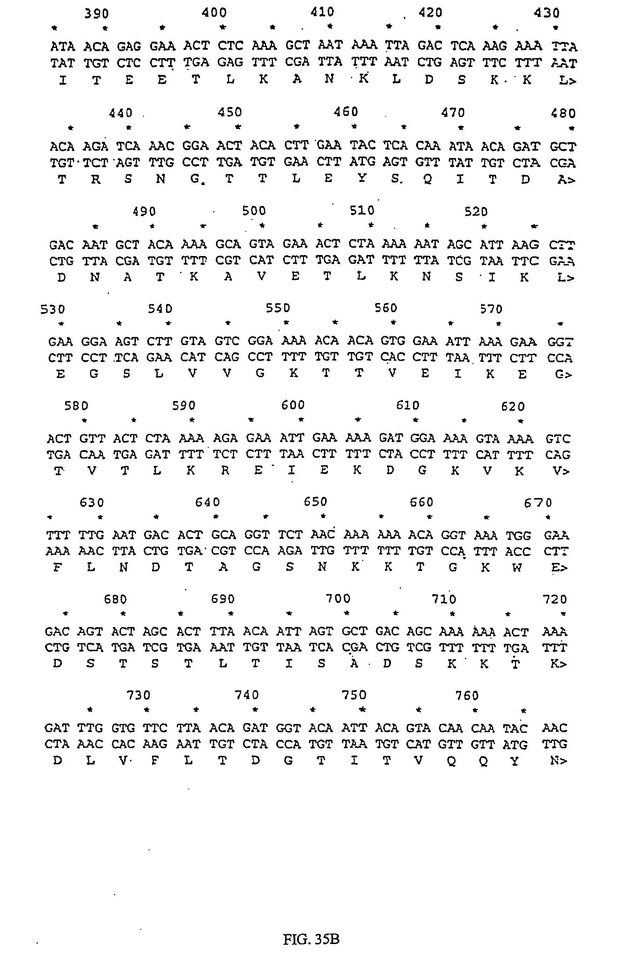

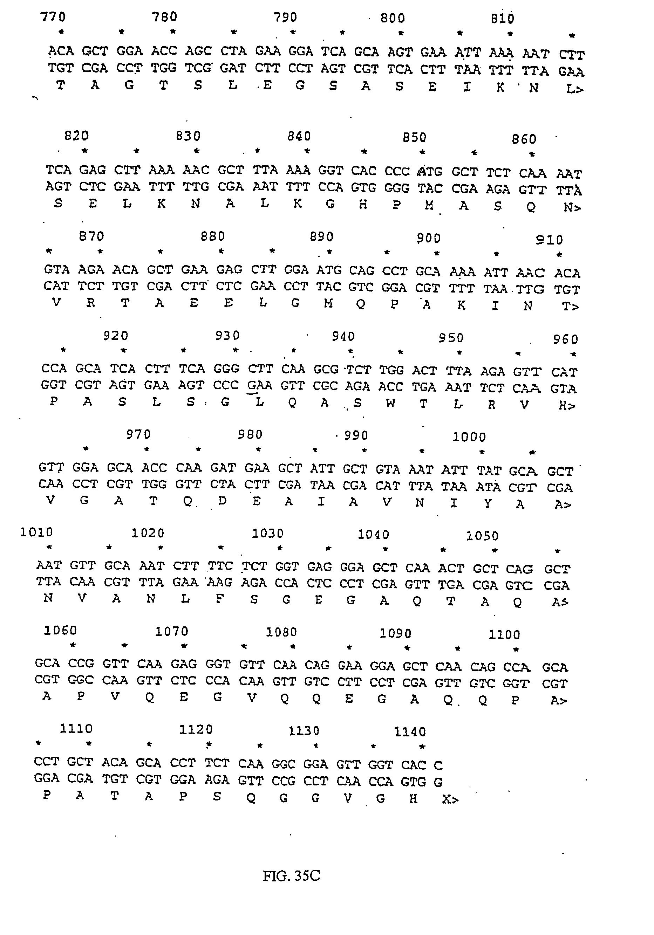

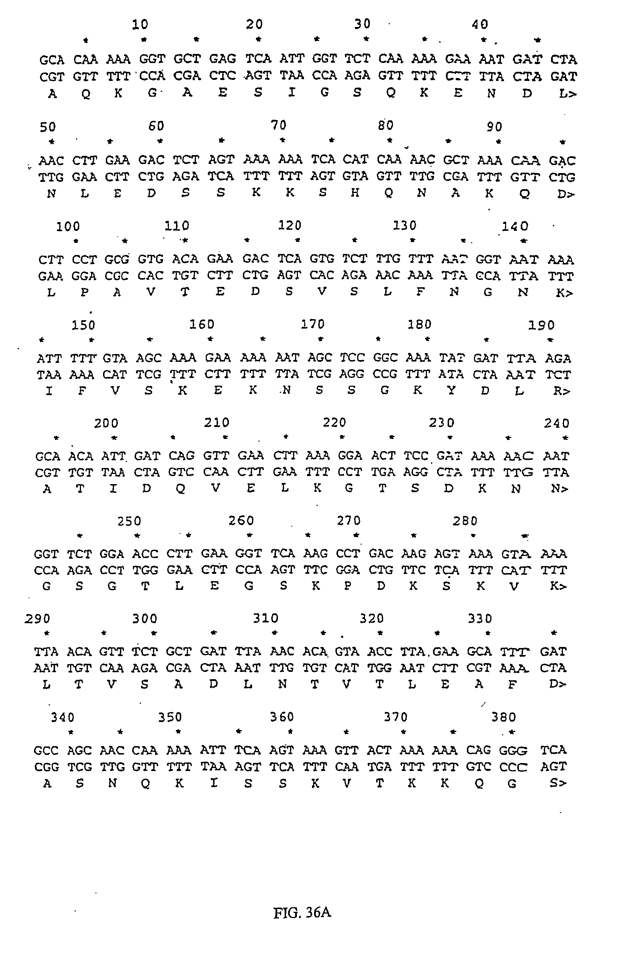

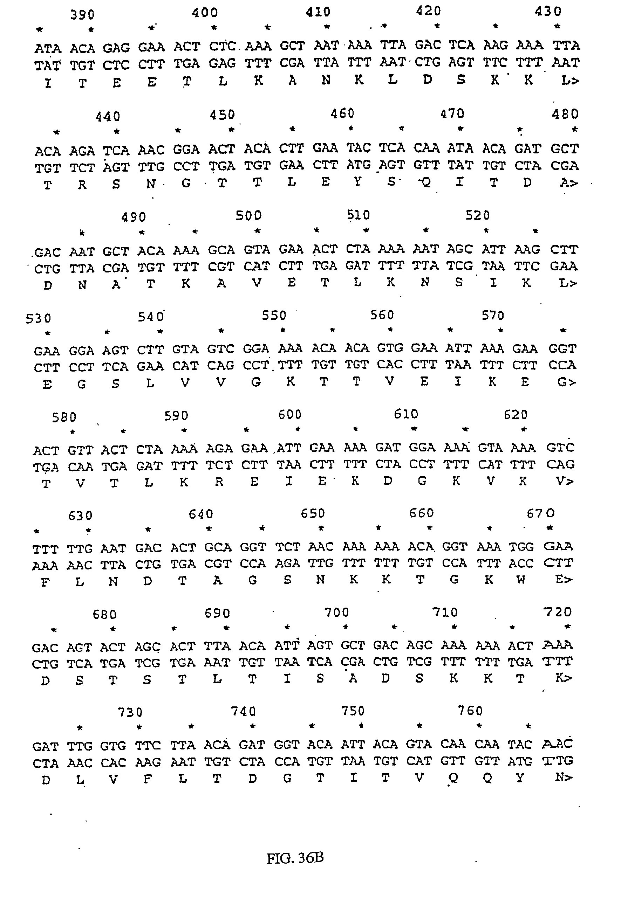

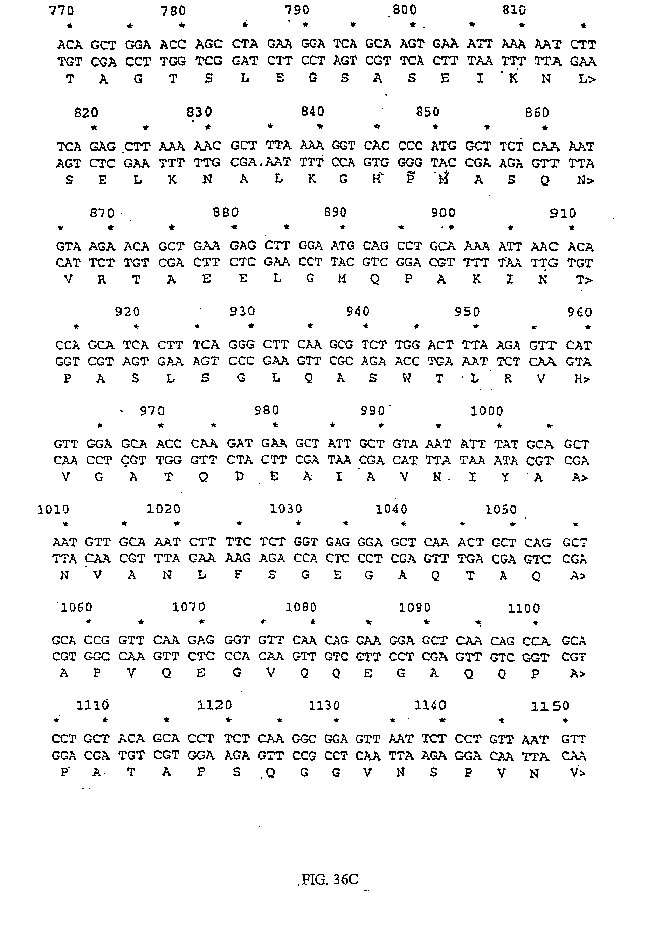

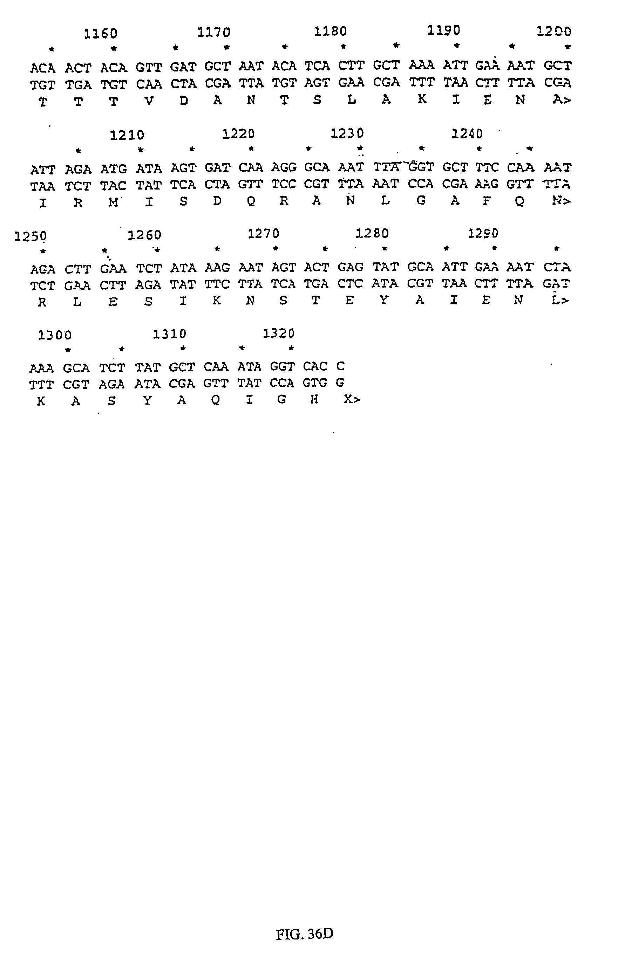

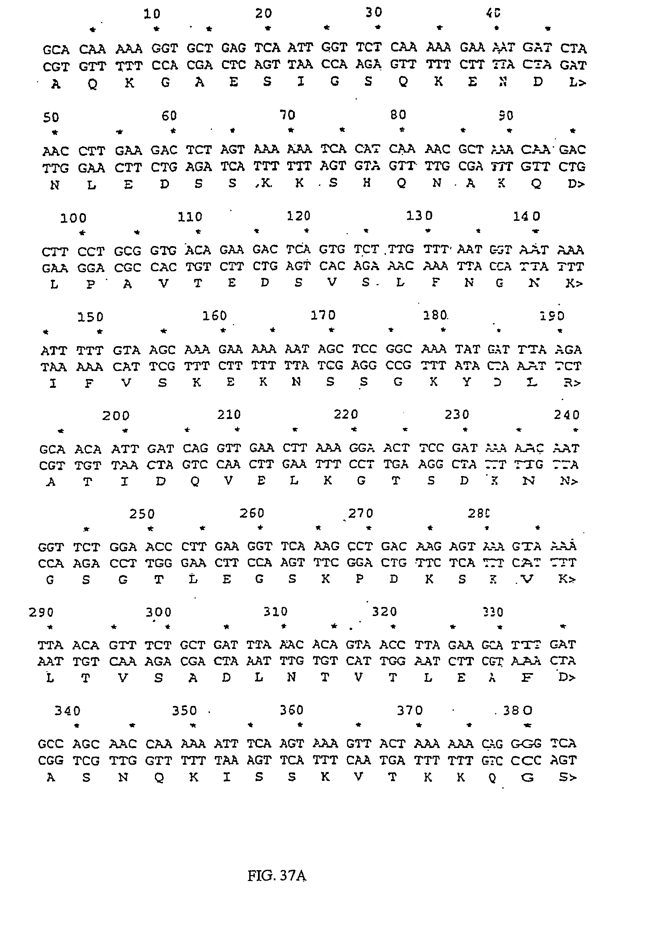

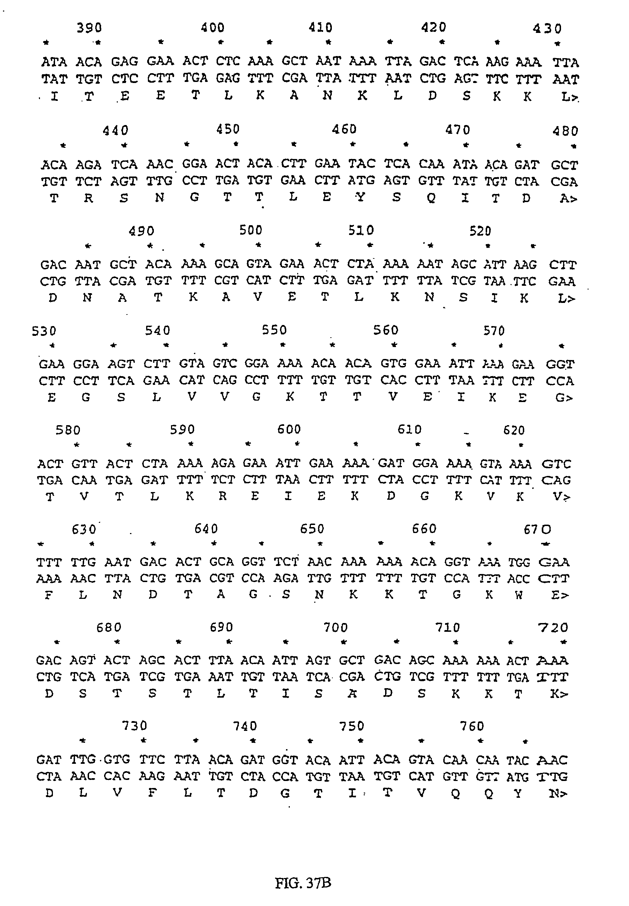

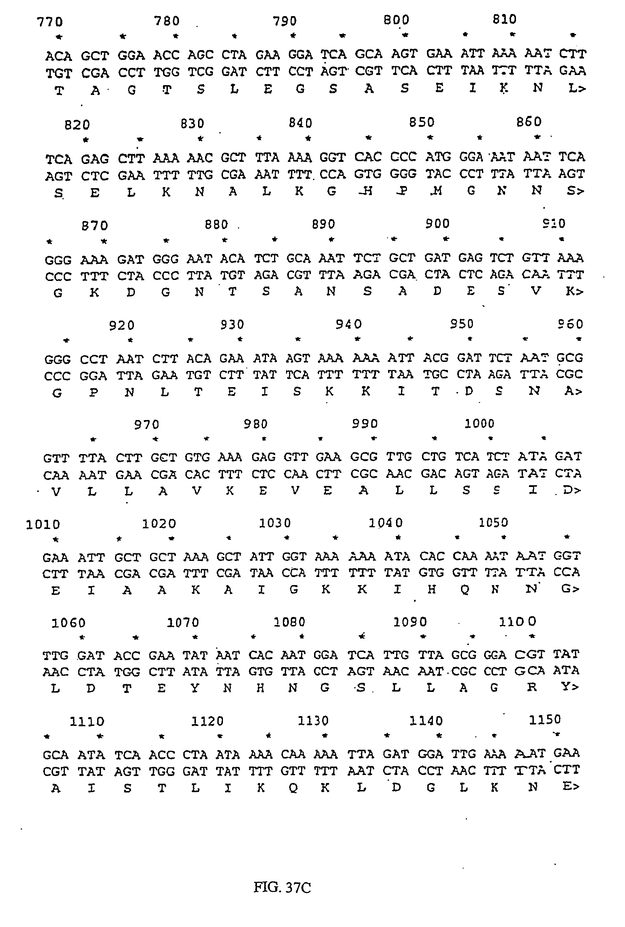

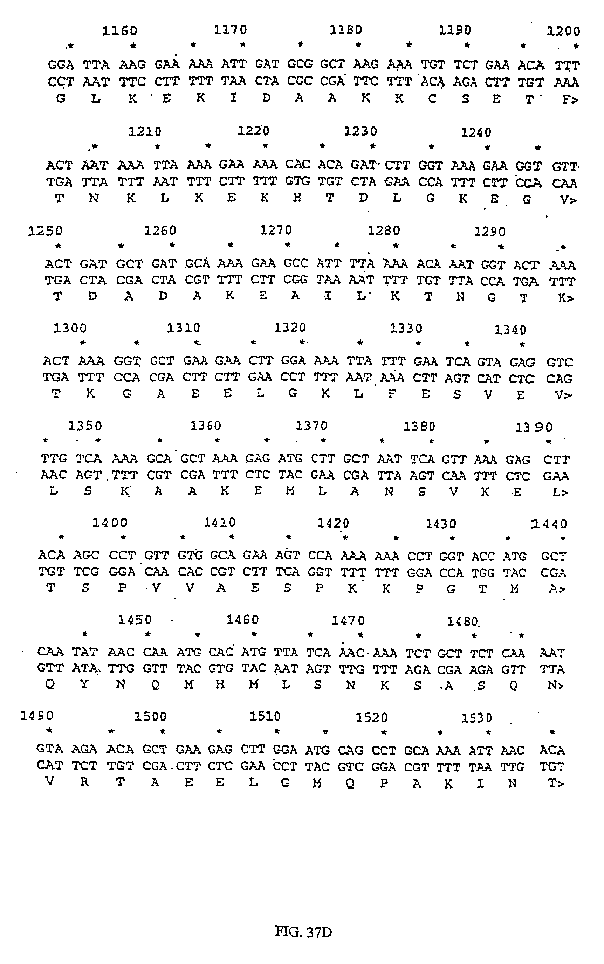

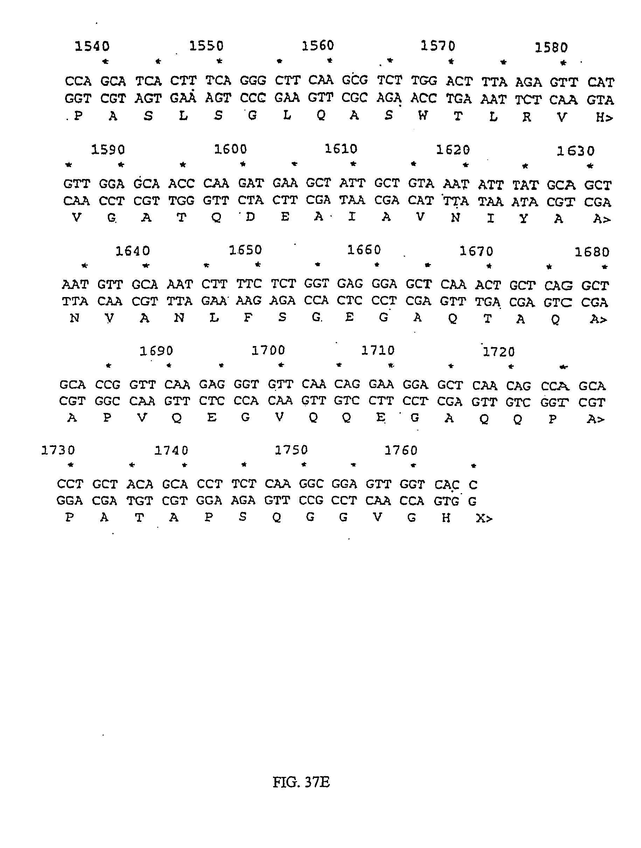

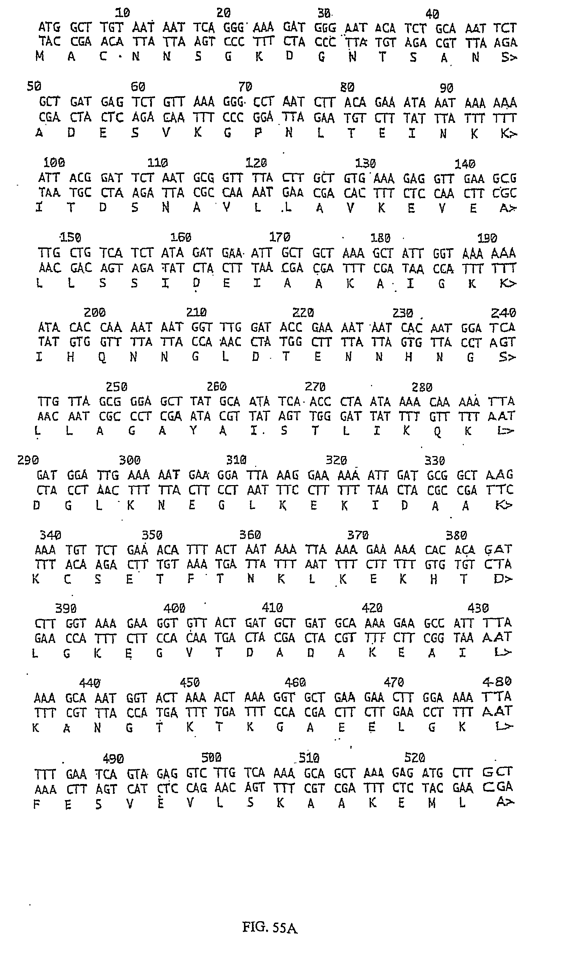

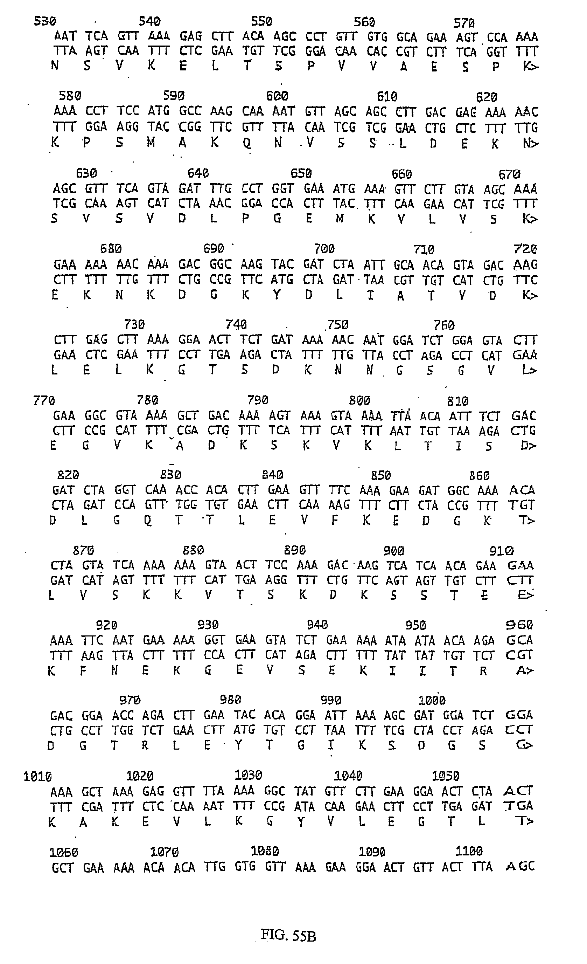

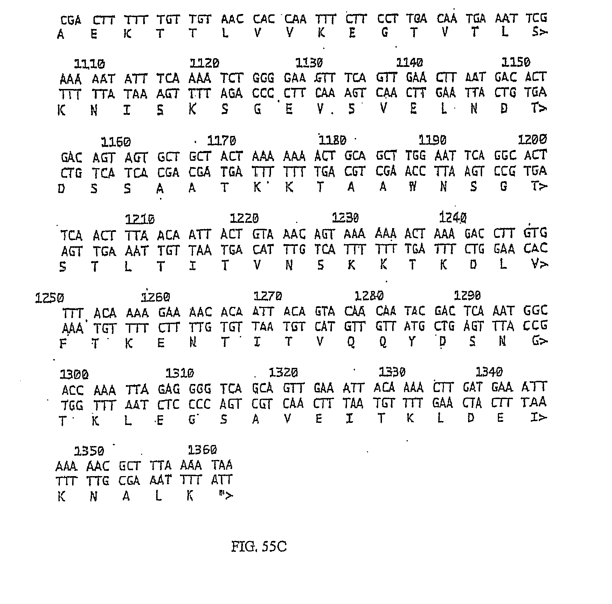

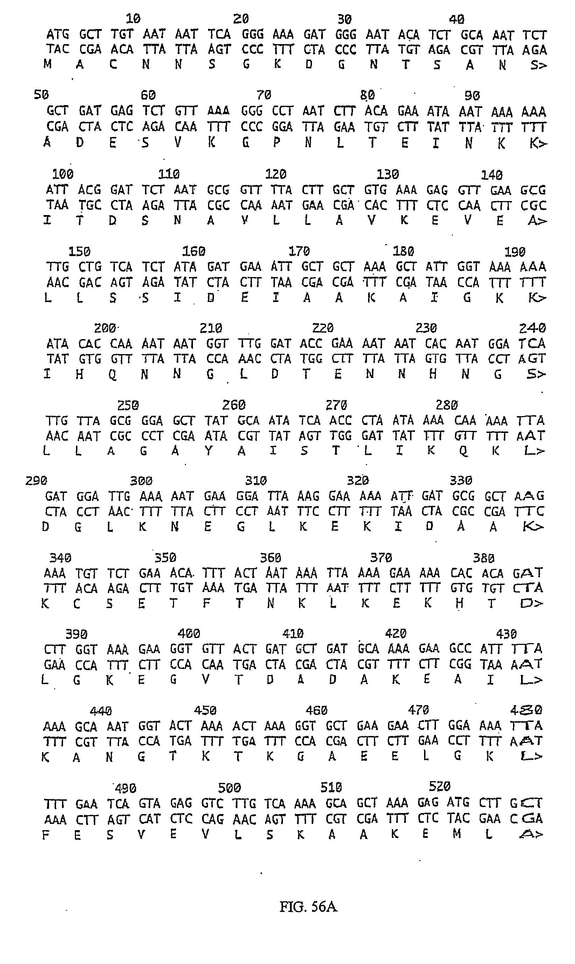

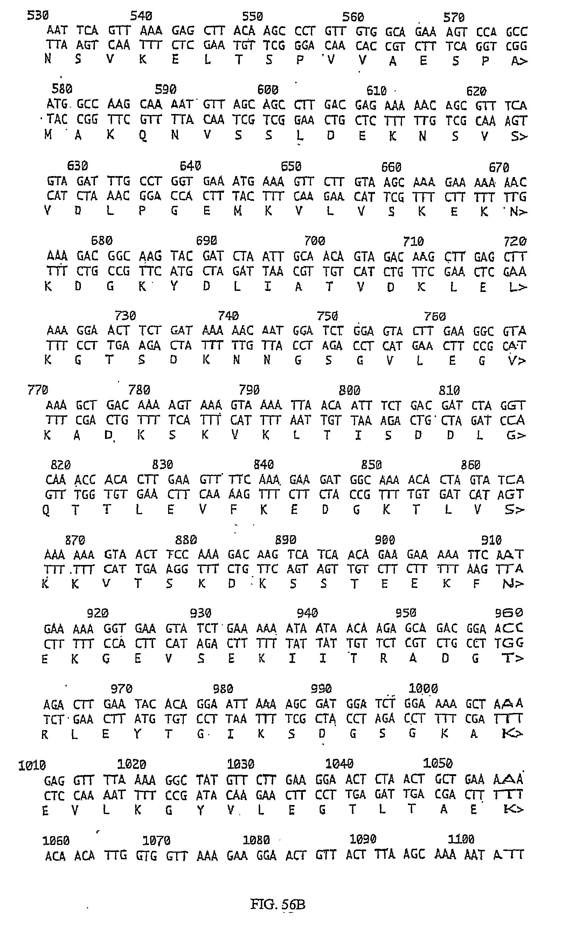

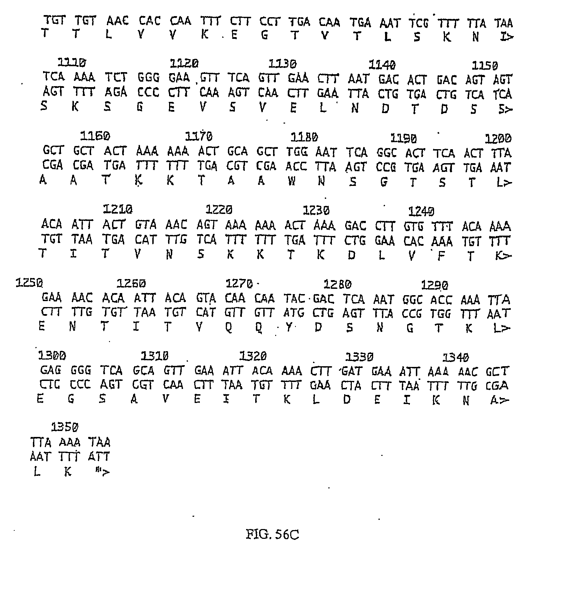

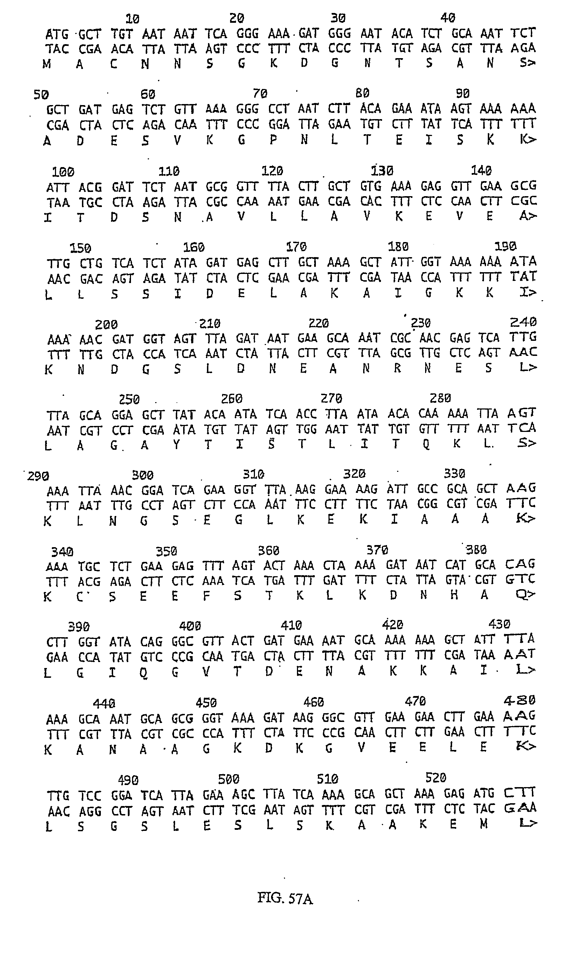

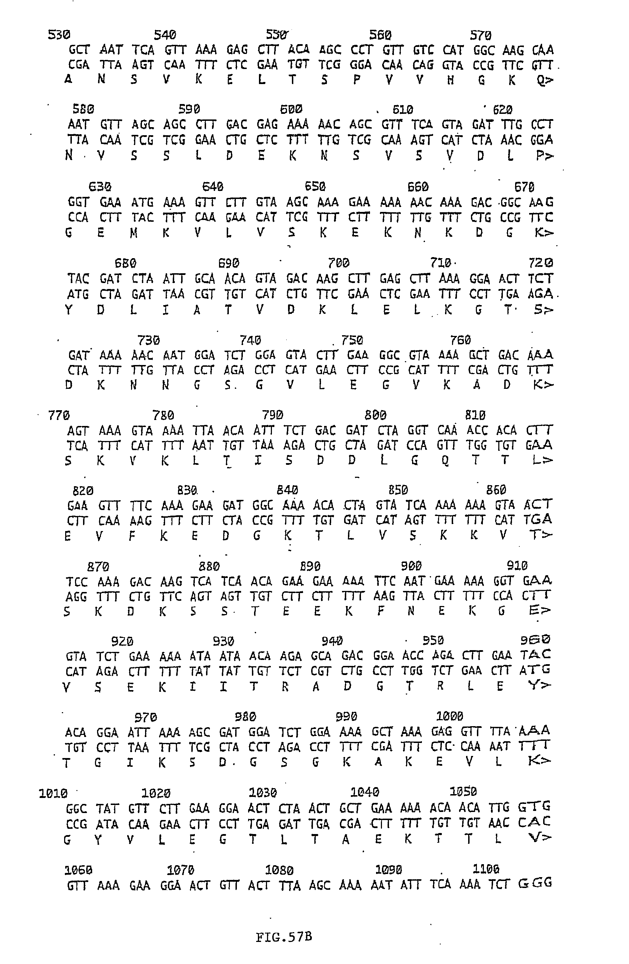

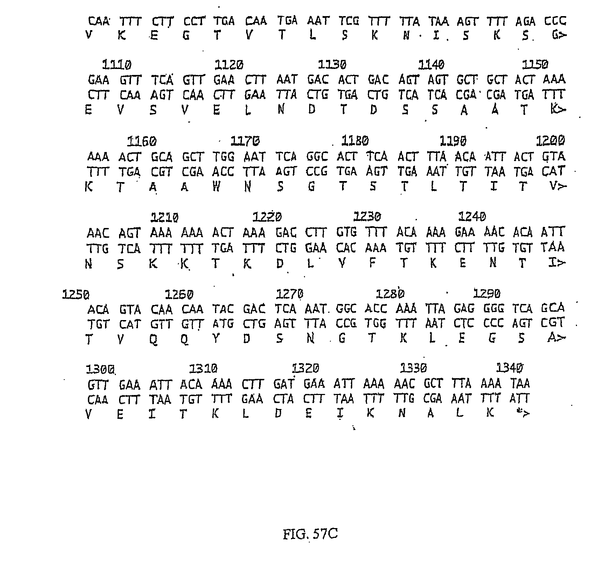

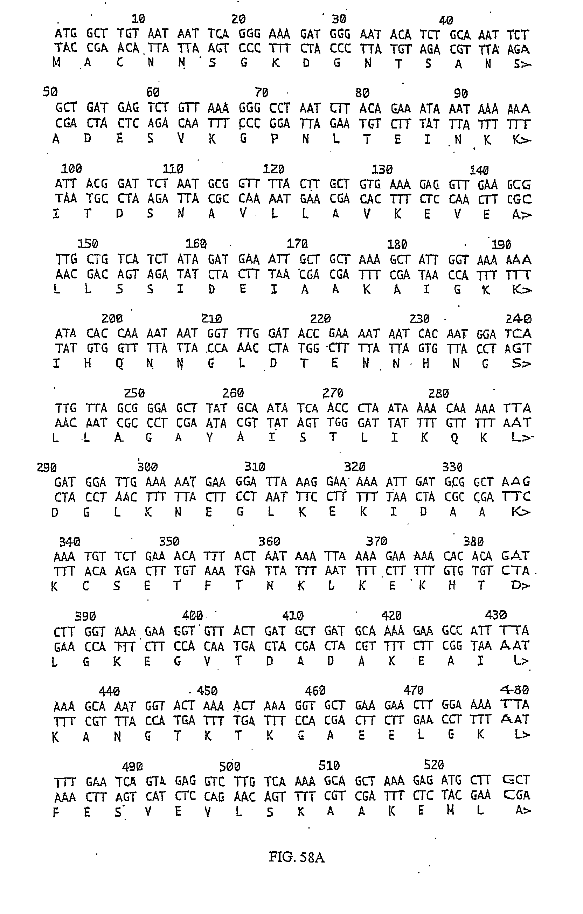

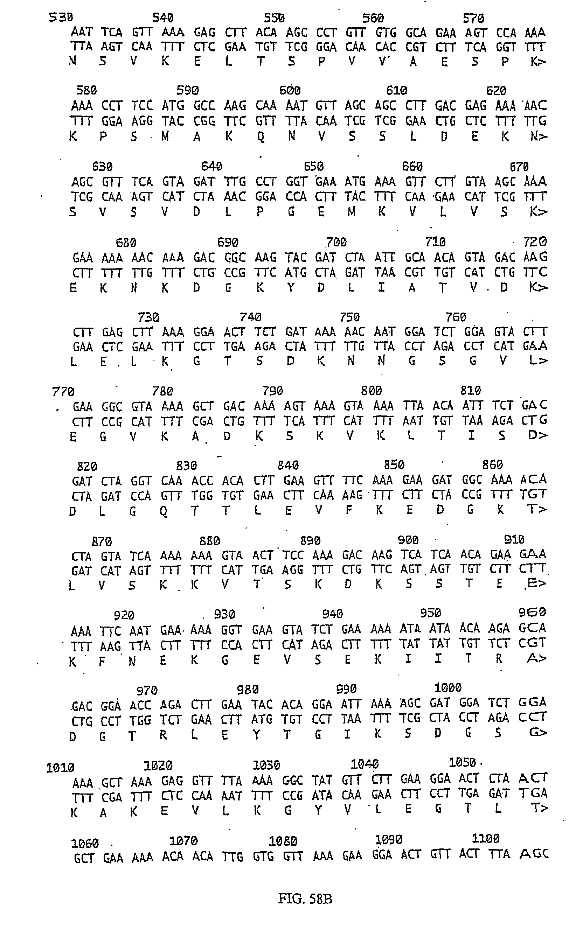

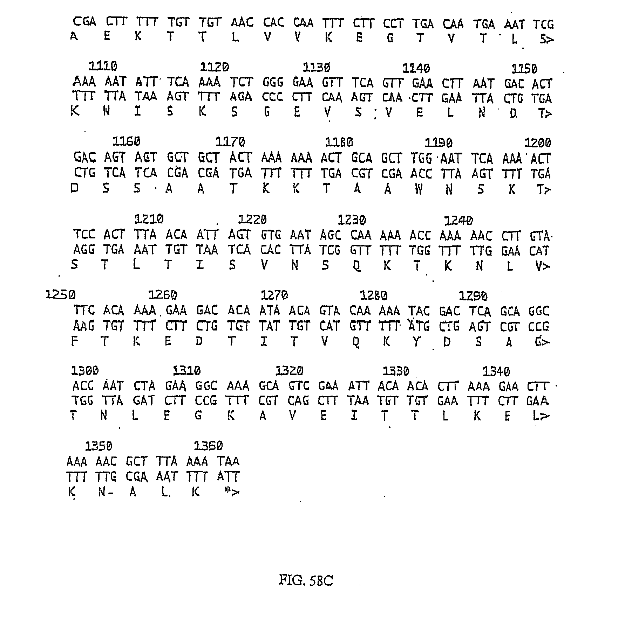

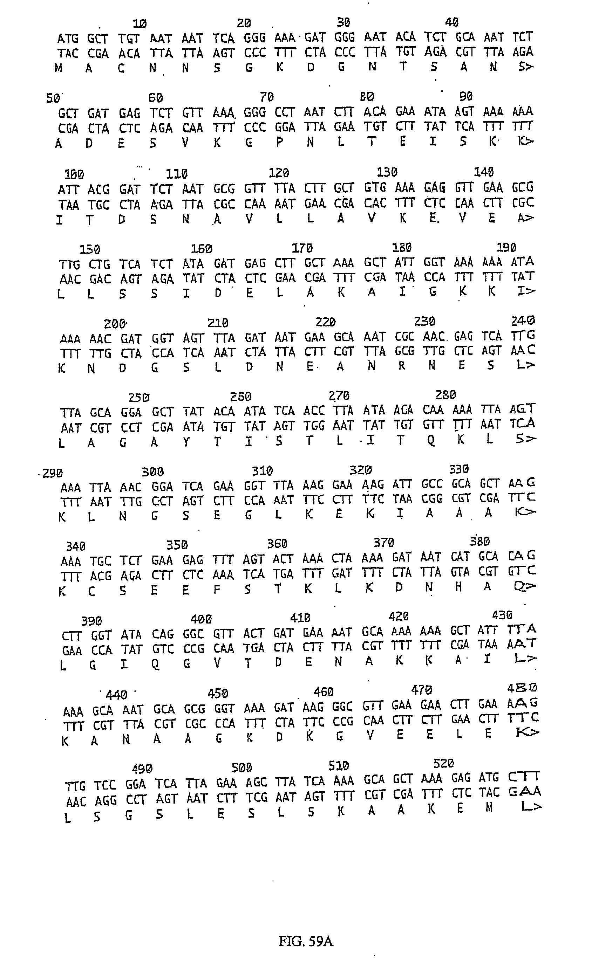

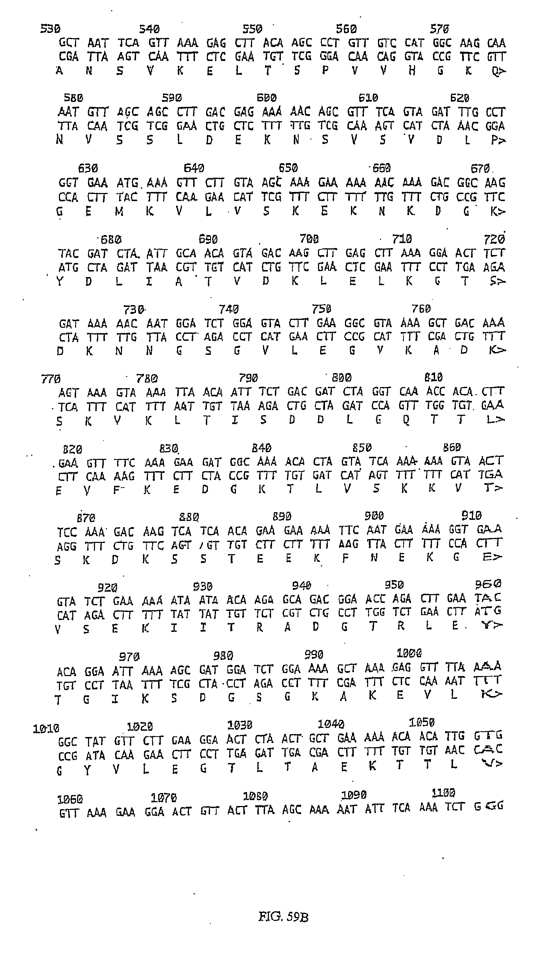

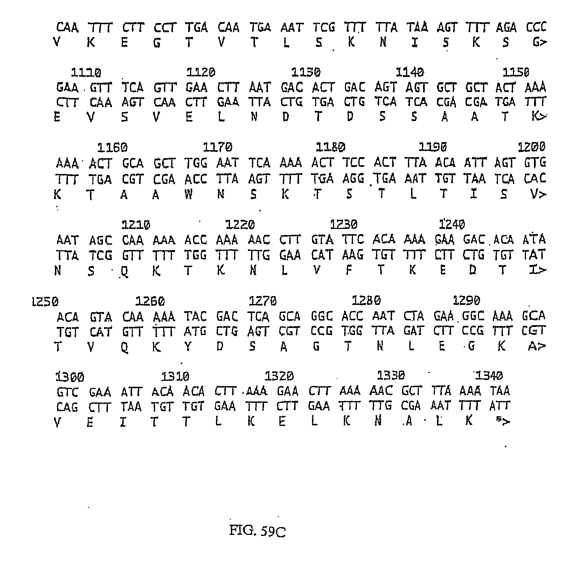

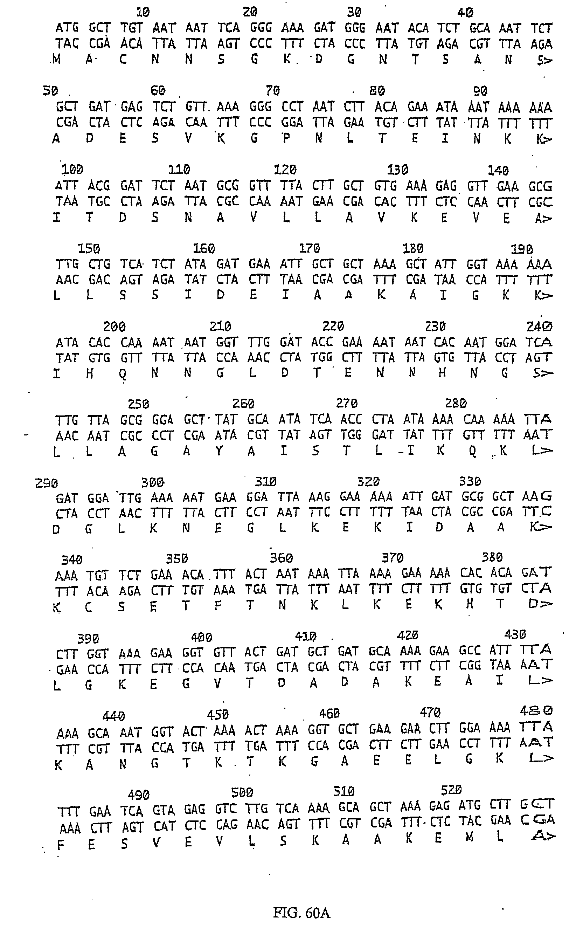

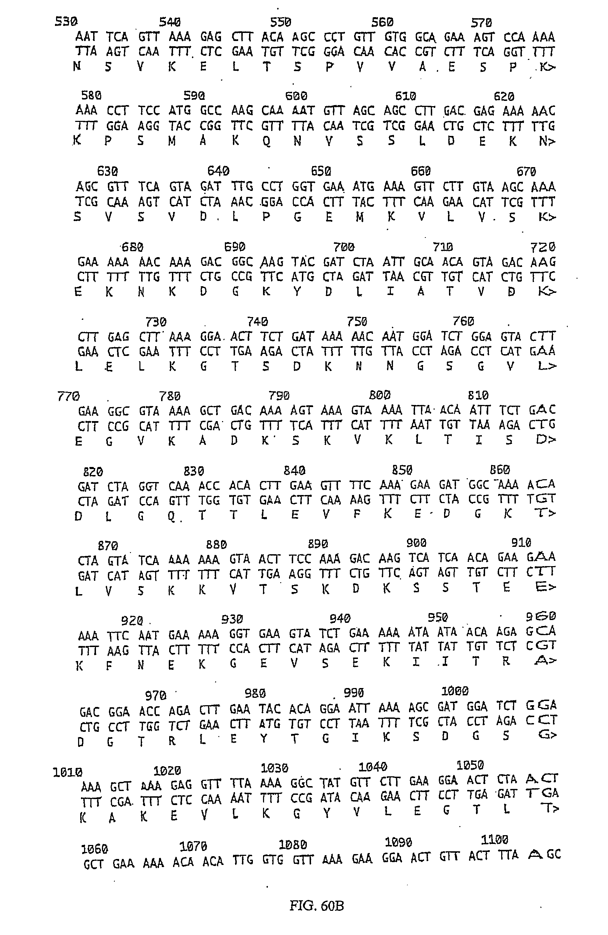

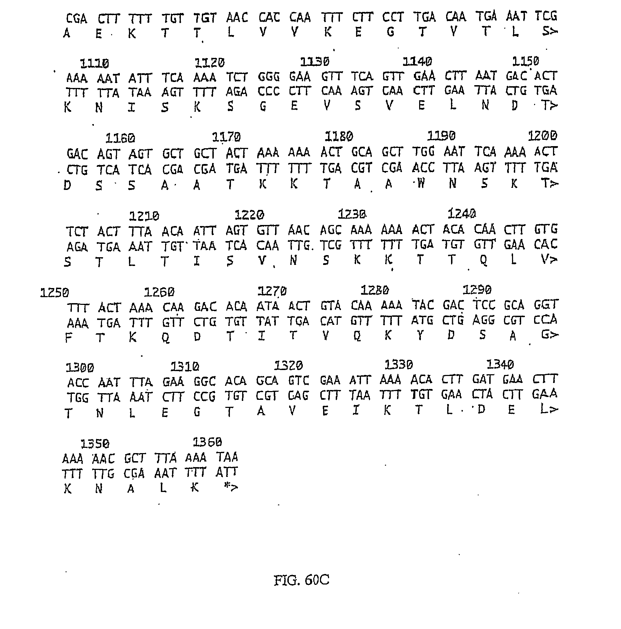

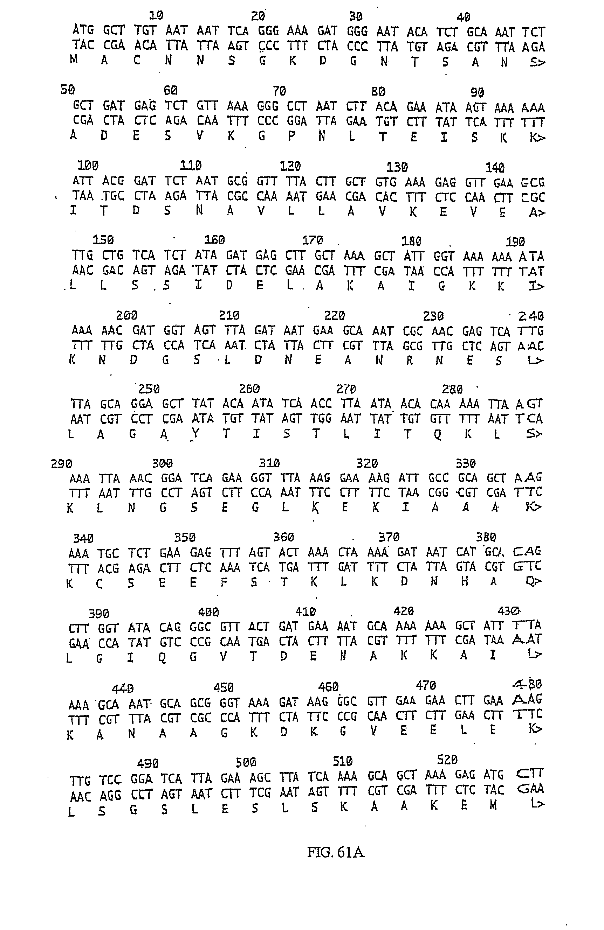

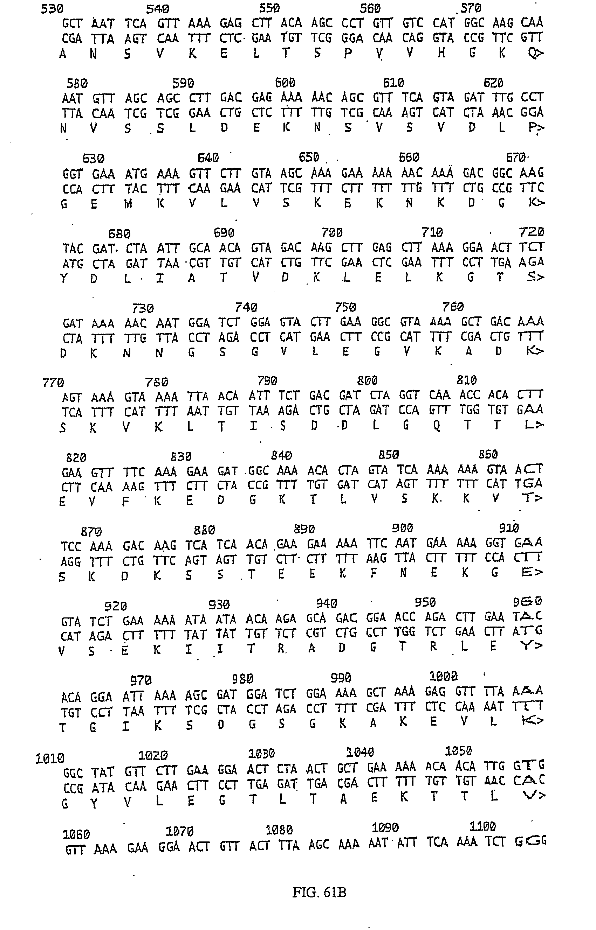

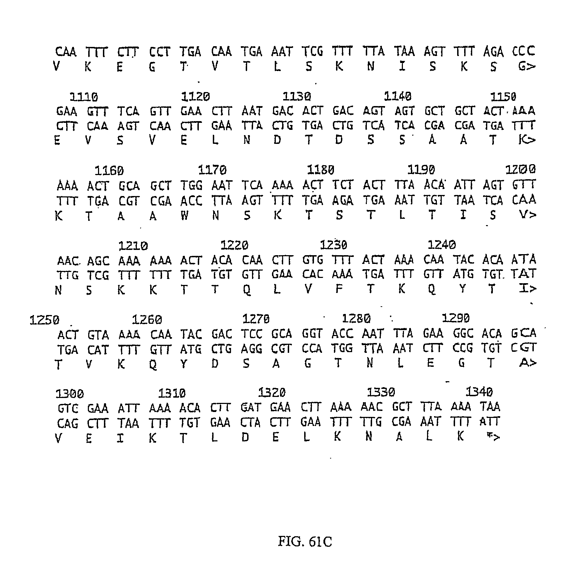

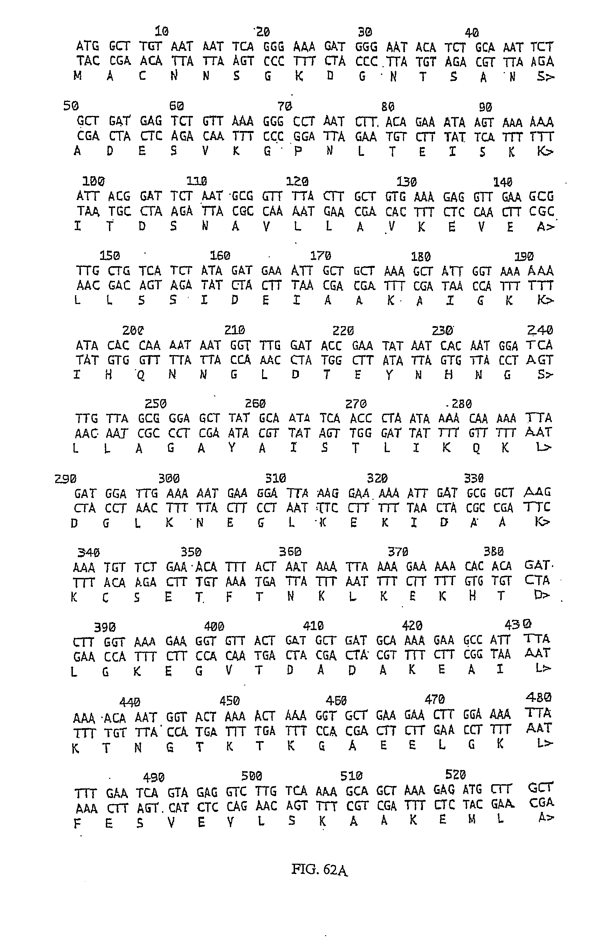

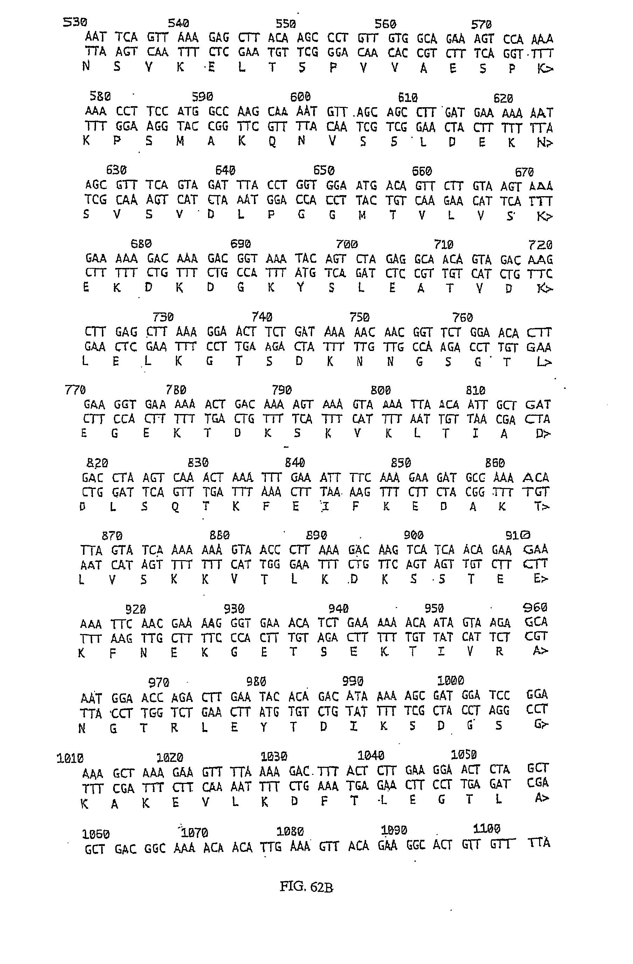

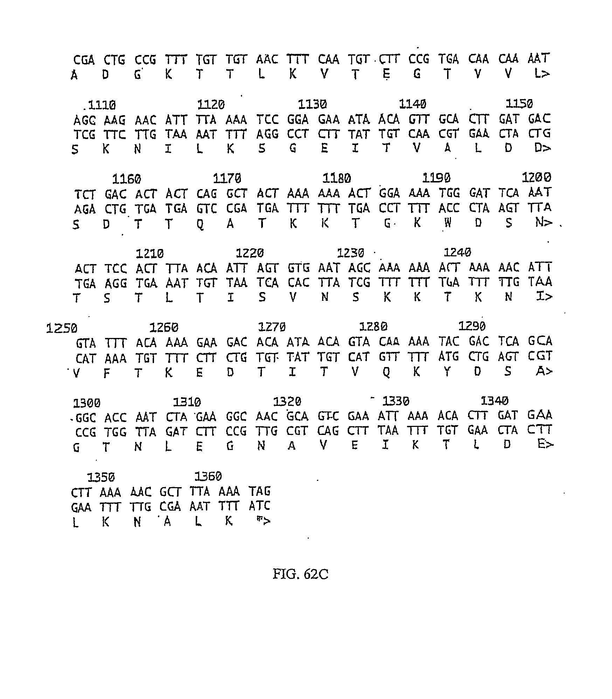

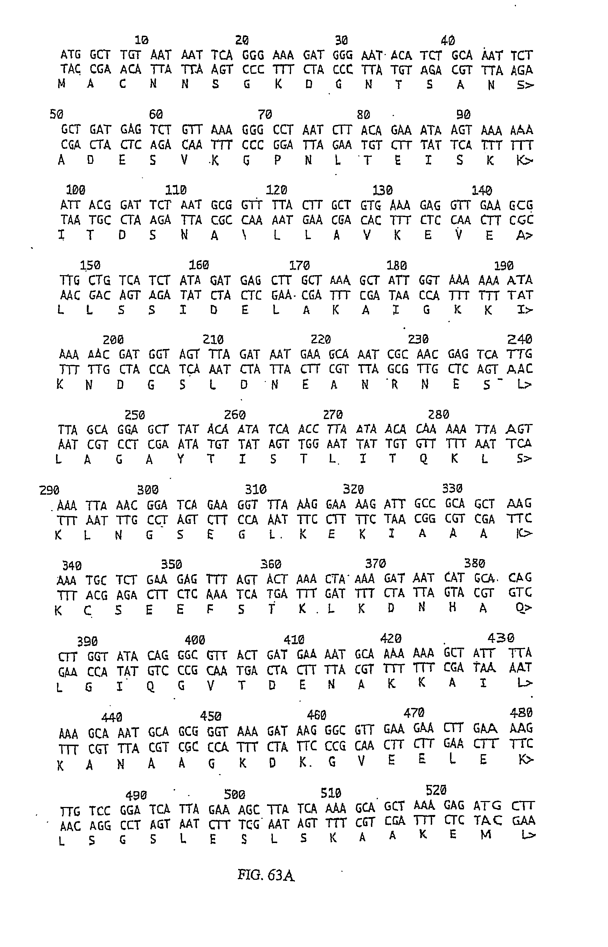

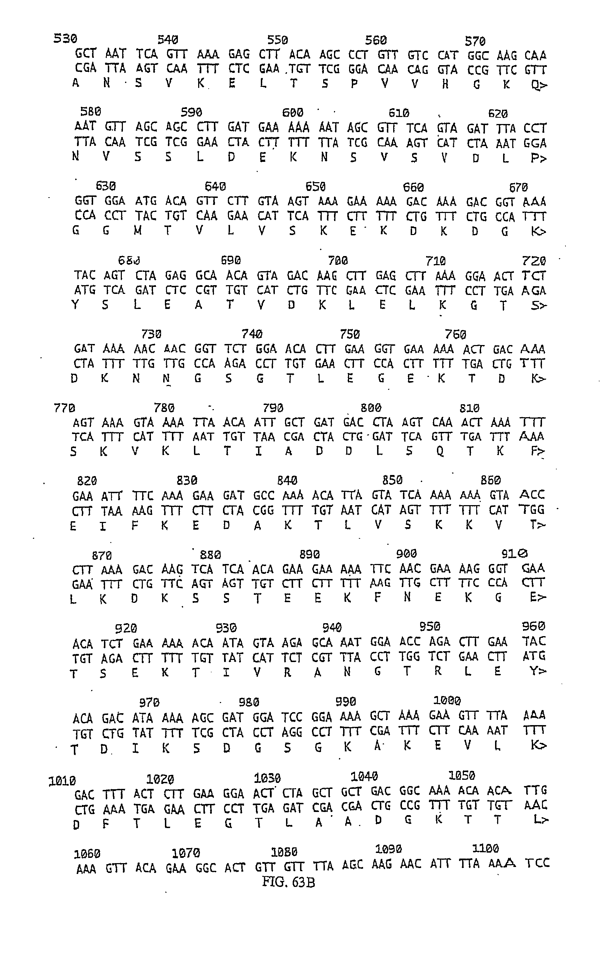

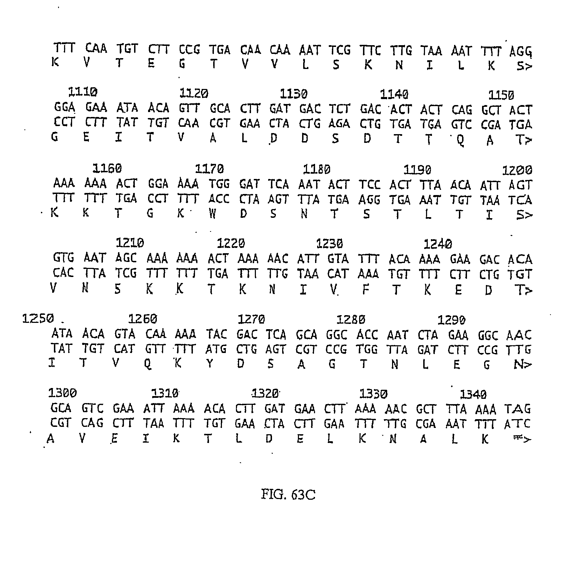

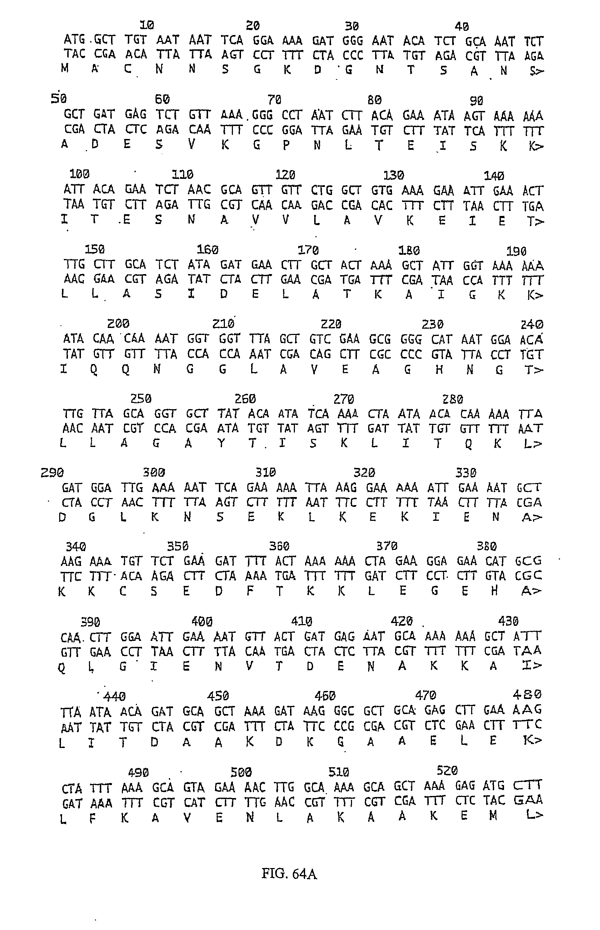

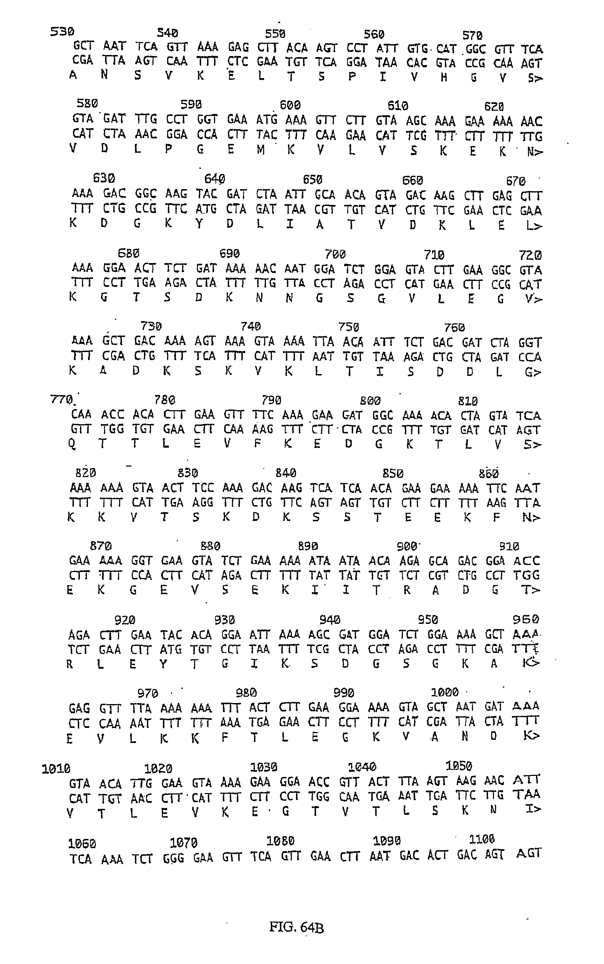

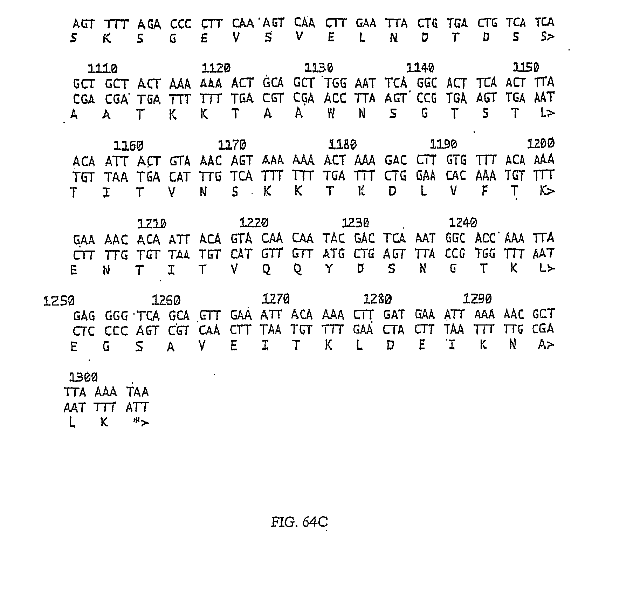

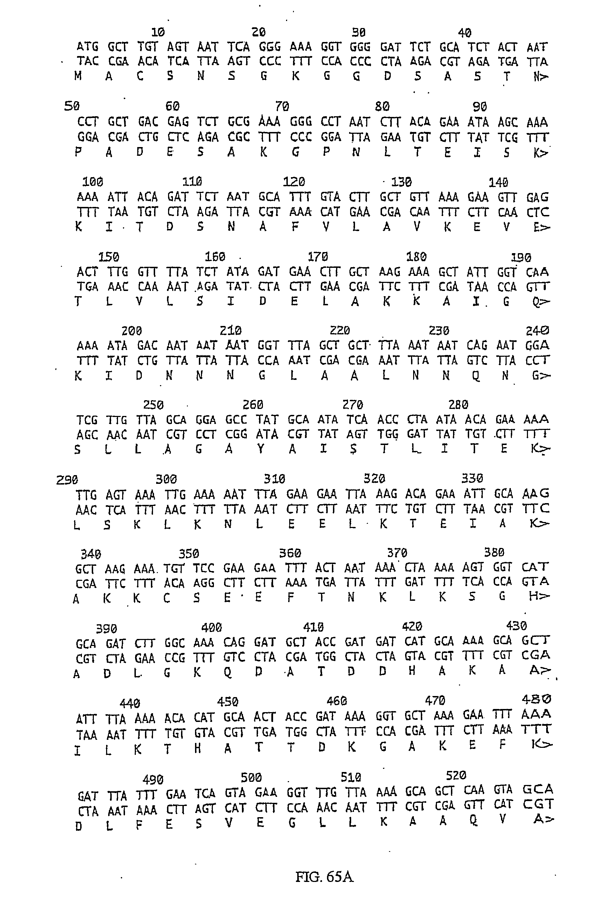

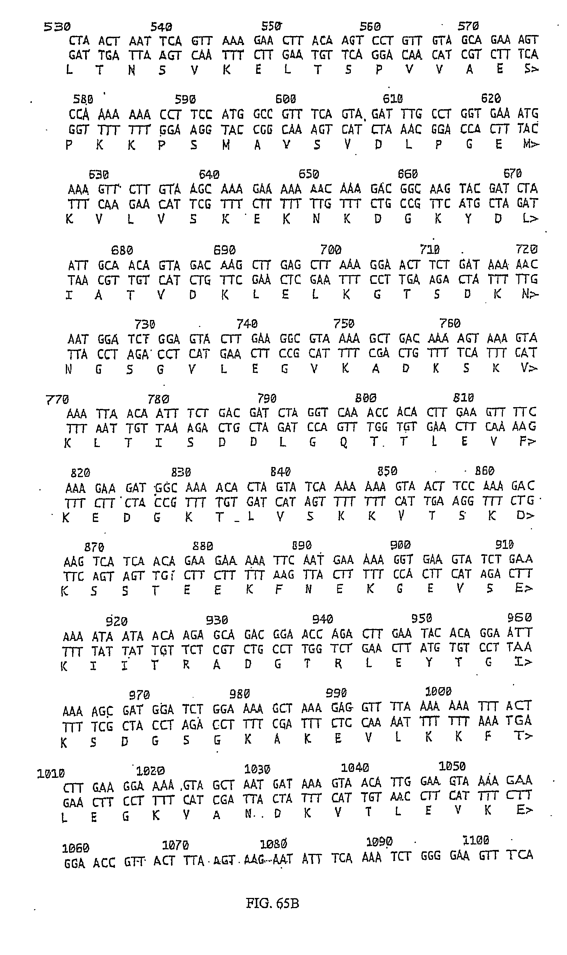

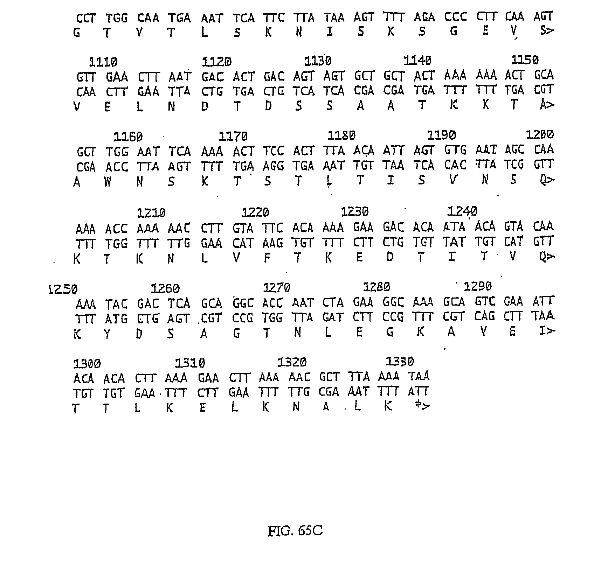

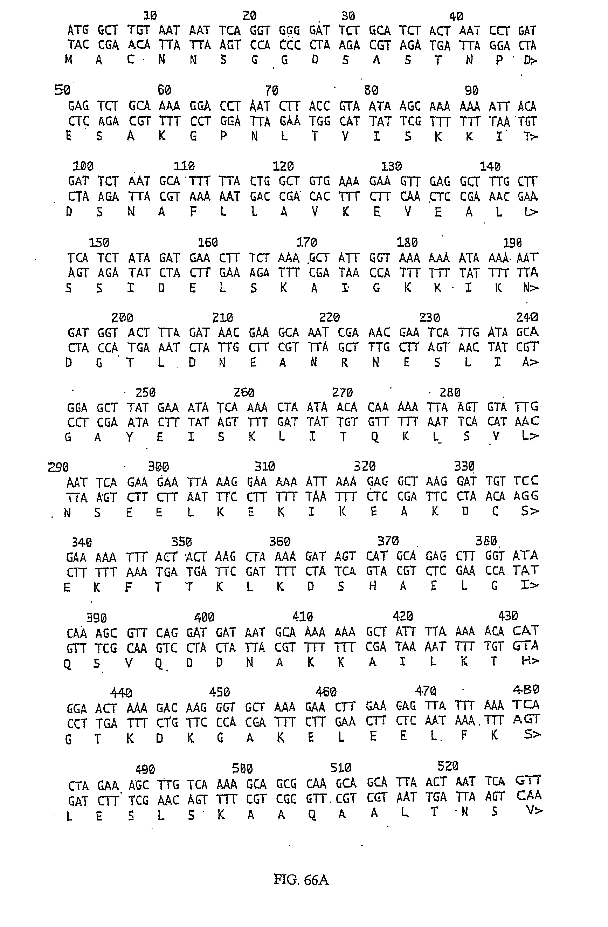

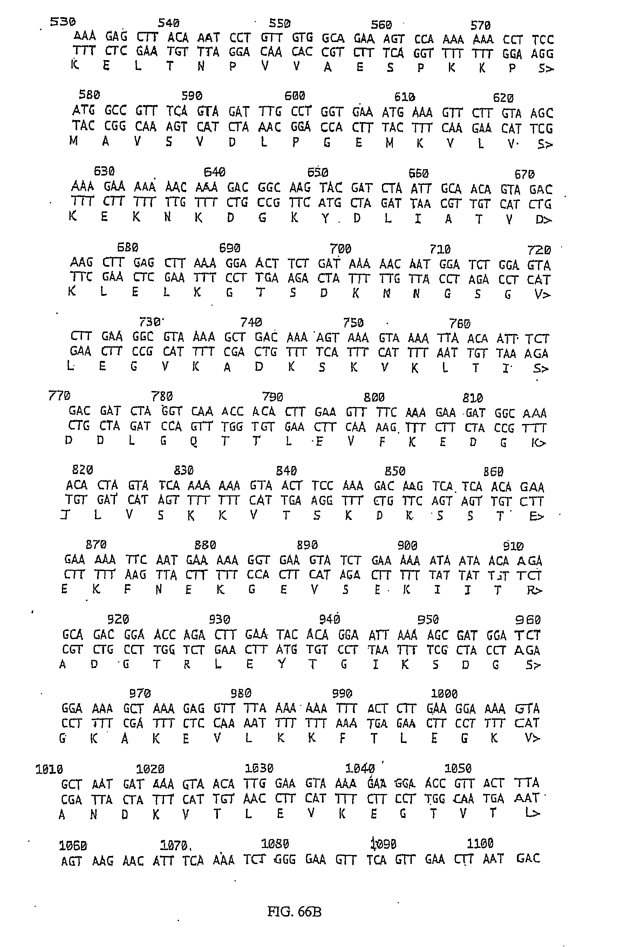

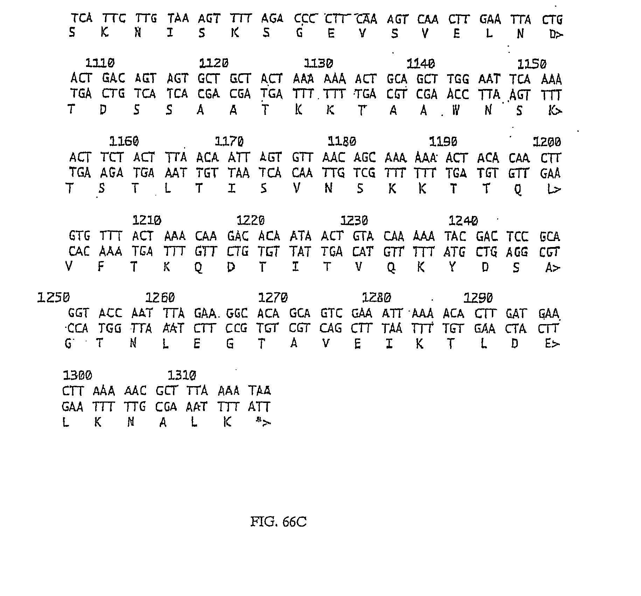

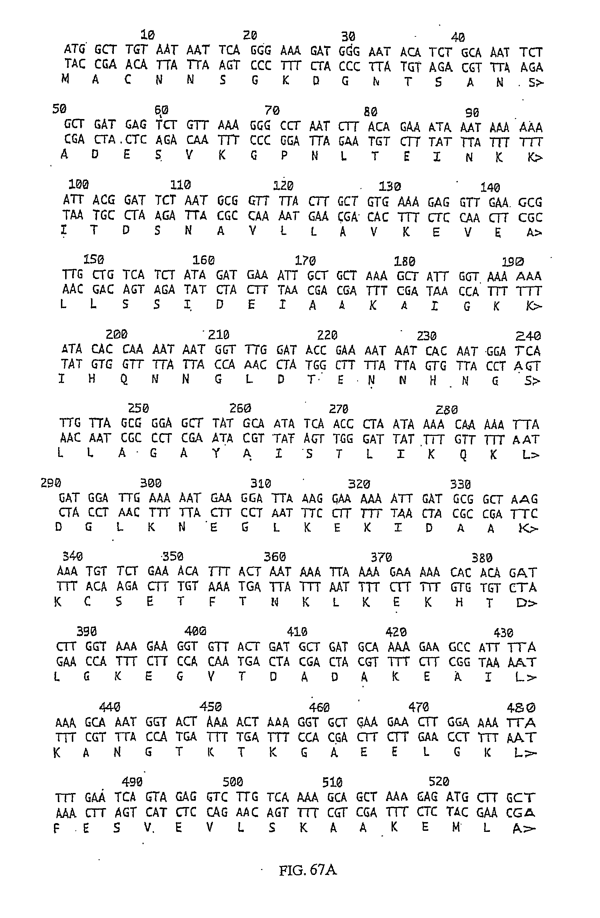

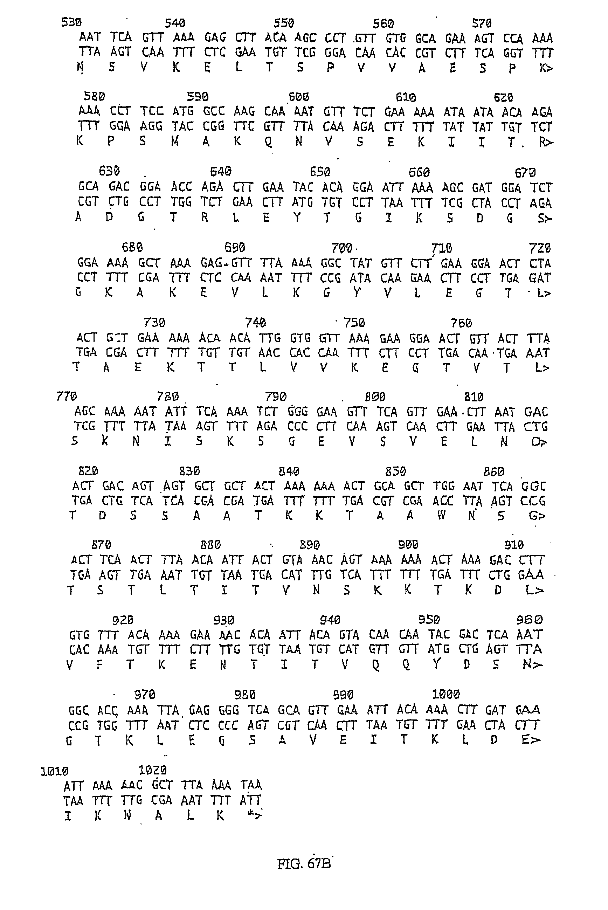

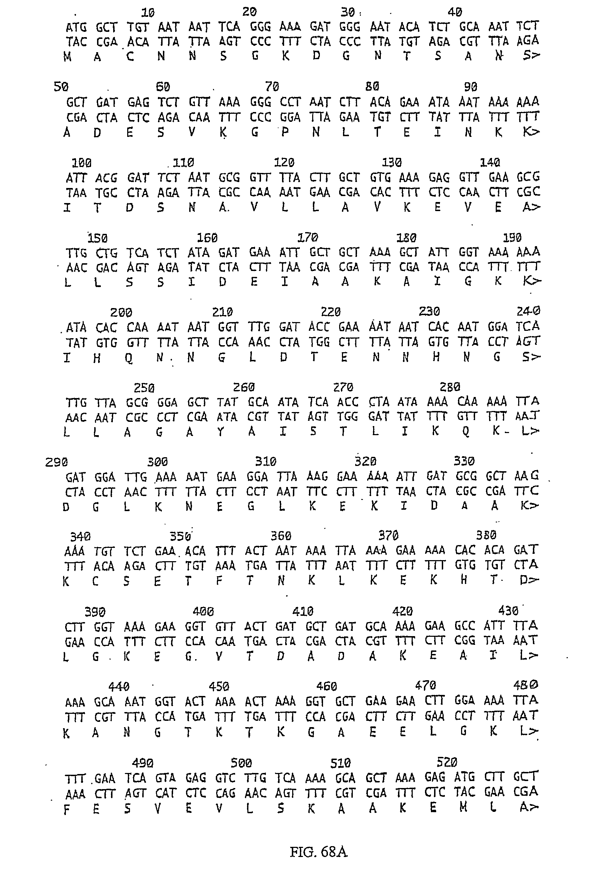

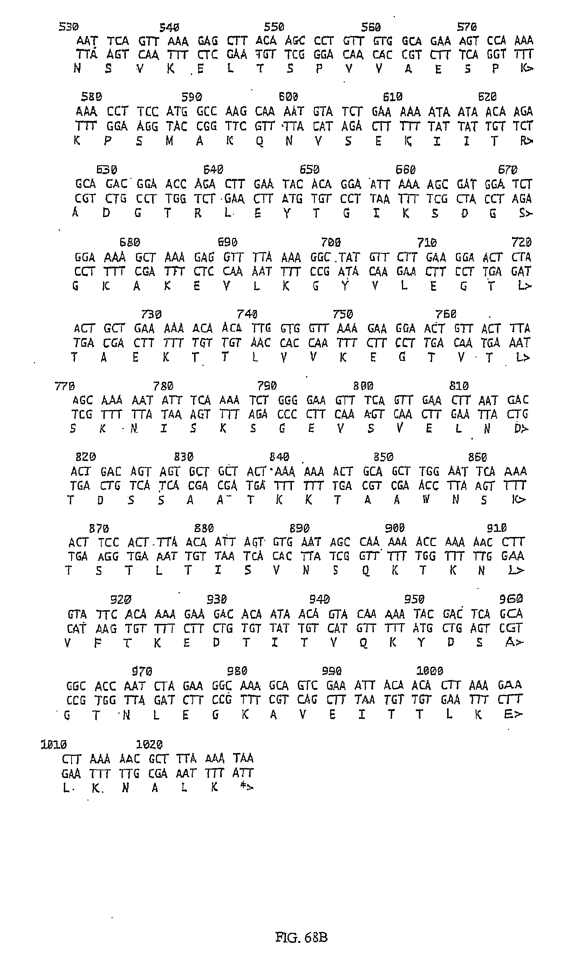

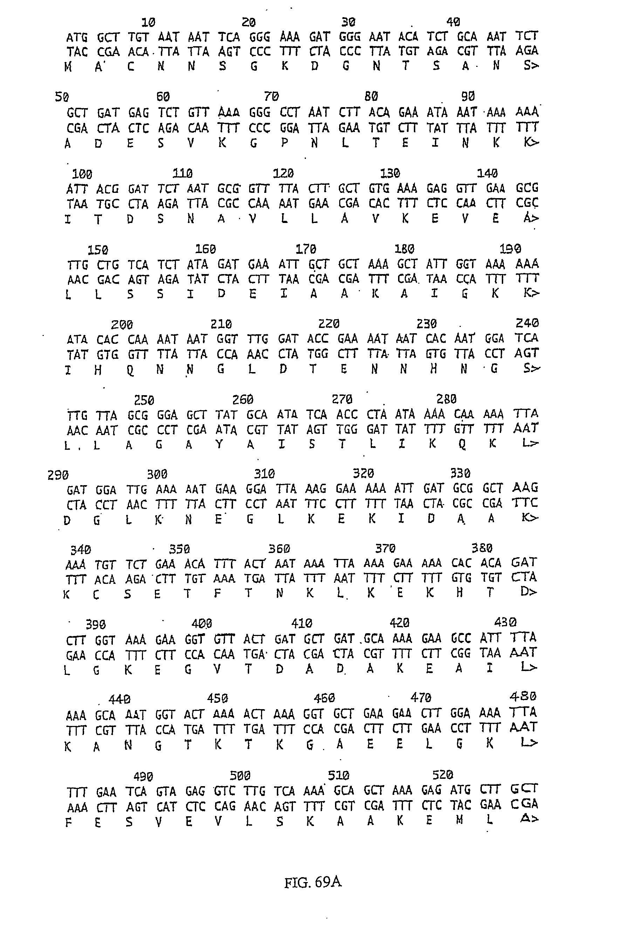

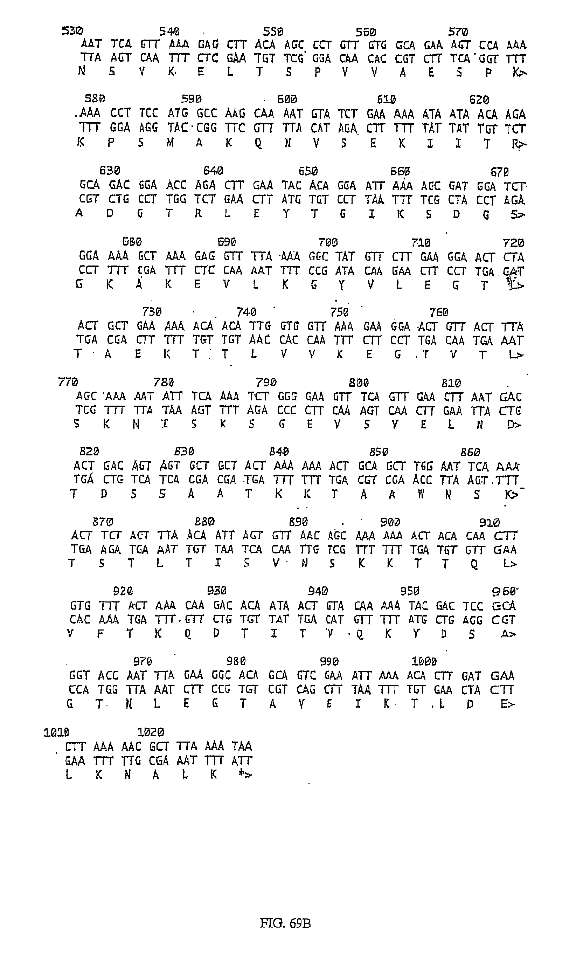

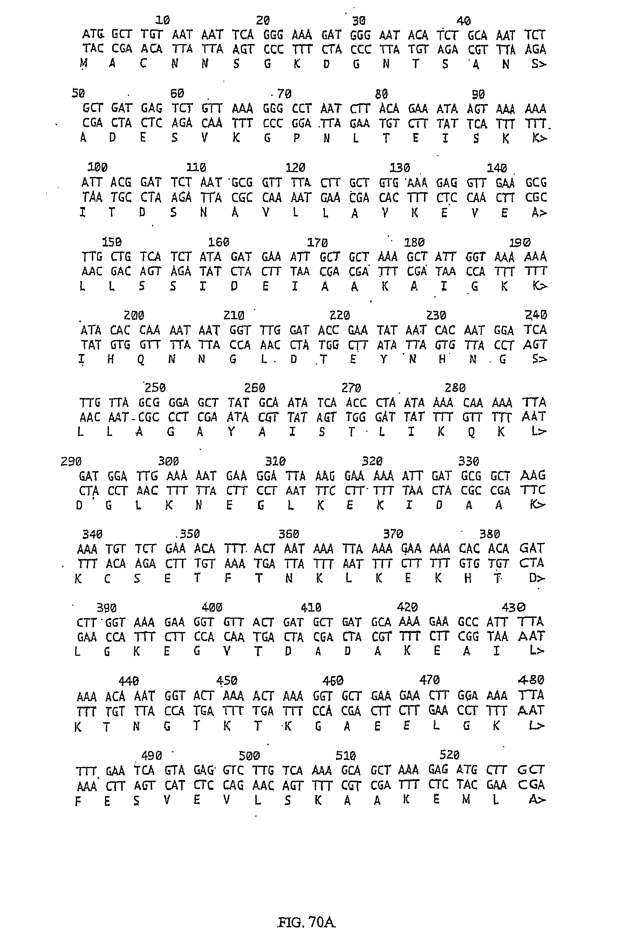

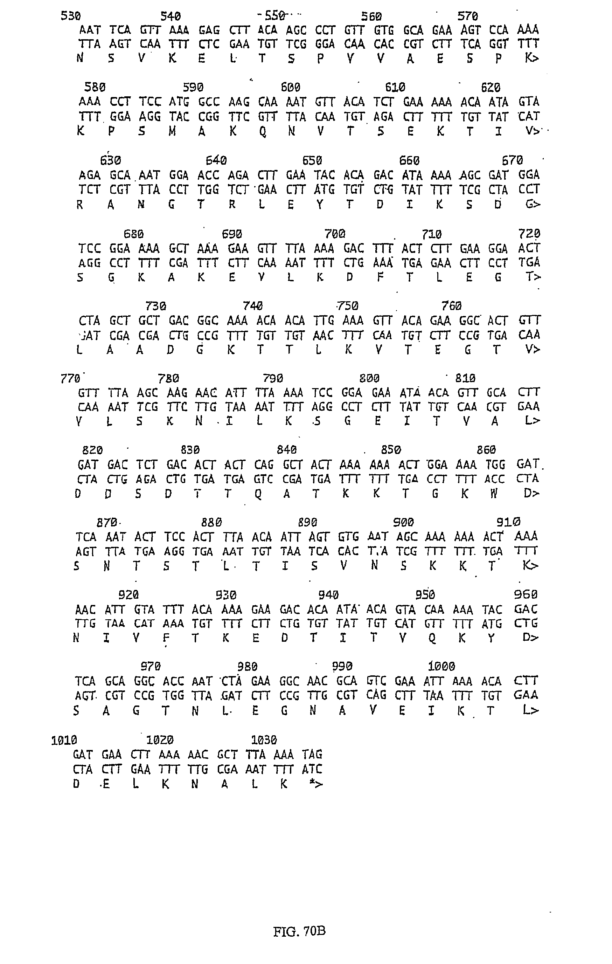

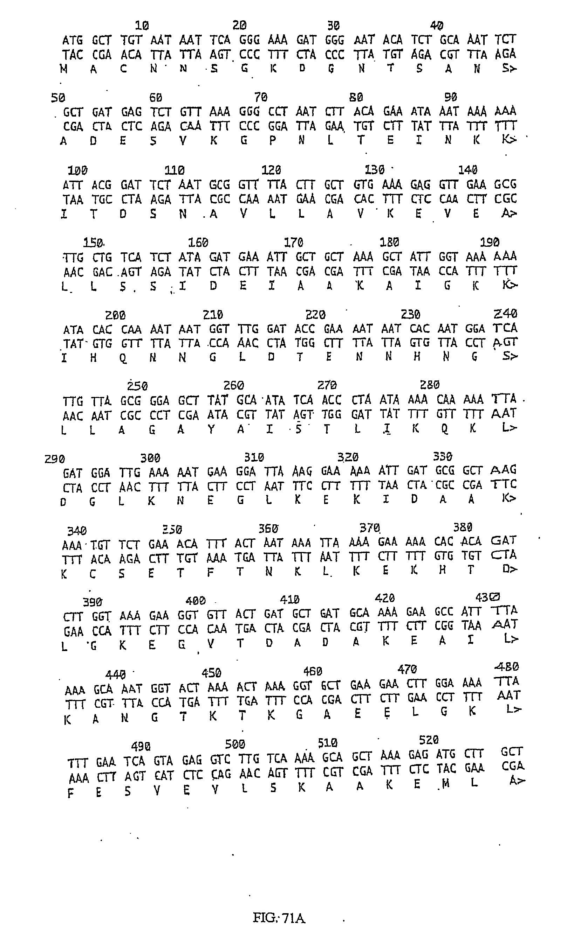

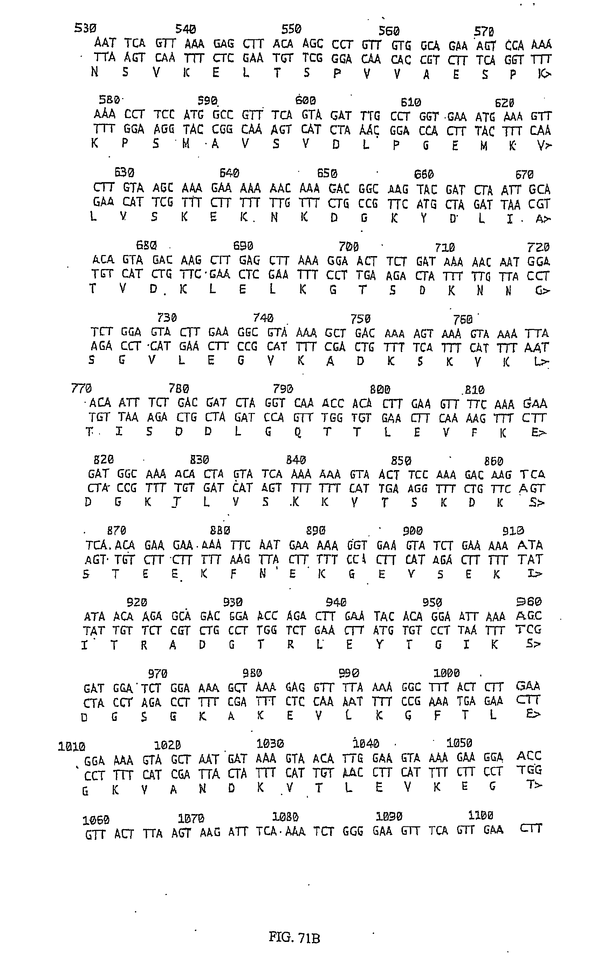

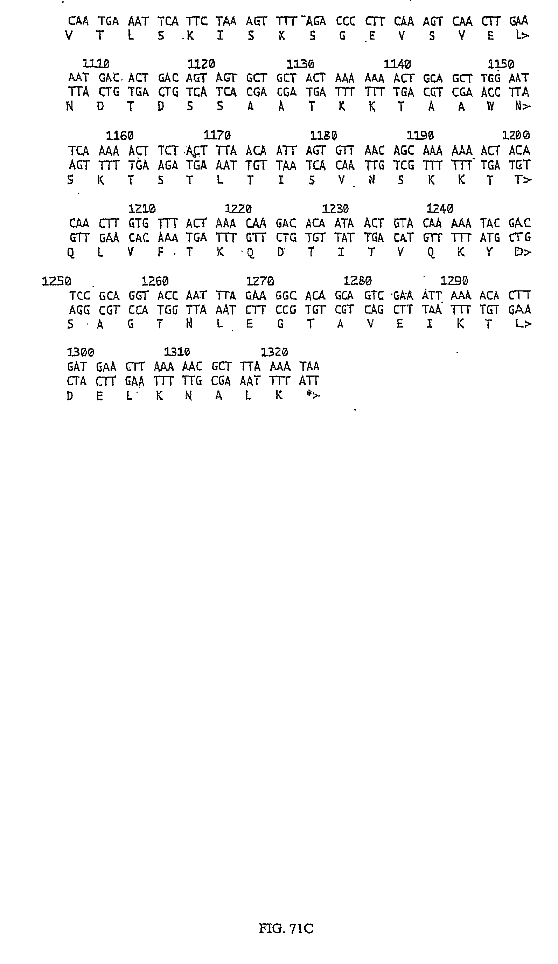

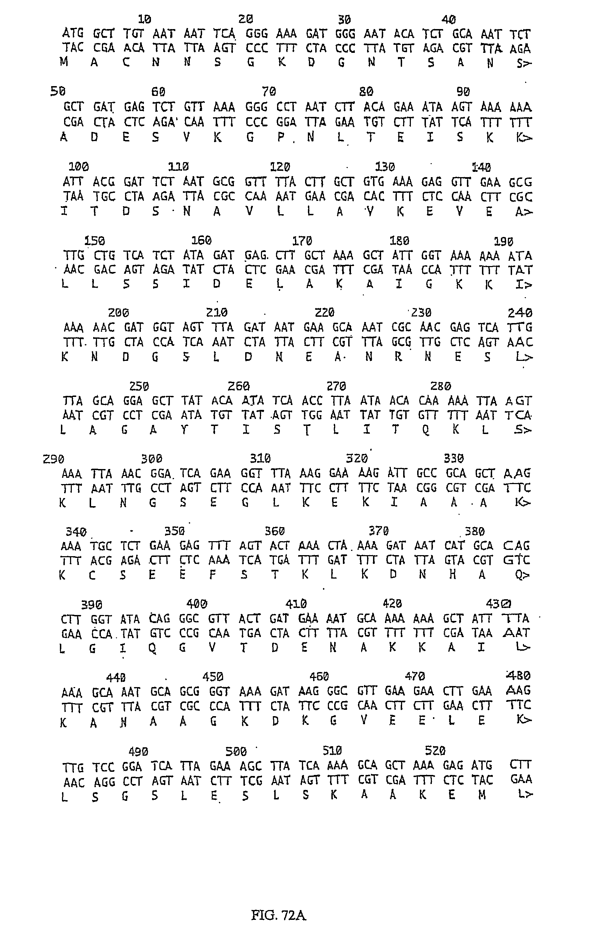

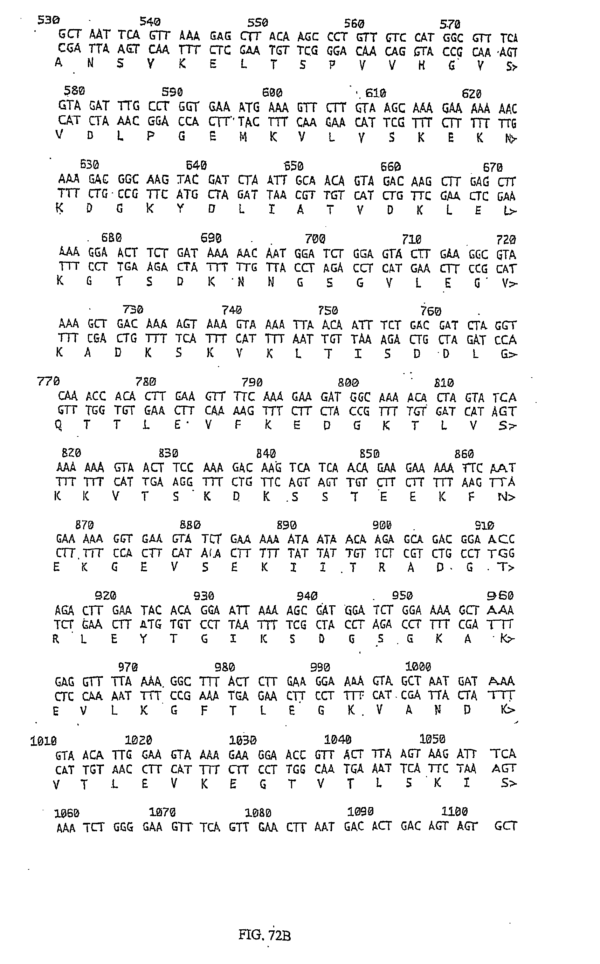

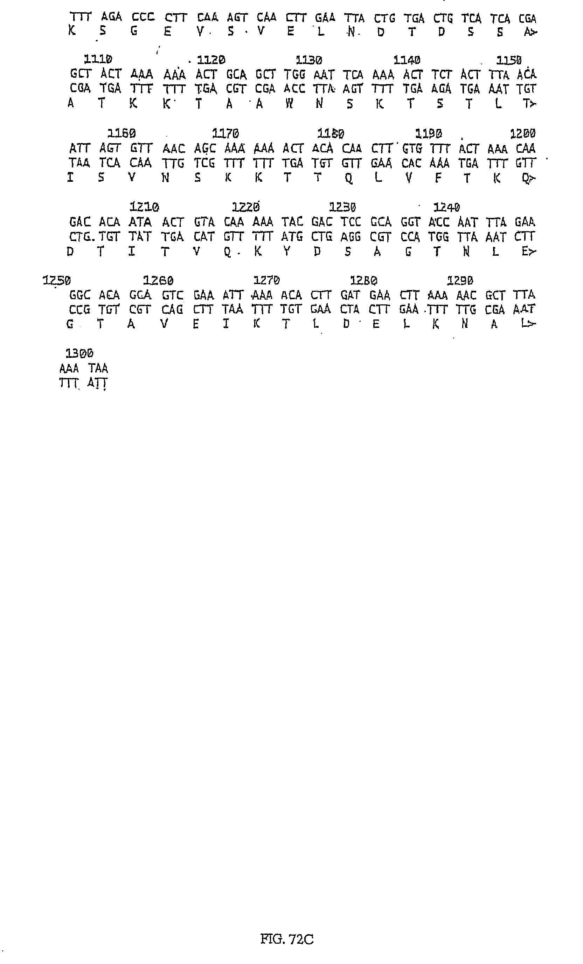

The current invention pertains to chimeric *Borrelia* proteins which include two or more antigenic *Borrelia* polypeptides which do not occur naturally (in nature) in the same protein in *Borrelia,* as well as the nucleic acids encoding such chimeric proteins. The proteins from which the antigenic polypeptides are derived can be from the same strain or genospecies of *Borrelia,* from different strains or genospecies, or from combinations of proteins from the same and from different strains or genospecies. Particular chimeric proteins, and the nucleotide sequences encoding them, are set forth in Figs. 30-37 and 55-72.

The chimeric proteins of the current invention provide antigenic polypeptides of a variety of *Borrelia* strains and/or proteins within a single protein. Such proteins are particularly useful in immunodiagnostic assays to detect the presence of antibodies to native *Borrelia* in potentially infected individuals as well as to measure T-cell reactivity, and can therefore be used as immunodiagnostic reagents. These chimeric proteins are also useful in the generation of immune responses (such as antibody production) against proteins expressed by *Borrelia burgdorferi.* The chimeric proteins of the current invention are additionally useful as vaccine immunogens against *Borrelia* infection.

In one embodiment of the present invention, the chimeric proteins are made up of polypeptide fragments from Lyme Disease-causing strains of *Borrelia.* In another embodiment, the polypeptide fragments that make up the chimeric protein are from outer surface protein A (OspA) and outer surface protein C (OspC), which have the general structure of OspC linked via a peptide bond to the N-terminus of OspA. The present invention encompasses both lipidated and unlipidated chimeric proteins. In one embodiment, the OspA and OspC portions of the chimeric protein possess a lipidation signal. In other embodiments, either the OspA polypeptide portion, the OspC polypeptide portion, or both, do not include a lipidation signal.

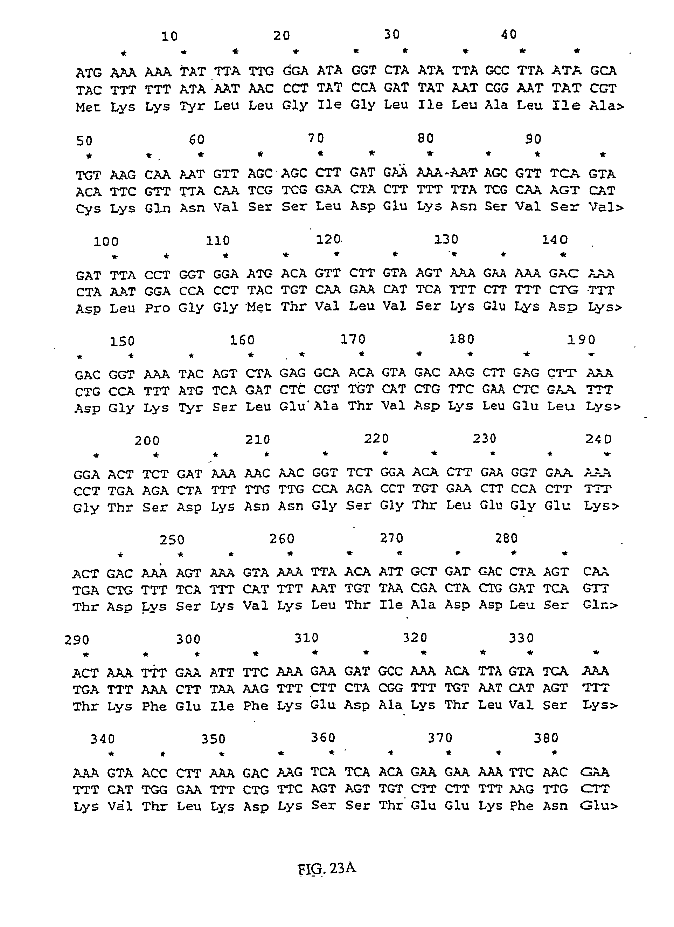

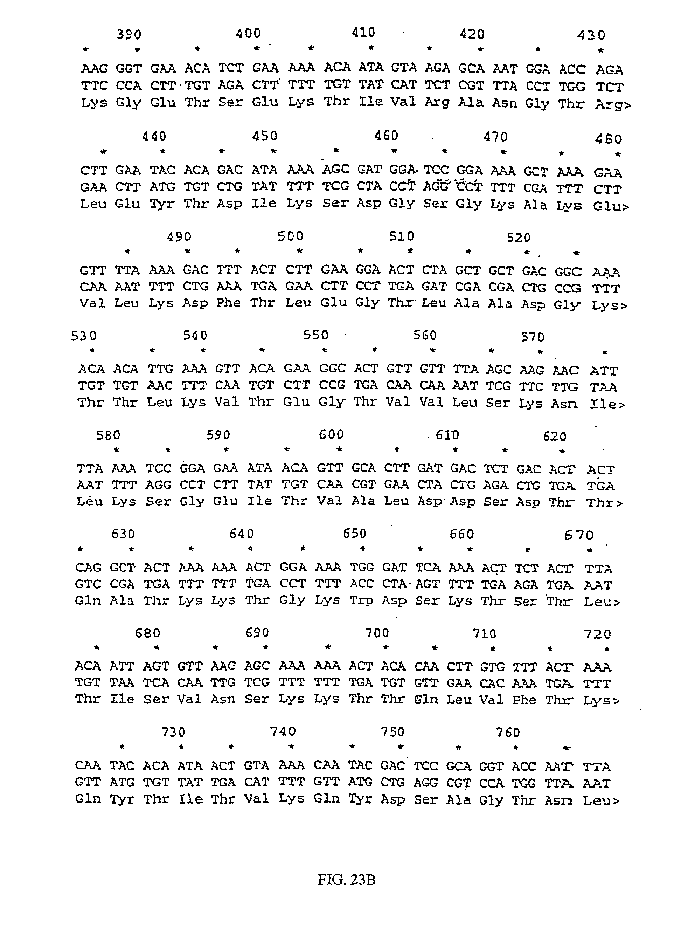

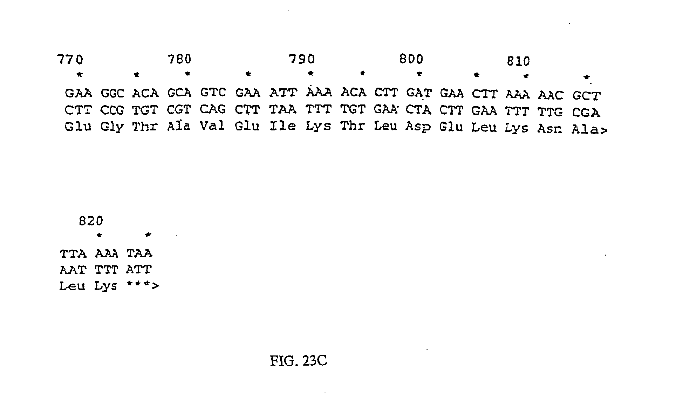

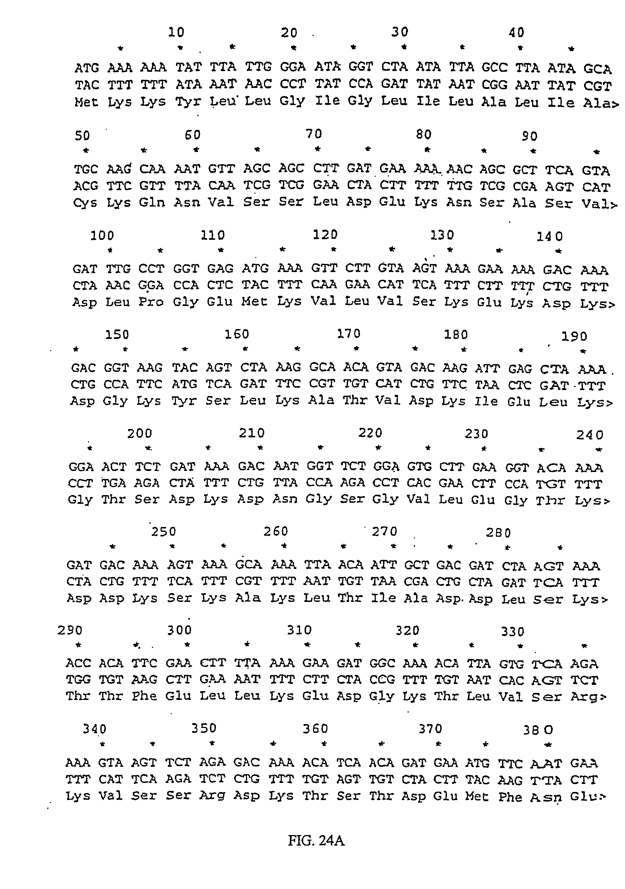

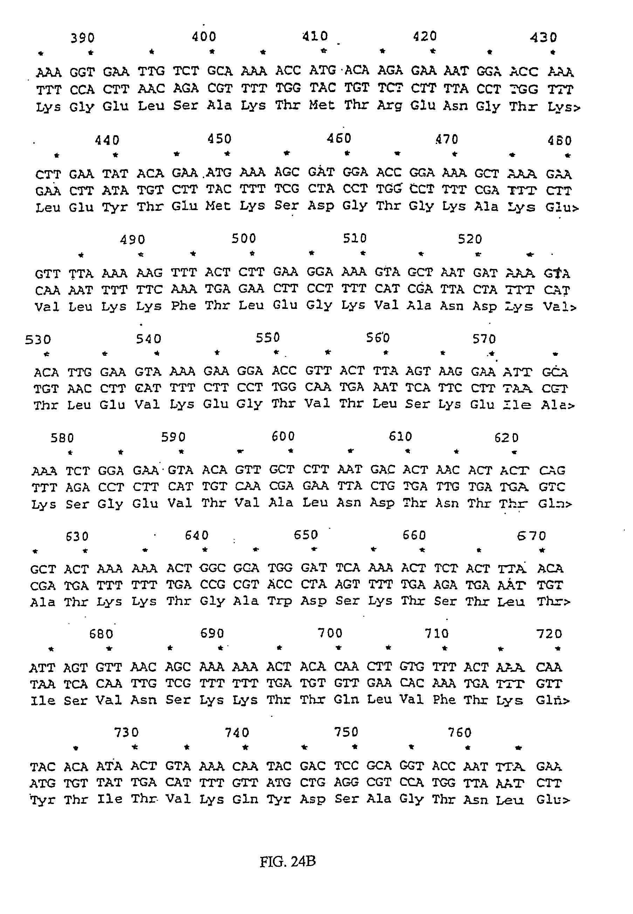

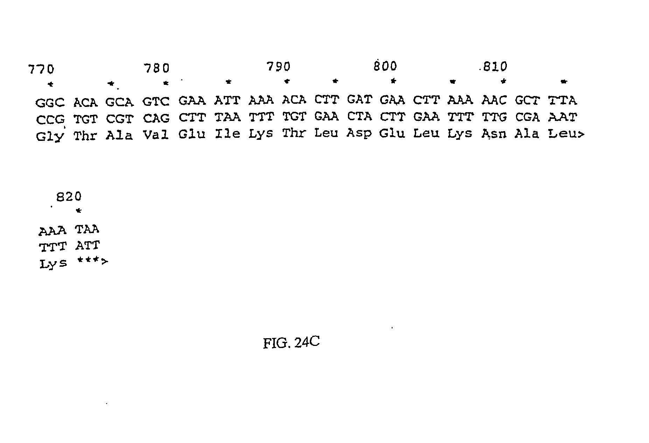

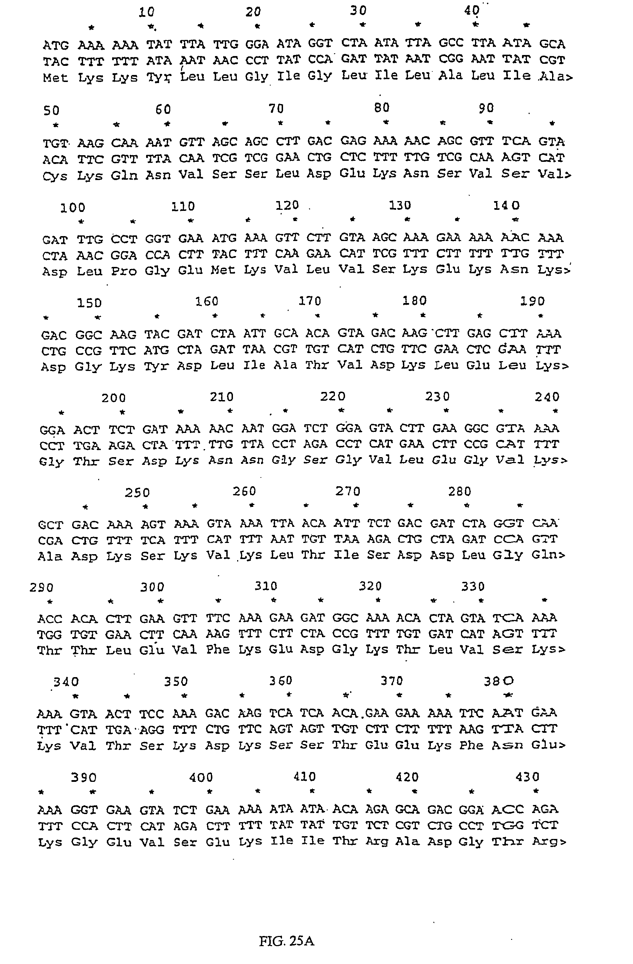

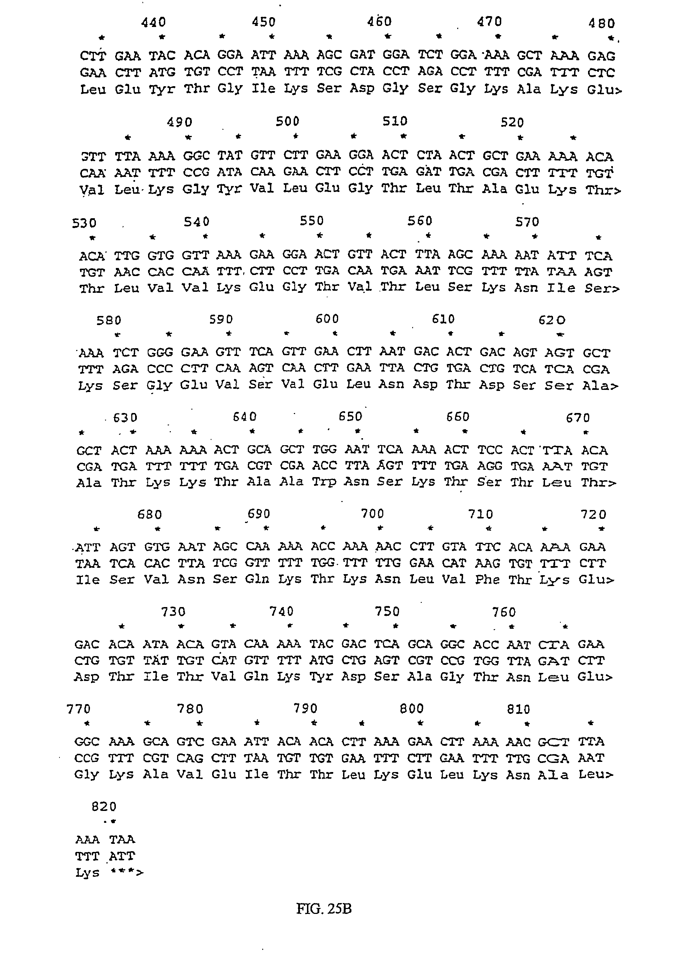

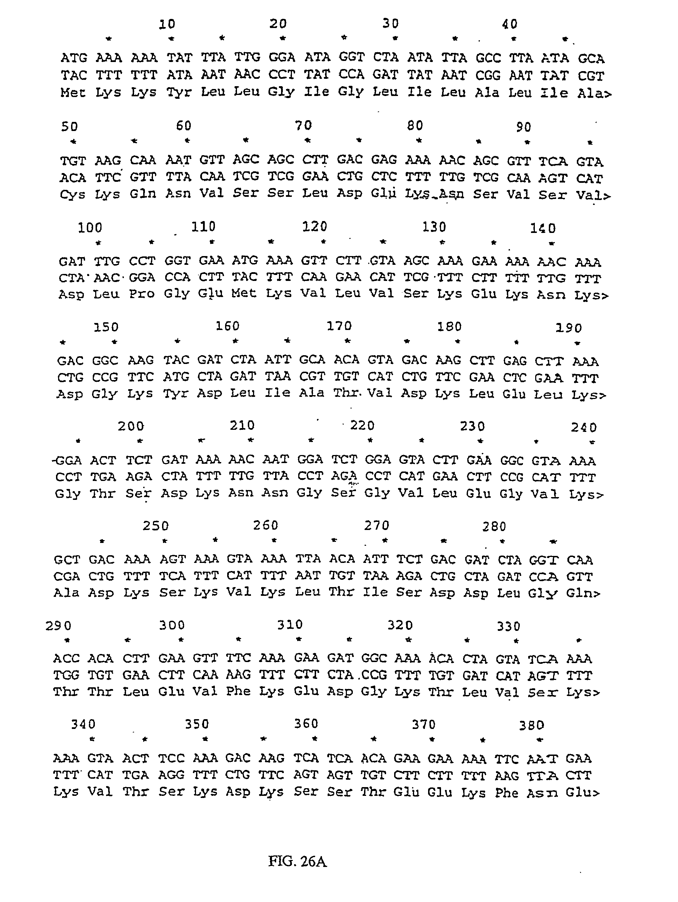

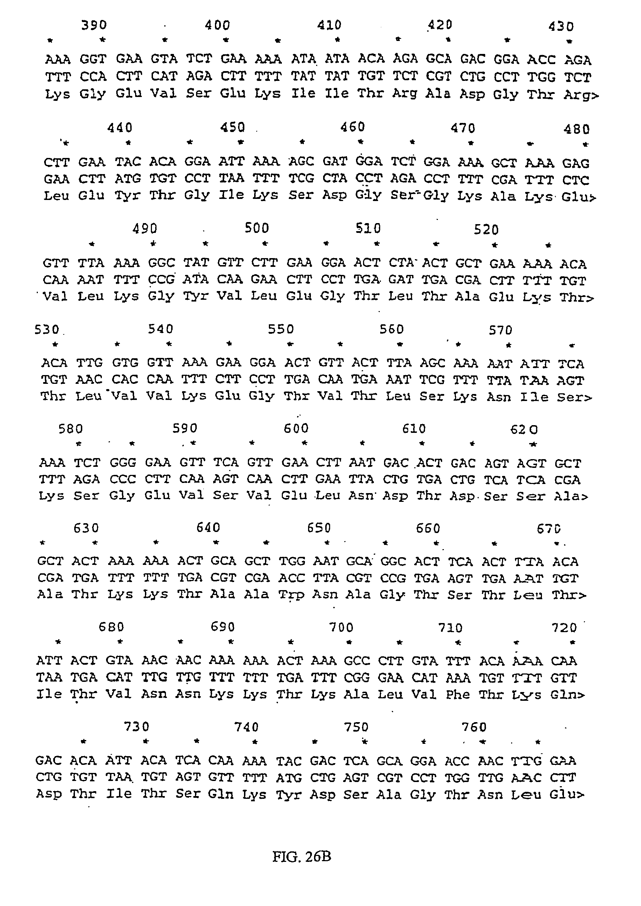

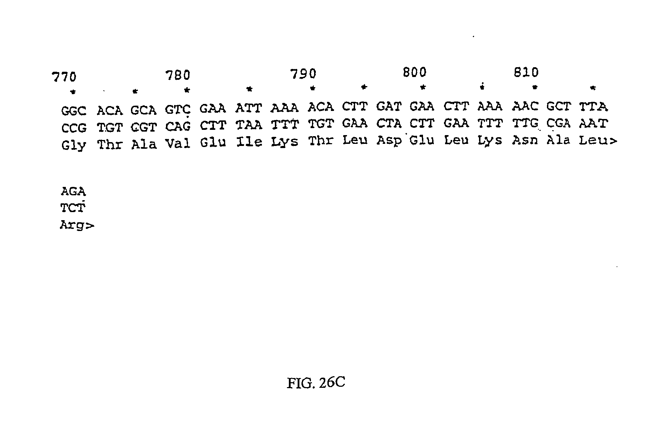

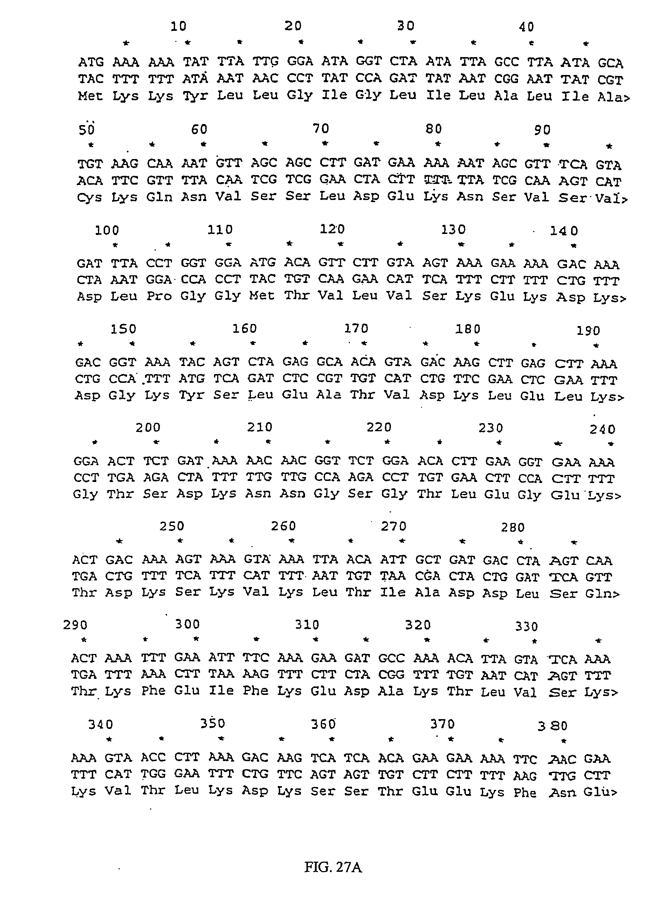

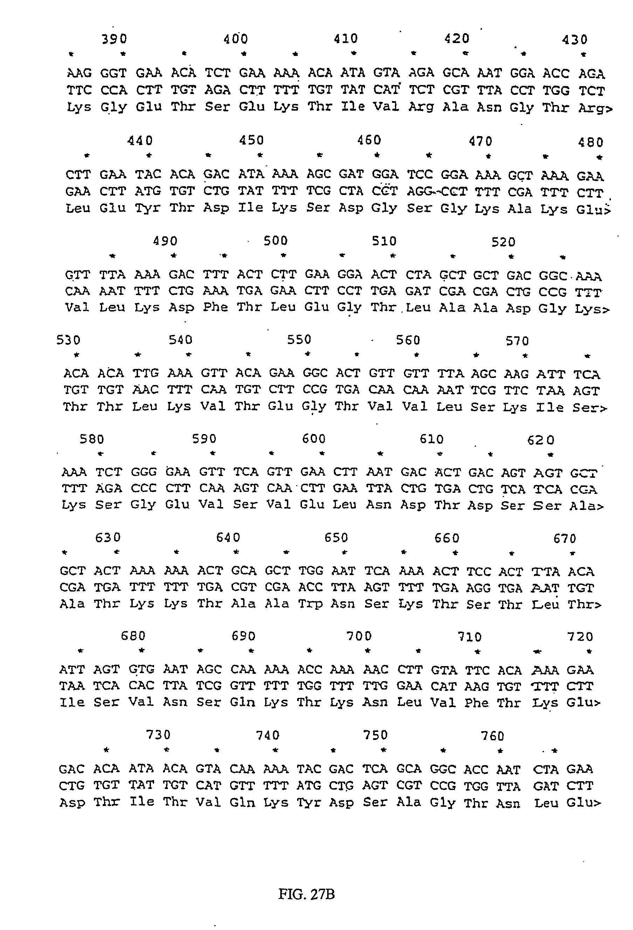

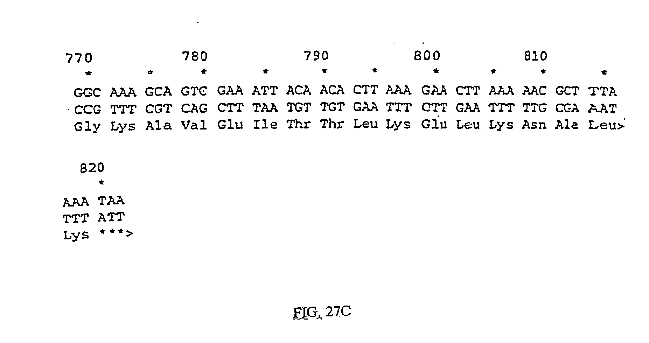

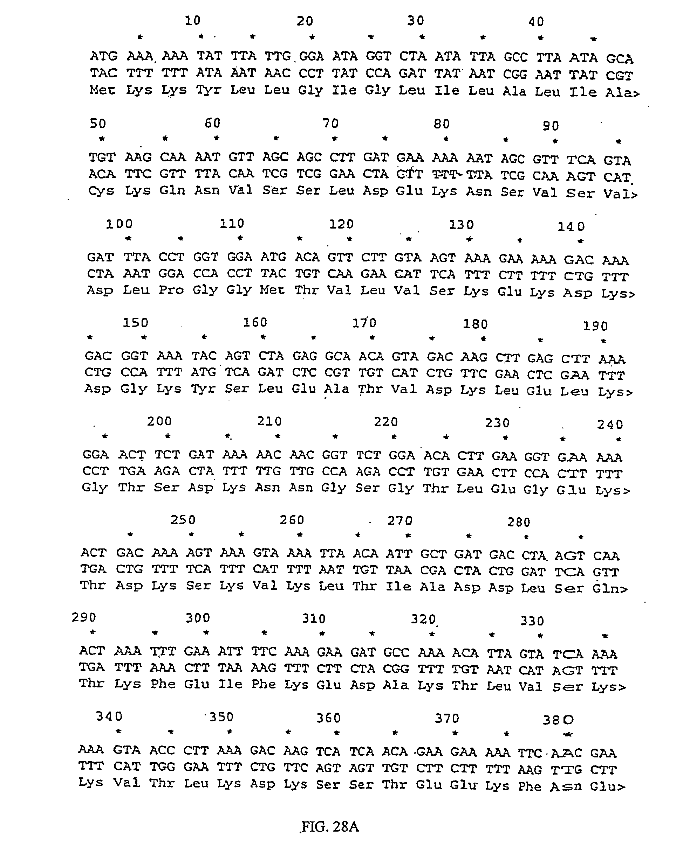

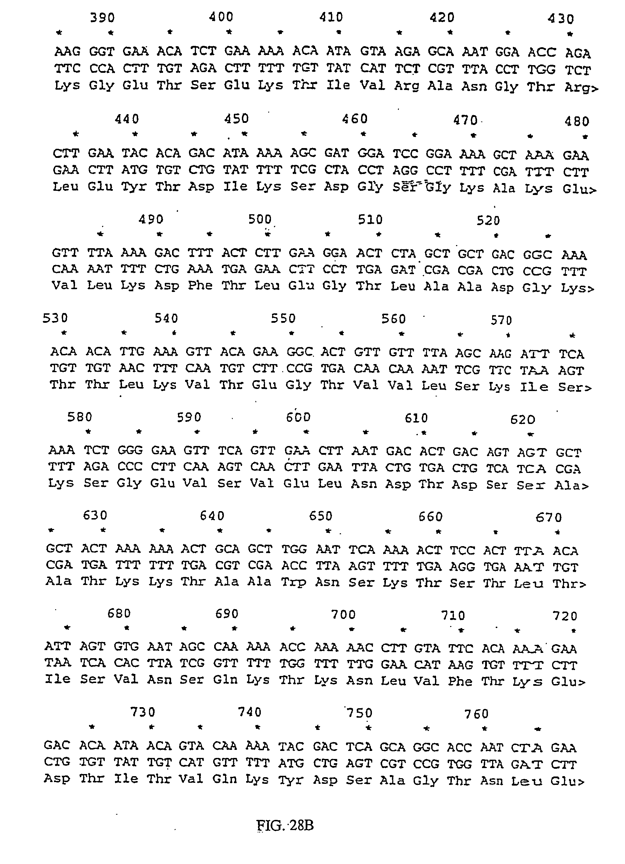

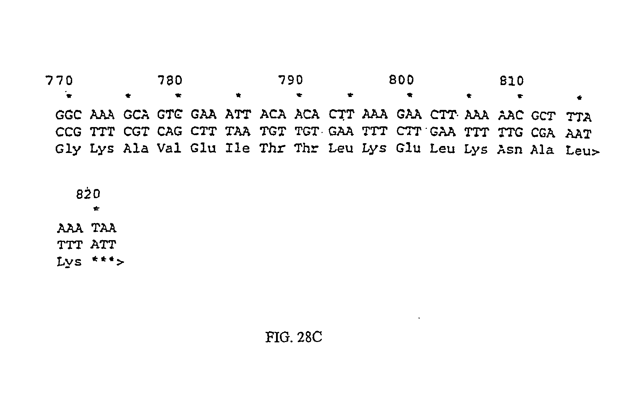

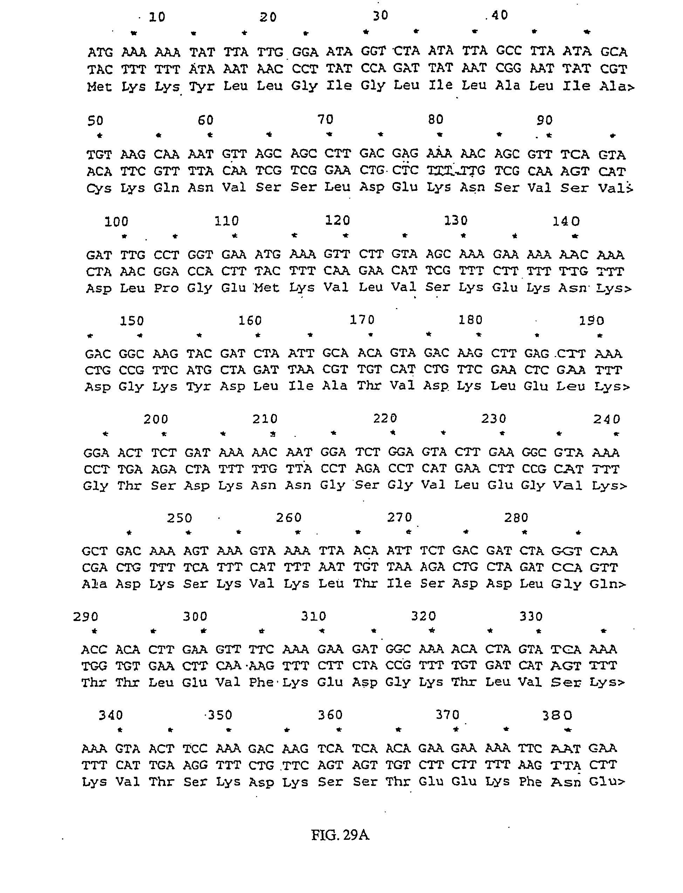

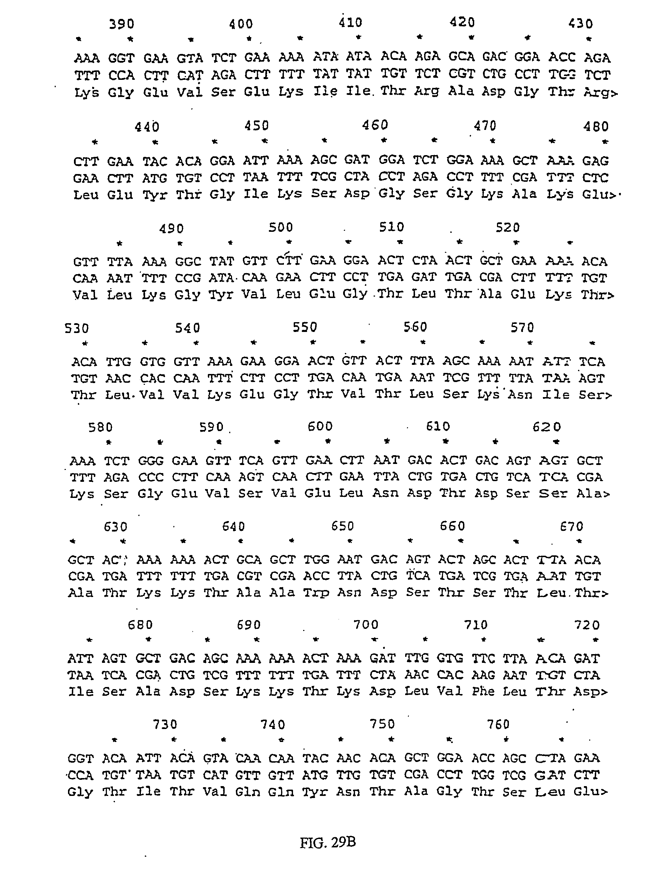

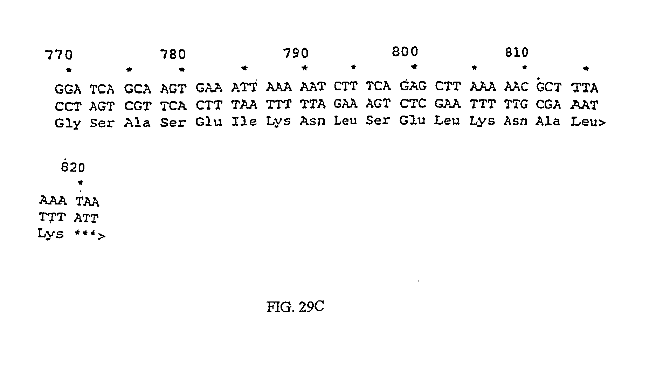

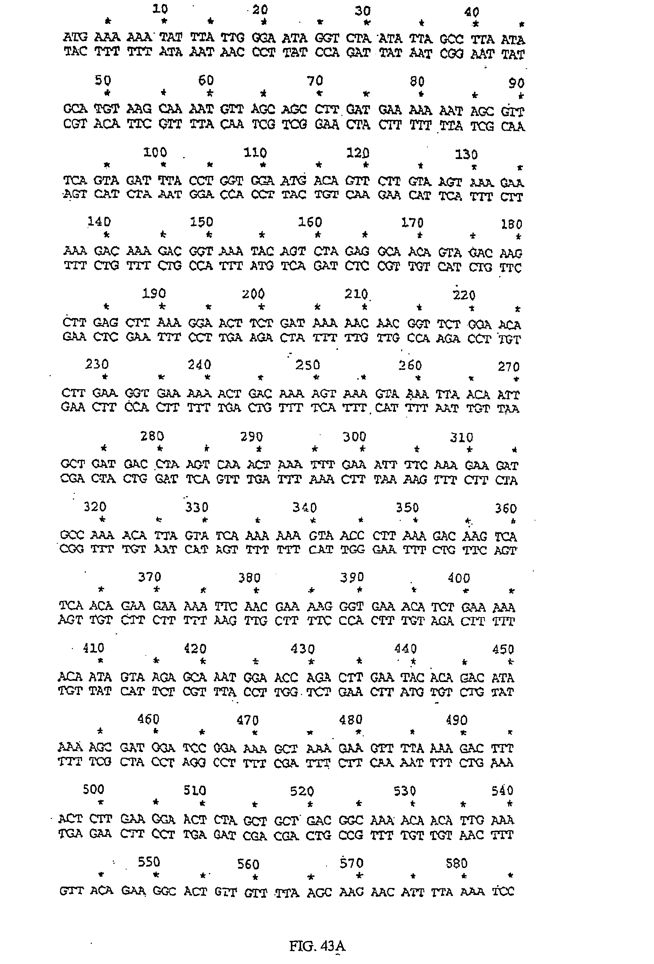

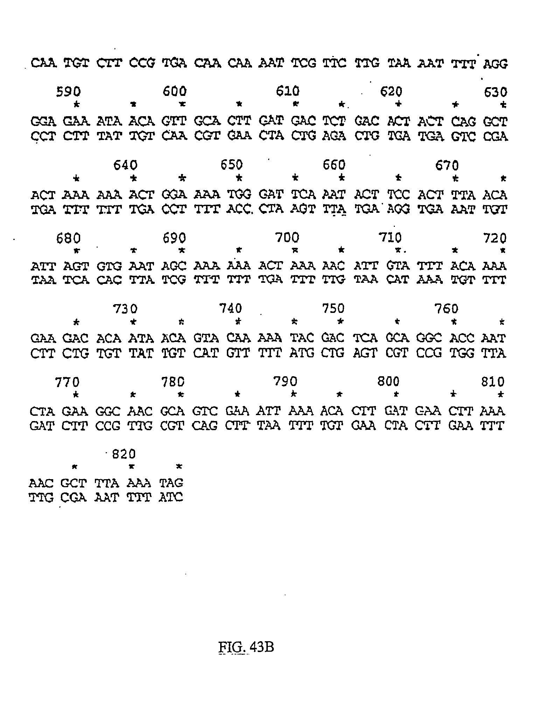

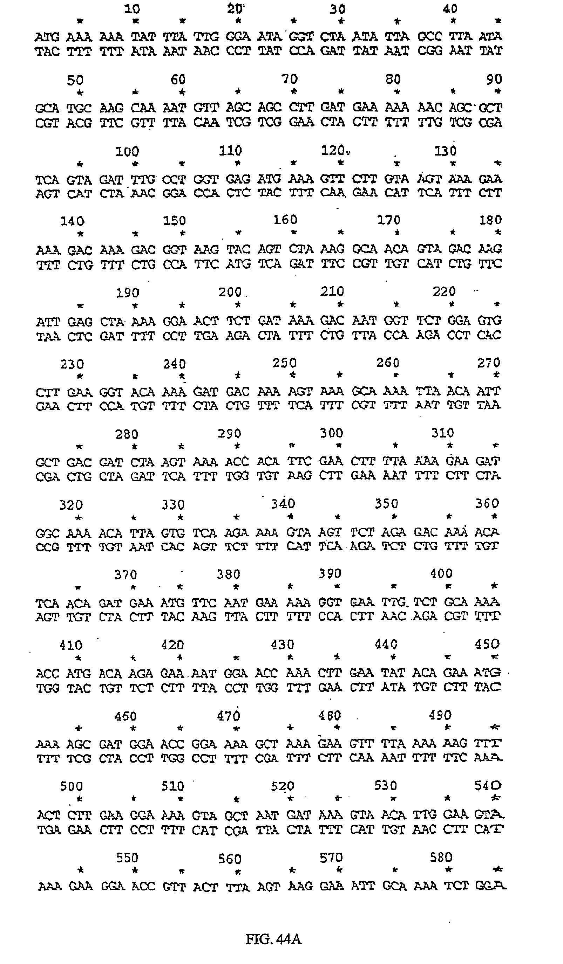

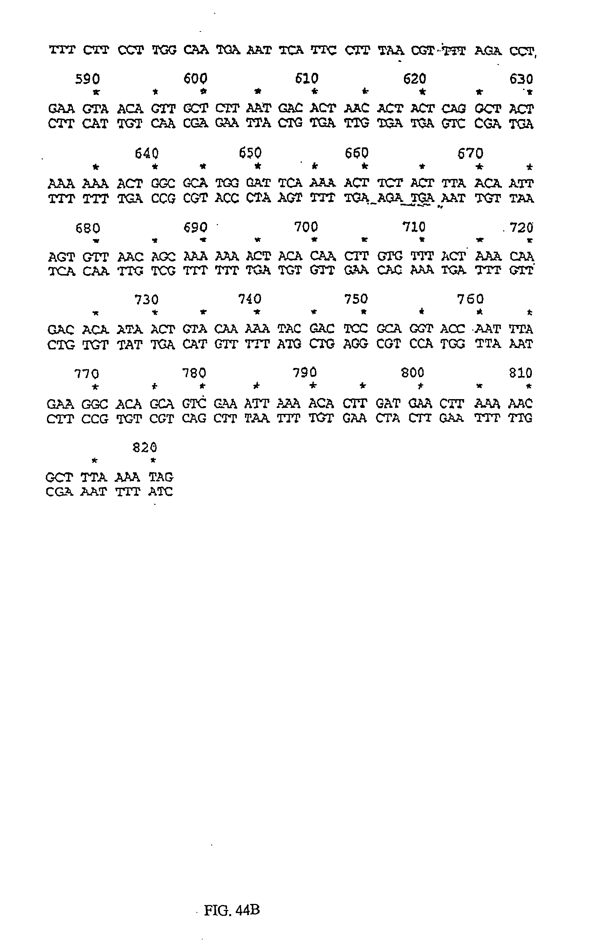

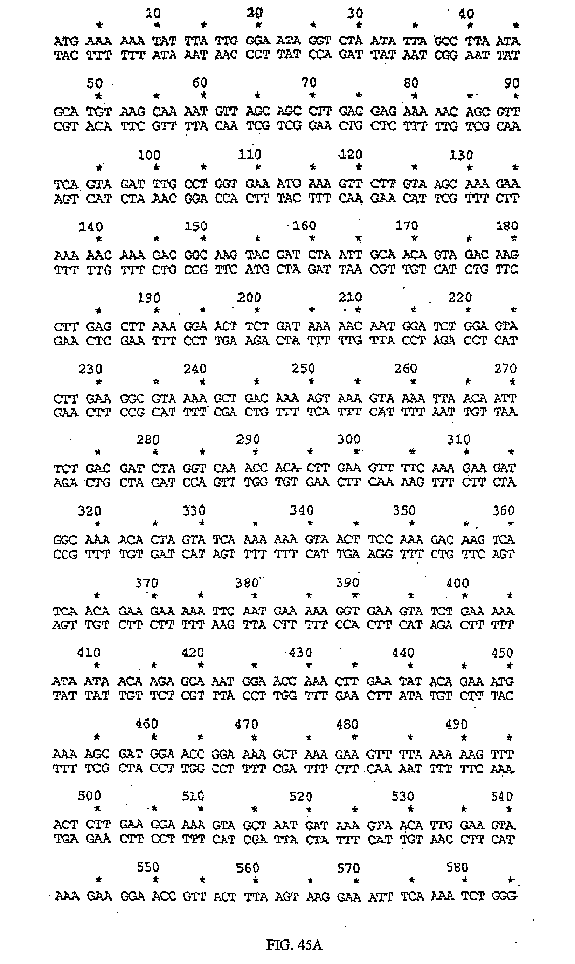

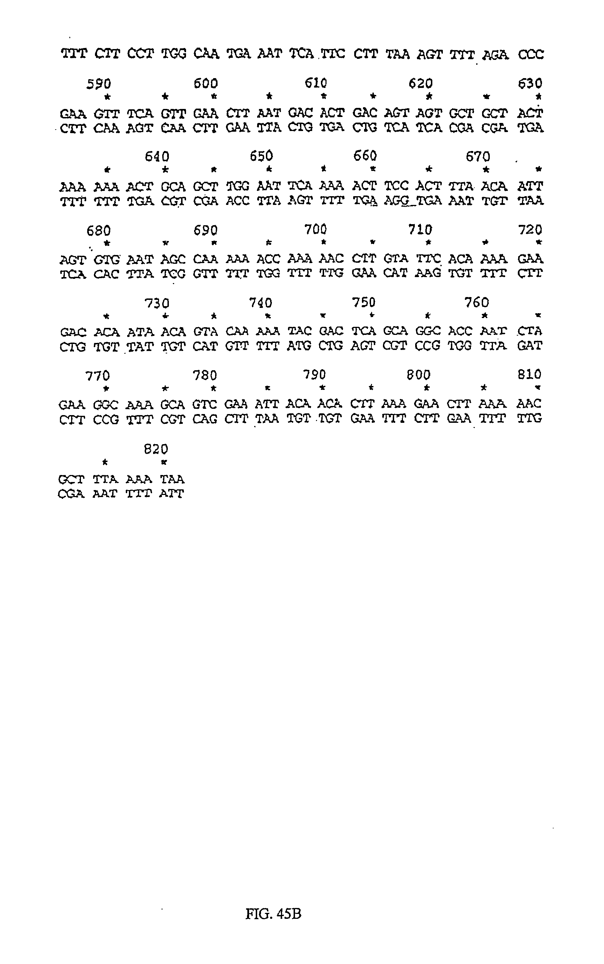

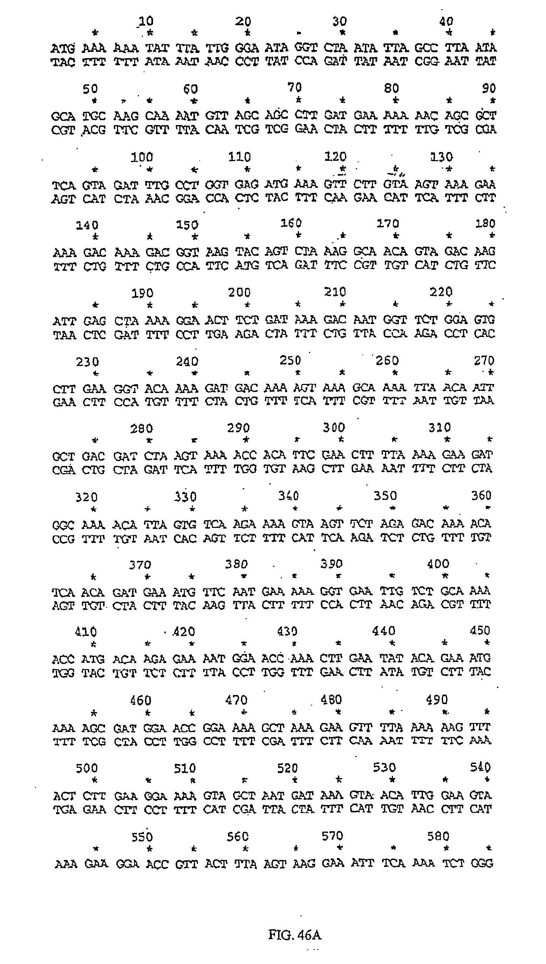

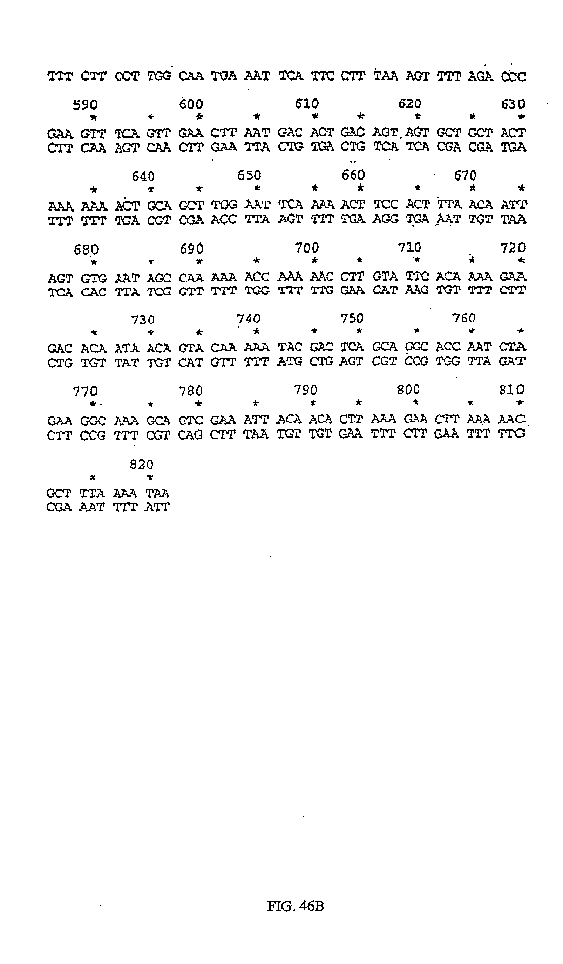

The OspA portion of the chimeric polypeptide can itself comprise OspA portions from two or more strains of Lyme Disease-causing *Borrelia* as described herein and provided, for example, in Figs. 23-29 and 43-46. In another embodiment, the OspA polypeptide comprises OspA portions from two or more genospecies of Lyme Disease-causing *Borrelia,* for example, wherein the genospecies are defined as *Borrelia burgdorferi sensu stricto, Borrelia afzelii* and *Borrelia garinii.* In this manner, the OspC and OspA polypeptide fragments that make up the chimeric protein can be from the same strain or genospecies of *Borrelia,* from different strains or genospecies *of Borrelia,* or from combinations of proteins from the same and from different strains or genospecies *of Borrelia.*

The present invention is also drawn to nucleic acids which encode a *Borrelia* chimeric protein. In a particular embodiment, the composition comprises a nucleic acid that encodes a chimeric protein of at least two polypeptides, wherein the first polypeptide comprises *Borrelia burgdorferi* OspC, and the second polypeptide comprises *Borrelia burgdorferi* OspA, such that OspC is upstream of OspA. The OspC and OspA nucleic acid fragments that make up the chimeric protein can be from the same strain or genospecies of *Borrelia,* from different strains or genospecies *of Borrelia,* or from combinations of proteins that are from the same and/or different strains or genospecies *of Borrelia.*

The present invention is also drawn to expression vectors which comprise an isolated DNA encoding a *Borrelia* chimeric protein. In one embodiment, the composition includes an expression vector comprising an isolated DNA which encodes an OspC/OspA chimeric protein as described herein. The present invention also encompasses host cells which comprise a recombinant nucleic acid encoding an OspC/OspA chimeric protein as described herein.

The present invention is also drawn to methods of making the *Borrelia* chimeric polypeptides described herein. In one embodiment, the method of making a chimeric *Borrelia* protein comprises selecting a polynucleotide sequence encoding OspC, or an antigenic portion thereof, selecting a polynucleotide sequence encoding OspA, or an antigenic portion thereof, and ligating these polynucleotide sequences together.

The present invention is also drawn to methods of delivering the *Borrelia* chimeric polypeptides described herein. In one embodiment, the method comprises administering the chimeric protein in a physiologically-acceptable carrier to an individual. As a result of the administration of the chimeric protein, the individual develops at least some immune response to the chimeric protein, e.g., the individual generates a humoral immune response, wherein antibodies are produced by the individual that recognize at least a portion of said chimeric polypeptide.

The present invention is also drawn to methods of delivering nucleic acids which encode the chimeric polypeptides described herein. In one embodiment, the method comprises administering the nucleic acid in a physiologically-acceptable carrier to an individual. As a result of the administration of the nucleic acid, the individual expresses the chimeric protein at least transiently and develops at least some immune response to the chimeric protein encoded by the nucleic acid, e.g., the individual generates a humoral immune response, wherein antibodies that recognize at least a portion of the chimeric polypeptide produced from the nucleic acid, are produced by the individual.

The invention also encompasses methods of using the chimeric proteins described herein in a diagnostic assay. As described herein, the method can be used to detect the presence of OspA- and/or OspC-specific antibodies in a sample, e.g., a host sample of interest. The method comprises contacting a sample, e.g., a host sample of interest, with the chimeric protein, under conditions, wherein antibodies, if present in the host sample, bind to the chimeric protein thereby forming antigen-antibody complexes. The antigen-antibody complexes are then detected. In this manner, the chimeric proteins of the present invention can be used to detect an immune response to Lyme Disease causing *Borrelia.*

The present invention is also drawn to diagnostic kits which comprise the chimeric polypeptides described herein. In one embodiment, the kit comprises a *Borrelia burgdorferi* OspC/OspA chimeric protein. The kit also includes reagents for detecting antibody-antigen complexes that are formed between the OspC/OspA chimeric protein and antibodies that are present in a sample, e.g., a user-supplied host sample.

The present invention is also drawn to pharmaceutical compositions which can be used to vaccinate and/or treat *Borrelia* infection in an animal or human. The pharmaceutical composition can be administered together with a physiologically-acceptable carrier and/or with suitable excipients and/or adjuvants.

The present invention is also drawn to methods of immunizing an animal or human against Lyme disease. In a particular embodiment, the method comprises administering a *Borrelia* chimeric OspC/OspA protein. The chimeric protein can be administered together with a physiologically-acceptable carrier, a suitable excipient and/or a suitable adjuvant, to an animal or human such that the animal or human develops an immune response to at least one of the OspC and/or OspA polypeptides of the composition.

By incorporating polypeptide fragments from multiple *Borrelia burgdorferi* proteins, the present invention provides a composition that has great utility for vaccines and diagnostic kits. As a result of the present invention, there exist diagnostic tools and vaccines that comprise both OspA and OspC antigens from various *Borrelia burgdorferi* strains and/or genospecies in a single protein. Since OspA is primarily expressed in the tick vector, and OspC is upregulated in response to the feeding of an infected tick on a mammal, this allows for a diagnostic tool or vaccine that can recognize antigens that are expressed at different stages of the life cycle *of Borrelia burgdorferi.* Thus, the chimeric proteins of the present invention can act at the level of the tick as well as the level of the host, in preventing infection and/or disease caused by *Borrelia.* Moreover, by incorporating unique polypeptide fragments from pathogenic families *of Borrelia,* such as *Borrelia burgdorferi sensu stricto, Borrelia afzelii* and *Borrelia garinii,* an improved diagnostic tool or vaccine is produced which can detect clinically important exposure to a wider variety of pathogenic *Borrelia,* while overlooking the remainder of non-pathogenic families of *Borrelia.* Furthermore, OspC polypeptides can be selected from strains of *Borrelia* that are associated with disseminated disease, as described in WO 00/78966, the teachings of which are incorporated herein in their entirety.

The present invention also provides a combination *of Borrelia* antigens in a single polypeptide that, when used as a vaccine, are expected to prevent Lyme disease from becoming systemic. The chimeric proteins of the present invention can be effective in preventing Lyme disease, as well as having a therapeutic effect on established infection, for example after the tick bite is noticed by the patient.

The present invention is drawn to both lipidated and unlipidated chimeric proteins. Unlipidated chimeric proteins, such as the OspC/OspA chimeric proteins described herein, have certain advantages over their lipidated counterparts. These advantages include simpler production methods, improved yields of protein and simpler purification methods. While the lack of a lipidation signal provides several advantages, it had been thought that the lipidation signal was required for immunogenicity. However, as described herein, the non-lipidated OspC/OspA chimeric proteins of the present invention elicit an immune response that is at least as broadly reactive as that of lipidated OspA and lipidated OspC control proteins. Moreover, the unlipidated OspC/OspA chimeric proteins of the present invention unexpectedly elicit an immune response to more than one genospecies and/or strain of Lyme disease-causing *Borrelia,* including genospecies and/or strains that were not used to generate the particular chimeric OspC/OspA immunogen.

For a better understanding of the present invention together with other and further objects, reference is made to the following description, taken together with the accompanying drawings.

### BRIEF DESCRIPTION OF THE DRAWINGS

Fig. 1 summarizes peptides and antigenic domains localized by proteolytic and chemical fragmentation of OspA.
Fig. 2 is a comparison of the antigenic domains depicted in Fig. 1, for OspA in nine strains *of B. burgdorferi.*
Fig. 3 is a graph depicting a plot of weighted polymorphism versus amino acid position among 14 OspA variants. The marked peaks are: a) amino acids 132-145; b) amino acids 163-177; c) amino acids 208-221. The lower line at polymorphism value 1.395 demarcates statistically significant excesses of polymorphism at p = 0.05. The upper line at polymorphism value 1.520 is the same, except that the first 29 amino acids at the monomorphic N-terminus have been removed from the original analysis.
Fig. 4 depicts the amino acid alignment of residues 200 through 220 for OspAs from strains B31 and K48 as well as for the site-directed mutants 613, 625, 640, 613/625, and 613/640. The arrow indicates Trp216. Amino acid changes are underlined.
Fig. 5 is a helical wheel projection of residues 204-217 of B31 OspA. Capital letters indicate hydrophobic residues; lower case letters indicate hydrophilic residues; + and - indicate positively and negatively charged residues, respectively. The dashed line indicates division of the alpha-helix into a hydrophobic arc (above the line) and a polar arc (below the line). Adapted from France et al. (Biochem. Biophys. Acta 1120: 59 (1992)).

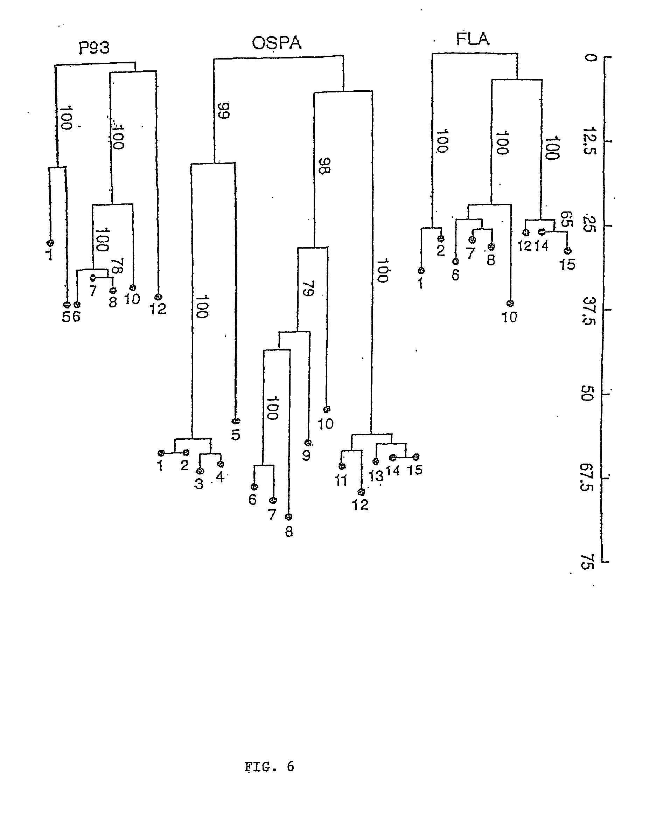

Fig. 6 depicts a phylogenic tree for strains *of Borrelia* described in Table I. The strains are as follows: 1 = B31; 2 = PKa1; 3 = ZS7; 4 = N40; 5 = 25015; 6 = K48; 7 = DK29; 8 = PHei; 9 = Ip90; 10 = PTrob; 11= ACAI; 12 = PGau; 13 = Ip3; 14 = PBo; 15 = PKo.
Figs. 7A and 7B depict the nucleic acid sequence of OspA-B31 (SEQ ID NO. 6), and the encoded protein sequence (SEQ ID NO. 7).

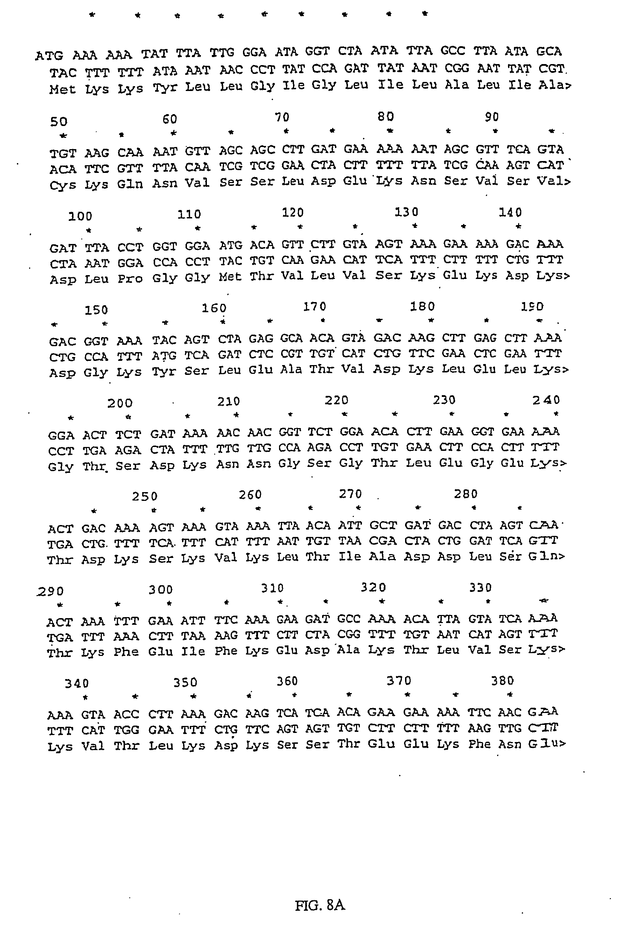

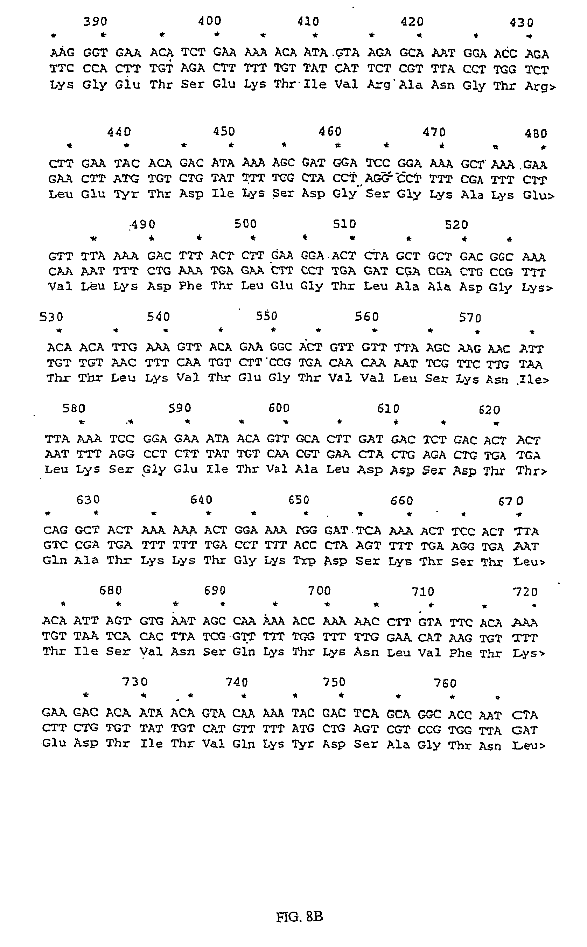

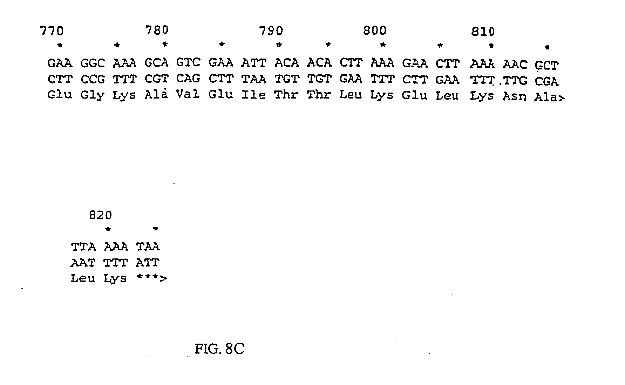

Figs. 8A, 8B and 8C depict the nucleic acid sequence of OspA-K48 (SEQ ID NO. 8), and the encoded protein sequence (SEQ ID NO. 9).

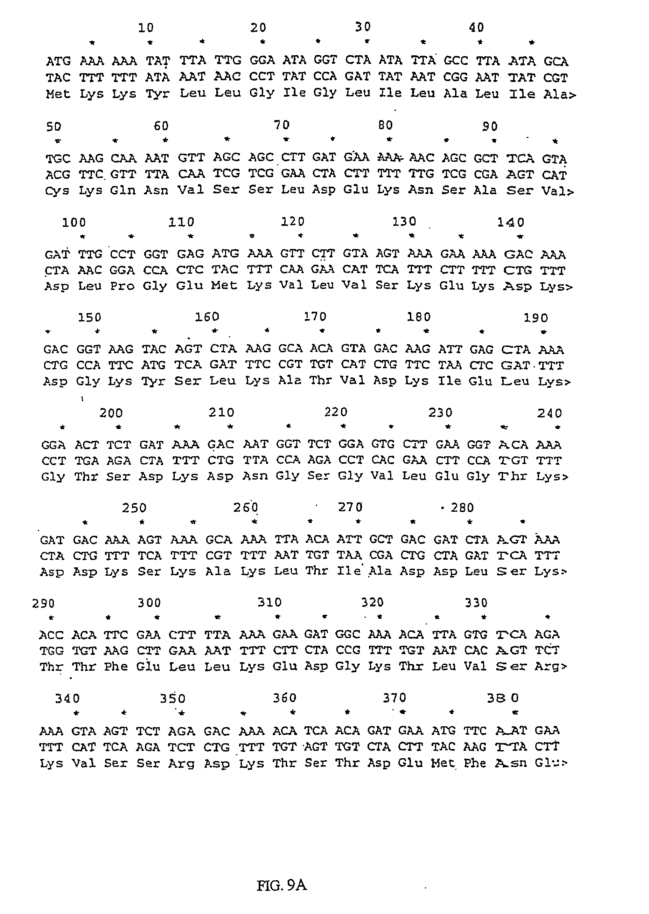

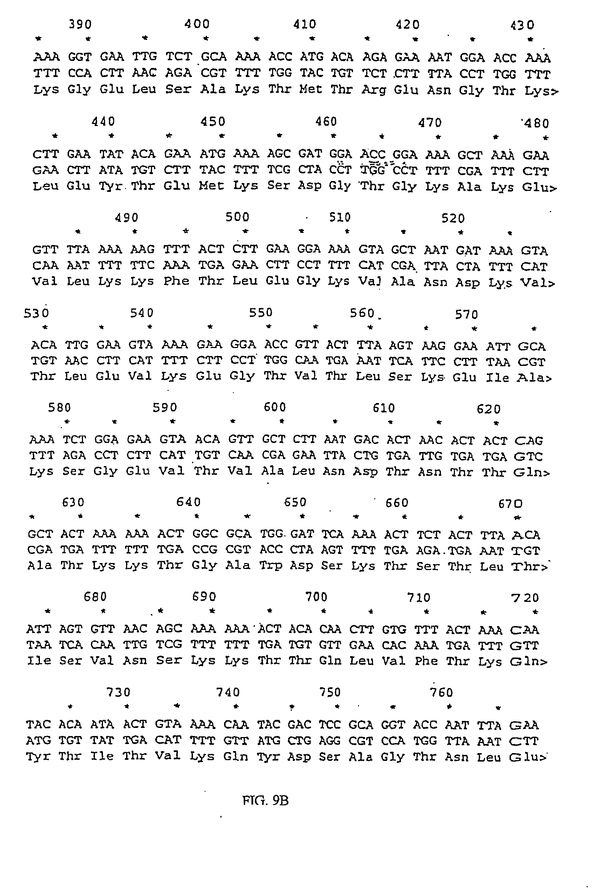

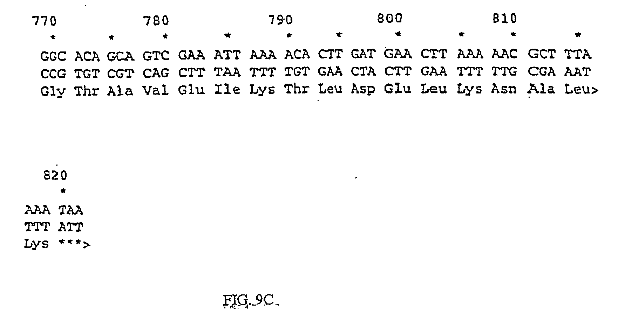

Figs. 9A, 9B and 9C depict the nucleic acid sequence of OspA-PGau (SEQ ID NO. 10), and the encoded protein sequence (SEQ ID NO. 11).

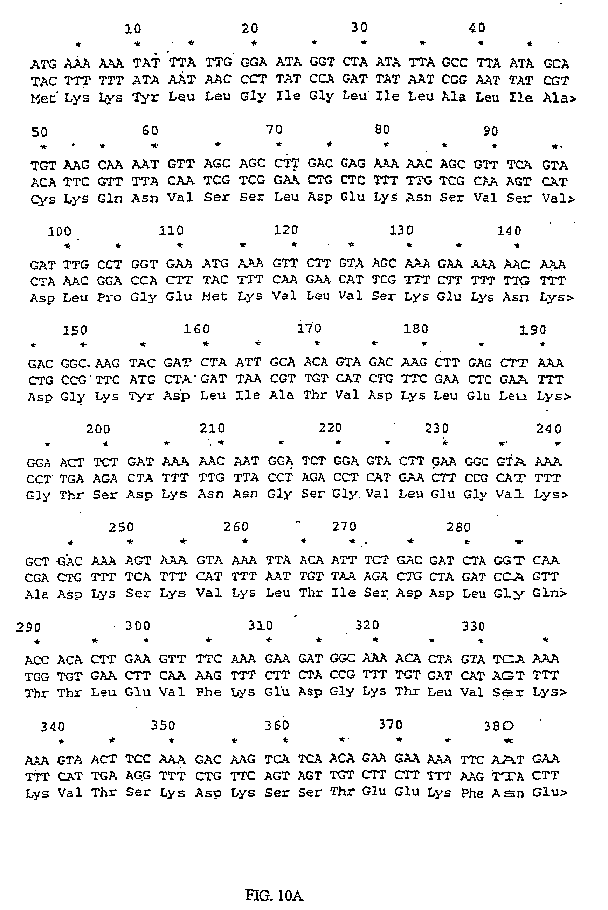

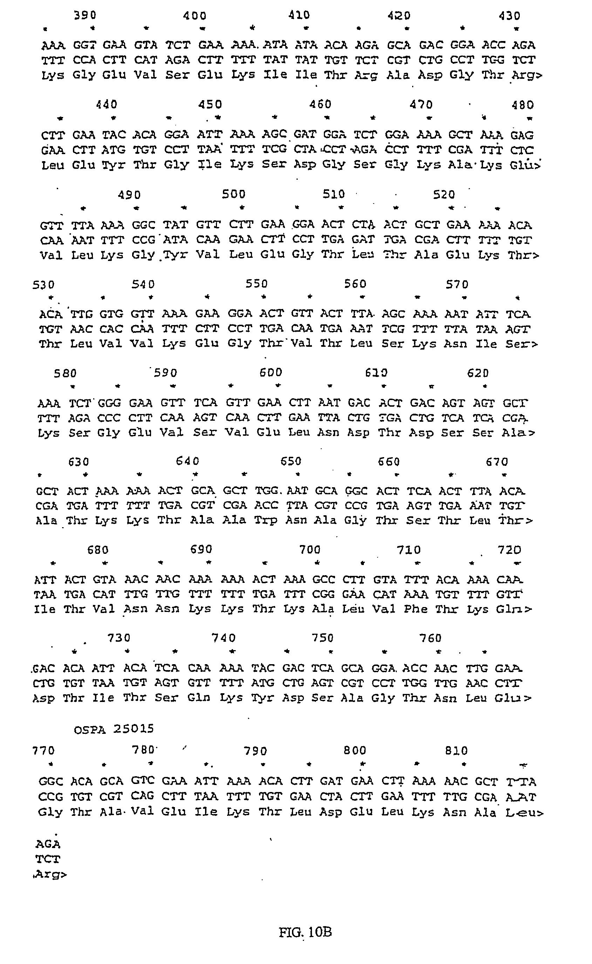

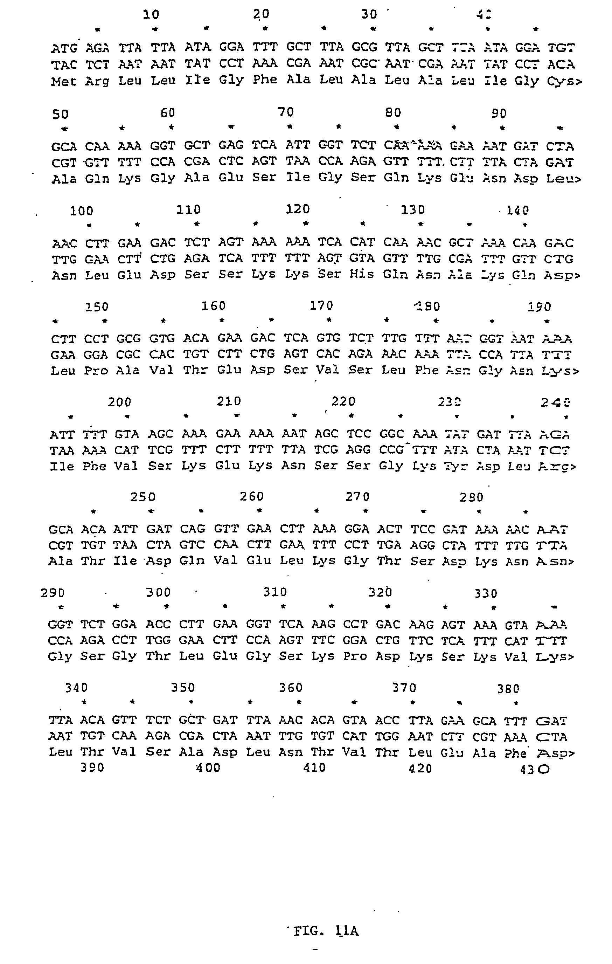

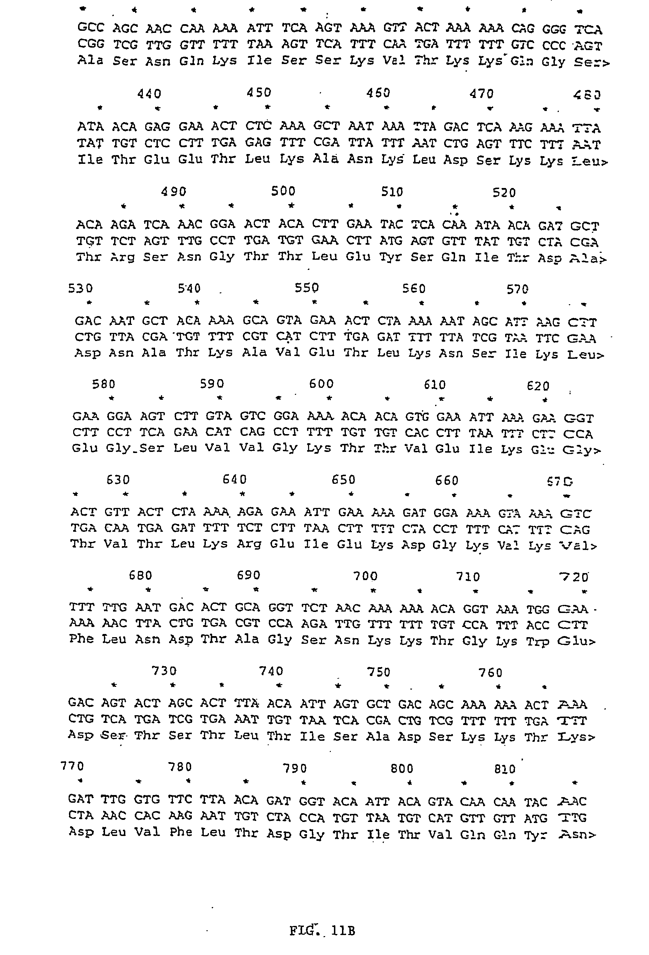

Figs. 10A and 10B depict the nucleic acid sequence of a portion of an OspA gene (SEQ ID NO. 185) and its encoded protein sequence (SEQ ID NO. 186).

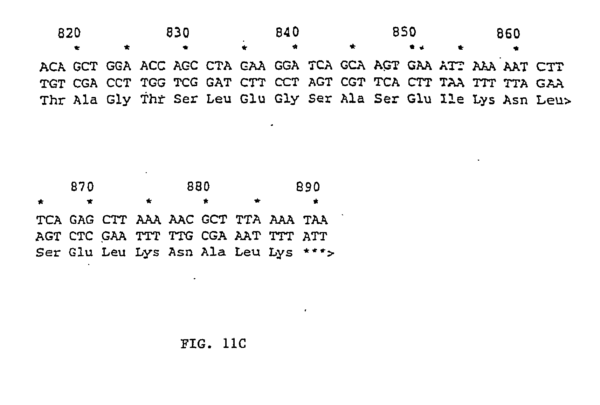

Figs. 11A, 11B and 11C depict the nucleic acid sequence of OspB-B31 (SEQ ID NO. 21), and the encoded protein sequence (SEQ ID NO. 22).
Figs. 12A and 12B depict the nucleic acid sequence of OspC-B31 (SEQ ID NO. 29), and the encoded protein sequence (SEQ ID NO. 30).

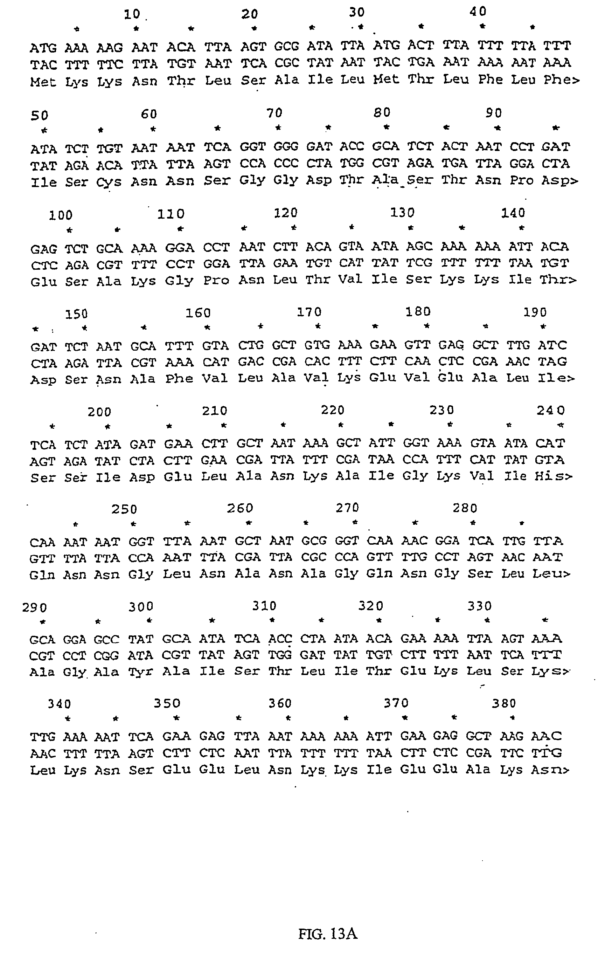

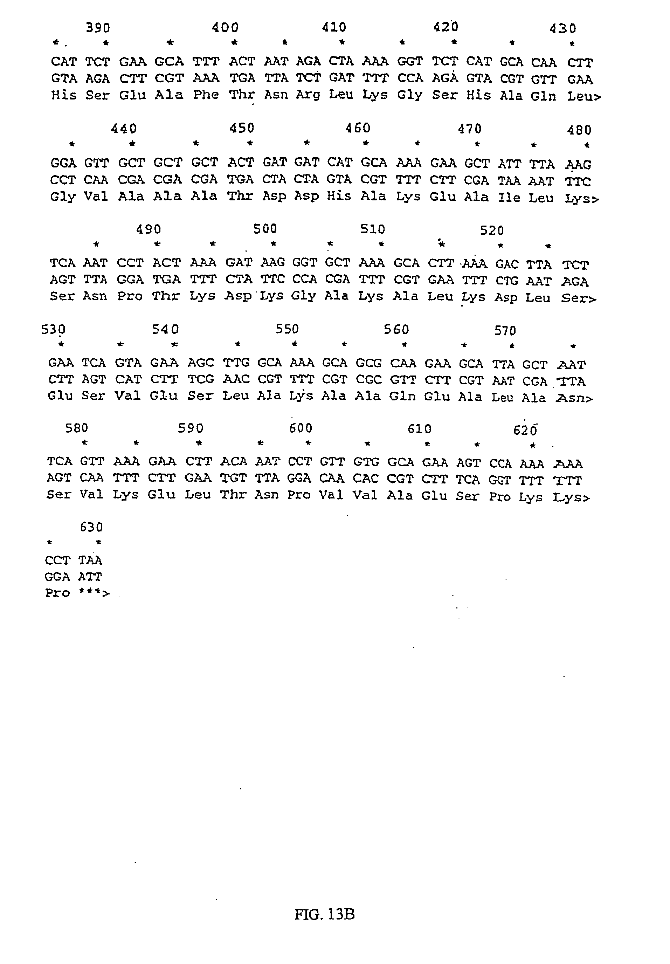

Figs. 13A and 13B depict the nucleic acid sequence of OspC-K48 (SEQ ID NO. 31), and the encoded protein sequence (SEQ ID NO. 32).

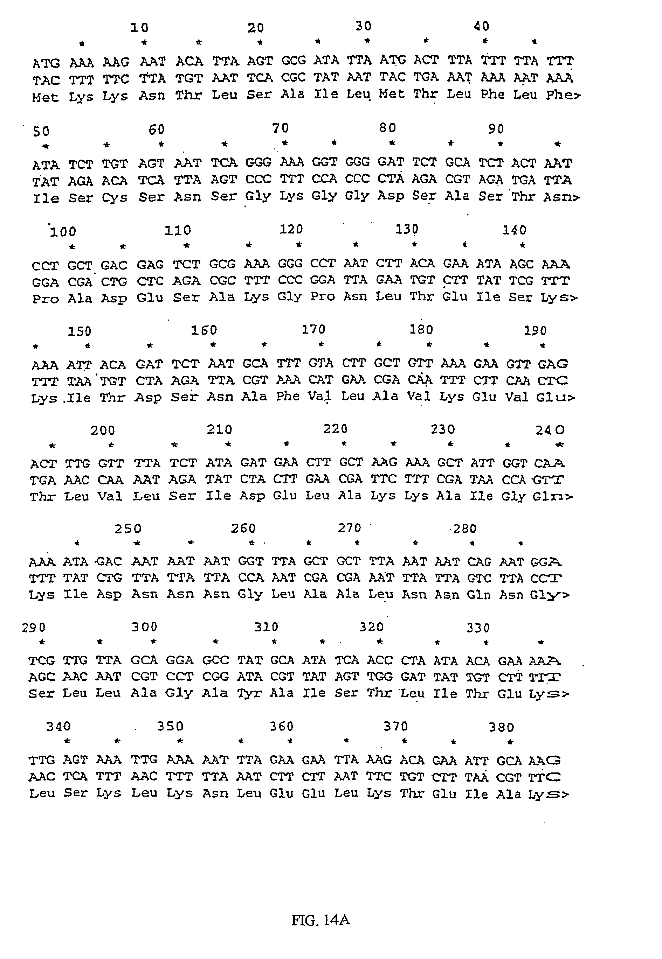

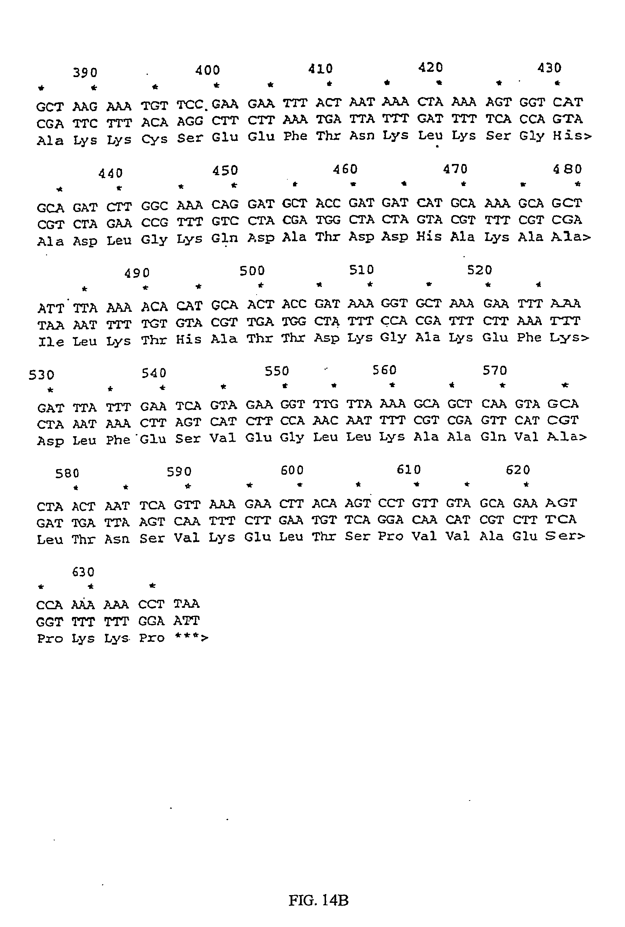

Figs. 14A and 14B depict the nucleic acid sequence of OspC-PKo (SEQ ID NO. 33), and the encoded protein sequence (SEQ ID NO. 34).

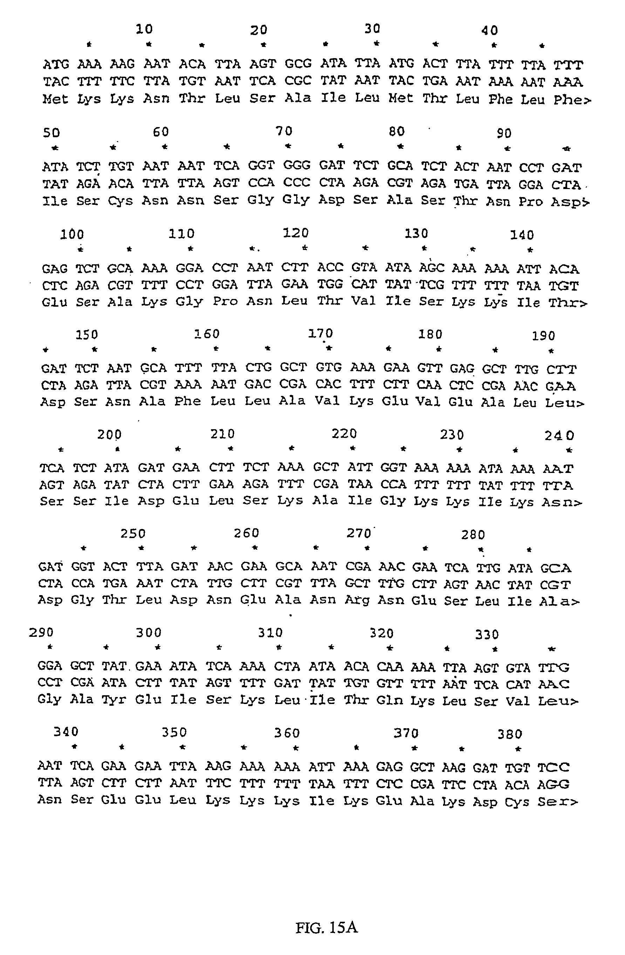

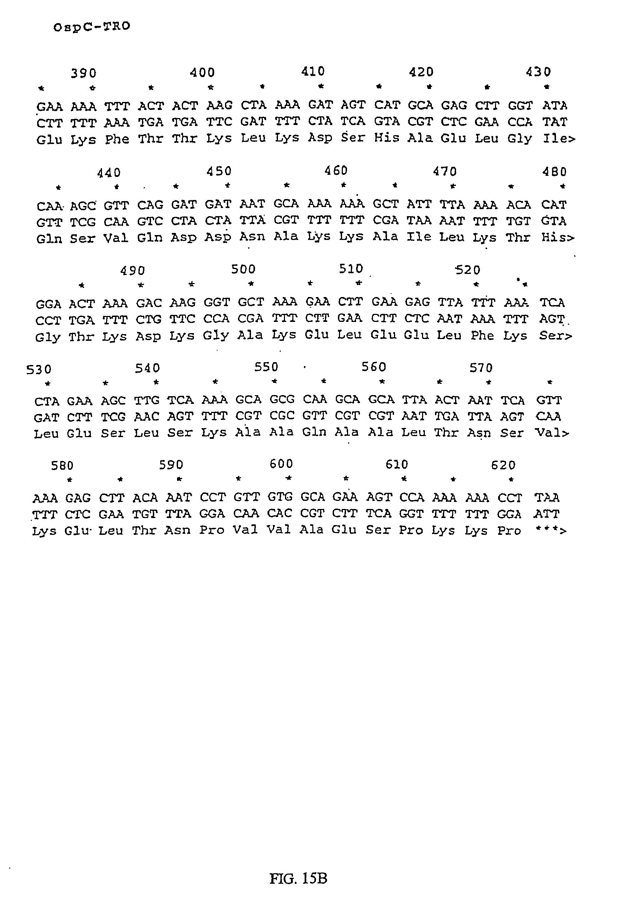

Figs. 15A and 15B depict the nucleic acid sequence of OspC-PTrob (SEQ ID NO. 35) and the encoded protein sequence (SEQ ID NO. 36).

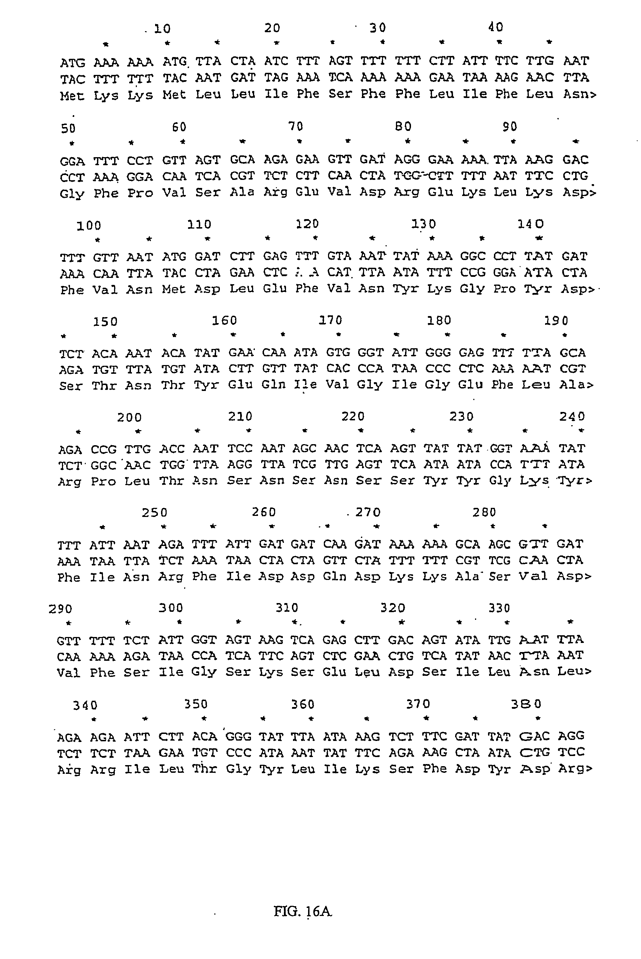

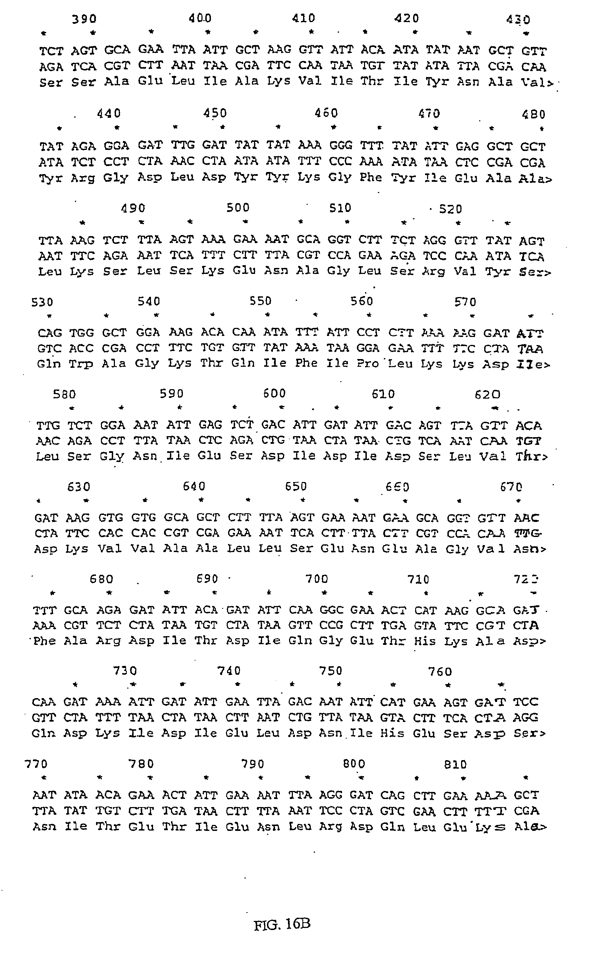

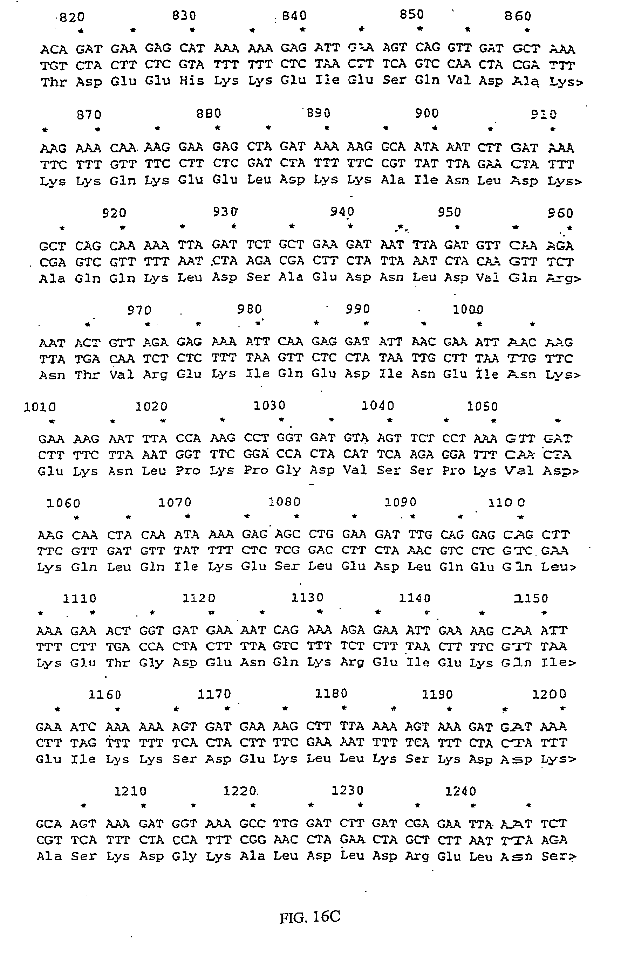

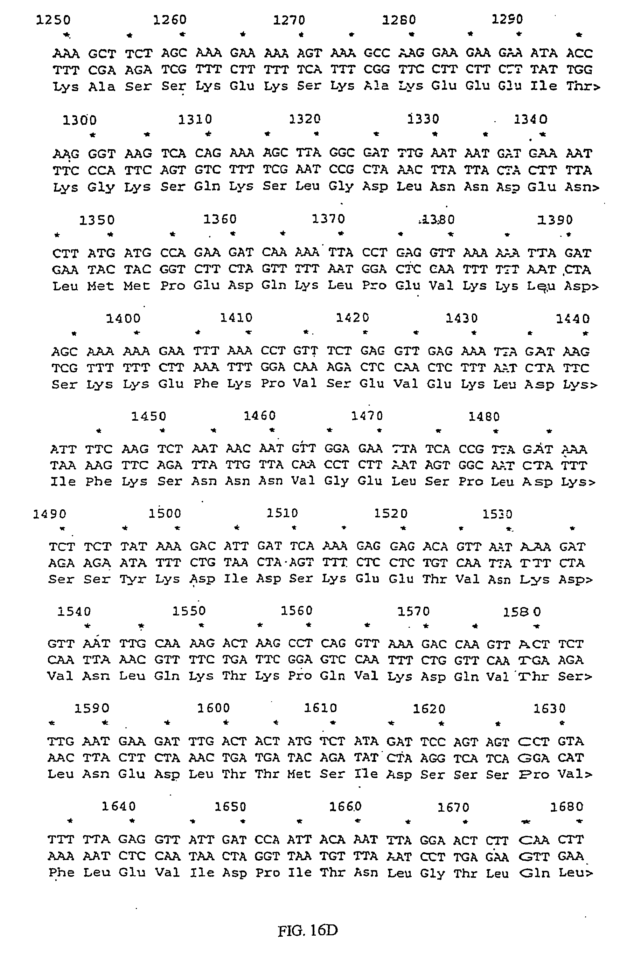

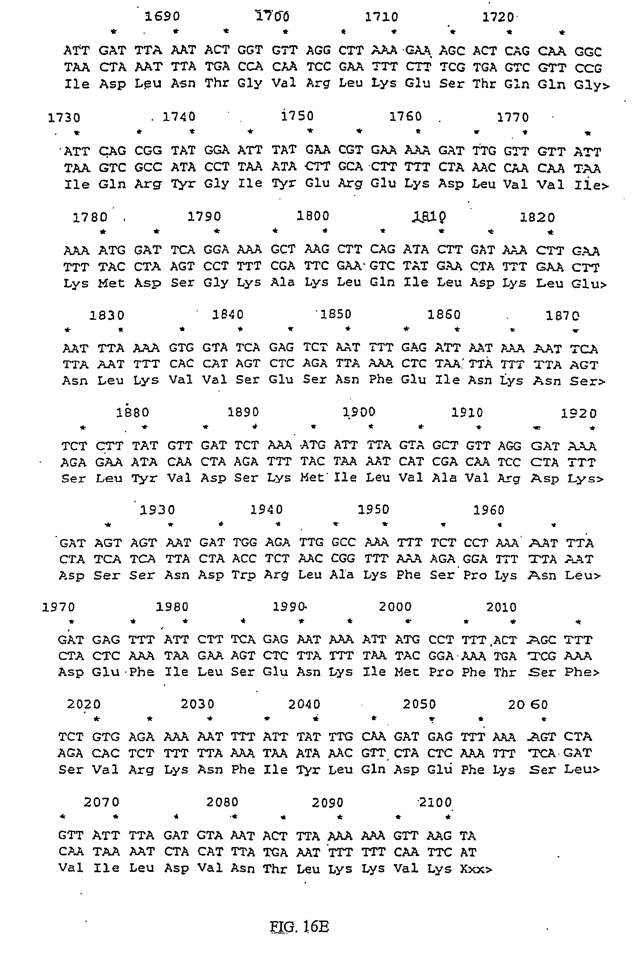

Figs. 16A, 16B, 16C, 16D and 16E depict the nucleic acid sequence of p93-B31 (SEQ ID NO. 65) and the encoded protein sequence (SEQ ID NO. 66).

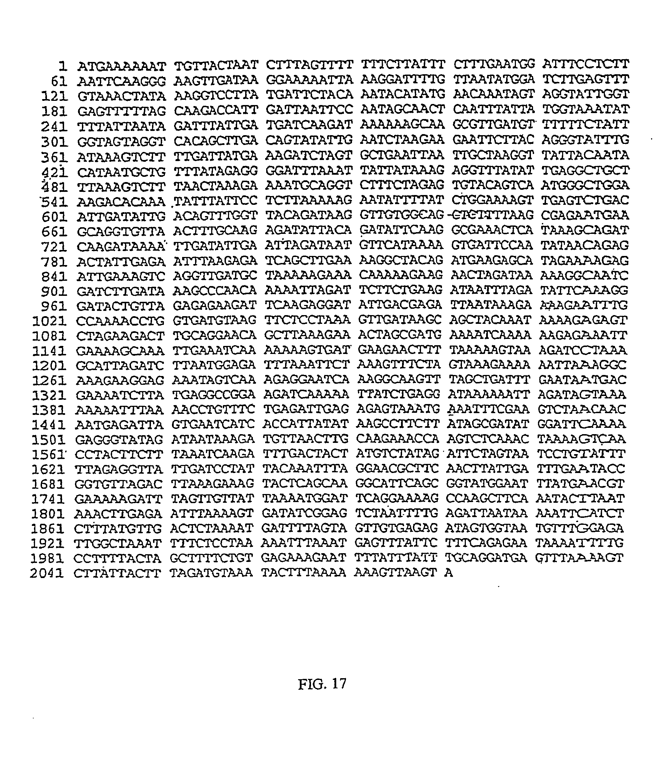

Fig. 17 depicts the nucleic acid sequence of p93-K48 (SEQ ID NO. 67).

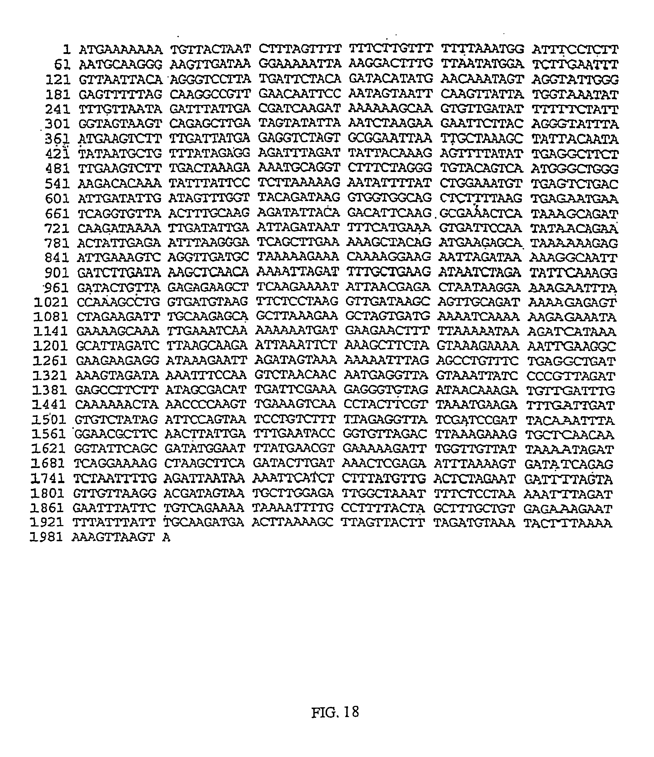

Fig. 18 depicts the nucleic acid sequence of p93-PBo (SEQ ID NO. 69).

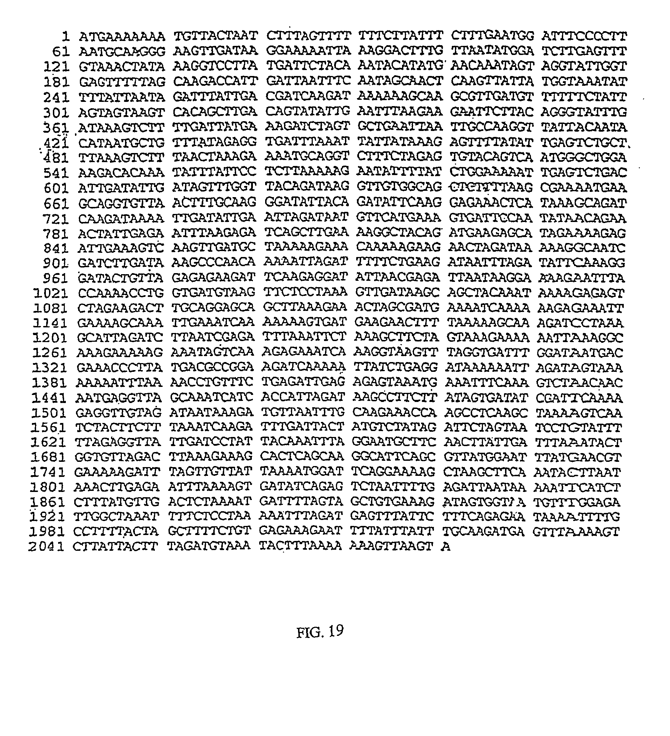

Fig. 19 depicts the nucleic acid sequence of p93-PTrob (SEQ ID NO. 71).

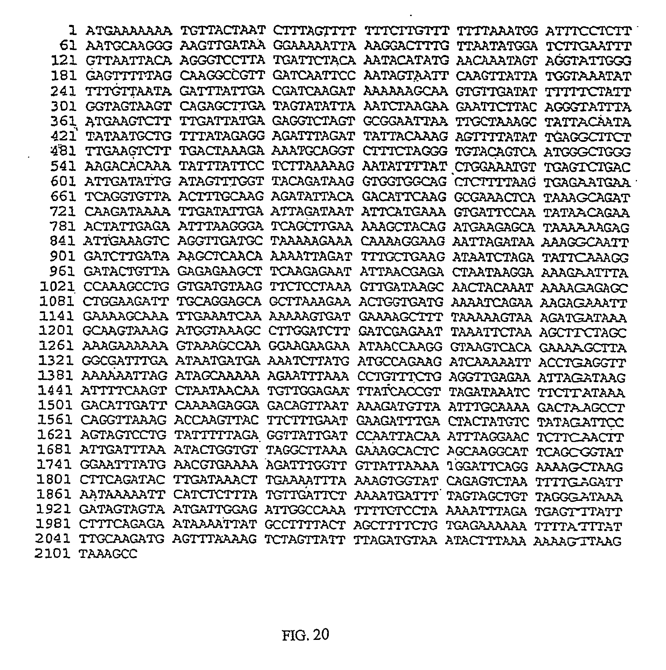

Fig. 20 depicts the nucleic acid sequence of p93-PGAU (SEQ ID NO. 73).

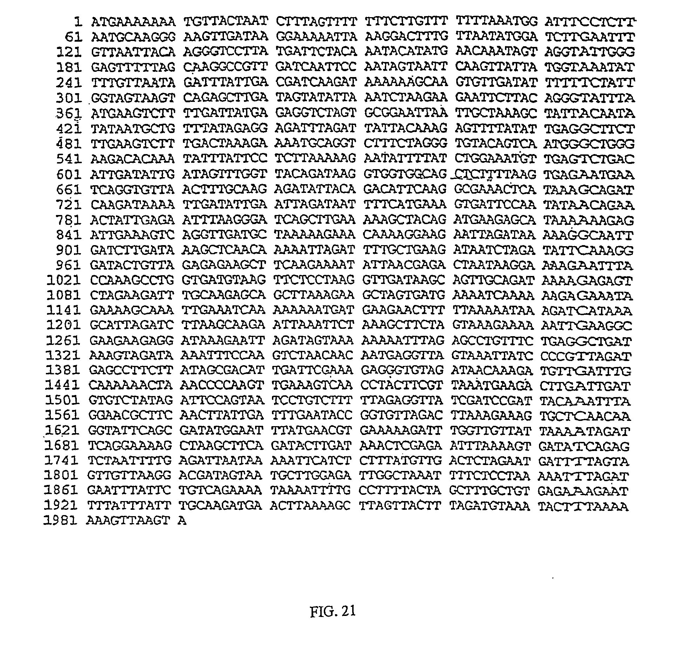

Fig. 21 depicts the nucleic acid sequence of p93-25015 (SEQ ID NO. 77).

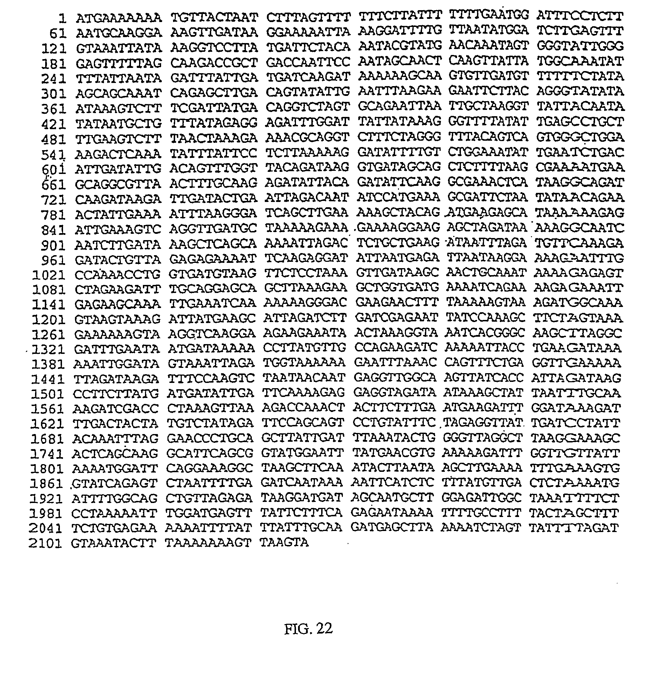

Fig. 22 depicts the nucleic acid sequence of p93-PKo (SEQ ID NO. 75).
Figs. 23A, 23B and 23C depict the nucleic acid sequence of the OspA-K48/OspA-PGAU chimer (SEQ ID NO. 85) and the encoded chimeric protein sequence (SEQ ID NO. 86).
Figs. 24A, 24B and 24C depict the nucleic acid sequence of the OspA-B31/OspA-PGAU chimer (SEQ ID NO. 88) and the encoded chimeric protein sequence (SEQ ID NO. 89).
Figs. 25A and 25B depict the nucleic acid sequence of the OspA-B31/OspA-K48 chimer (SEQ ID NO. 91) and the encoded chimeric protein sequence (SEQ ID NO. 92).
Figs. 26A, 26B and 26C depict the nucleic acid sequence of the OspA-B31/OspA-2501.5 chimer (SEQ ID NO. 94) and the encoded chimeric protein sequence (SEQ ID NO. 95).
Figs. 27A, 27B and 27C depict the nucleic acid sequence of the OspA-K48/OspA-B31/OspA-K48 chimer (SEQ ID NO. 97) and the encoded chimeric protein sequence (SEQ ID NO. 98).
Figs. 28A, 28B and 28C depict the nucleic acid sequence of the OspA-B31/OspA-K48/OspA-B31/OspA-K48 chimer (SEQ ID NO. 100) and the encoded chimeric protein sequence (SEQ ID NO. 101).
Figs. 29A, 29B and 29C depict the nucleic acid sequence of the OspA B31/OspB-B31 chimer (SEQ ID NO. 103) and the encoded chimeric protein sequence (SEQ ID NO. 104).
Figs. 30A, 30B, 30C and 30D depict the nucleic acid sequence of the OspA-B31/OspB-B31/OspC-B31 chimer (SEQ ID NO. 106) and the encoded chimeric protein sequence (SEQ ID NO. 107).
Figs. 31A, 31B, 31C and 31D depict the nucleic acid sequence of the OspC-B31/OspA-B31/OspB-B31 chimer (SEQ ID NO. 109) and the encoded chimeric protein sequence (SEQ ID NO. 110).
Figs. 32A, 32B, 32C, 32D and 32E depict the nucleic acid sequence of the OspA-B31/p93-B31 chimer (SEQ ID NO. 111) and the encoded chimeric protein sequence (SEQ ID NO. 112).
Figs. 33A, 33B, 33C and 33D depict the nucleic acid sequence of the OspB-B31/p41-B31 (122-234) chimer (SEQ ID NO. 113) and the encoded chimeric protein sequence (SEQ ID NO. 114).
Figs. 34A, 34B, 34C and 34D depict the nucleic acid sequence of the OspB-B31/p41-B31 (122-295) chimer (SEQ ID NO. 115) and the encoded chimeric protein sequence (SEQ ID NO. 116).
Figs. 35A, 35B and 35C depict the nucleic acid sequence of the OspB-B31/p41-B31 (140-234) chimer (SEQ ID NO. 117) and the encoded chimeric protein sequence (SEQ ID NO. 118).
Figs. 36A, 36B, 36C and 36D depict the nucleic acid sequence of the OspB-B31/p41-B31 (140-295) chimer (SEQ ID NO. 119) and the encoded chimeric protein sequence (SEQ ID NO. 120).
Figs. 37A, 37B, 37C, 37D and 37E depict the nucleic acid sequence of the OspB-B31/p41-B31 (122-234)/OspC-B31 chimer (SEQ ID NO. 121) and the encoded chimeric protein sequence (SEQ ID NO. 122).
Figs. 38A, 38B, 38C and 38D depict an alignment of the nucleic acid sequences for OspC-B31 (SEQ ID NO. 29), OspC-PKo (SEQ ID NO. 33), OspC-PTrob (SEQ ID NO. 35), and OspC-K48 (SEQ ID NO. 31). Nucleic acids which are identical to those in the lead nucleic acid sequence (here, OspC-B31) are represented by a period (.); differing nucleic acids are shown in lower case letters.
Figs. 39A, 39B, 39C and39D depict an alignment of the nucleic acid sequences for OspD-PBo (SEQ ID NO. 123), OspD-PGAU (SEQ ID NO. 124), OspD-DK29 (SEQ ID NO. 125), and OspD-K48 (SEQ ID NO. 126). Nucleic acids which are identical to those in the lead nucleic acid sequence (here, OspD-PBo) are represented by a period (.); differing nucleic acids are shown in lower case letters.

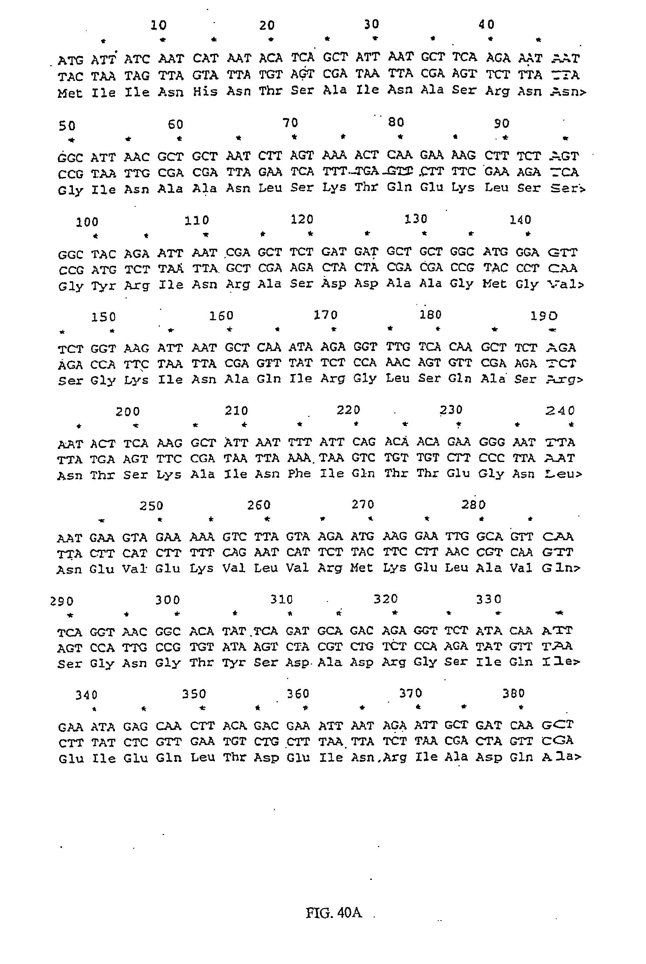

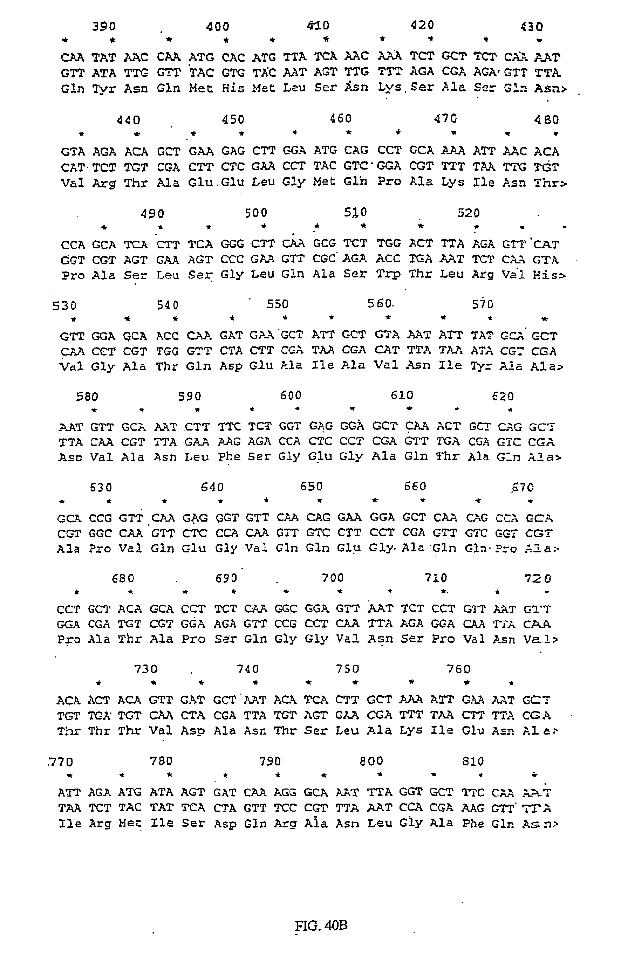

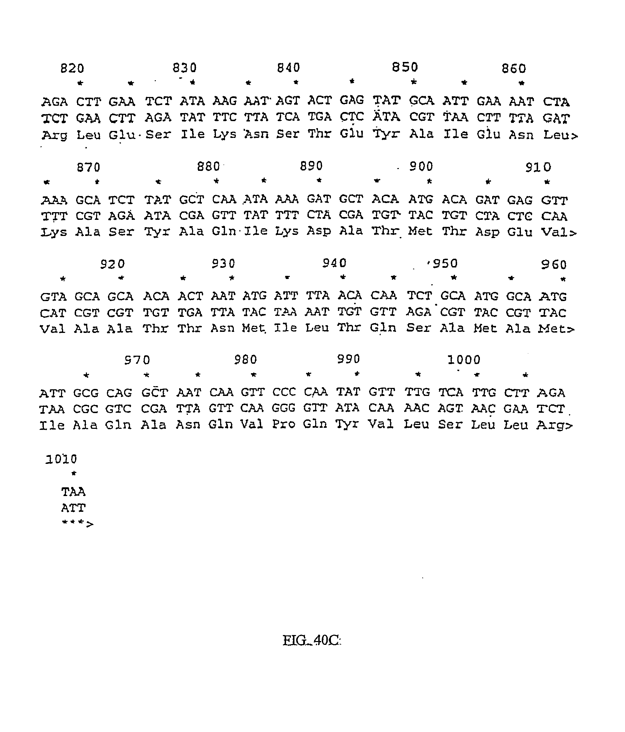

Figs. 40A, 40B and 40C depict the nucleic acid sequence of p41-B31 (SEQ ID NO. 127) and then encoded protein sequence (SEQ ID NO. 128).
Figs. 41A, 41B, 41C, 41D, 41E, 41F, 41G and 41H depict an alignment of the nucleic acid sequences for p41-B31 (SEQ ID NO. 127), p41-PKal (SEQ ID NO. 129), p41-PGAU (SEQ ID NO. 51), p41-PBo (SEQ ID NO. 130), p41-DK29 (SEQ ID NO. 53), and p41-PKo (SEQ ID NO. 131). Nucleic acids which are identical to those in the lead nucleic acid sequence (here, p41-B31) are represented by a period (.); differing nucleic acids are shown in lower case letters.

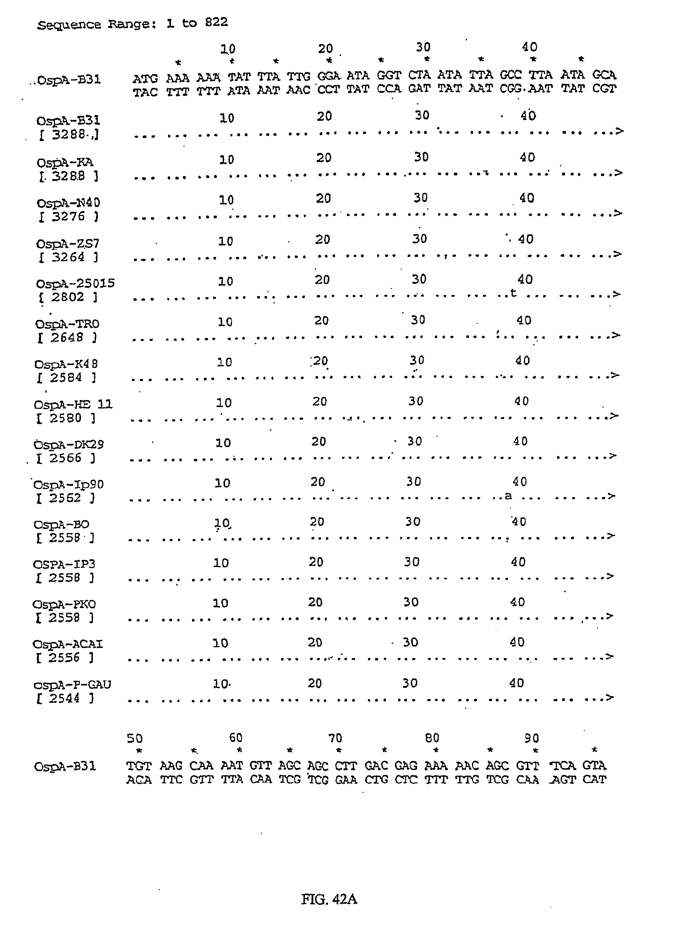

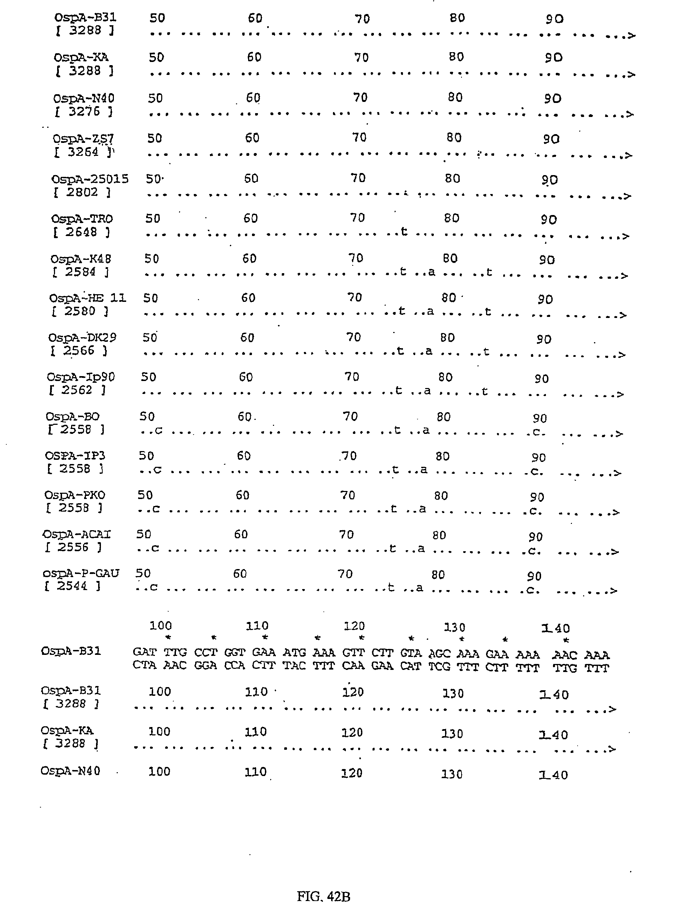

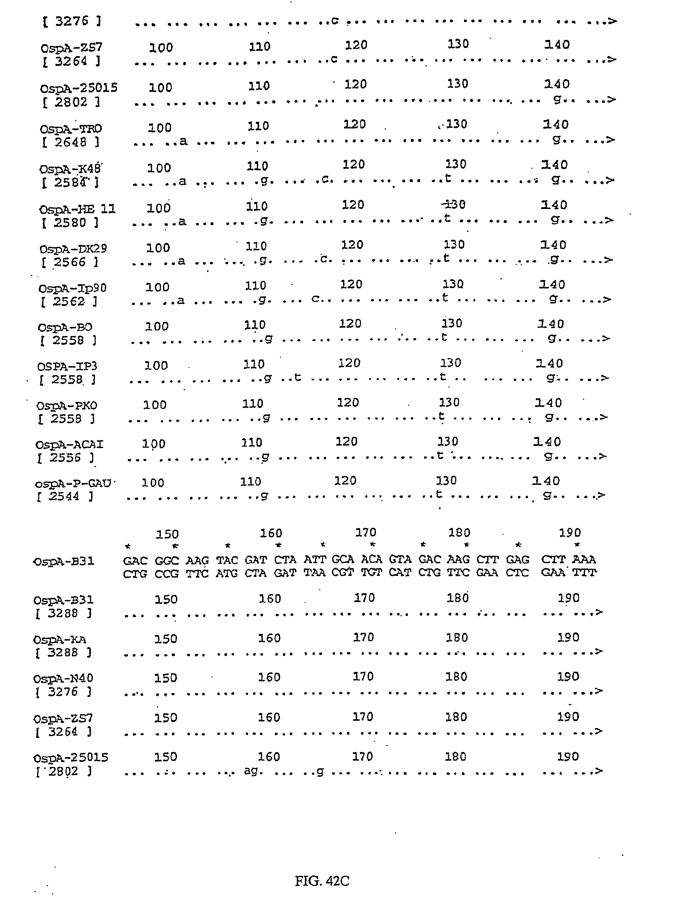

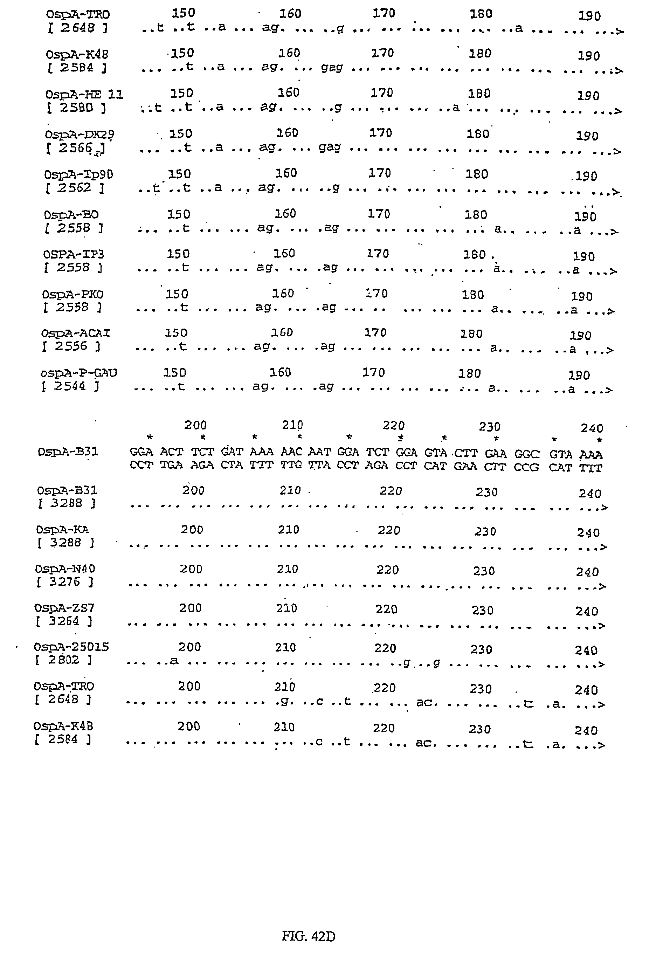

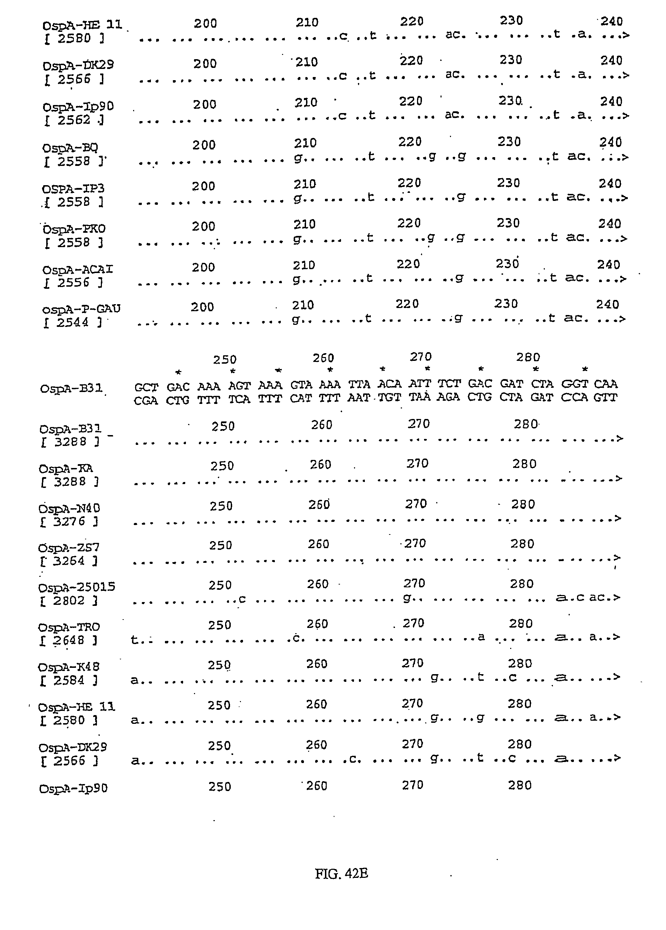

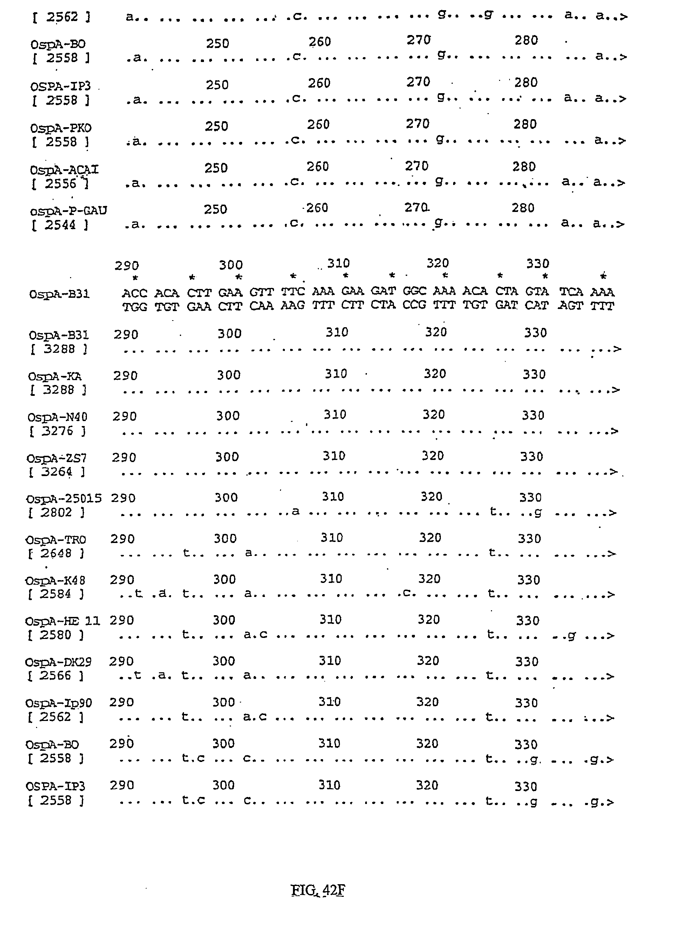

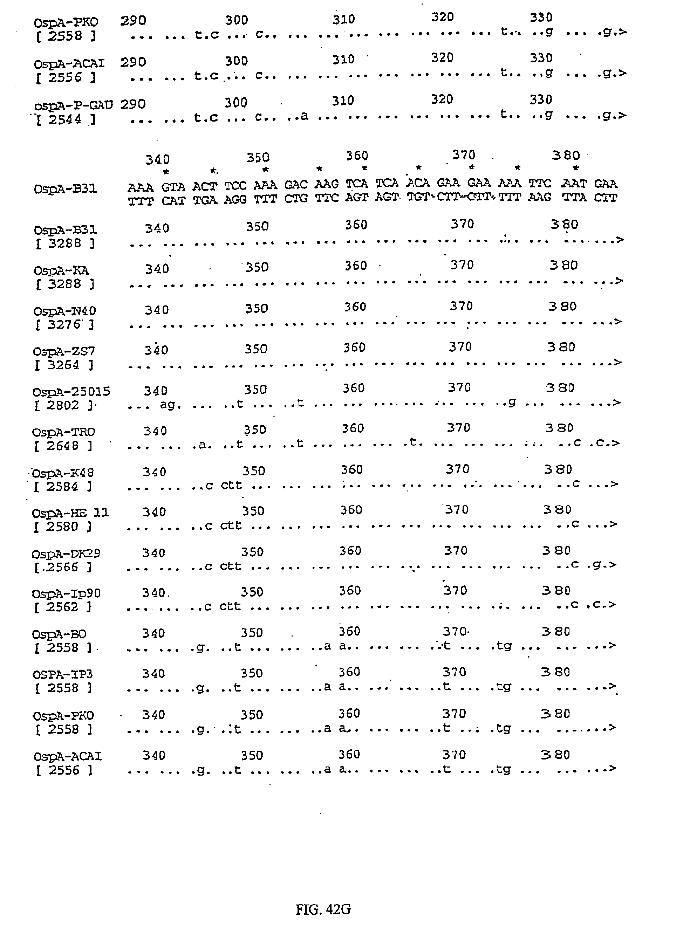

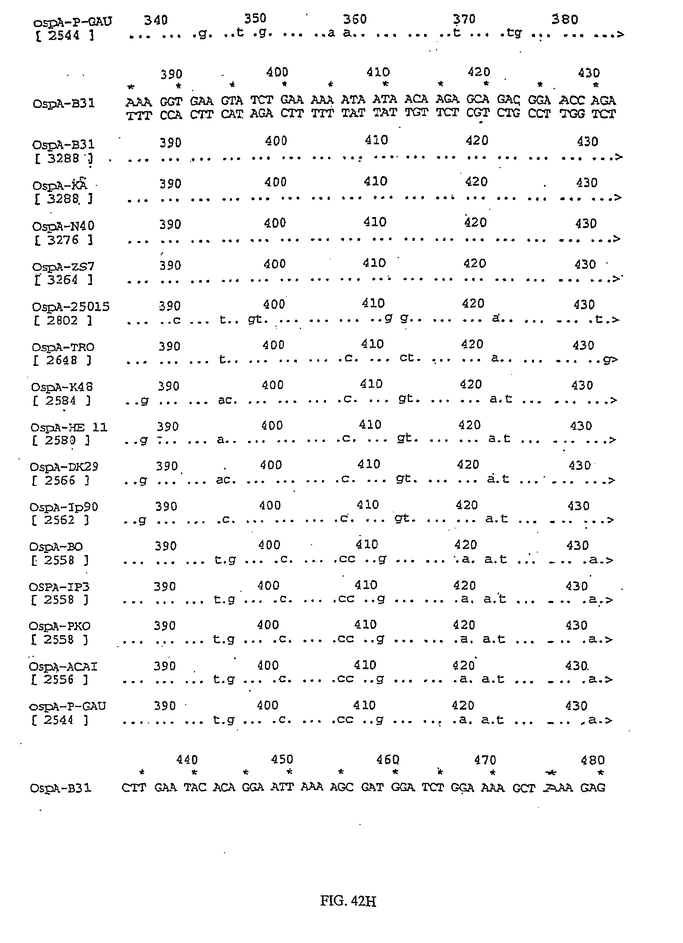

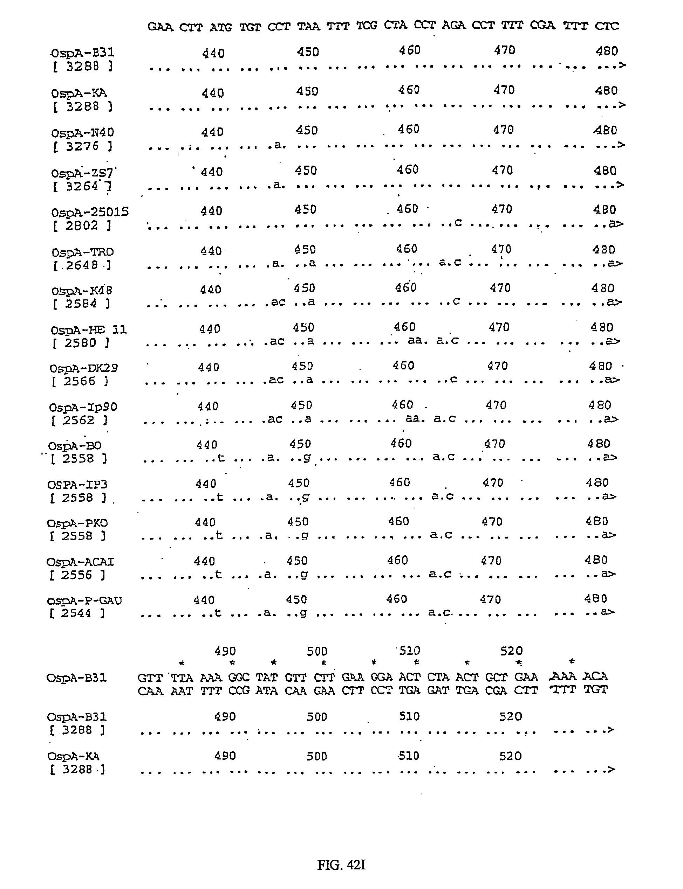

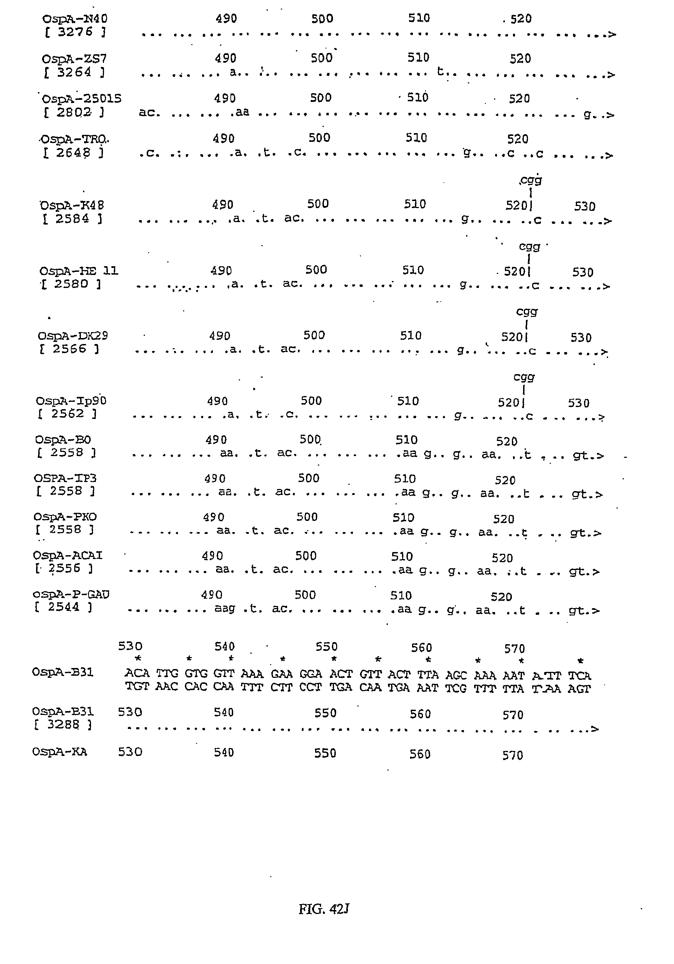

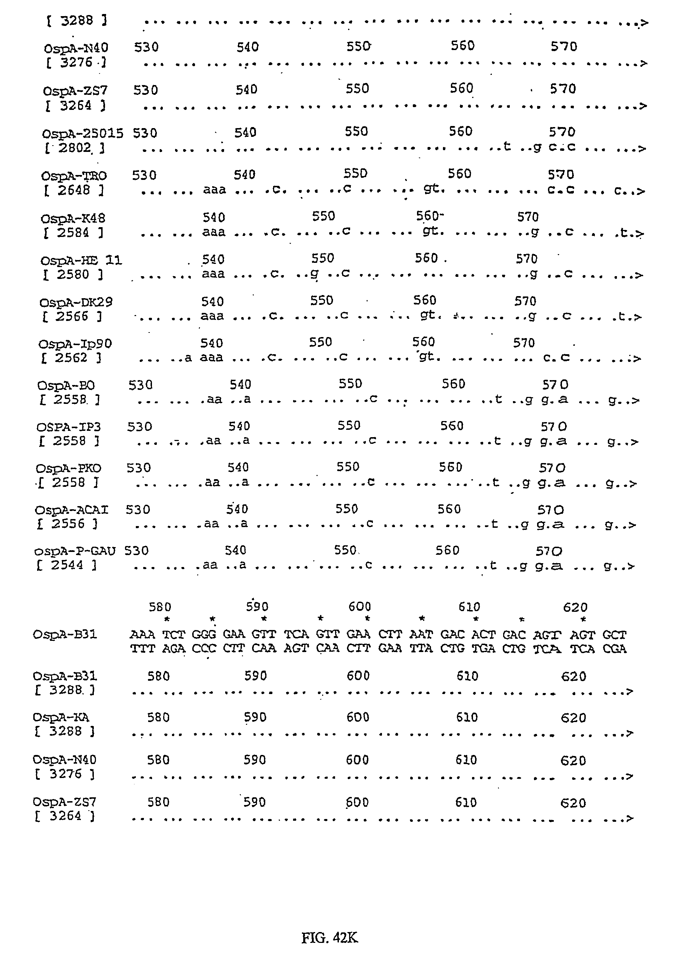

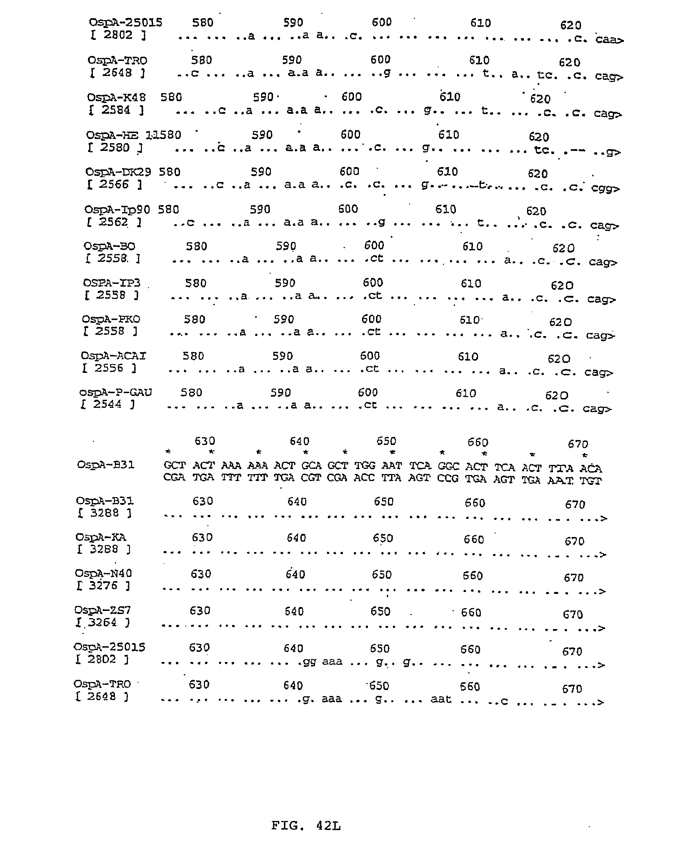

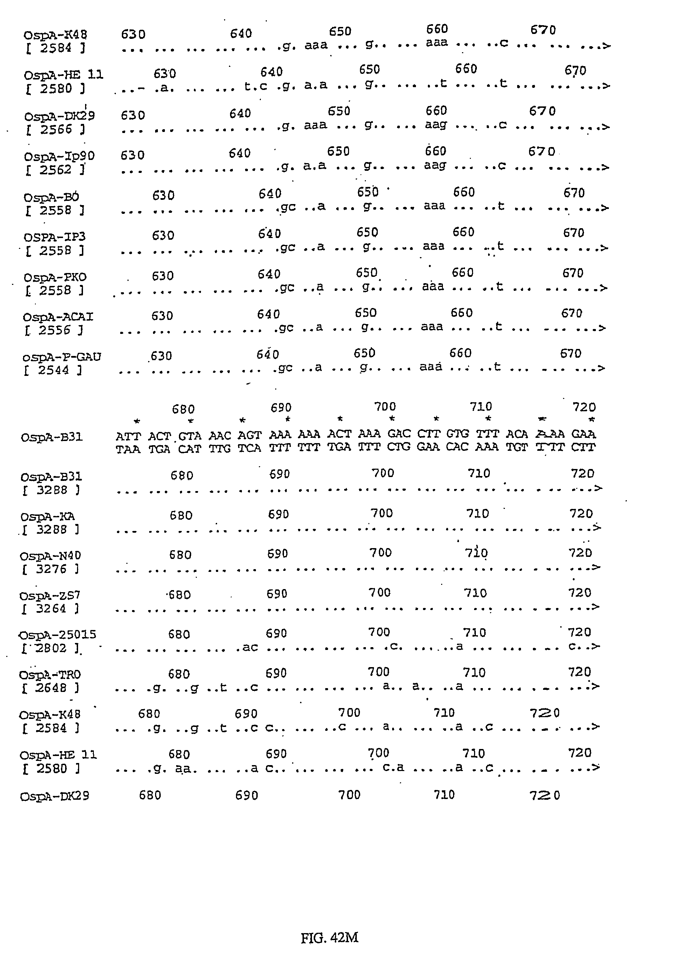

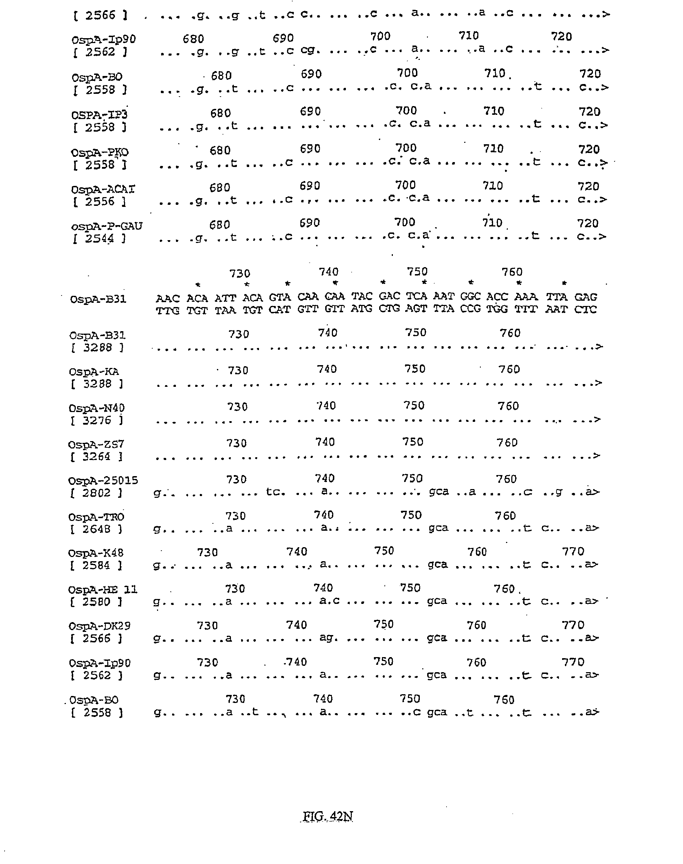

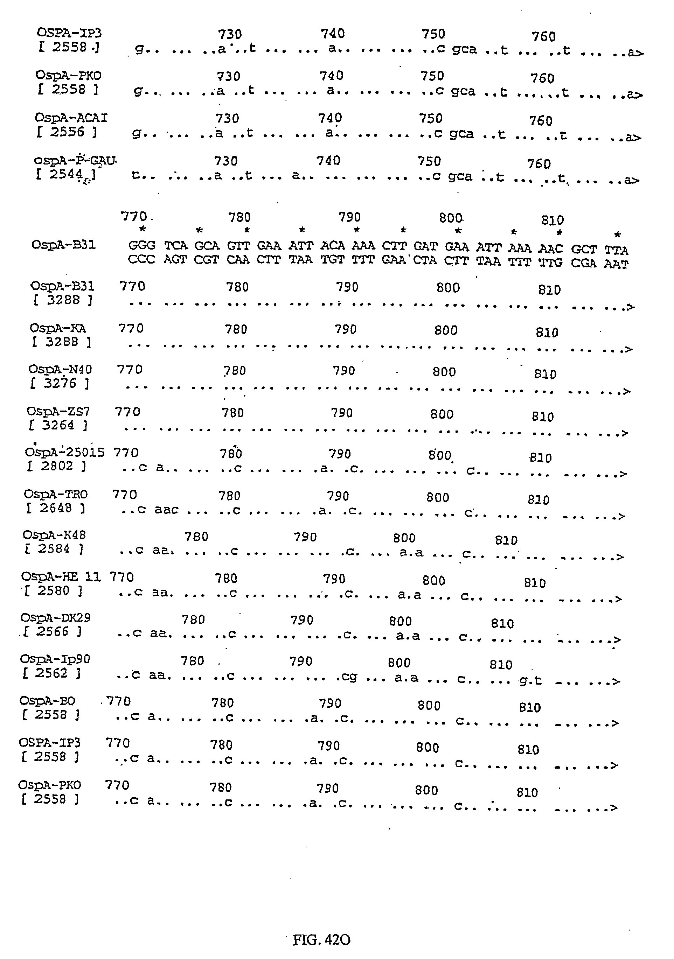

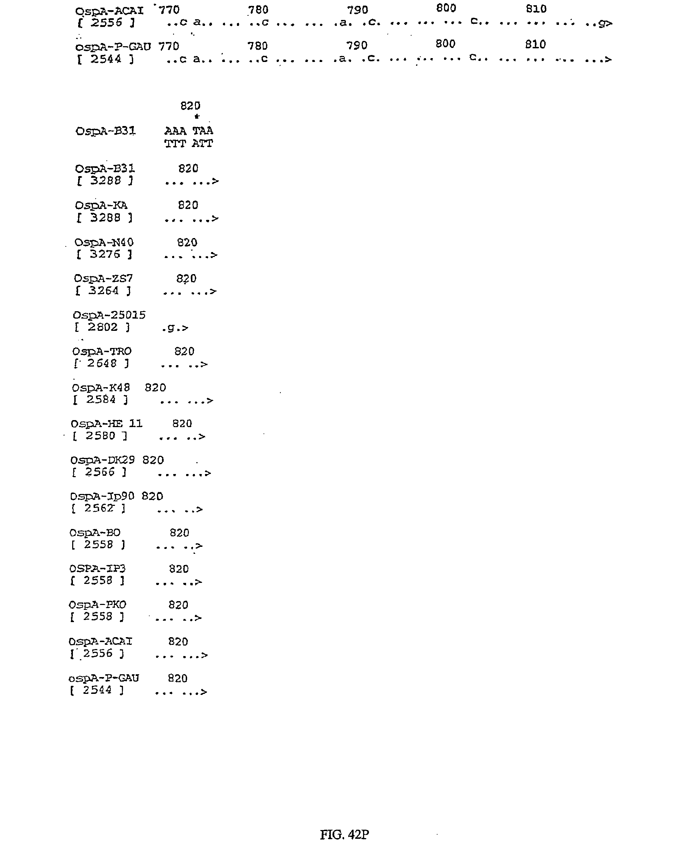

Figs. 42A, 42B, 42C, 42D, 42E, 42F, 42G, 42H, 42I, 42J, 42K, 42L, 42M, 42N, 420 and 42P depict an alignment of the nucleic acid sequences for OspA-B31 (SEQ ID NO. 6), OspA-PKal (SEQ ID NO. 132), OspA-N40 (SEQ ID NO. 133), OspA-ZS7 (SEQ ID NO. 134), OspA-25015 (SEQ ID NO. 12), OspA-PTrob (SEQ ID NO. 135), OspA-K48 (SEQ ID NO. 8), OspA-Hei (SEQ ID NO. 136), OspA-DK29 (SEQ ID NO. 49), OspA-Ip90 (SEQ ID NO. 50), OspA-PBo (Seq ID NO. 55), OspA-Ip3 (SEQ ID NO. 56), OspA-PKo (SEQ ID NO. 57), OspA-ACAI (SEQ ID NO. 58), and OspA-PGAU (SEQ ID NO. 10). Nucleic acids which are identical to those in the lead nucleic acid sequence (here, OspA-B31) are represented by a period (.); differing nucleic acids are shown in lower case letters.
Figs. 43A and 43B depict the nucleic acid sequence of the OspA-Tro/OspA-Bo chimer (SEQ ID NO. 137) which encodes the chimeric protein sequence SEQ ID NO. 138.
Figs. 44A and 44B depict the nucleic acid sequence of the OspA-PGAU/OspA-Bo chimer (SEQ ID NO. 139) which encodes the chimeric protein sequence SEQ ID NO. 140.
Figs. 45A and 45B depict the nucleic acid sequence of the OspA-B31/OspA-PGAU/OspA-B31/OspA-K48 chimer (SEQ ID NO. 143) which encodes the chimeric protein sequence SEQ ID NO. 144.
Figs. 46A and 46B depict the nucleic acid sequence of the OspA-PGAU/OspA-B31/OspA-K48 chimer (SEQ ID NO. 141) which encodes the chimeric protein sequence SEQ ID NO. 142.
Fig. 47 is a bar graph showing the reactivity (as measured by ELISA) of sera from mice immunized with the indicated *Borrelia* protein (OspA or OspC) or recombinant chimeric protein (OspC2-OspA) (X-axis) against OspA B31 or OspC B31 antigens (legend).
Fig. 48 is a bar graph showing the reactivity (as measured by ELISA) of sera from mice immunized with the indicated *Borrelia* protein (OspA or OspC) or recombinant chimeric protein (OspC2-OspA) (X-axis) against OspA B31 or OspC B31 antigens (legend). For the ELISA results to the B31 OspA antigen, a purified fragment of B31 OspA (amino acids 18-139) was added in excess to the sera so that the detected immune response was specific for the C-terminal region of OspA.
Fig. 49 is a bar graph showing the reactivity of sera from mice immunized with the indicated *Borrelia* chimeric protein (lipOspA/Bo, lipOspAB/P or OspC-OspAB/P) (X-axis) against the indicated OspA antigens (legend) from strains B31 *(Borrelia burgdorferi* sensu stricto), K48 (*Borrelia garinii*) and PGau *(Borrelia afzelli).*
Fig. 50 is a bar graph showing the reactivity of sera from mice immunized with the indicated *Borrelia* chimeric protein (lipOspAP/Bo, lipOspAB/P) or OspC-OspAB/P) (X-axis) against the indicated OspA (legend) from strains B31 *(Borrelia burgdorferi* sensu stricto), K48 (*Borrelia garinii)* and PGau *(Borrelia afzelli).* In all cases, a purified fragment of B31 OspA (amino acids 18-139) was added in excess to the sera so that the detected immune response is specific for the C-terminal region of OspA.
Fig. 51 is a bar graph showing the reactivity of sera from mice immunized with the indicated *Borrelia* chimeric protein (OspCB31-OspAB31, OspC2-OspAB31 or lip OspC-B31) (X-axis) against the indicated OspC antigen (legend) from the strain B31 *(Borrelia burgdorferi* sensu stricto).
Fig. 52 is a bar graph showing the reactivity of sera from mice immunized with the indicated *Borrelia* chimeric protein (OspCB31-OspAB31, OspC2-OspAB31 or Lip OspA K/T) (X-axis) against the indicated OspA antigens (legend) from strains B31 *(Borrelia burgdorferi* sensu stricto), K48 *(Borrelia garinii)* and PGau *(Borrelia afzelli).*
Fig. 53 is a bar graph showing the reactivity of sera from mice immunized with the indicated *Borrelia* chimeric protein (OspCB31-OspAB/P, OspCB31-OspABPBP or OspCB31-OspAB31) (X-axis) against the indicated OspA antigens (legend) from strains B31 *(Borrelia burgdorferi* sensu stricto), K48 *(Borrelia garinii)* and PGau *(Borrelia afzelli).*
Fig. 54 is a bar graph showing the reactivity of sera from mice immunized with the indicated *Borrelia* chimeric protein (OspCB31-OspAB/P, OspCB31-OspABPBP or OspCB31-OspAB31) (X-axis) against the indicated OspA (legend) from strains B31 *(Borrelia burgdorferi* sensu stricto), K48 *(Borrelia garinii)* and PGau *(Borrelia afzelli).* In all cases, a purified fragment of B31 OspA (amino acids 18-139) was added in excess to the sera so that the detected immune response is specific for the C-terminal region of OspA.
Figs. 55A, 55B and 55C depict the nucleic acid sequence of the OspC-B31 (bp 55-633)/OspA-B31 (bp 52-822) chimer (SEQ ID NO. 145) and the encoded chimeric protein sequence (SEQ ID NO. 146).
Figs. 56A, 56B and 56C depict the nucleic acid sequence of the OspC-B31 (bp 55-624)/OspA-B31 (bp 52-822) chimer (SEQ ID NO. 147) and the encoded chimeric protein sequence (SEQ ID NO. 148).
Figs. 57A, 57B and 57C depict the nucleic acid sequence of the OspC-C2 (bp 55-612)/OspA-B31 (bp 52-822) chimer (SEQ ill NO. 149) and the encoded chimeric protein sequence (SEQ ID NO. 150).
Figs. 58A, 58B and 58C depict the nucleic acid sequence of the OspC-B31 (bp 55-633)/OspA-B31 (bp 52-651)/OspA-K48 (bp 652-820) chimer (SEQ ID NO. 151) and the encoded chimeric protein sequence (SEQ ID NO. 152).
Figs. 59A, 59B and 59C depict the nucleic acid sequence of the OspC-C2 (bp 55-612)/OspA-B31 (bp 52-651)/OspA-K48 (bp 652-820) chimer (SEQ ID NO. 153) and the encoded chimeric protein sequence (SEQ ID NO. 154).
Figs. 60A, 60B and 60C depict the nucleic acid sequence of the OspC-B31 (bp 55-633)/OspA-B31 (bp 52-651)/OspA-PKo (bp 652-820) chimer (SEQ ID NO. 155) and the encoded chimeric protein sequence (SEQ ID NO. 156).
Figs. 61A, 61B and 61C depict the nucleic acid sequence of the OspC-C2 (bp 55-612)/OspA-B31 (bp 52-651)/OspA-PKo (bp 652-820) chimer (SEQ ID NO. 157) and the encoded chimeric protein sequence (SEQ ID NO. 158).
Figs. 62A, 62B and 62C depict the nucleic acid sequence of the OspC-B31 (bp 55-633)/OspA-K48 (bp 52-654)/OspA-Tro (bp 655-819) chimer (SEQ ID NO. 159) and the encoded chimeric protein sequence (SEQ ID NO. 160).
Figs. 63A, 63B and 63C depict the nucleic acid sequence of the OspC-C2 (bp 55-612)/OspA-K48 (bp 52-654)/OspA-Tro (bp 655-819) chimer (SEQ ID NO. 161) and the encoded chimeric protein sequence (SEQ ID NO. 162).
Figs. 64A, 64B and 64C depict the nucleic acid sequence of the OspC-C12 (bp 55-612)/OspA-B31 (bp 88-492)/OspA-PKo (bp 493-537)/OspA-B31 (bp 538-822) chimer (SEQ ID NO. 163) and the encoded chimeric protein sequence (SEQ ID NO. 164).
Figs. 65A, 65B and 65C depict the nucleic acid sequence of the OspC-PKo (bp 55-639)/OspA-B31 (bp 88-492)/OspA-PKo (bp 493-537)/OspA-B31 (bp 538-651)/OspA-K48 (bp 652-825) chimer (SEQ ID NO. 165) and the encoded chimeric protein sequence (SEQ ID NO. 166).
Figs. 66A, 66B and 66C depict the nucleic acid sequence of the OspC-Tro (bp 55-624)/OspA-B31 (bp 88-492)/OspA-PKo (bp 493-537)/OspA-B31 (bp 538-651)/OspA-PKo (bp 652-822) chimer (SEQ ID NO. 167) and the encoded chimeric protein sequence (SEQ ID NO. 168).
Figs. 67A and 67B depict the nucleic acid sequence of the OspC-B31 (bp 55-633)/OspA-B31 (bp 394-820) chimer (SEQ ID NO. 169) and the encoded chimeric protein sequence (SEQ ID NO. 170).
Figs. 68A and 68B depict the nucleic acid sequence of the OspC-B31 (bp 55-631)/OspA-B31 (bp 394-651)/OspA-K48 (bp 652-820) chimer (SEQ ID NO. 171) and the encoded chimeric protein sequence (SEQ ID NO. 172).
Figs. 69A and 69B depict the nucleic acid sequence of the OspC-B31 (bp 55-633)/OspA-B31 (bp 394-651)/OspA-PKo (bp 652-820) chimer (SEQ ID NO. 173) and the encoded chimeric protein sequence (SEQ ID NO. 174).
Figs. 70A and 70B depict the nucleic acid sequence of the OspC-B31 (bp 55-633)/OspA-K48 (bp 394-654)/OspA-Tro (bp 655-819) chimer (SEQ ID NO. 175) and the encoded chimeric protein sequence (SEQ ID NO. 176).
Figs. 71A, 71B and 71C depict the nucleic acid sequence of the OspC-B31 (bp 55-633)/OspA-B31 (bp 88-492)/OspA-PKo (bp 493-537)/OspA-B31 (bp 541-651)/OspA-PKo (bp 652-822) chimer (SEQ ID NO. 177) and the encoded chimeric protein sequence (SEQ ID NO. 178); a variant of this sequence was also generated, where the N at position 190 of B31 OspA was deleted.
Figs. 72A, 72B and 72C depict the nucleic acid sequence of the OspC-C2 (bp 55-612)/OspA-B31 (bp 88-492)/OspA-PKo (bp 493-537)/OspA-B31 (bp 541-651)/OspA-PKo (bp 652-822) chimer (SEQ ID NO. 179) and the encoded chimeric protein sequence (SEQ ID NO. 180); a variant of this sequence was also generated, where the N at position 190 of B31 OspA was deleted.

### DETAILED DESCRIPTION OF THE INVENTION.

The present invention pertains to chimeric proteins comprising various antigenic *Borrelia* polypeptides. In a preferred embodiment, the chimeric protein comprises *Borrelia* outer surface protein C (OspC) and outer surface protein A (OspA). These chimeric proteins have the general structure of OspC linked to OspA via a peptide bond. Each of the OspA and OspC portions of the chimeric OspC/OspA protein can be lipidated or unlipidated. In a preferred embodiment, the OspC/OspA chimer comprises OspC and OspA polypeptide fragments that do not possess their lipidation signals.

The chimeric forms of the OspA and OspC proteins described herein were bioengineered such that the resultant chimeric protein maintained at least some antigenicity of one or both of the parent molecules. As described herein, antigenic refers to the ability of a compound to bind products of an immune response, such as antibodies, T-cell receptors or both. Such responses can be measured using standard antibody detection assays, such as ELISA or standard T-cell activation assays. In a particular embodiment, the chimeric OspC/OspA proteins comprise OspA polypeptides which lack the putative autoreactive domain that has similarity to a region of human leukocyte function-associated antigen-1 (hLFA-1) (Gross, D.M. et al., Science, 281: 703-706 (1998)).

The current invention pertains to chimeric proteins comprising antigenic *Borrelia* polypeptides which do not occur in nature in the same *Borrelia* protein. The chimeric proteins are a combination of two or more antigenic polypeptides derived from *Borrelia* proteins. The antigenic polypeptides can be derived from different proteins from the same species *of Borrelia,* or different proteins from different *Borrelia* species, as well as from corresponding proteins from different species. As used herein, the term "chimeric protein" describes a protein comprising two or more polypeptides which are derived from corresponding and/or non-corresponding native *Borrelia* protein. A polypeptide "derived from" a native *Borrelia* protein is a polypeptide which has an amino acid sequence the same as an amino acid sequence present in a *Borrelia* protein, an amino acid sequence equivalent to the amino acid sequence of a naturally occurring *Borrelia* protein, or an amino acid sequence substantially similar to the amino acid sequence of a naturally occurring *Borrelia* protein (e.g., differing by a few amino acids), such as when a nucleic acid encoding a protein is subjected to site-directed mutagenesis. "Corresponding" proteins are equivalent proteins from different species or strains of *Borrelia,* such as outer surface protein A (OspA) from strain B31 and OspA from strain K48. The invention additionally pertains to nucleic acids encoding these chimeric proteins.

In one embodiment, the present invention is drawn to chimeric proteins comprising antigenic polypeptides from Lyme Disease-causing strains *of Borrelia.* In another embodiment, the chimeric proteins described herein comprise antigenic polypeptides from different pathogenic genospecies *of Borrelia,* such as *Borrelia burgdorferi sensu stricto, Borrelia afzelii* and *Borrelia garinii.* In a preferred embodiment, the chimeric proteins comprise antigenic polypeptides from each of the pathogenic genospecies *of Borrelia,* including *Borrelia burgdorferi sensu stricto, Borrelia afzelii* and *Borrelia garinii.*

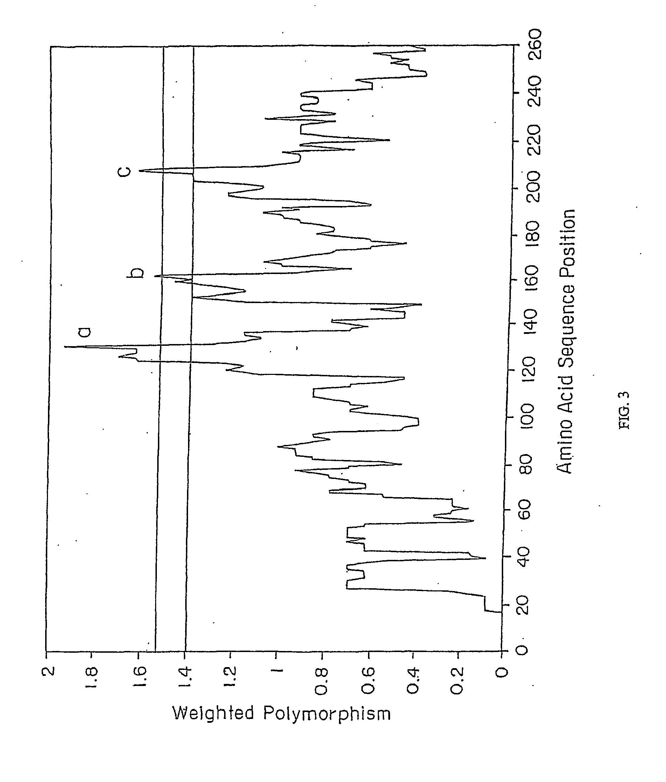

The OspA portion of the chimeric molecules of the present invention can themselves be chimeric combinations of more than one OspA polypeptide. Similarly, the OspC portion of the chimeric molecules of the present invention can themselves be chimeric combinations of more than one OspC polypeptide. As described below, Applicants have identified two separate antigenic domains of OspA and OspB which flank the sole conserved tryptophan present in OspA and in OspB. These domains share cross-reactivity with different genospecies *of Borrelia.* The precise amino acids responsible for antigenic variability were determined through site-directed mutagenesis, so that proteins with specific amino acid substitutions are available for the development of chimeric versions of OspA which can be included in the OspC/OspA chimeric proteins of the present invention. Furthermore, Applicants have identified immunologically important hypervariable domains in OspA proteins, as described below in Example 2. The first hypervariable domain of interest for chimeric proteins, Domain A, includes amino acid residues 120-140 of OspA, the second hypervariable domain, Domain B, includes residues 150-180 and the third hypervariable domain, Domain C, includes residues 200-216 or 217 (depending on the position of the sole conserved tryptophan residue in the OspA of that particular species of *Borrelia)* (see Fig. 3). In addition, Applicants have sequenced the genes for several *Borrelia* proteins.

These discoveries have aided in the development of novel recombinant *Borrelia* proteins which include two or more amino acid regions or sequences which do not occur in the same *Borrelia* protein in nature. The recombinant proteins comprise polypeptides from a variety of *Borrelia* proteins, including, but not limited to, OspA, OspB, OspC, OspD, p12, p39, p41, p66, and p93. Preferred combinations include all or a portion of OspC linked to all or a portion of OspA. Antigenically relevant polypeptides from each of a number of proteins are combined into a single chimeric protein.

In one embodiment of the current invention, chimeras are now available which include antigenic OspA polypeptides flanking a tryptophan residue. OspB has a similar primary structure as OspA and is included in the following discussion. The antigenic polypeptides are derived from either the proximal portion from the tryptophan (the portion of the OspA protein present between the amino terminus and the conserved tryptophan of the protein), or the distal portion from the tryptophan (the portion of the OspA protein present between the conserved tryptophan of the protein and the carboxy terminus) in OspA. The resultant chimeras can be OspA-OspA chimeras (e.g., chimeras incorporating polypeptides derived from OspA from different strains of Borrelia), OspA-OspB chimeras, or OspB-OspB chimeras, and are constructed such that amino acid residues amino-proximal to an invariant tryptophan are from one protein and residues carboxy-proximal to the invariant tryptophan are from the other protein. For example, one available chimer consists of a polypeptide derived from the amino-proximal region of OspA from strain B31, followed by the tryptophan residue, followed by a polypeptide derived from the carboxy-proximal region of OspA from strain K48 (SEQ ID NO. 92). Another available chimer includes a polypeptide derived from the amino-proximal region of OspA from strain B31, and a polypeptide derived from the carboxy-proximal region of OspB from strain B31 (SEQ ID NO. 104). If the polypeptide proximal to the tryptophan of these chimeric proteins is derived from OspA, the proximal polypeptide can be further subdivided into the three hypervariable domains (Domains A, B, and C), each of which can be derived from OspA from a different strain of *Borrelia.* These chimeric proteins can further comprise antigenic polypeptides from another protein, e.g., OspC, in addition to the antigenic polypeptides flanking the tryptophan residue.

In one embodiment, the chimeric OspC/OspA proteins of the present invention comprise at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein.

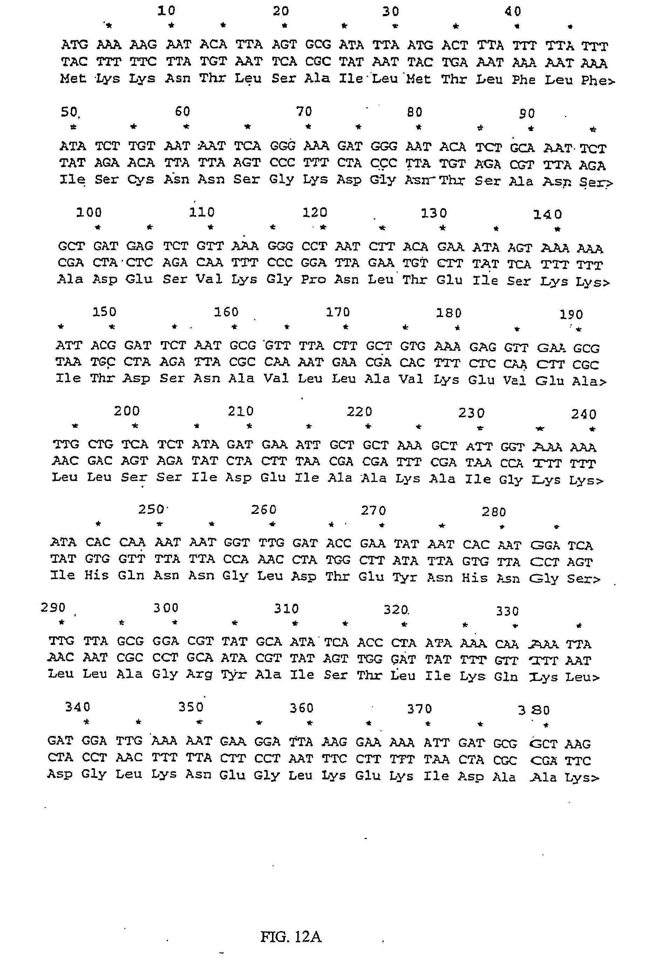

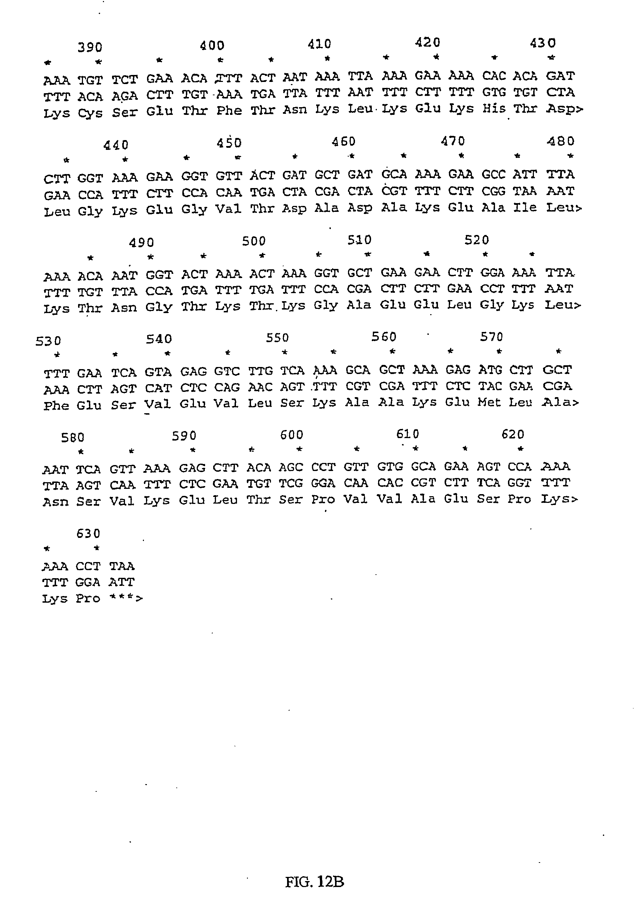

In a particular embodiment, the first polypeptide comprises a *Borrelia burgdorferi* OspC polypeptide from about amino acid residue 19 to about amino acid residue 213, and the second polypeptide comprises a *Borrelia burgdorferi* OspA polypeptide. In another embodiment, the first polypeptide comprises a *Borrelia burgdorferi* OspC polypeptide from about amino acid residue 19 to about amino acid residue 211. In another embodiment, the first polypeptide comprises a *Borrelia burgdorferi* OspC polypeptide from about amino acid residue 19 to about amino acid residue 208. In another embodiment, the first polypeptide comprises a *Borrelia burgdorferi* OspC polypeptide from about amino acid residue 19 to about amino acid residue 204. The numbering of the OspC residues is according to the numbering of SEQ ID NO: 30 (Figs. 12 A and 12B). It is evident that the person of skill in the art recognizes that OspC genes from different strains and/or genospecies may differ in their primary sequence and that based on homology, similar regions of such OspC proteins could be identified and used in the present invention with no or only routine experimentation.

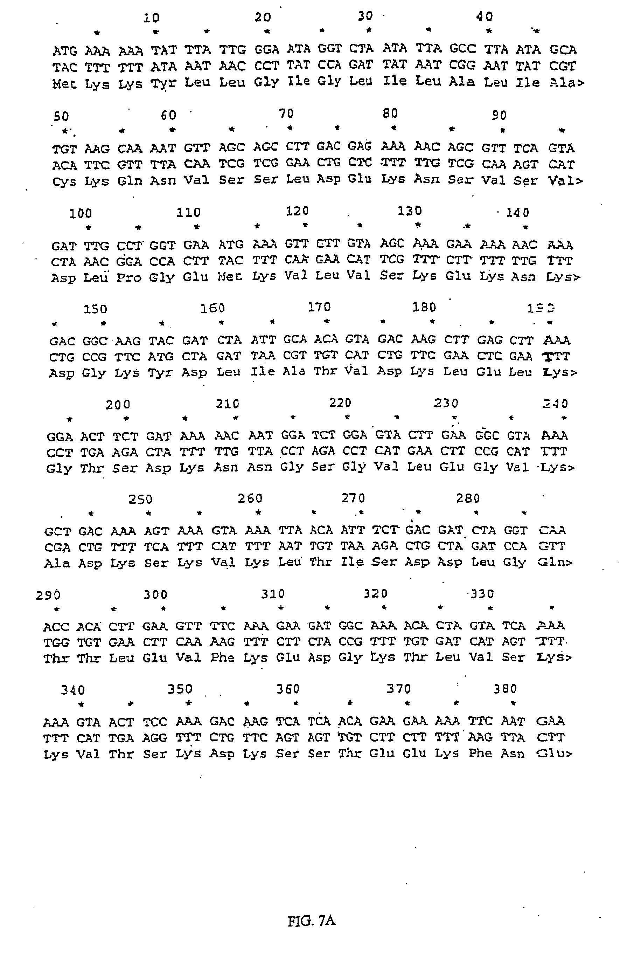

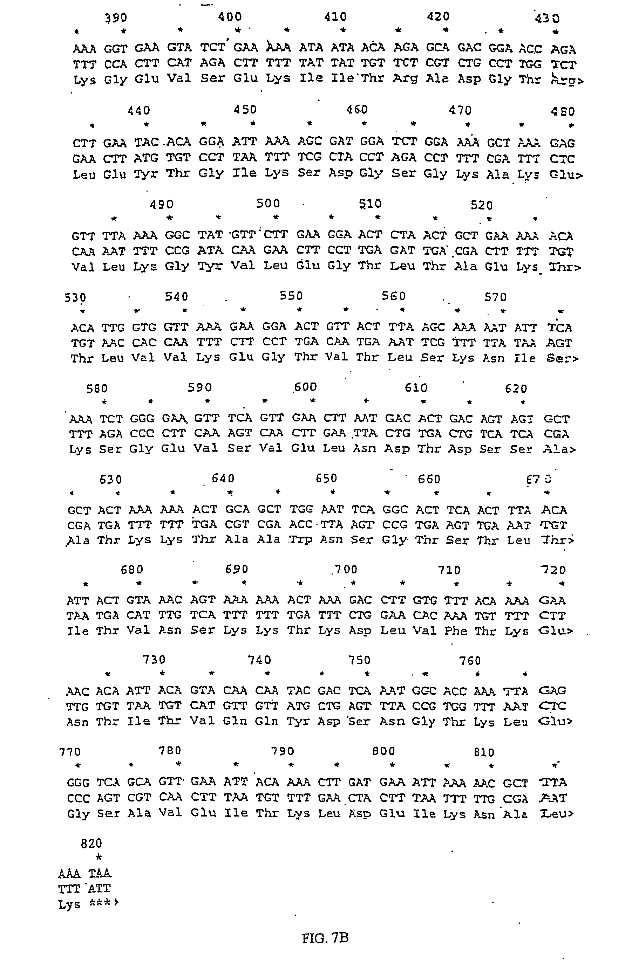

In one embodiment, the invention is drawn to chimeric OspC/OspA proteins wherein the first polypeptide comprises a *Borrelia burgdorferi* OspC polypeptide and the second polypeptide comprises a *Borrelia burgdorferi* OspA polypeptide from about amino acid residue 18 to about amino acid residue 273. In other embodiments, the chimeric OspC/OspA protein comprises a first polypeptide which is a *Borrelia burgdorferi* OspC polypeptide and a second polypeptide which is a *Borrelia burgdorferi* OspA polypeptide selected from the group consisting of an OspA polypeptide from about amino acid residue 132 to about amino acid residue 216, an OspA polypeptide from about amino acid residue 218 to about amino acid residue 273, an OspA polypeptide from about amino acid residue 18 to about amino acid residue 216 and an OspA polypeptide from about 132 to about amino acid residue 273. The numbering of the OspA residues is according to the numbering of SEQ ID NO: 7 (Fig. 7A and 7B). It is evident that the person of skill in the art recognizes that OspA genes from different strains and/or genospecies may differ in their primary sequence and that based on homology, similar regions of such OspA proteins could be identified and used in the present invention with no or only routine experimentation.

The present invention is also drawn to OspC/OspA chimeric proteins wherein the first polypeptide comprises a *Borrelia burgdorferi* OspC polypeptide and the second polypeptide comprises *a Borrelia burgdorferi* OspA polypeptide, wherein the OspA polypeptide comprises two or more OspA polypeptide fragments as described above. In a preferred embodiment, the OspA polypeptide comprises portions of OspA from two or more strains of *Borrelia.* In another preferred embodiment, the OspA polypeptide comprises portions of OspA from two or more Lyme Disease-causing genospecies *of Borrelia,* e.g., wherein the genospecies are *Borrelia burgdorferi sensu stricto, Borrelia afzelii* and/or *Borrelia garinii.* In still another preferred embodiment, the OspC/OspA chimeric protein comprises one or more polypeptides from each of the pathogenic genospecies, *Borrelia burgdorferi sensu stricto, Borrelia afzelii* and *Borrelia garinii.*

The chimeras described herein can be produced so that they are highly soluble, hyper-produced in *E*. *coli,* and non-lipidated. Lipidated chimeric proteins can also be produced. In addition, the chimeric proteins can be designed to end in an affinity tag (His-tag) to facilitate purification. The recombinant proteins described herein have been constructed to maintain antigenicity of at least one of the parent polypeptides. In addition, recombinant proteins specific for the various genospecies of *Borrelia* that cause Lyme disease are now available, because the genes from each of the major genospecies have been sequenced. These recombinant proteins with their novel biophysical and antigenic properties will be important diagnostic reagent and vaccine candidates.

The chimeric proteins of the current invention are advantageous in that they retain at least some specific reactivity to monoclonal or polyclonal antibodies that recognize wild-type *Borrelia* proteins. The proteins are immunogenic, and elicit antibodies that inhibit growth and/or induce lysis of *Borrelia in vitro.* Furthermore, in some embodiments, the proteins provide antigenic domains of two or more *Borrelia* strains and/or proteins within a single protein. Such proteins are particularly useful in immunodiagostic assays. For example, proteins of the present invention can be used as reagents in assays to detect the presence of antibodies to native *Borrelia* in potentially infected individuals. These proteins can also be used as immunodiagnostic reagents, such as in dot blots, Western blots, enzyme-linked immunosorbent assays (ELISA), or agglutination assays. The chimeric proteins of the present invention can be produced by known techniques, such as by recombinant methodology, polymerase chain reaction, or mutagenesis.

Furthermore, the proteins of the current invention are useful as vaccine immunogens against *Borrelia* infection. Because *Borrelia* has been shown to be clonal, a protein comprising antigenic polypeptides from a variety of *Borrelia* proteins and/or species, will provide immunoprotection for a considerable time when used in a vaccine. The lack of significant intragenic recombination, a process which might rapidly generate novel epitopes with changed antigenic properties, ensures that *Borrelia* can only change antigenic type by accumulating mutational change, which is slow when compared with recombination in generating different antigenic types. The chimeric protein can be combined with a physiologically-acceptable carrier and administered to a vertebrate animal through standard methods (e.g., intravenously or intramuscularly, for example).

In addition to the chimeric proteins described herein, the present invention is also drawn to nucleic acids which encode the *Borrelia* chimeric protein described herein. In one embodiment of the present invention, the composition comprises a nucleic acid that encodes a chimeric protein of at least two polypeptides, wherein the first polypeptide comprises *Borrelia burgdorferi* OspC, and the second polypeptide comprises *Borrelia burgdorferi* OspA, such that OspC is upstream of OspA. The OspC and OspA nucleic acid fragments that make up the chimeric protein can be from the same strain or genospecies of *Borrelia,* from different strains or genospecies *of Borrelia,* or from combinations of nucleic acids that are from the same and/or different strains or genospecies *of Borrelia.*

It is understood that the nucleic acids that encode the polypeptides that comprise the chimeric protein can include extra nucleotides or fewer nucleotides in order to simplify the construction of the gene encoding the chimeric polypeptide, e.g., to allow for the use of convenient restriction endonuclease sites or to allow the ligation of the gene fragments such that a contiguous coding region is created. Based on the guidance provided herein, one of ordinary skill in the art would readily be able to add or remove nucleotides from the termini of the gene fragments encoding the polypeptides of the chimeric OspC/OspA protein in order to generate the chimeric proteins of the present invention with no or only routine experimentation. Furthermore, there can be an extra about 1 to about 10 amino acids on the N- and/or C-terminus of the polypeptides and chimeric proteins of the present invention and still retain the properties of the present invention. It is also understood that those of skill in the art, using art-known methods and/or the methods described herein, could generate additional OspC-OspA chimeric proteins, and that these chimeric proteins are encompassed by the invention.

The present invention is also drawn to expression vectors which comprise an isolated DNA encoding the *Borrelia* chimeric protein described herein. In one embodiment, the composition includes an expression vector comprising an isolated DNA which encodes an OspC/OspA chimeric protein, wherein the OspC portion of the protein is upstream of the OspA portion. The present invention also encompasses host cells which comprise a recombinant nucleic acid that encodes an OspC/OspA chimeric protein, as described herein.

The present invention is also drawn to methods of making the *Borrelia* chimeric polypeptides described herein. In one embodiment, the method of making a chimeric *Borrelia* protein comprises selecting a polynucleotide sequence encoding OspC, or an antigenic portion thereof, selecting a polynucleotide sequence encoding OspA, or an antigenic portion thereof, and ligating these polynucleotide sequences together, such that OspC comprises the N-terminus of the protein. The polypeptides of the present invention can also be recombinantly expressed in suitable microbial hosts, wherein said hosts include, but are not limited to, bacterial hosts, such as *E*. *coli,* fungal hosts, such as *S*. *cerevisiae* or cell culture hosts, such as those of mammalian cell culture or insect cell culture.

The present invention is also drawn to methods of delivering the *Borrelia* chimeric polypeptides described herein. In one embodiment, the method comprises administering the chimeric protein in a physiologically-acceptable carrier to an individual. The individual develops at least some immune response to the chimeric protein. As an example, the individual could generate a humoral immune response, wherein antibodies that recognize at least a portion of said chimeric polypeptide are produced by the individual. The antibodies that recognize the chimeric polypeptide can be of any class of immunoglobulin, such as IgM, IgD, IgA and IgG or combinations thereof.

The present invention is also drawn to methods of delivering a nucleic acid which encodes a chimeric polypeptide described herein. In one embodiment, the method comprises administering the nucleic acid in a physiologically-acceptable carrier to an individual using art-accepted methods of DNA delivery, including but not limited to, biolistic delivery and lipid encapsulation. The chimeric polypeptide is at least transiently expressed and the individual develops at least some immune response to the chimeric protein encoded by the nucleic acid.

The invention also encompasses methods of using the chimeric proteins described herein in diagnostic assays. In one embodiment, the method can be used to detect the presence of OspA- and/or OspC-specific antibodies in a sample, e.g., a host sample of interest. In one embodiment, the method comprises contacting a host sample of interest with the chimeric OspC/OspA protein, under conditions, wherein antibodies, if present in the host sample, bind to the chimeric protein thereby forming antigen-antibody complexes. The antigen-antibody complexes are then detected. In this manner, an immune response to Lyme-Disease causing *Borrelia* can be detected.

As described herein, the chimeric proteins of the present invention incorporate antigenic domains from different *Borrelia* proteins, as well as from different *Borrelia* strains and/or genospecies. As such, they are useful in the detection or diagnosis of the presence of Lyme disease-causing *Borrelia,* especially *Borrelia* from groups capable of causing disseminated symptoms of Lyme disease. Disseminated symptoms refer to infection outside of the erythema migrans skin lesion, e.g., infection in blood, CNS or synovia.

The chimeric polypeptides of the present invention elicit specific immune responses to OspC and OspA. In one embodiment, the chimeric polypeptides elicit immune responses to strains of Lyme disease-causing *Borrelia* of the same genospecies as that represented by the OspC/OspA chimeric protein. In another embodiment, the chimeric polypeptides elicit immune responses to strains of Lyme disease-causing *Borrelia* of different genospecies than that represented by the OspC/OspA chimeric protein, as well as to Lyme disease-causing *Borrelia* of the same genospecies as that represented by the OspC/OspA chimeric protein. The immune response includes, but is not limited to, a humoral response, a secretory response, a cell-mediated response, or any combination thereof.

The immunogenic compositions of the present invention can also be used to immunize animals, e.g., mammals, including humans. Immunization is understood to elicit specific immunogenic responses as described herein. In one embodiment, administration of an immunogenic composition, e.g., an OspC/OspA chimeric protein, an OspC/OspA chimeric nucleic acid, to an animal results in the animal developing immunity to infection by Lyme disease-causing *Borrelia,* e.g., *Borrelia burgdorferi, Borrelia afzelii* and/or *Borrelia garinii.*

Immunity, as described herein, is understood to mean the ability of the treated animal to resist infection (e.g., systemic infection), to overcome infection (e.g., systemic infection) or to overcome infection (e.g., systemic infection) more easily and/or more quickly when compared to non-immunized and/or non-treated individuals. Immunity can also include an improved ability of the treated individual to sustain an infection with reduced or no clinical symptoms of systemic infection. The individual may be treated with the chimeric proteins of the present invention either proactively, e.g., once a year or alternatively after sustaining a tick bite.

In one embodiment, the OspC/OspA chimeric protein of the present invention, together with suitable excipients and/or adjuvants, is administered to an animal such that the animal develops an immune response to at least one of the OspC and/or OspA polypeptides of the composition. The pharmaceutical composition can also be administered with other components suitable for *in vitro* and/or *in vivo* use. These additional components include buffers, carrier proteins, adjuvants, excipients, preservatives and combinations thereof.

The present invention is also drawn to pharmaceutical compositions which can be used to vaccinate and/or treat *Borrelia* infection in an animal or human. In a particular embodiment, the pharmaceutical composition comprises a *Borrelia burgdoferi* OspC/OspA chimeric protein. The pharmaceutical composition can also be administered together with a physiologically-acceptable carrier, an excipient and/or an adjuvant. Suitable adjuvants are well known in the art (see for example PCT Publication WO 96/40290, the entire teachings of which are incorporated herein by reference), and can be used, for example, to enhance immunogenicity, potency or half-life of the chimeric proteins in the treated animal.

The pharmaceutical compositions used to vaccinate and/or treat *Borrelia* infection can be prepared using methods for preparing vaccines which are well known in the art. For example, the OspC/OspA chimeric proteins described herein can be isolated and/or purified by known techniques, such as by size exclusion chromatography, affinity chromatography, ion exchange chromatography, preparative electrophoresis, selective precipitation or combinations thereof. The prepared chimeric proteins can be mixed with suitable other reagents as described herein, such that the chimeric protein is at a suitable concentration. The dosage of the chimeric protein will vary and depends upon the age, weight and/or physical condition of the animal, e.g., mammal, human, to be treated. The optimal dosage can be determined by routine optimization techniques, using suitable animal models.

Administration of the pharmaceutical composition to be used as a vaccine can be by any suitable technique. Suitable techniques for administration of the pharmaceutical composition include, but are not limited to, injection, e.g., subcutaneous injection, intramuscular injection, intravenous injection, intra peritoneal injection; mucosal administration, e.g., exposing nasal mucosa to nose drops containing the proteins or chimeric proteins of the present invention; oral administration; and DNA immunization.

The incorporation of polypeptide fragments from different strains and/or genospecies of *Borrelia burgdorferi* allows for a greater detection range and a more effective vaccination tool. The present invention provides a chimeric combination of proteins that, when used as a vaccine, can prevent Lyme disease from becoming systemic. The chimeric proteins of the present invention can be effective in preventing Lyme disease, as well as having a therapeutic effect on established infection, for example, after the tick bite is noticed by the patient. Since the chimeric proteins of the present invention comprise both OspC and OspA polypeptides, they are expected to act at the level of the tick as well as the level of the host in preventing both infection and disease due to *Borrelia burgdorferi, Borrelia afzelii* and/or *Borrelia garinii*.

The present invention is also drawn to diagnostic kits which comprise the chimeric polypeptides described herein. In one embodiment, the kit comprises a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein. The kit also includes reagents for detecting antibody-antigen complexes that are formed between the OspC/OspA chimeric protein and antibodies that are present in a sample, e.g., a user-supplied host sample.

As a result of the present invention, it is now possible to prepare improved diagnostic tools comprising both OspA and OspC antigens from various *Borrelia burgdorferi* strains and/or genospecies. Since OspA is primarily expressed in the tick vector, and OspC is upregulated in response to the feeding of an infected tick on a mammal, the diagnostic compositions of the invention can recognize antigens that are expressed at different stages of the life cycle of *Borrelia burgdorferi.* Moreover, by incorporating unique polypeptide fragments from pathogenic families of *Borrelia,* the present invention allows for improved diagnostic compositions which can detect clinically important exposure to pathogenic *Borrelia* while overlooking the remainder of non-pathogenic families *of Borrelia.*

As described herein, the OspC/OspA chimeric proteins were bioengineered such that the protective domains of each protein were maintained. In experiments described herein, mice were either immunized with OspA, OspC or OspC/OspA chimeric proteins in aluminum hydroxide. Mice were then bled and tested for antibody responses against OspC and OspA derived from various strains *of Borrelia.* In additional experiments, these immunized mice were challenged with ticks infected with *Borrelia burgdorferi* and transmission of infection was assessed.

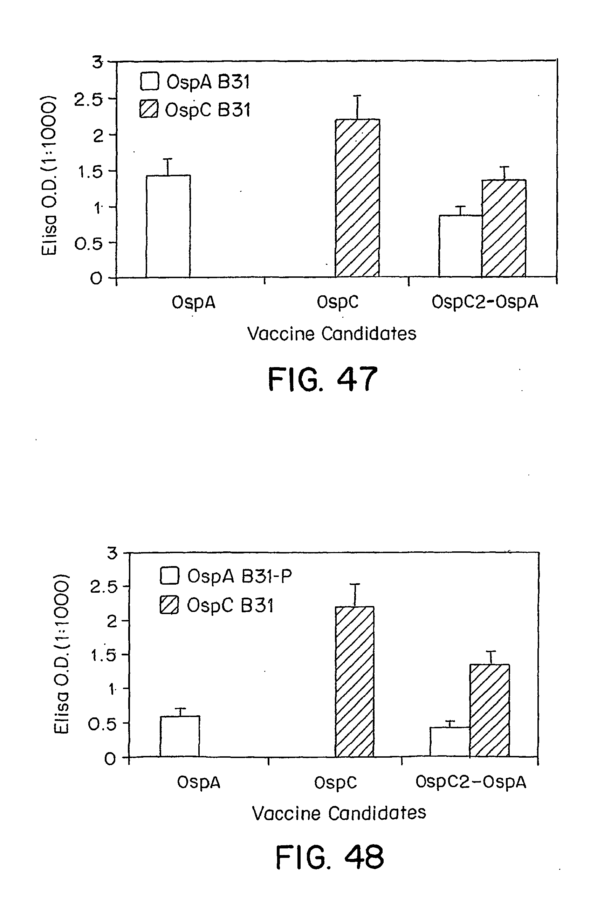

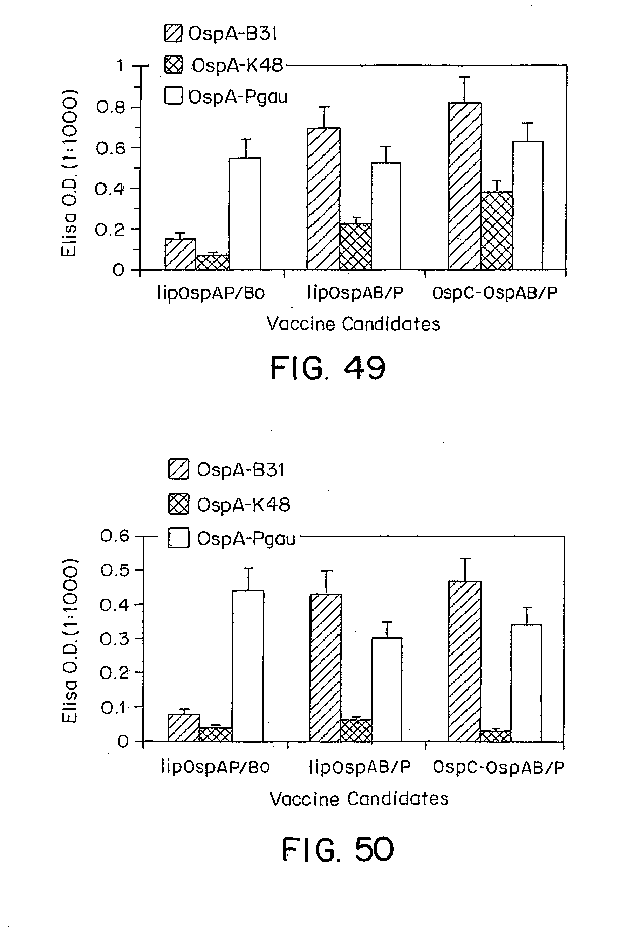

Mice immunized with the OspC/OspA chimeric protein gave a remarkable and equivalent antibody response to both OspA and OspC, as compared to mice immunized with OspA and OspC control proteins (Figs. 47 and 48). In addition, antibodies in the sera of mice immunized with the OspC/OspA chimeric protein were also reactive against antigens derived from different strains *of Borrelia burgdorferi* (Figs. 49-50 and 52-54). Chimer-immunized mice were fully protected against challenge with ticks infected with *Borrelia burgdorferi,* as compared to sham-vaccinated controls (infection rates of 100%) (Table VI).

In other experiments described herein, mice were either immunized with a lipidated OspA chimeric protein, a lipidated OspC chimeric protein, or a non-lipidated OspC/OspA chimeric protein, once again in the presence of aluminum hydroxide. Mice were then bled and tested for antibody responses against OspA and OspC derived from various strains of *Borrelia.* Surprisingly, the results of these studies indicate that mice immunized with the non-lipidated OspC/OspA chimeric protein have antibody responses to OspA and OspC that are equivalent or greater than those generated by mice immunized with the corresponding lipidated OspA or lipidated OspC chimeric proteins (Figs. 49-51).

The results of the studies presented herein indicate that mice immunized with OspC-OspA chimeric proteins generate a potent antibody response against two immunoprotective targets that are expressed at different stages of the life cycle of *Borrelia burgdorferi.*

The current invention is illustrated by the following Examples, which are not to be construed to be limiting in any way.

### EXEMPLIFICATION

### Example 1. Purification of Borrelia burgdorferi Outer Surface Protein A and Analysis of Antibody Binding Domains

This example details a method for the purification of large amounts of native outer surface protein A (OspA) to homogeneity, and describes mapping of the antigenic specificities of several anti-OspA MAbs. OspA was purified to homogeneity by exploiting its resistance to trypsin digestion. Intrinsic labeling with ¹⁴C-palmitic acid confirmed that OspA was lipidated, and partial digestion established lipidation at the amino-terminal cysteine of the molecule.

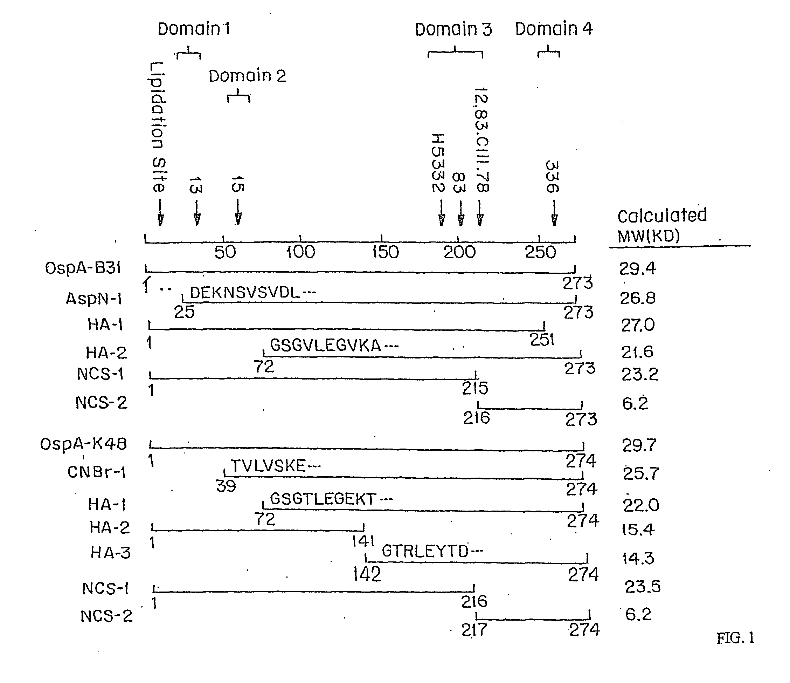

The reactivity of seven anti-OspA murine monoclonal antibodies to nine different *Borrelia* isolates was ascertained by Western blot analysis. Purified OspA was fragmented by enzymatic or chemical cleavage, and the monoclonal antibodies were able to define four distinct immunogenic domains (see Fig. 1). Domain 3, which included residues 190-220 of OspA, was reactive with protective antibodies known to agglutinate the organism *in vitro,* and included distinct specificities, some of which were not restricted to a genotype of *B. burgdorferi.*

### A. Purification of Native OspA

Detergent solubilization *of B. burgdorferi* strips the outer surface proteins and yields partially-purified preparations containing both OspA and outer surface protein B (OspB) (Barbour, A.G. et al., Infect. Immun. 52 (5): 549-554 (1986); Coleman, J.L. and J.L. Benach, J Infect. Dis. 155 (4): 756-765 (1987); Cunningham, T.M. et al., Ann. NYAcad. Sci. 539: 376-378 (1988); Brandt, M.E. et al., Infect. Immun. 58: 983-991 (1990); Sambri, V. and R. Cevenini, Microbiol. 14: 307-314 (1991)). Although both OspA and OspB are sensitive to proteinase K digestion, in contrast to OspB, OspA is resistant to cleavage by trypsin (Dunn, J. et al., Prot. Exp. Purif 1: 159-168 (1990); Barbour, A.G. et al., Infect. Immun. 45: 94-100 (1984)). The relative insensitivity to trypsin is surprising in view of the fact that OspB A has a high (16% for B31) lysine content, and may relate to the relative configuration of OspB A and B in the outer membrane.

### Intrinsic Radiolabeling of Borrelia

Labeling for lipoproteins was performed as described by Brandt *et al.* (Brandt et al., Infect. Immun. 58: 983-991 (1990)). ¹⁴C-palmitic acid (ICN, Irvine, California) was added to the BSK II media to a final concentration of 0.5 µCi per milliliter (ml). Organisms were cultured at 34°C in this medium until a density of 10⁸ cells per ml was achieved.

### Purification of OspA Protein from Borrelia Strain B31

*Borrelia burgdorferi,* either ¹⁴C-palmitic acid-labeled or unlabeled, were harvested and washed as described (Brandt, M.E. et al., Infect. Immun. 58: 983-991 (1990)). Whole organisms were trypsinized according to the protocol of Barbour et al. (Infect. Immun. 45: 94-100 (1984)) with some modifications. The pellet was suspended in phosphate buffered saline (PBS, 10mM, pH 7.2), containing 0.8% tosyl-L-phenylalanine chloromethyl ketone (TPCK)-treated trypsin (Sigma, St. Louis, Missouri), the latter at a ratio of 1 µg per 10⁸ cells. Reaction was carried out at 25°C for 1 hour, following which the cells were centrifuged. The pellet was washed in PBS with 100 µg/ml phenylmethylsulfonyl fluoride (PMSF). Triton X-114 partitioning of the pellet was carried out as described by Brandt *et al.* (Brandt et al., Infect. Immun. 58: 983-991 (1990)). Following trypsin treatment, cells were resuspended in ice-cold 2% (v/v) Triton X-114 in PBS at 10⁹ cells per ml. The suspension was rotated overnight at 4°C, and the insoluble fraction removed as a pellet after centrifugation at 10,000 X g for 15 minutes at 4°C. The supernatant (soluble fraction) was incubated at 37°C for 15 minutes and centrifuged at room temperature at 1000 X g for 15 minutes to separate the aqueous and detergent phases. The aqueous phase was decanted, and ice cold PBS added to the lower Triton phase, mixed, warmed to 37°C, and again centrifuged at 1000 X g for 15 minutes. Washing was repeated twice more. Finally, detergent was removed from the preparation using a spin column ofBio-beads SM2 (BioRad, Melville, New York) as described (Holloway, P.W., Anal. Biochem. 53: 304-308 (1973)).

Ion exchange chromatography was carried out as described by Dunn *et al.* (Dunn et al., Prot. Exp. Purif. 1: 159-168 (1990)) with minor modifications. Crude OspA was dissolved in buffer A (1% Triton X-100, 10mM phosphate buffer (pH 5.0)) and loaded onto a SP Sepharose resin (Pharmacia, Piscataway, New Jersey), pre-equilibrated with buffer A at 25°C. After washing the column with 10 bed-volumes of buffer A, the bound OspA was eluted with buffer B (1% Triton X-100, 10mM phosphate buffer (pH 8.0)). OspA fractions were detected by protein assay using the BCA method (Pierce, Rockford, Illinois), or as radioactivity when intrinsically labeled material was fractionated. Triton X-100 was removed using a spin column of Bio-beads SM2.

This method purifies OspA from an outer surface membrane preparation. In the absence of trypsin-treatment, OspA and B were the major components of the soluble fraction obtained after Triton partitioning of strain B31. In contrast, when Triton extraction was carried out after trypsin-treatment, the OspB band is not seen. Further purification of OspA-B31 on a SP Sepharose column resulted in a single band by SDS-PAGE. The yield following removal of detergent was approximately 2 mg per liter of culture. This method of purification of OspA, as described herein for strain B31, can be used for other isolates *of Borrelia* as well. For strains such as strain K48, which lack OspB, trypsin treatment can be omitted.

### Lipidation site of OspA-B31

¹⁴C-palmitic acid labeled OspA from strain B31 was purified as described above and partially digested with endoproteinase Asp-N (data not shown). Following digestion, a new band of lower molecular weight was apparent by SDS-PAGE, found by direct ammo-terminal sequencing to begin at Asp₂₅. This band had no trace of radioactivity by autoradiography (data not shown). OspA and B contain a signal sequence (L-X-Y-C) similar to the consensus described for lipoproteins of *E. coli,* and it has been predicted that the lipidation site of OspA and B should be the amino-terminal cysteine (Brandt, M.E. et al., Infect. Immun 58: 983-991 (1990)). The results presented herein support this prediction.

### B. Comparison of OspA Antibody Binding Regions in Nine Strains of Borrelia burgdorferi

The availability of the amino acid sequenced for OspA from a number of different isolates, combined with peptide mapping and Western blot analysis, permitted the identification of the antigenic domains recognized by monoclonal antibodies (MAbs) and allowed inference of the key amino acid residues responsible for specific antibody reactivity.

### Strains of Borrelia burgdorferi

Nine strains of Borrelia, including seven European strains and two North American strains, were used in this study of antibody binding domains of several proteins. Information concerning the strains is summarized in Table I, below.

**Table I. Representative Borrelia Strains**

| Strain | Location and Source | Reference for Strain |
|---|---|---|
| K48 | Czechoslovakia, *Ixodes ricinus* | none |
| PGAU | Germany, human ACA | Wilske, B. et al., J. Clin. Microbiol. 32: 340-350 (1993) |
| DK29 | Denmark, human EM | Wilske, *B. et al.* |
| PKo | Germany, human EM | Wilske, B. *et al.* |
| PTrob | Germany, human skin | Wilske, B. *et al.* |
| Ip3 | Khabarovsk, Russia, *I*. *persulcatus* | Asbrink, E. et al., Acta Derm. Venereol. 64: 506-512 (1984) |
| Ip90 | Khabarovsk, Russia, *I*. *persulcatus* | Asbrink, E*. et al.* |
| 25015 | Millbrook, NY, *I*. *persulcatus* | Barbour, A.G. et al., Curr Microbiol. 8:123-126 (1983) |
| B31 | Shelter Island, NY, *I*. *scapularis* | Luft, B.J. et al., Infect. Immun. 60: 4309-4321 (1992); ATCC 35210 |
| PKa1. | Germany, human CSF | Wilske, B. *et al.* |
| ZS7 | Freiburg, Germany, *I*. *ricinus* | Wallich, R. et al., Nucl. Acids Res. 17: 8864 (1989) |
| N40 | Westchester Co., NY | Fikrig, E. et al., Science 250: 553-556 (1990) |
| PHei | Germany, human CSF | Wilske, B. *et al.* |
| ACAI | Sweden, human ACA | Luft, B. J. et al., FEMS Microbiol. Lett. 93: 73-68 (1992) |
| PBo | Germany, human CSF | Wilske, *B. et al.* |

| | | |
|---|---|---|
| ACA = patient with acrodermatitis chronica atrophicans; EM = patient with erythema migrans; CSF = cerebrospinal fluid of patient with Lyme disease | | |

Strains K48, PGAU and DK29 were supplied by R. Johnson, University of Minnesota; PKo and PTrob were provided by B. Wilske and V. Preac-Mursic of the Pettenkhofer Institute, Munich, Germany; and Ip3 and Ip90 were supplied by L. Mayer of the Center for Disease Control, Atlanta, Georgia. The North American strains included strain 25015, provided by J. Anderson of the Connecticut Department of Agriculture; and strain B31 (ATCC 35210).

### Monoclonal Antibodies

Seven monoclonal antibodies (MAbs) were utilized in this study. Five of the MAbs (12, 13; 15, 83 and 336) were produced from hybridomas cloned and subcloned as previously described (Schubach, W.H., et al., Infect. Immun. 59(6):1911-1915 (1991)). MAb H5332 (Barbour, A.G. et al., Infect. Immun. 41: 795-804 (1983)) was a gift from Drs. Alan Barbour, University of Texas, and MAb CIII.78 (Sears, J.E. et al., J. Immunol. 147(6):1995-2000 (1991)) was a gift from Richard A. Flavell, Yale University. MAbs 12 and 15 were raised against whole sonicated B3; MAb 336 was produced against whole PGAU; and MAbs 13 and 83 were raised to a truncated form of OspA cloned from the K48 strain and expressed in *E. coli* using the T7 RNA polymerase system (McGrath, B.C. et al., Vaccines, Cold Spring Harbor Laboratory Press, Plainview, New York, pp. 365-370 (1993)). All MAbs were typed as being Immunoglobulin G (IgG).

### Methods of Protein Cleavage, Western Blotting and Amino-Terminal Sequencing

Prediction of the various cleavage sites was achieved by knowledge of the primary amino acid sequence derived from the full nucleotide sequences of OspA, many of which are currently available (see Table II, below). Cleavage sites can also be predicted based on the peptide sequence of OspA, which can be determined by standard techniques after isolation and purification of OspA by the method described above. Cleavage of several OspA isolates was conducted to determine the localization of monoclonal antibody binding of the proteins.

Hydroxylamine-HCl (HA), N-chlorosuccinimide (NCS), and cyanogen bromide cleavage of OspA followed the methods described by Bornstein (Biochem. 9 (12):2408-2421 (1970)), Shechter et al., (Biochem. 15 (23):5071-5075 (1976)), and Gross (in Hirs, C.H.W. (ed): Methods in Enzymology, (N.Y. Acad. Press), 11:238-255 (1967)) respectively. Protease cleavage by endoproteinase, Asp-N (Boehringer Mannheim, Indianapolis, Indiana), was performed as described by Cleveland D.W. et al., (J. Biol. Chem. 252: 1102-1106 (1977)). Ten micrograms of OspA were used for each reaction. The ratio of enzyme to OspA was approximately 1 to 10 (w/w).

Proteins and peptides generated by cleavage were separated by SDS-polyacrylamide gel electrophoresis (SDS-PAGE) (Laemmli, U.K., Nature (London) 227:680-685 (1970)), and electroblotted onto immobilon Polyvinylidine Difluoride (PVDF) membranes (Ploskal, M.G. et al., Biotechniques 4: 272-283 (1986)). They were detected by amido black staining or by immunostaining with murine MAbs, followed by alkaline phosphatase-conjugated goat antimouse IgG. Specific binding was detected using a 5-bromo-4-chloro-3-indolylphosphate (BCIP)/nitroblue tetrazolium (NBT) developer system (KPL Inc., Gathersburg, Maryland).

In addition, amino-terminal amino acid sequence analysis was carried out on several cleavage products, as described by Luft et al. (Infect. Immun. 57: 3637-3645 (1989)). Amido black stained bands were excised from PVDF blots and sequenced by Edman degradation using a Biosystems model 475A sequenator with model 120A PTH analyzer and model 900A control/data analyzer.

### Cleavage Products of Outer Surface Protein A Isolates

Purified OspA-B31, labeled with ¹⁴C-palmitic acid, was fragmented with hydroxylamine-HCl (HA) into two peptides, designated HA1 and HA2 (data not shown). The HA1 band migrated at 27 KD and retained its radioactivity, indicating that the peptide included the lipidation site at the N-terminus of the molecule (data not shown). From the predicted cleavage point, HA1 should correspond to residues 1 to 251 of OspA-B31. HA2 had a MW of 21.6 KD by SDS-PAGE, with amino-terminal sequence analysis showing it to begin at Gly72, i.e. residues 72 to 273 of OspA-B31. By contrast, HA cleaved OspA-K48 into three peptides, designated HA1, HA2, and HA3 with apparent MWs of 22KD, 16 KD and 12 KD, respectively. Amino-terminal sequencing showed HA1 to start at Gly72, and HA3 at Gly142. HA2 was found to have a blocked amino-terminus, as was observed for the full-length OspA protein. HA1,2 and 3 of OspA-K48 were predicted to be residues 72-274, 1 to 141 and 142 to 274, respectively.

N-Chlorosuccinimide (NCS) cleaves tryptophan (W), which is at residue 216 of OspA-B31 or residue 217 of OspA-K48 (data not shown). NCS cleaved OspA-B31 into 2 fragments, NCS1, with MW of 23 KD, residues 1-216 of the protein, and NCS2 with a MW of 6.2 KD, residues 217 to 273 (data not shown). Similarly, K48 OspA was divided into 2 pieces, NCS1 residues 1-217, and NCS2 residues 218 to 274 (data not shown).

Cleavage of OspA by cyanogen bromide (CNBr) occurs at the carboxy side of methionine, residue 39. The major fragment, CNBr1, has a MW of 25.7 KD, residues 39-274 by amino-terminal amino acid sequence analysis (data not shown). CNBr2 (about 4 KD) could not be visualized by amido black staining; instead, lightly stained bands of about 20 KD MW were seen. These bands reacted with anti-OspA MAbs, and most likely were degradation products due to cleavage by formic acid.

### Determination of Antibody Binding Domains for Anti-OspA Monoclonal Antibodies

The cleavage products of OspA-B31 and OspA-K48 were analyzed by Western blot to assess their ability to bind to the six different MAbs. Preliminary Western blot analysis of the cleavage products demonstrated that strains K48 and DK29 have similar patterns of reactivity, as do Ip3, PGAU and PKo. The OspA of strain PTrob was immunologically distinct from the others, being recognized only by MAb 336. MAb 12 recognized only the two North American strains, B31 and 25015. When the isolates were separated into genogroups, it was remarkable that all the MAbs, except MAb 12, crossed over to react with multiple genogroups.

MAb12, specific for OspA-B31, bound to both HA1 and HA2 ofOspA-B31. However, cleavage of OspA-B31 by NCS at residue Trp216 created fragments which did not react with MAb12, suggesting that the relevant domain is near or is structurally dependent upon the integrity of this residue (data not shown). MAb 13 bound only to OspA-K48, and to peptides containing the amino-terminus of that molecule (e.g. HA2; NCS1). It did not bind to CNBr1 residues 39 to 274. Thus the domain recognized by MAb13 is in the amino-terminal end of OspA-K48, near Met38.

MAb15 reacts with the OspA of both the B31 and K48 strains, and to peptides containing the N-terminus of OspA, such as HA1 of OspA-B31 and NCS1, but not to peptides HA2 of OspA-B31 and HA1 of OspA-K48 (data not shown). Both peptides include residue 72 to the C-terminus of the molecules. MAb15 bound to CNBr1 of OspA-K48, indicating the domain for this antibody to be residues 39 to 72, specifically near Gly72 (data not shown).

MAb83 binds to OspA-K48, and to peptides containing the C-terminal portion of the molecule, such as HA1. They do not bind to HA2 of OspA-K48, most likely because the C-terminus of HA2 of OspA-K48 ends at 141. Similar to MAb12 and OspA-B31, binding of MAbs 83 and CIII.78 is eliminated by cleavage of OspA at the tryptophan residue. Thus binding of MAbs 12, 83 and CIII.78 to OspA depends on the structural integrity of the Trp₂₁₆ residue, which appears to be critical for antigenicity. Also apparent is that, although these MAbs bind to a common antigenic domain, the precise epitopes which they recognize are distinct from one another given the varying degrees of cross-reactivity to these MAbs among strains.

Although there is similar loss of binding activity of MAb336 with cleavage at Trp₂₁₆, this MAb does not bind to HA1 of OspA-B31, suggesting the domain for this antibody includes the carboxy-terminal end of the molecule, inclusive of residues 251 to 273. Low MW peptides, such as HA3 (10 KD) and NCS2 (6KD), of OspA-K48 do not bind this MAb on Western blots. In order to confirm this observation, we tested binding of the 6 MAbs with a recombinant fusion construct p3A/EC that contains a trpE leader protein fused with residues 217 to 273 of OspA-B31 (Schubach, W.H. et al., Infect. Immun. 59(6): 1911-1915 (1991)). Only MAb336 reacted with this construct (data not shown). Peptides and antigenic domains localized by fragmentation of OspA are summarized in Fig. 1.

### Mapping of Domains to Define the Molecular Basis for the Serotype Analysis

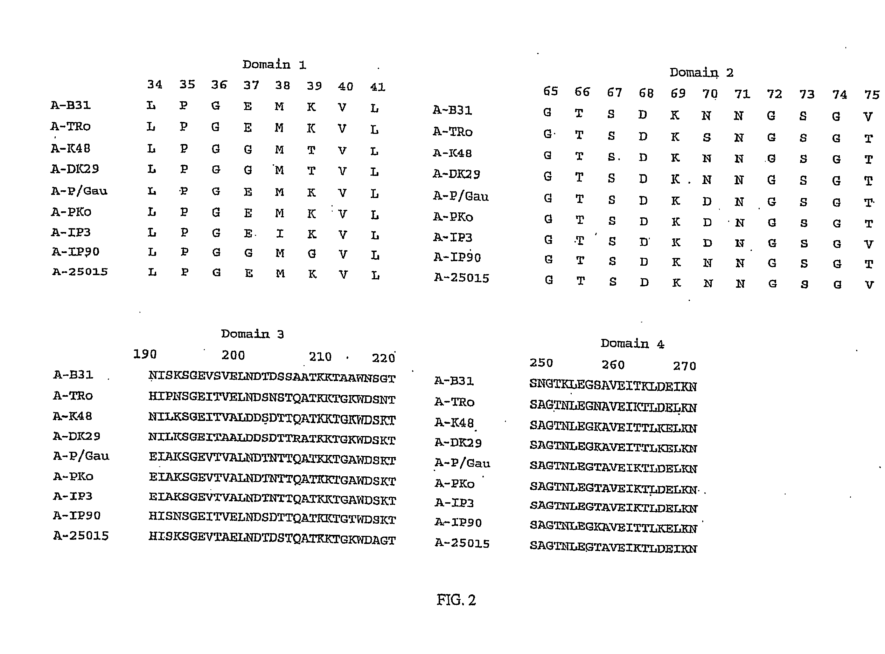

To define the molecular basis for the serotype analysis of OspA, we compared the derived amino acid sequences of OspA for the nine isolates (Fig. 2). At the amino terminus of the protein, these predictions can be more precise given the relatively small number of amino acid substitutions in this region compared to the carboxy terminus. Domain 1, which is recognized by MAb13, includes residues Leu34 to Leu41. MAb13 only binds to the OspA of species K48, DK29 and IP90. Within this region, residue 37 is variable, however Gly37 is conserved amongst the three reactive strains. When Gly37 is changed to Glu37, as it is in OspA of strains B31, PTrob, PGAU, and PKo, MAb13 does not recognize the protein (data not shown). By similar analysis, it can be seen that Asp70 is a crucial residue for Domain 2, which includes residues 65 to 75 and is recognized by MAb1.5. Domain 3 is reactive with MAbs H5332, 12 and 83, and includes residues 190-220. It is clear that significant heterogeneity exists between MAbs reactive with this domain, and that more than one conformational epitope must be contained within the sequence. Domain 4 binds MAb336, and includes residues 250 to 270. In this region, residue 266 is variable and therefore may be an important determinant. It is apparent, however, that other determinants of the reactivity of this monoclonal antibody reside in the region comprising amino acids 217-250. Furthermore, the structural integrity of Trp216 is essential for antibody reactivity in the intact protein. Finally, it is important to stress that Fig. 2 indicates only the locations of the domains, and does not necessarily encompass the entire domain. Exact epitopes are being analyzed by site-directed mutagenesis of specific residues.

Overall, evidence suggests that the N-terminal portion is not the immunodominant domain of OspA, possibly by virtue of its lipidation, and the putative function of the lipid moiety in anchoring the protein to the outer envelope. The C-terminal end is immunodominant and includes domains that account in part for structural heterogeneity (Wilske, B. et al., Med. Microbiol. Immunol. 181: 191-207 (1992)), and may provide epitopes for antibody neutralization (Sears, J.E. et al., J. Immunol. 147(6): 1995-2000 (1991)), and relate to other activities, such as the induction of T-cell proliferation (Shanafel, M.M., et al., J. Immunol. 148: 218-224 (1992)). There are common epitopes in the carboxy-end of the protein that are shared among genospecies which may have immunoprotective potential (Wilske, B., et al., Med. Microbiol. Immunol. 181: 191-207 (1992)).

Prediction of secondary structure on the basis of hydropathy analysis and circular dichroism and fluorescence spectroscopy measurements (McGrath, B.C., et al., Vaccines, Cold Spring Harbor Laboratory Press, Plainview, New York; pp. 365-370 (1993)) suggest domains 3 and 4 to be in a region of the molecule with a propensity to form alpha-helix, whereas domains 1 and 2 occur in regions predicted to be beta-sheets (see Fig. 1). These differences may distinguish domains in accessibility to antibody or to reactive T-cells (Shanafel, M.M. et al., J. Immunol. 148: 218-224 (1992)). Site-directed mutagenesis of specific epitopes, as described below in Example 2, aids in identifying exact epitopes.

### Example 2. Identification of an Immunologically Important Hypervariable Domain of the Major Outer Surface Protein A of Borrelia.

This Example describes epitope mapping studies using chemically cleaved OspA and TrpE-OspA fusion proteins. The studies indicate a hypervariable region surrounding the single conserved tryptophan residue of OspA (at residue 216, or in some cases 217), as determined by a moving window population analysis of OspA from fifteen European and North American isolates of *Borrelia.* The hypervariable region is important for immune recognition.

Site-directed mutagenesis was also conducted to examine the hypervariable regions more closely. Fluorescence and circular dichroism spectroscopy have indicated that the conserved tryptophan is part of an alpha-helical region in which the tryptophan is buried in a hydrophobic environment (McGrath, B.C., et al., Vaccines, Cold Spring Harbor Laboratory Press, Plainview, New York; pp. 365-370 (1993)). More polar amino acid side-chains flanking the tryptophan are likely to be exposed to the hydrophilic solvent. The hypervariability of these solvent-exposed residues among the various strains *of Borrelia* suggested that these amino acid residues may contribute to the antigenic variation in OspA. Therefore, site-directed mutagenesis was performed to replace some of the potentially exposed amino acid side chains in the protein from one strain with the analogous residues of a second strain. The altered proteins were then analyzed by Western Blot using monoclonal antibodies which bind OspA on the surface of the intact, non-mutated spirochete. The results indicated that certain specific amino acid changes near the tryptophan can abolish reactivity of OspA to these monoclonal antibodies.

### A. Verification of Clustered Polymorphisms in Outer Surface Protein A Sequences

Cloning and sequencing of the OspA protein from fifteen European and North American isolates (described above in Table I) demonstrated that amino acid polymorphism is not randomly distributed throughout the protein; rather, polymorphism tended to be clustered in three regions of OspA. The analysis was carried out by plotting the moving, weighted average polymorphism of a window (a fixed length subsection of the total sequence) as it is slid along the sequence. The window size in this analysis was thirteen amino acids, based upon the determination of the largest number of significantly deviating points as established by the method of Tajima (J. Mol. Evol. 33: 470-473 (1991)). The average weighted polymorphism was calculated by summing the number of variant alleles for each site. Polymorphism calculations were weighted by the severity of amino acid replacement (Dayhoff, M.O. et al., in: Dayhoff, M.O. (ed.) Atlas of Protein Sequence and Structure NBRF, Washington, Vol. 5, Suppl. 3: 345 (1978)). The sum was normalized by the window size and plotted. The amino acid sequence position corresponds to a window that encompasses amino acids 1 through 13. Bootstrap resampling was used to generate 95% confidence intervals on the sliding window analysis. Since *Borrelia* has been shown to be clonal, the bootstrap analysis should give a reliable estimate of the expected variance from polymorphism calculations. The bootstrap was iterated five hundred times at each position, and the mean was calculated from the sum of all positions. The clonal nature *of Borrelia* ensures that the stochastic variance that results from differing genealogical histories of the sequence positions (as would be expected if recombination were prevalent) will be minimized.

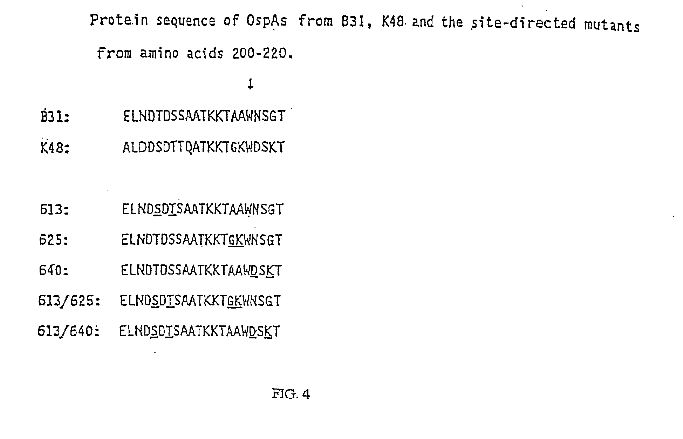

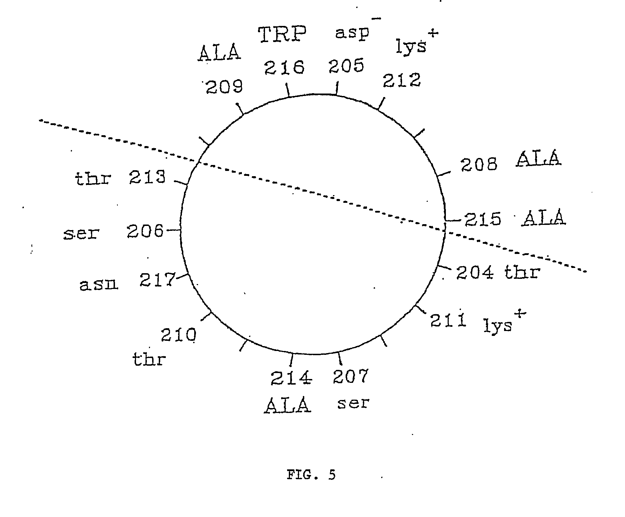

This test verified that the three regions around the observed peaks all have significant excesses of polymorphism. Excesses of polymorphism were observed in the regions including amino acid residues 132-145, residues 163-177, and residues 208-221 (Fig. 3). An amino acid alignment between residues 200 and 220 for B31, K48 and the four site-directed mutants is shown in Fig. 4. The amino acid 208-221 region includes the region of OspA which has been modeled as an oriented alpha-helix in which the single tryptophan residue at amino acid 216 is buried in a hydrophobic pocket, thereby exposing more polar amino acids to the solvent (Fig. 5) (France, L.L., et al., Biochem. Biophys. Acta 1120: 59 (1992)). These potentially solvent-exposed residues showed considerable variability among the OspAs from various strains and may be an important component of OspA antigenic variation. For the purposes of generating chimeric proteins, the hypervariable domains of interest are Domain A, which includes amino acid residues 120-140 of OspA; Domain B, which includes residues 150-180; and Domain C, which includes residues 200-216 or 217.

### B. Site-Directed Mutagenesis of the Hypervariable Region

Site-directed mutagenesis was performed to convert residues within the 204-219 domain of the recombinant B31 OspA to the analogous residues of a European OspA variant, K48. In the region of OspA between residues 204 and 219, which includes the helical domain (amino acids 204-217), there are seven amino acid differences between OspA-B31 and OspA-K48. Three oligonucleotides were generated, each containing nucleotide changes which would incorporate K48 amino acids at their analogous positions in the B31 OspA protein. The oligos used to create the site-directed mutants were:
5'-CTTAATGACTCTGACACTAGTGC-3' (#613, which converts serine at position 204 to threonine, and serine at 206 to threonine (Ser204-Thr, Ser206-Thr)) (SEQ ID NO. 1);
5'-GCTACTAAAAAAACCGGGAAATGGAATTCA-3' (#625, which converts alanine at 214 to glycine, and alanine at 215 to lysine (Ala214-Gly, A1a215-Lys)) (SEQ ID NO. 2); and
5'-GCAGCTTGGGATTCAAAAACATCCACTTTAACA-3' (#640, which converts asparagine at 217 to aspartate, and glycine at 219 to lysine (Asn217-Asp, Gly219-Lys)) (SEQ ID NO. 3).

Site-directed mutagenesis was carried out by performing mutagenesis with pairs of the above oligos. Three site-directed mutants were created, each with two changes: OspA 613 (Ser204-Thr, Ser206-Thr), OspA 625 (Ala214-Gly, Ala215-Lys), and 640 (Asn217-Asp, Gly219-Lys). There were also two proteins with four changes: OspA 613/625 (Ser204-Thr, Ser206-Thr, Ala214-Gly, Ala215-Lys) and OspA 613/640 (Ser204-Thr, Ser206-Thr, Asn217-Asp, Gly219-Lys).

### Specificity of Antibody Binding to Epitopes of the Non-mutated Hypervariable Region

Monoclonal antibodies that agglutinate spirochetes, including several which are neutralizing *in vitro,* recognize epitopes that map to the hypervariable region around Trp216 (Barbour, A.G. et al., Infect. and Immun. 41: 759 (1983); Schubach, W.H. et al., Infect. and Immun. 59: 1911 (1991)). Western Blot analysis demonstrated that chemical cleavage of OspA from the B31 strain at Trp 216 abolishes reactivity of the protein with the agglutinating MAb 105, a monoclonal raised against B31 spirochetes (data not shown). The reagent, n-chlorosuccinimide (NCS), cleaves OspA at the Trp 216, forming a 23.2kd fragment and a 6.2kd peptide which is not retained on the Imobilon-P membrane after transfer. The uncleaved material binds MAb 105; however, the 23.2kd fragment is unreactive. Similar Western blots with a TrpE-OspA fusion protein containing the carboxy-terminal portion of the OspA protein demonstrated that the small 6.2kd piece also fails to bind MAb 105 (Schubach, W.H. et al., Infect. and Immun. 59: 1911 (1991)).

Monoclonal antibodies H5332 and H3TS (Barbour, A.G. et al., Infect. and Immun. 41: 759 (1983)) have been shown by immunofluorescence to decorate the surface of fixed spirochetes (Wilske, B. et al., World J. Microbiol. 7: 130 (1991)). These monoclonals also inhibit the growth of the organism in culture. Epitope mapping with fusion proteins has confirmed that the epitopes which bind these MAbs are conformationally determined and reside in the carboxy half of the protein. MAb H5332 is cross-reactive among all of the known phylogenetic groups, whereas MAb H3TS and MAb 105 seem to be specific to the B31 strain to which they were raised. Like MAb 105, the reactivities of H5332 and H3TS to OspA are abrogated by fragmentation of the protein at Trp216 (data not shown). MAb 336 was raised to whole spirochetes of the strain PGau. It cross-reacts to OspA from group 1 (the group to which B31 belongs) but not to group 2 (of which K48 is a member). Previous studies using fusion proteins and chemical cleavage have indicated that this antibody recognizes a domain of OspA in the region between residues 217 and 273 (data not shown). All of these MAbs will agglutinate the B31 spirochete.

### Western Blot Analysis of Antibody Binding to Mutated Hypervariable Regions

MAbs were used for Western Blot analysis of the site-directed OspA mutants induced in *E. coli* using the T7 expression system (Dunn, J.J. et al., Protein Expression and Purification 1: 159 (1990)). *E. coli* cells carrying pET9c plasmids having a site-directed OspA mutant insert were induced at mid-log phase growth with IPTG for four hours at 37°C. Cell lysates were made by boiling an aliquot of the induced cultures in SDS gel loading dye, and this material was then loaded onto a 12% SDS gel (BioRad mini-Protean II), and electrophoresed. The proteins were then transferred to Imobilon-P membranes (Millipore) 70V, 2 hour at 4°C using the BioRad mini transfer system. Western analysis was carried out as described by Schubach et al. (Infect. Immun. 59: 1911 (1991)).

Western Blot analysis indicated that only the 625 mutant (A1a214-Gly and Ala215-Lys) retained binding to the agglutinating monoclonal H3TS antibody (data not shown). However, the 613/625 mutant which has additional alterations to the amino terminus of Trp216 (Ser204-Thr and Ser206-Thr) did not bind this monoclonal antibody. Both 640 and 613/640 OspAs which have the Asn217-Asp and Gly219-Lys changes on the carboxy-terminal side of Trp216 also failed to bind MAb H3TS. This indicated that the epitope of the B31 OspA which binds MAb H3TS is comprised of amino acid side-chains on both sides of Trp216.

The 613/625 mutant failed to bind MAbs 105 and H5332, while the other mutants retained their ability to bind these MAbs. This is important in light of the data using fusion proteins that indicate that MAb 105 behaves more like MAb H3TS in terms of its serotype specificity and binding to OspA (Wilske, B. et al., Med. Microbiol. Immunol. 181: 191 (1992)). The 613/625 protein has, in addition to the differences at residues Ser204 and Ser206, changes immediately amino-terminal to Trp216 (Ala214-Gly and Ala215-Lys). The abrogation of reactivity of MAbs 105 and H5332 to this protein indicated that the epitopes of OspA which bind these monoclonals are comprised of residues on the amino-terminal side of Trp216.

The two proteins carrying the Asn217-Asp and Gly219-Lys replacements on the carboxy-terminal side of Trp216 (OspAs 640 and 613/640) retained binding to MAbs 105 and H5332; however, they failed to react with MAb 336, a monoclonal which has been mapped with TrpE-OspA fusion proteins and by chemical cleavage to a more carboxy-terminal domain. This result may explain why MAb 336 failed to recognize the K48-type of OspA (Group 2).

It is clear that amino acids Ser204 and Ser206 play an important part in the agglutinating epitopes in the region of the B31 OspA flanking Trp216. Replacement of these two residues altered the epitopes of OspA that bind MAbs 105, H3TS and H5332. The ability of the 640 changes alone to abolish reactivity of MAb 336 indicated that Ser204 and Ser206 are not involved in direct interaction with MAb 336.

The results indicated that the epitopes of OspA which are available to MAbs that agglutinate spirochetes are comprised at least in part by amino acids in the immediate vicinity of Trp216. Since recent circular dichroism analysis indicated that the structures of B31 and K48 OspA differ very little within this domain, it is unlikely that the changes made by mutation have radically altered the overall structure of the OspA protein (France, L.L. et al., Biochem. Biophys. Acta 1120: 59 (1992); and France et al., Biochem. Biophys Acta, submitted (1993)). This hypothesis is supported by the finding that the recombinant, mutant OspAs exhibit the same high solubility and purification properties as the parent B31 protein (data not shown).

In summary, amino acid side-chains at Ser204 and Ser206 are important for many of the agglutinating epitopes. However, a limited set of conservative changes at these sites were not sufficient to abolish binding of all of the agglutinating MAbs. These results suggested that the agglutinating epitopes of OspA are distinct, yet may have some overlap. The results also supported the hypothesis that the surface-exposed epitope around Trp216 which is thought to be important for immune recognition and neutralization is a conformationally-determined and complex domain of OspA.

### EXAMPLE 3. Borrelia Strains and Proteins

Proteins and genes from any strain *of Borrelia* can be utilized in the current invention. Representative strains are summarized in Table I, above.

### A. Genes Encoding Borrelia Proteins

The chimeric peptides of the current invention can comprise peptides derived from any *Borrelia* proteins. Representative proteins include OspA, OspB, OspC, OspD, p12, p39, p41 (fla), p66, and p93. Nucleic acid sequences encoding several *Borrelia* proteins are presently available (see Table II, below); alternatively, nucleic acid sequences encoding *Borrelia* proteins can be isolated and characterized using methods such as those described below.

**Table II. References for Nucleic Acid Sequences for Several Proteins of Various Borrelia Strains**

| Strain | p93 | OspA | p41 (fla) |
|---|---|---|---|
| K48 | X69602 (SID 67) | X62624 (SID 8) | X69610 |
| PGau | SID 73 | X62387 (SID 10) | X69612 (SID 51) |
| DK29 | ND | X63412 (SID 49) | X69608 (SID 53) |
| PKo | X69803 (SID 77) | X65599 (SID 57) | X69613 (SID 131) |
| PTrob | X69604 (SID 71) | X65598 (SID 135) | X69614 |
| Ip3 | ND | X70365 (SID 56) | ND |
| Ip90 | ND | Kryuchechnikov, V.N. et al., J.Microbiol. Epid. Immunobiol. 12: 41-44 (1988) (SID 50) | ND |
| 25015 | X70365 (SID 75) | Fikrig, E.S. et al., J. Immunol. 7:2256-2260 (1992) (SID 12) | ND |
| B31 | Perng, G.C. et al., Infect. Immun. 59:2070-74 (1992); Luft, B.J. et al., Infect. Immun. 60:4309-4321 (1992) (SID 65) | Bergstrom, S. et al., Mol. Microbiol. 3: 479-486 (1989) (SID 6) | Gassmann, G.S. et al., Nucl. Acids Res. 17: 3590 (1989) (SID 127) |
| PKa1 | ND | X69606 (SID 132) | X69611 (SID 129) |
| ZS7 | ND | Jonsson, M. et al., Infect. Immun. 60:1845-1853 (1992) (SID 134) | ND |
| N40 | ND | Kryuchechnikov, V.N. *et al.* (SID 133) | ND |
| PHei | ND | X65600 (SID 136) | ND |
| ACAI | ND | Kryuchechnikov, V.N. *et al.* (SID 58) | ND |
| PBo | X69601 (SID 69) | X65605 (SID 55) | X69610 (SID 130) |

| | | | |
|---|---|---|---|
| Numbers with an "X" prefix are GenBank data base accession numbers. SID = SEQ ID NO. | | | |

### B. Isolation of Borrelia Genes

Nucleic acid sequences encoding full length, lipidated proteins from known *Borrelia* strains were isolated using the polymerase chain reaction (PCR) as described below. In addition, nucleic acid sequences were generated which encoded truncated proteins (proteins in which the lipidation signal has been removed, such as by eliminating the nucleic acid sequence encoding the first 18 amino acids, resulting in non-lipidated proteins). Other proteins were generated which encoded polypeptides of a particular gene (i.e., encoding a segment of the protein which has a different number of amino acids than the protein does in nature). Using similar methods as those described below, primers can be generated from known nucleic acid sequences encoding *Borrelia* proteins and used to isolate other genes encoding *Borrelia* proteins. Primers can be designed to amplify all of a gene, as well as to amplify a nucleic acid sequence encoding truncated protein sequences, such as described below for OspC, or nucleic acid sequences encoding a polypeptide derived from a *Borrelia* protein. Primers can also be designed to incorporate unique restriction enzyme cleavage sites into the amplified nucleic acid sequences. Sequence analysis of the amplified nucleic acid sequences can then be performed using standard techniques.

### Cloning and Sequencing of OspA Genes and Relevant Nucleic Acid Sequences

*Borrelia* OspA sequences were isolated in the following manner: 100 µl reaction mixtures containing 50 mM KCl, 10 mM TRIS-HCl (pH 8,3), 1.5 mM MgCl₂, 200 µM each NTP, 2.5 units of TaqI DNA polymerase (Amplitaq, Perkin-Elmer/Cetus) and 100 pmol each of the 5' and 3' primers (described below) were used. Amplification was performed in a Perkin-Elmer/Cetus thermal cycler as described (Schubach, W.H, et al., Infect. Immun. 59:1811-1915 (1991)). The amplicon was visualized on an agarose gel by ethidium bromide staining. Twenty nanograms of the chloroform-extracted PCR product were cloned directly into the PC-TA vector (Invitrogen) by following the manufacturer's instructions. Recombinant colonies containing the amplified fragment were selected, the plasmids were prepared, and the nucleic acid sequence of each OspA was determined by the dideoxy chain-termination technique using the Sequenase kit (United States Biochemical). Directed sequencing was performed with M13 primers followed by OspA-specific primers derived from sequences, previously obtained with M13 primers.

Because the 5' and 3' ends of the OspA gene are highly conserved (Fikrig, E.S. et al., J. Immunol. 7: 2256-2260 (1992); Bergstrom, S. et al., Mol. Microbiol. 3: 479-486 (1989); Zumstein, G. et al., Med. Microbiol. Immunol. 181: 57-70 (1992)), the 5' and 3' primers for cloning can be based upon any known OspA sequences. For example, the following primers based upon the OspA nucleic acid sequence from strain B31 were used:
5'-GGAGAATATATTATGAAA-3' (-12 to +6) (SEQ ID NO. 4); and
5'-CTCCTTATTTTAAAGCG-3' (+826 to +809) (SEQ ID NO. 5). (Schubach, W.H. et al., Infect. Immun 59: 1811-1915 (1991)).

OspA genes isolated in this manner include those for strains B31, K48, PGau, and 25015; the nucleic acid sequences are depicted in the sequence listing as SEQ ID NO. 6 (OspA-B31), SEQ ID NO. 8 (OspA-K48), SEQ ID NO. 10 (OspA-PGau), and SEQ ID NO. 12 (OspA-25015). An alignment of these and other OspA nucleic acid sequences is shown in Fig. 42. The amino acid sequences of the proteins encoded by these nucleic acid sequences are represented as SEQ ID NO. 7 (OspA-B31), SEQ ID NO. 9 (OspA-K48), SEQ ID NO. 11 (OspA-PGau), and SEQ ID NO. 13 (OspA-25015).

The following primers were used to generate specific nucleic acid sequences of the OspA gene, to be used to generate chimeric nucleic acid sequences (as described in Example 4):
5'-GTCTGCAAAAACCATGACAAG-3' (plus strand primer #369) (SEQ ID NO. 14);
5'-GTCATCAACAGAAGAAAAATTC-3' (plus strand primer #357) (SEQ ID NO 15);
5'-CCGGATCCATATGAAAAAATATTTATTGGG-3' (plus strand primer #607) (SEQ ID NO. 16);
5'-CCGGGATCCATATGGCTAAGCAAAATGTTAGC-3' (plus strand primer #584) (SEQ ID NO. 17);
5'-GCGTTCAAGTACTCCAGA-3' (minus strand primer #200) (SEQ ID NO. 18);
5'-GATATCTAGATCTTATTTTAAAGCGTT-3' (minus strand primer #586) (SEQ ID NO. 19); and
5'-GGATCCGGTGACCTTTTAAAGCGTTTTTAAT-3' (minus strand primer #1169) (SEQ ID NO. 20).

### Cloning and Sequencing of OspB

Similar methods were also used to isolate OspB genes. One OspB genes isolated is represented as SEQ ID NO. 21 (OspB-B31); its encoded amino acid sequence is SEQ ID NO. 22.

The following primers were used to generate specific nucleic acid sequences of the OspB gene, to be used in generation of chimeric nucleic acid sequences (see Example 4):
5'-GGTACAATTACAGTACAA-3' (plus strand primer #721) (SEQ ID NO. 23);
5'-CCGAGAATCTCATATGGCACAAAAAGGTGCTGAGTCAATTGG-3' (plus strand primer #1105) (SEQ ID NO. 24);
5'-CCGATATCGGATCCTATTTTAAAGCGTTTTTAAGC-3' (minus strand primer # 1106) (SEQ ID NO. 25); and
5'-GGATCCGGTGACCTTTTAAAGCGTTTTTAAG-3' (minus strand primer #1170) (SEQ ID NO. 26).

### Cloning and Sequencing of OspC

Similar methods were also used to isolate OspC genes. The following primers were used to isolate entire OspC genes from *Borrelia* strains B31, K48, PKo, and PTrob:
5'-GTGCGCGACCATATGAAAAAGAATACATTAAGTGCG-3' (plus strand primer having Nde1 site combined with start codon) (SEQ ID NO. 27), and
5'-GTCGGCGGATCCTTAAGGTTTTTTTGGACTTTCTGC-3' (minus strand primer having BamH1 site followed by stop codon) (SEQ ID NO. 28).

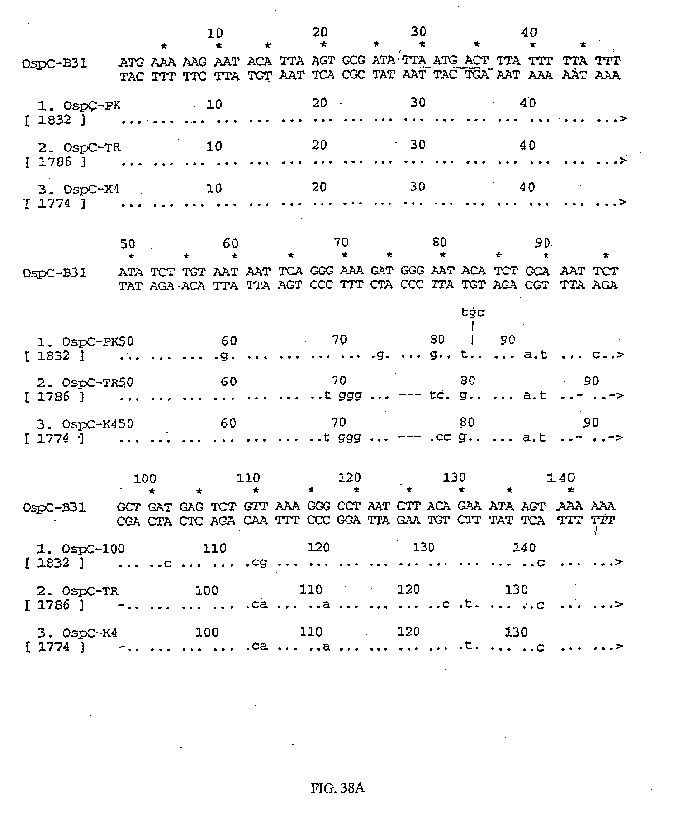

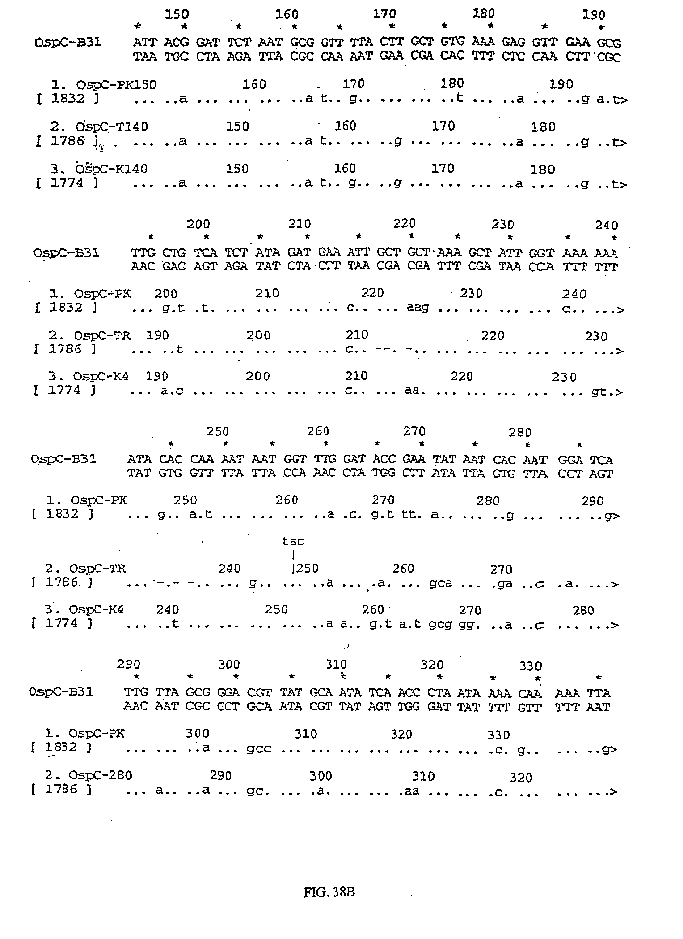

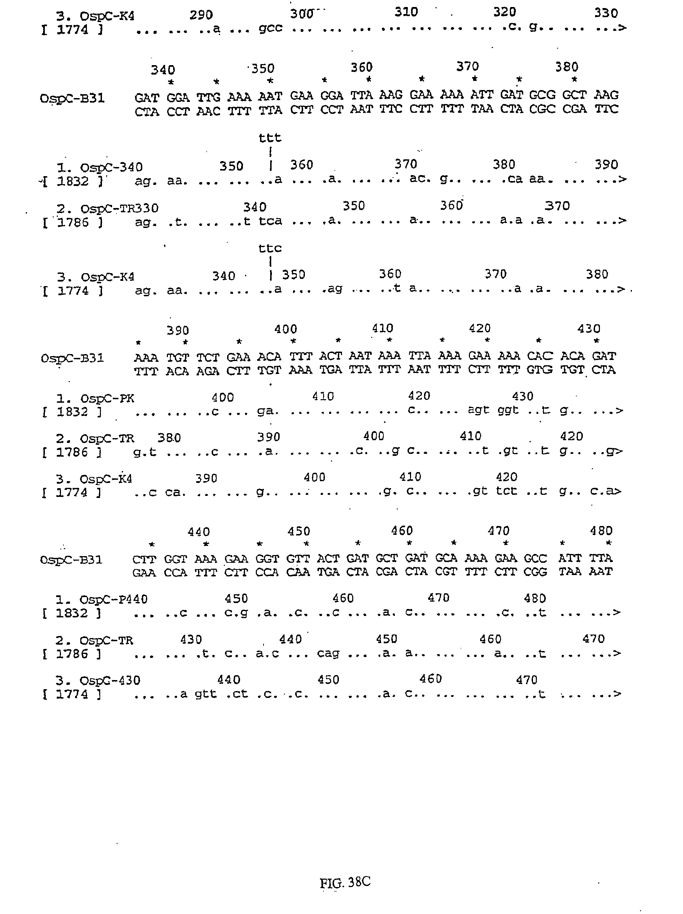

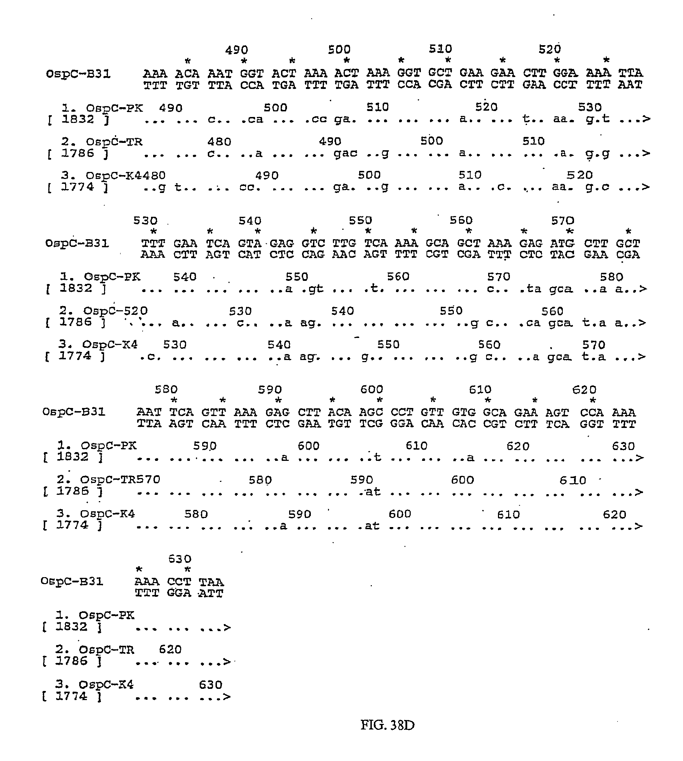

The nucleic acid sequences of the OspC genes were then determined by the dideoxy chain-termination technique using the Sequenase kit (United States Biochemical). OspC genes isolated and sequenced in this manner include those for strains B31, K48, PKo, and Tro; the nucleic acid sequences are depicted in the sequence listing as SEQ ID NO. 29 (OspC-B31), SEQ ID NO. 31 (OspC-K48), SEQ ID NO. 33 (OspC-PKo), and SEQ ID NO. 35 (OspC-Tro). An alignment of these sequences is shown in Fig. 38. The amino acid sequences of the proteins encoded by these nucleic acid sequences are represented as SEQ ID NO. 30 (OspC-B31), SEQ ID NO. 32 (OspC-K48), SEQ ID NO. 34 (OspC-PKo), and SEQ ID NO. 36 (OspC-Tro).

Truncated OspC genes were generated using other primers. These primers were designed to amplify nucleic acid sequences, derived from the OspC gene, that lacked the nucleic acids encoding the signal peptidase sequence of the full-length protein. The primers corresponded to bp 58-75 of the natural protein, with codons for Met-Ala attached ahead. For strain B31, the following primer was used:
5'-GTGCGCGACCATATGGCTAATAATTCAGGGAAAGAT-3' (SEQ ID NO. 37).

For strain PKo,
5'-GTGCGCGACCATATGGCTAGTAATTCAGGGAAAGGT-3' (SEQ ID NO. 38) was used.

For strains PTrob and K48,
5'-GTGCGCGACCATATGGCTAATAATTCAGGTGGGGAT-3' (SEQ ID NO. 39) was used.

Additional primers were also designed to amplify nucleic acids encoding particular polypeptides, for use in creation of chimeric nucleic acid sequences (see Example 4). These primers included:
5'-CTTGGAAAATTATTTGAA-3' (plus strand primer #520) (SEQ ID NO. 40);
5'-CACGGTCACCCCATGGGAAATAATTCAGGGAAAGG-3' (plus strand primer #58) (SEQ ID NO. 41);
5'-TATAGATGACAGCAACGC-3' (minus strand primer #207) (SEQ ID NO. 42); and
5'-CCGGTGACCCCATGGTACCAGGTTTTTTTGGACTTTCTGC-3'(minus strand primer #636) (SEQ ID NO. 43).

### Cloning and Sequencing of OspD

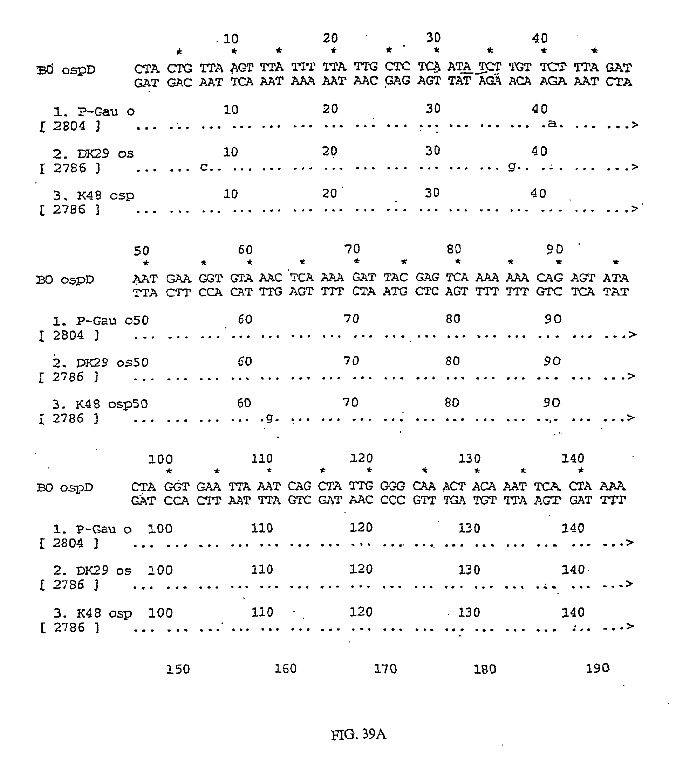

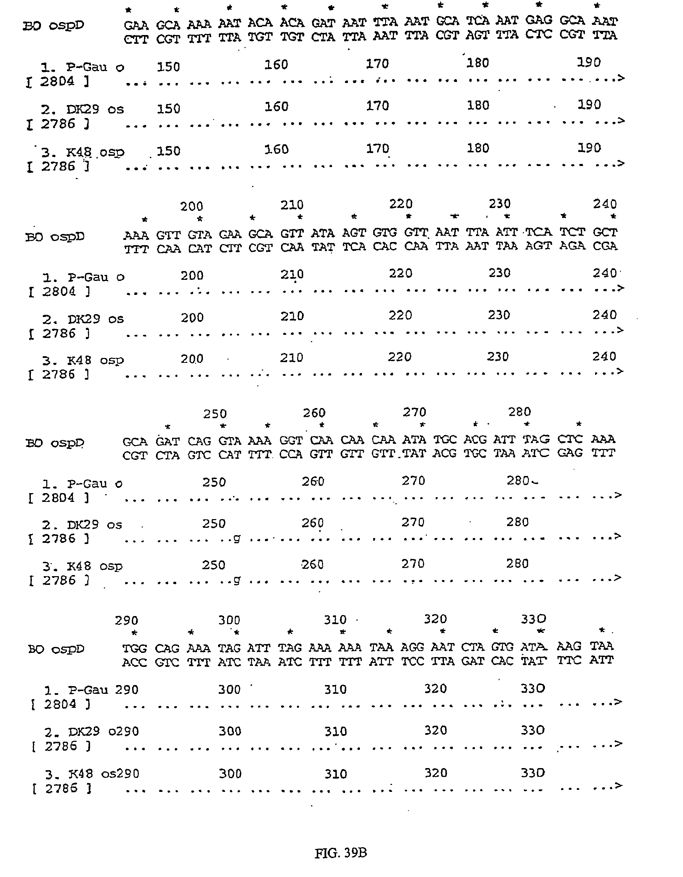

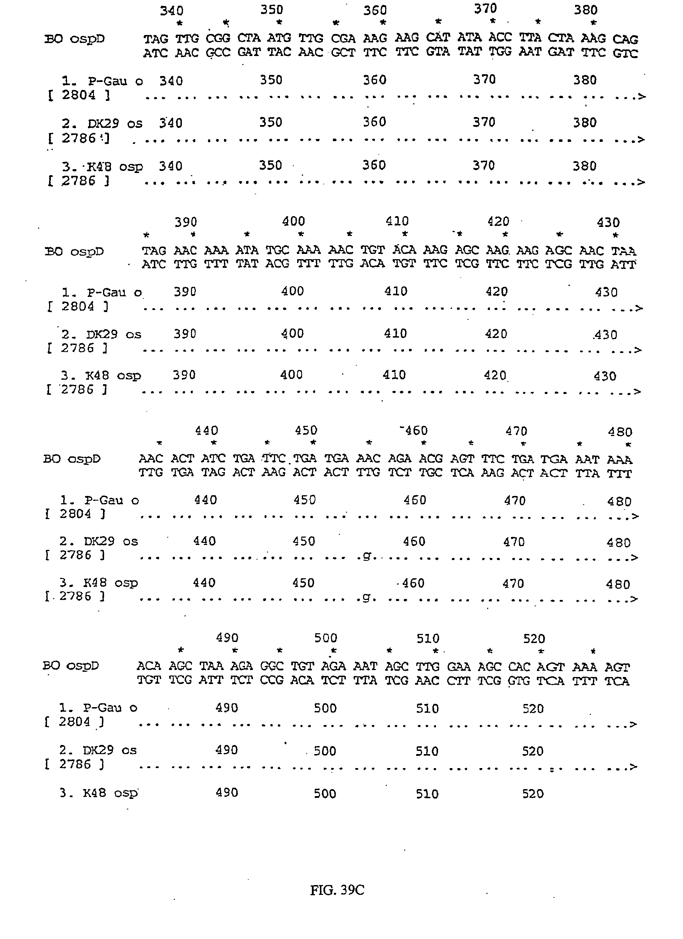

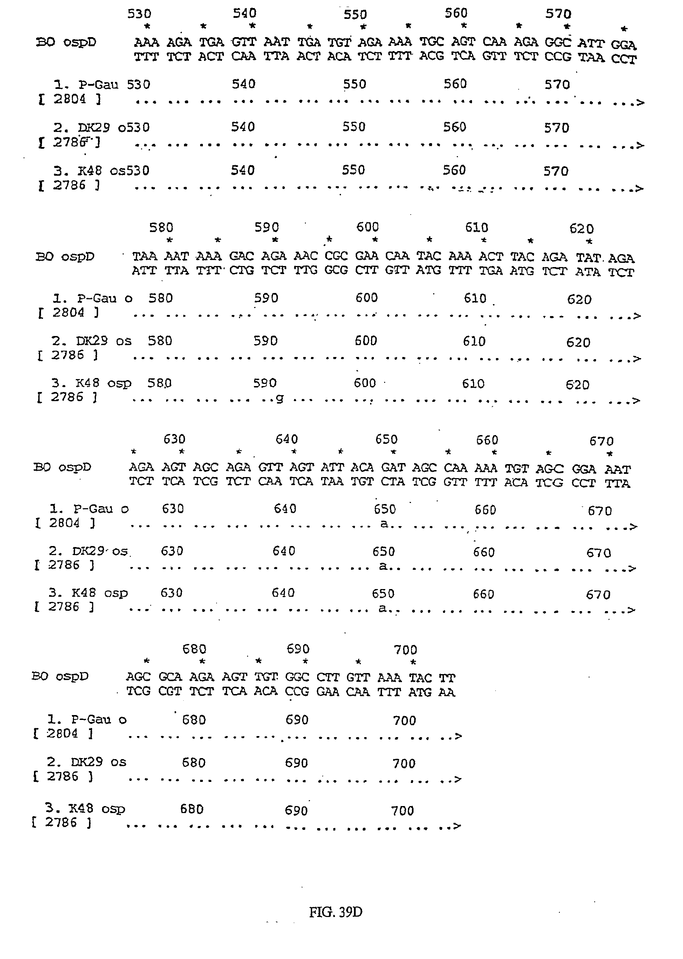

Similar methods can be used to isolate OspD genes. An alignment of four OspD nucleic acid sequences (from strains PBo, PGau, DK29, and K48) is shown in Fig. 39.

### Cloning and Sequencing of p12

The p12 gene was similarly identified. Primers used to clone the entire p 12 gene included: 5'-CCGGATCCATATGGTTAAAAAAATAATATTTATTTC-3' (forward primer # 757) (SEQ ID NO. 44); and 5'-GATATCTAGATCTTTAATTGCTCTGCTCACTCTCTTC-3' (reverse primer #758) (SEQ ID NO. 45).

To amplify a truncated p12 gene (one in which the transcribed protein is non-lipidated, and begins at amino acid 18 of the native sequence), the following primers were used: 5'-CCGGGATCCATATGGCTAGTGCAATTGGTCGTGG-3' (forward primer # 759) (SEQ ID NO. 46); and primer #758 (SEQ ID NO. 45).

### Cloning and Sequencing of p41 (fla)

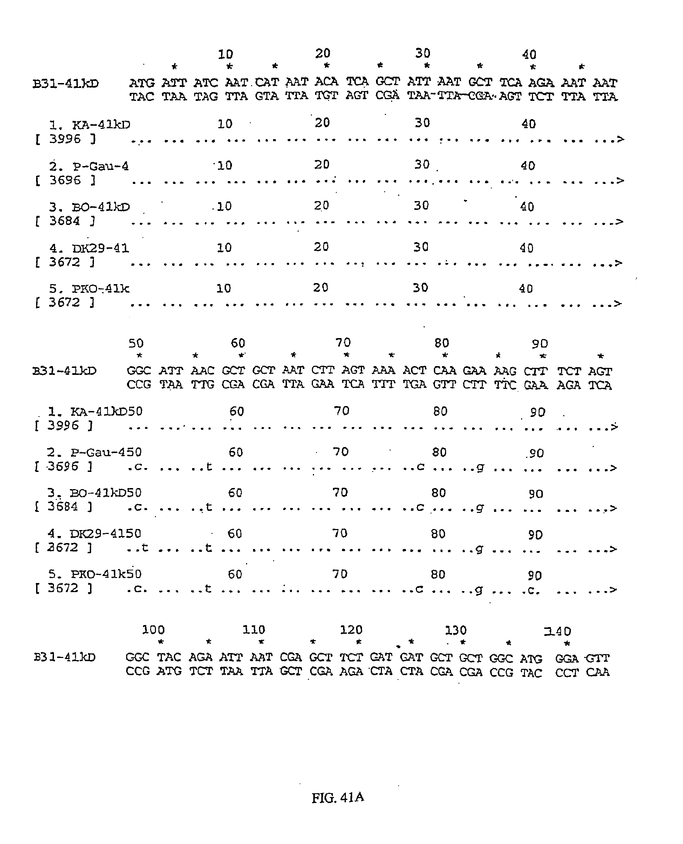

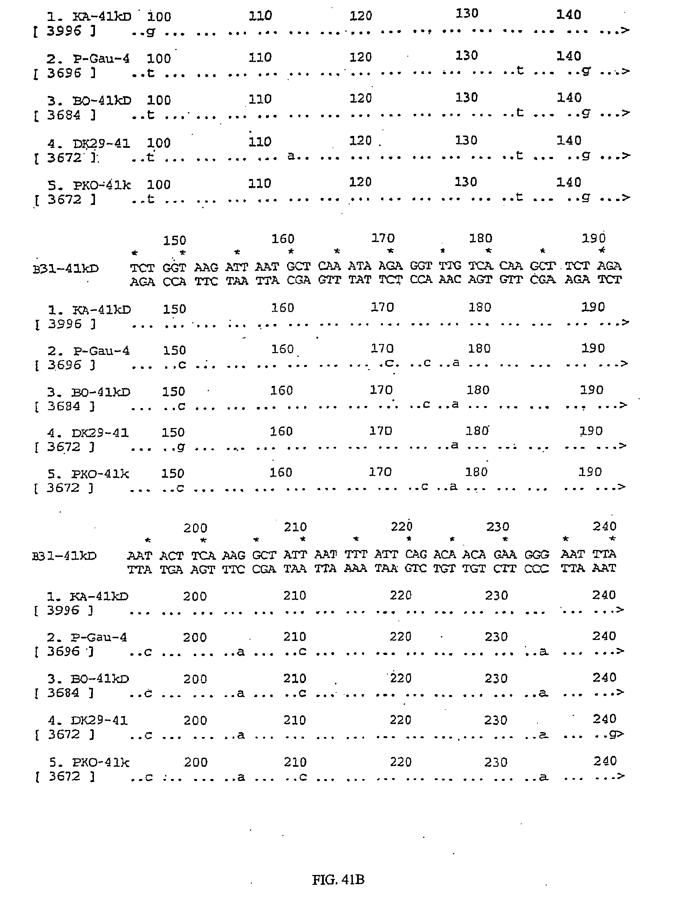

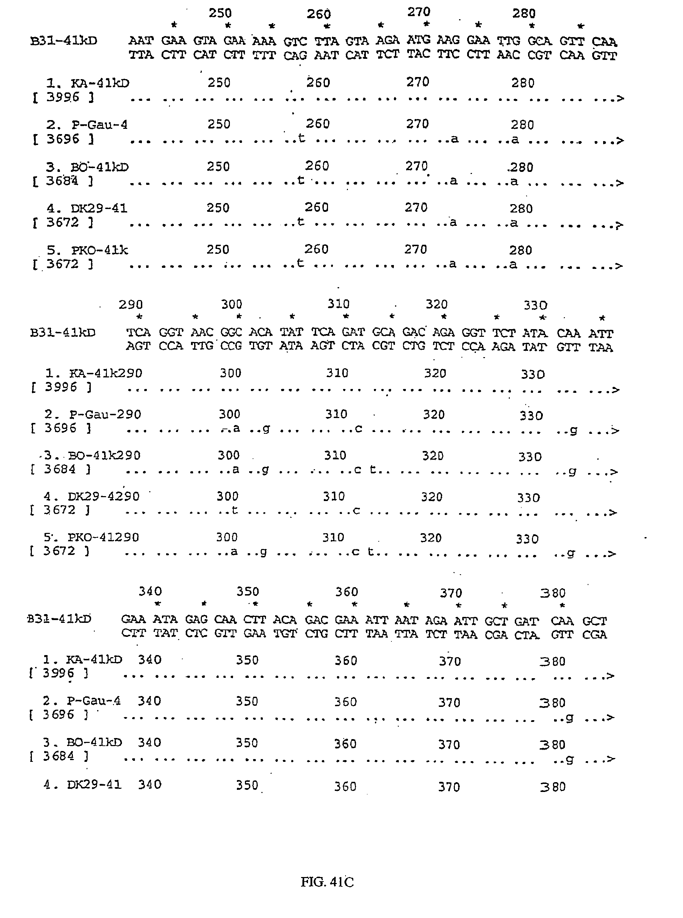

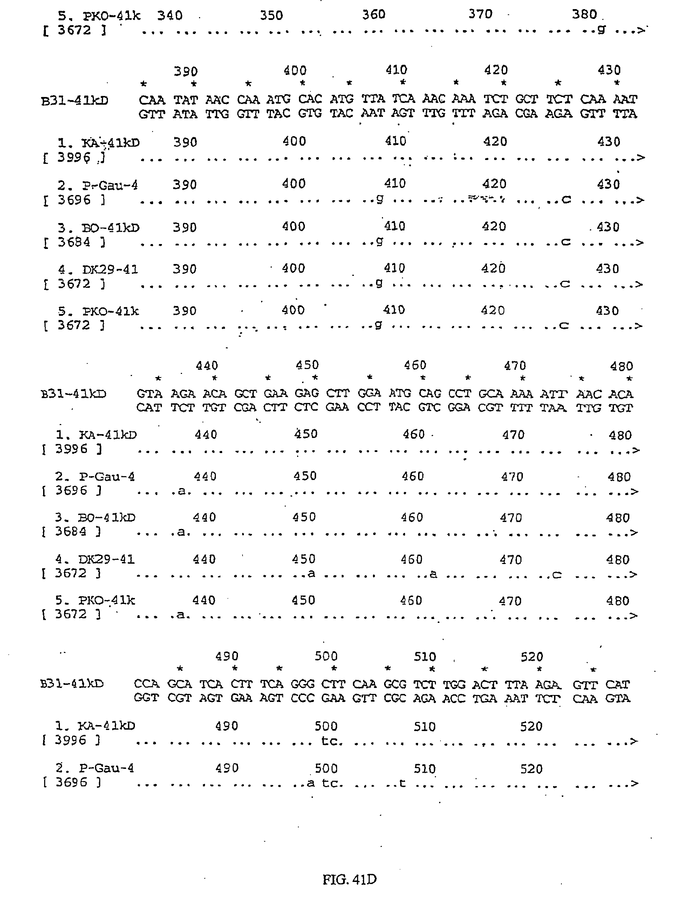

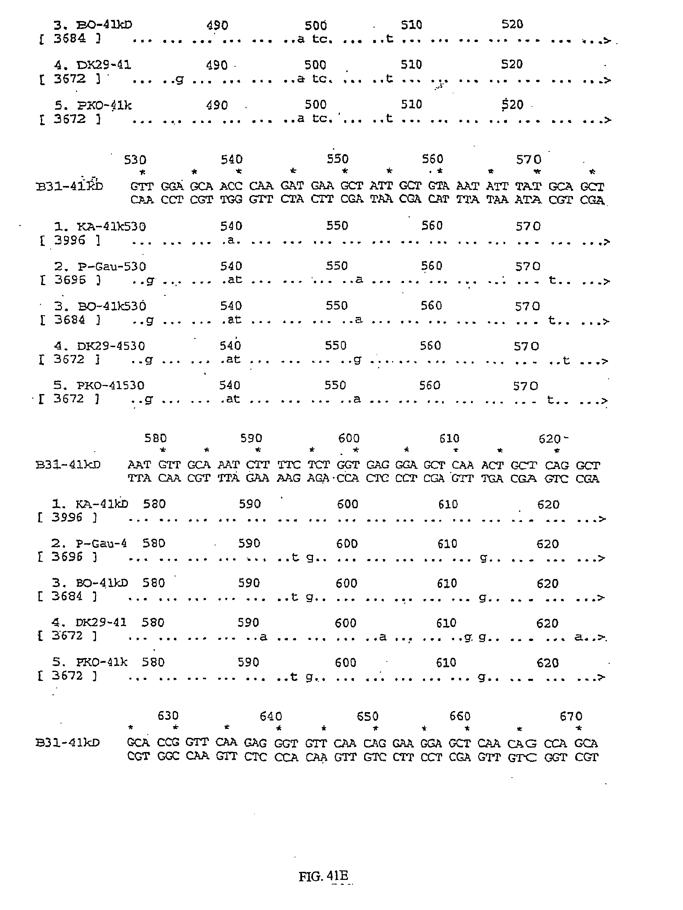

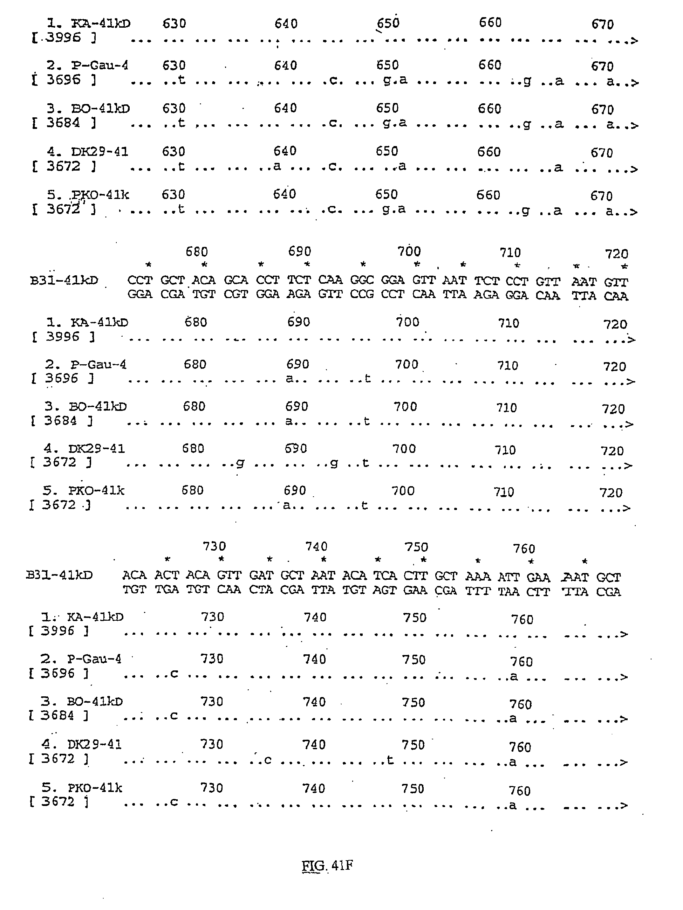

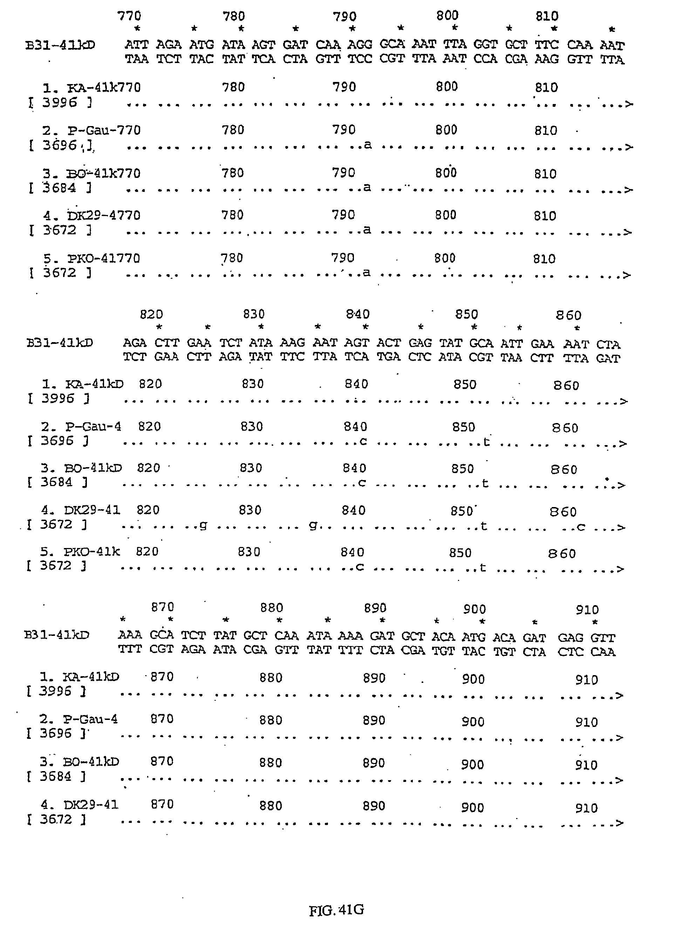

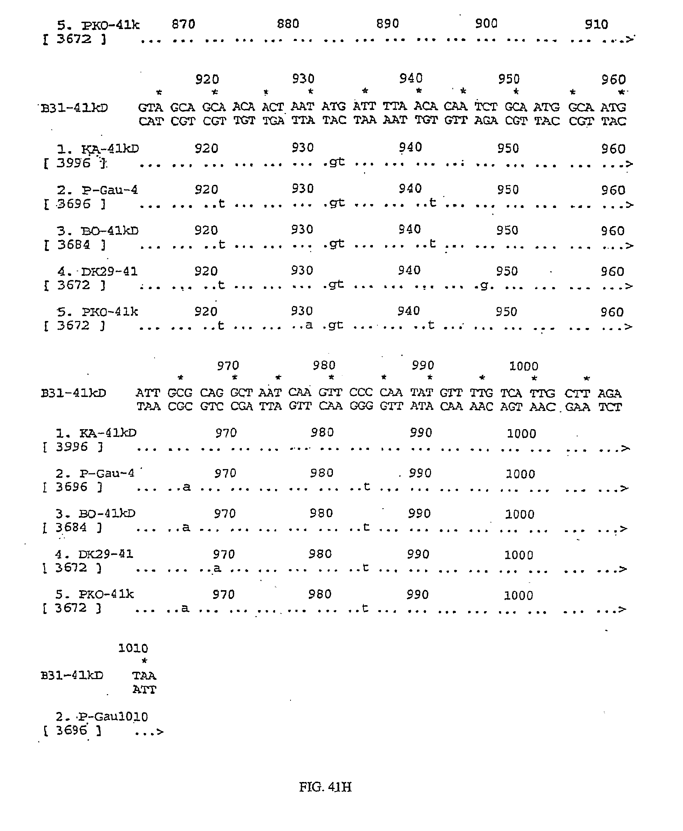

A similar approach was used to clone and sequence genes encoding the p41 (fla) protein. The p41 sequences listed in Table II with GenBank accession numbers were isolated using the following primers from strain B31:
5'-ATGATTATCAATCATAAT-3' (+1 to +18) (SEQ ID NO. 47); and 5'-TCTGAACAATGACAAA.AC-3' (+1008 to +991) (SEQ ID NO. 48). The nucleic acid sequences of p41 isolated in this manner are depicted in the sequence listing as SEQ ID NO. 51 (p41-PGau), and SEQ ID NO. 53 (p41-DK29). An alignment of several p41 nucleic acid sequences, including those for strains B31, PKa1, PGau, PBo, DK29, and PKo, is shown in Fig. 41. The amino acid sequences of the proteins encoded by these nucleic acid sequences are represented as SEQ ID NO. 52 (p41-PGau) and SEQ ID NO. 54 (p41-DK29).

Other primers were designed to amplify nucleic acid sequences encoding polypeptides of p41, to be used in chimeric nucleic acid sequences. These primers included:
5'-TTGGATCCGGTCACCCCATGGCTCAATATAACCAATG-3' (minus strand primer #122) (SEQ ID NO. 59);
5'-TTGGATCCGGTCACCCCATGGCTTCTCAAAATGTAAG-3' (plus strand primer # 140) (SEQ ID NO. 60);
5'-TTGGATCCGGTGACCAACTCCGCCTTGAGAAGG-3' (minus strand primer # 234) (SEQ ID NO. 61); and
5'-TTGGATCCGGTGACCTATTTGAGCATAAGATGC-3' (minus strand primer #141) (SEQ ID NO. 62).

### Cloning and Sequencing of p93

The same approach was also used to clone and sequence p93 proteins. Genes encoding p93, as listed in Table II with GenBank accession numbers, were isolated by this method with the following primers from strain B31:
5'-GGTGAATTTAGTTGGTAAGG-3' (-54 to -35) (SEQ ID NO. 63); and
5'-CACCAGTTTCTTTAAGCTGCTCCTGC-3' (+1117 to +1092) (SEQ ID NO. 64).

The nucleic acid sequences of p93 isolated in this manner are depicted in the sequence listing as SEQ ID NO. 65 (p93-B31), SEQ ID NO. 67 (p93-K48) SEQ ID NO. 69 (p93-PBo), SEQ ID NO. 71 (p93-PTrob), SEQ ID NO. 73 (p93-PGau), SEQ ID NO. 77 (p93-25015), and SEQ ID NO. 75 (p93-PKo). The amino acid sequences of the proteins encoded by these nucleic acid sequences are represented as SEQ ID NO. 66 (p93-B31), SEQ ID NO. 68 (p93-K48) SEQ ID NO. 70 (p93-PBo), SEQ ID NO. 72 (p93-PTrob), SEQ ID NO. 74 (p93-PGau), SEQ ID NO. 78 (p93-25015), and SEQ ID NO. 76 (p93-PKo).

Other primers were used to amplify nucleic acid sequences encoding polypeptides of p93 to be used in generating chimeric nucleic acid sequences. These primers included:
5'-CCGGTCACCCCATGGCTGCTTTAAAGTCTTTA-3' (plus strand primer #475) (SEQ ID NO. 79);
5'-CCGGTCACCCCATGAATCTTGATAAAGCTCAG-3' (plus strand primer #900) (SEQ ID NO. 80);
5'-CCGGTCACCCCATGGATGAAAAGCTTTTAAAAAGT-3' (plus strand primer #1168) (SEQ ID NO. 81);
5'-CCGGTCACCCCCATGGTTGAGAAATTAGATAAG-3' (plus strand primer #1423) (SEQ ID NO. 82); and
5'-TTGGATCCGGTGACCCTTAACTTTTTTTAAAG-3' (minus strand primer # 2100) (SEQ ID NO. 83).

### C. Expression of Proteins from Borrelia Genes

The nucleic acid sequences described above can be incorporated into expression plasmids, using standard techniques, and transfected into compatible host cells in order to express the proteins encoded by the nucleic acid sequences. As an example, the expression of the p12 gene and the isolation of p12 protein is set forth.

Amplification of the p12 nucleic acid sequence was conducted with primers that included a NdeI restriction site into the nucleic acid sequence. The PCR product was extracted with phenol/chloroform and precipitated with ethanol. The precipitated product was digested and ligated into an expression plasmid as follows: 15 µl (approximately 1 µg) of PCR DNA was combined with 2 µl 10X restriction buffer for NdeI (Gibco/BRL), 1 µl NdeI (Gibco/BRL), and 2 µl distilled water, and incubated overnight at 37°C. This mixture was subsequently combined with 3 µl 10X buffer (buffer 3, New England BioLabs), 1 µl BamHI (NEB), and 6 µl distilled water, and incubated at 37° for two hours. The resultant material was purified by preparative gel electrophoresis using low melting point agarose, and the band was visualized under long wave ultraviolet light and excised from the gel. The gel slice was treated with Gelase using conditions recommended by the manufacturer (Epicentre Technologies). The resulting DNA pellet was resuspended in 25-50 µl of 10 mM TRIS-CL (pH 8.0) and 1 mM EDTA (TE). An aliquot of this material was ligated into the pET9c expression vector (Dunn, J. J. et al., Protein Expression and Purification 1: 159 (1990)).

To ligate the material into the pET9c expression vector, 20-50 ng of p12 nucleic acid sequences cut and purified as described above was combined with 5 µl 10 One-Phor-All (OPA) buffer (Pharmacia), 30-60 ng pET9c cut with NdeI and BamHI, 2.5 µl 20 mM ATP, 2 µl T4 DNA ligase (Pharmacia) diluted 1:5 in 1X OPA buffer, and sufficient distilled water to bring the final volume to 50 µl. The mixture was incubated at 12°C overnight.

The resultant ligations were transformed into competent DH5-alpha cells and plated on nutrient agar plates containing 50 µg/ml kanamycin and incubated overnight at 37 °C. DH5-alpha is used as a "storage strain" for T7 expression clones, because it is RecA deficient, so that recombination and concatenation are not problematic, and because it lacks the T7 RNA polymerase gene necessary to express the cloned gene. The use of this strain allows for cloning of potentially toxic gene products while minimizing the chance of deletion and/or rearrangement of the desired genes. Other cell lines having similar properties may also be used.

Kanamycin resistant colonies were single-colony purified on nutrient agar plates supplemented with kanamycin at 50 µg/ml. A colony from each isolate was inoculated into 3-5 ml of liquid medium containing 50 µg/ml kanamycin, and incubated at 37°C without agitation. Plasmid DNA was obtained from 1 ml of each isolate using a hot alkaline lysis procedure (Mantiatis, T. et al., Molecular Cloning: A Laboratory Manual, Cold Spring Harbor Laboratory, Cold Spring Harbor, NY (1982)).

Plasmid DNA was digested with EcoRI and BglII in the following manner: 15 µl plasmid DNA was combined with 2 µl 10X buffer 3 (NEB), 1 µl EcoRI (NEB), 1 µl BglII (NEB) and 1 µl distilled water, and incubated for two hours at 37°C. The entire reaction mixture was electrophoresed on an analytical agarose gel. Plasmids carrying the p12 insert were identified by the presence of a band corresponding to 925 base-pairs (full length p12) or 875 base-pairs (nonlipidated p12). One or two plasmid DNAs from the full length and nonlipidated p12 clones in pET9c were used to transform BL21 DE3 pLysS to kanamycin resistance as described by Studier et al. (Methods in Enzymology, Goeddel, D. (Ed.), Academic Press,185: 60-89 (1990)). One or two transformants of the full length and nonlipidated clones were single-colony purified on nutrient plates containing 25 µg/ml chloramphenicol (to maintain pLysS) and 50 µg/ml kanamycin at 37 °C. One colony of each isolate was inoculated into liquid medium supplemented with chloramphenicol and kanamycin and incubated overnight at 37°C. The overnight culture was subcultured the following morning into 500 ml of liquid broth with chloramphenicol (25 µg/ml) and kanamycin (50 µg/ml) and grown with aeration at 37°C in an orbital air-shaker until the absorbance at 600 nm reached 0.4-0.7. Isopropyl-thio-galactoside (IPTG) was added to a final concentration of 0.5 mM, for induction, and the culture was incubated for 3-4 hours at 37° as before. The induced cells were pelleted by centrifugation and resuspended in 25 ml of 20 mM NaPO₄ (pH 7.7). A small aliquot was removed for analysis by gel electrophoresis. Expressing clones produced proteins which migrated at the 12 kDa position.

A crude cell lysate was prepared from the culture as described for recombinant OspA by Dunn, J.J. et al., (Protein Expression and Purification 1: 159 (1990)). The crude lysate was first passed over a Q-sepharose column (Pharmacia) which had been pre-equilibrated in Buffer A: 10 mM NaPO₄ (pH 7.7), 10 mM NaCl, 0.5 mM PMSF. The column was washed with 10 mM NaPO₄, 50 mM NaCl and 0.5 mM PMSF and then p12 was eluted in 10 mM NaPO₄, 0.5 mM PMSF with a NaCl gradient from 50-400 mM. p12 eluted approximately halfway through the gradient between 100 and 200 mM NaCl. The peak fractions were pooled and dialyzed against 10 mM NaPo4 (pH 7.7), 10 mM NaCl, 0.5 mM PMSF. The protein was then concentrated and applied to a Sephadex G50 gel filtration column of approximately 50 ml bed volume (Pharmacia), in 10 mM NaPO₄, 200 mM NaCl, 0.5 mM PMSF. p12 would typically elute shortly after the excluded volume marker. Peak fractions were determined by running small aliquots of all fractions on an SDS gel. The p12 peak was pooled and stored in small aliquots at -20°C.

### Example 4. Generation of Chimeric Nucleic Acid Sequences and Chimeric Proteins

### A. General Protocol for Creation of Chimeric Nucleic Acid Sequences

The megaprimer method of site directed mutagenesis and its modification were used to generate chimeric nucleic acid sequences (Sarkar and Sommer, Biotechniques 8(4): 404-407 (1990); Aiyar, A. and J. Leis, Biotechniques 14(3): 366-369 (1993)). A 5' primer for the first genomic template and a 3' fusion oligo are used to amplify the desired region. The fusion primer consists of a 3' end of the first template (DNA that encodes the amino-proximal polypeptide of the fusion protein), coupled to a 5' end of the second template (DNA that encodes the carboxy-proximal polypeptide of the fusion protein).

The PCR amplifications are performed using Taq DNA polymerase, 10X PCR buffer, and MgCl₂ (Promega Corp., Madison, WI), and Ultrapure dNTPs (Pharmacia, Piscataway, NJ). One µg of genomic template 1, 5 µl of 10 µM 5' oligo and 5 µl of 10 µM fusion oligo are combined with the following reagents at indicated final concentrations: 10X Buffer-Mg FREE (1X), MgCl₂ (2 mM), dNTP mix (200 µM each dNTP), Taq DNA polymerase (2.5 units), water to bring final volume to 100 µl. A Thermal Cycler (Perkin Elmer Cetus, Norwalk, CT) is used to amplify under the following conditions: 35 cycles at 95°C for one minute, 55°C for two minutes, and 72° for three minutes. This procedure results in a "megaprimer".

The resulting megaprimer is run on a 1X TAE, 4% low-melt agarose gel. The megaprimer band is cut from the gel and purified using the Promega Magic PCR Preps DNA purification system. Purified megaprimer is then used in a second PCR step. One µg of genomic template 2, approximately 0.5 µg of the megaprimer, and 5 µl of 10 µM 3' oligo are added to a cocktail of 10X buffer, MgCl₂, dNTPs and Taq at the same final concentrations as noted above, and brought to 100 µl with water. PCR conditions are the same as above. The fusion product resulting from this amplification is also purified using the Promega Magic PCR Preps DNA purification system.

The fusion product is then ligated into TA vector and transformed into *E*. *coli* using the Invitrogen (San Diego, CA) TA Cloning Kit. Approximately 50 ng of PCR fusion product is ligated to 50 ng of pCRII vector with 1X Ligation Buffer, 4 units of T4 ligase, and brought to 10 µl with water. This ligated product mixture is incubated at 12°C overnight (approximately 14 hours). Two µl of the ligation product mixture is added to 50 µl competent INC F' cells and 2 µl beta mercaptoethanol. The cells are then incubated for 30 minutes, followed by heat shock treatment at 42°C for 60 seconds, and an ice quenching for two minutes. 450 µl of warmed SOC media is then added to the cells, resulting in a transformed cell culture which is incubated at 37°C for one hour with slight shaking. 50 µl of the transformed cell culture is plated on LB + 50 µg/µl ampicillin plates and incubated overnight at 37°C. Single white colonies are picked and added to individual overnight cultures containing 3 ml LB with ampicillin (50 µg/µl).

The individual overnight cultures are prepared using Promega's Magic Miniprep DNA purification system. A small amount of the resulting DNA is cut using a restriction digest as a check. DNA sequencing is then performed to check the sequence of the fusion nucleic acid sequence, using the United States Biochemical (Cleveland, OH) Sequenase Version 2.0 DNA sequencing kit. Three to five µg of plasmid DNA is used per reaction. 2 µl 2M NaOH/2mM EDTA are added to the DNA, and the volume is brought to 20 µl with water. The mixture is then incubated at room temperature for five minutes. 7 µl water, 3µl 3M NaAc, 75 µl EtOH are added. The resultant mixture is mixed by vortex and incubated for ten minutes at -70°C, and then subjected to microcentrifugation. After microcentrifugation for ten minutes, the supernatant is aspirated off, and the pellet is dried in the speed vac for 30 second. 6 µl water, 2 µl annealing buffer, and 2 µl of 10 µM of the appropriate oligo is then added. This mixture is incubated for 10 minutes at 37°C and then allowed to stand at room temperature for 10 minutes. Subsequently, 5.5 µl of label cocktail (described above) is added to each sample of the mixture, which are incubated at room temperature for an additional five minutes. 3.5 µl labeled DNA is then added to each sample which is then incubated for five minutes at 37°C. 4 µl stop solution is added to each well. The DNA is denatured at 95° for two minutes, and then placed on ice.

Clones with the desired fusion nucleic acid sequences are then reckoned in frame in the pET expression system in the lipidated (full length) and non-lipidated (truncated, i.e., without first 17 amino acids) forms. The product is amplified using restriction sites contained in the PCR primers. The vector and product are cut with the same enzymes and ligated together with T4 ligase. The resultant plasmid is transformed into competent *E. coli* using standard transformation techniques. Colonies are screened as described earlier and positive clones are transformed into expression cells, such as *E*. *coli* BL21, for protein expression with IPTG for induction. The expressed protein in its bacterial culture lysate form and/or purified form is then injected in mice for antibody production. The mice are bled, and the sera collected for agglutination, *in vitro* growth inhibition, and complement-dependent and -independent lysis tests.

### B. Specific Chimeric Nucleic. Acid Sequences

Various chimeric nucleic acid sequences were generated. The nucleic acid sequences are described as encoding polypeptides from *Borrelia* proteins. The chimeric nucleic acid sequences are produced such that the nucleic acid sequence encoding one polypeptide is in the same reading frame as the nucleic acid sequence encoding the next polypeptide in the chimeric protein sequence encoded by the chimeric nucleic acid sequence. The proteins are listed sequentially (in order of presence of the encoding sequence) in the description of the chimeric nucleic acid sequence. For example, if a chimeric nucleic acid sequence consists of bp 1-650 from OspA-1 and bp 651-820 from OspA-2 were sequenced, the sequence of the chimer would include the first 650 base pairs from OspA-1 followed immediately by base pairs 651-820 of OspA-2.

### OspA-K48/OspA-PGau

A chimer of OspA from strain K48 (OspA-K48) and OspA from strain PGau (OspA-PGau) was generated using the method described above. This chimeric nucleic acid sequence included bp 1-654 from OspA-K48, followed by bp 655-820 from OspA-PGau. Primers used included: the amino-terminal sequence of OspA primer #607 (SEQ ID NO. 16); the fusion primer,
5'-AAAGTAGAAGTTTTTGAATCCCATTTTCCAGTTTTTTT3' (minus strand primer #668-654) (SEQ ID NO. 84); the carboxy-terminal sequence of OspA primer #586 (SEQ ID NO. 19); and the sequence primers #369 (SEQ ID NO. 14) and #357 (SEQ ID NO. 15). The chimeric nucleic acid sequence is presented as SEQ ID NO. 85; the chimeric protein encoded by this chimeric nucleic acid sequence is presented as SEQ ID NO. 86.

### OspA-B31/OspA-PGau

A chimer of OspA from strain B31 (OspA-B31) and OspA from strain PGau (OspA-PGau) was generated using the method described above. This chimeric nucleic acid sequence included bp 1-651 from OspA-B31, followed by bp 652-820 from OspA-PGau. Primers used included: the fusion primer,
5'-AAAGTAGAAGTTTTTGAATTCCAAGCTGCAGTTTT-3' (minus strand primer #668-651) (SEQ ID NO. 87); and the sequence primer, #369 (SEQ ID NO. 14). The chimeric nucleic acid sequence is presented as SEQ ID NO. 88; the chimeric protein encoded by this chimeric nucleic acid sequence is presented as SEQ ID NO. 89.

### OspA-B31/OspA-K48

A chimer of OspA from strain B31 (OspA-B31) and OspA from strain K48 (OspA-K48) was generated using the method described above. This chimeric nucleic acid sequence included bp 1-651 from OspA-B31, followed by bp 652-820 from OspA-K48. Primers used included: the fusion primer,
5'-AAAGTGGAAGTTTTTGAATTCCAAGCTGCAGTTTTTTT-3' (minus strand primer #671-651) (SEQ ID NO. 90); and the sequence primer, #369 (SEQ ID NO. 14). The chimeric nucleic acid sequence is presented as SEQ ID NO. 91; the chimeric protein encoded by this chimeric nucleic acid sequence is presented as SEQ ID NO. 92.

### OspA-B31/OspA-25015

A chimer of OspA from strain B31 (OspA-B31) and OspA from strain 25015 (OspA-25015) was generated using the method described above. This chimeric nucleic acid sequence included bp 1-651 from OspA-B31, followed by bp 652-820 from OspA-25015. Primers used included: the fusion primer, 5'-TAAAGTTGAAGTGCCTGCATTCCAAGCTGCAGTTT-3' (SEQ ID NO. 93).
The chimeric nucleic acid sequence is presented as SEQ ID NO. 94; the chimeric protein encoded by this chimeric nucleic acid sequence is presented as SEQ ID NO. 95.

### OspA-K48/OspA-B31/OspA-K48

A chimer of OspA from strain B31 (OspA-B31) and OspA from strain K48 (OspA-K48) was generated using the method described above. This chimeric nucleic acid sequence included bp 1-570 from OspA-K48, followed by bp 570-651 from OspA-B31, followed by bp 650-820 from OspA-K48. Primers used included: the fusion primer, 5'-CCCCAGATTTTGAAATCTTGCTTAAAACAAC-3' (SEQ ID NO. 96); and the sequence primer, #357 (SEQ ID NO. 15). The chimeric nucleic acid sequence is presented as SEQ ID NO. 97; the chimeric protein encoded by this chimeric nucleic acid sequence is presented as SEQ ID NO. 98.

### OspA-B31/OspA-K48/OspA-B31/OspA-K48

A chimer of OspA from strain B31 (OspA-B31) and OspA from strain K48 (OspA-K48) was generated using the method described above. This chimeric nucleic acid sequence included bp 1-420 from OspA-B31, followed by 420-570 from OspA-K48, followed by bp 570-650 from OspA-B31, followed by bp 651-820 from OspA-K48. Primers used included: the fusion primer, 5'-CAAGTCTGGTTCCAATTTGCTCTTGTTATTAT-3' (minus strand primer #436-420) (SEQ ID NO. 99); and the sequence primer, #357 (SEQ ID NO. 15). The chimeric nucleic acid sequence is presented as SEQ ID NO. 100; the chimeric protein encoded by this chimeric nucleic acid sequence is presented as SEQ ID NO. 101.

### OspA-B31/OspB-B31

A chimer of OspA and OspB from strain B31 (OspA-B31, OspB-B31) was generated using the method described above. The chimeric nucleic acid sequence included bp 1-651 from OspA-B31, followed by bp 652-820 from OspB-B31. Primers used included: the fusion primer, 5'-GTTAAAGTGCTAGTACTGTCATTCCAAGCTGCAGTTTTTTT-3' (minus strand primer #740-651) (SEQ ID NO. 102); the carboxy-terminal sequence of OspB primer #1106 (SEQ ID NO. 25); and the sequence primer #357 (SEQ ID NO. 15). The chimeric nucleic acid sequence is presented as SEQ ID NO. 103; the chimeric protein encoded by this chimeric nucleic acid sequence is presented as SEQ ID NO. 104.

### OspA-B31/OspB-B31/OspC-B31

A chimer of OspA, OspB and OspC from strain B31 (OspA-B31, OspB-B31, and OspC-B31) was generated using the method described above. The chimeric nucleic acid sequence included bp 1-650 from OspA-B31, followed by bp 652-820 from OspB-B31, followed by bp 74-630 of OspC-B31. Primers used included: the fusion primer, 5'-TGCAGATGTAATCCCATCCGCCATTTTTAAAGCGTTTTT-3' (SEQ ID NO. 105); and the carboxy-terminal sequence of OspC primer (SEQ ID NO. 28). The chimeric nucleic acid sequence is presented as SEQ ID NO. 106; the chimeric protein encoded by this chimeric nucleic acid sequence is presented as SEQ ID NO. 107.

### OspC-B31/OspA-B31/OspB-B31

A chimer of OspA, OspB and OspC from strain B31 (OspA-B31, OspB-B31, and OspC-B31) was generated using the method described above. The chimeric nucleic acid sequence included bp 1-630 from OspC-B31, followed by bp 52-650 from OspA-B31, followed by bp 650-820 of OspB-B31. Primers used included: the amino-terminal sequence of OspC primer having SEQ ID NO. 27; the fusion primer, 5'-GCTGCTAACATTTTGCTTAGGTTTTTTTGGACTTTC-3' (minus strand primer #69-630) (SEQ ID NO. 108); and the sequence primers #520 (SEQ ID NO. 40) and #200 (SEQ ID NO. 18). The chimeric nucleic acid sequence is presented as SEQ ID NO. 109; the chimeric protein encoded by this chimeric nucleic acid sequence is presented as SEQ ID NO. 110.

### Additional Chimeric Nucleic Acid Sequences

Using the methods described above, other chimeric nucleic acid sequences were produced. These chimeric nucleic acid sequences, and the proteins encoded, are summarized in Table III.

**Table III. Chimeric Nucleic acid Sequences and the Encoded Proteins**

| Chimers Generated (base pairs) | SEQ ID NO. (nt) | SEQ ID NO. (protein) |
|---|---|---|
| OspA (52-882) / p93 (1168-2100) | 111 | 112 |
| OspB (45-891) / p41 (122-234) | 113 | 114 |
| OspB (45-891) / p41 (122-295) | 115 | 116 |
| OspB (45-891) / p41 (140-234) | 117 | 118 |
| OspB (45-891)/p41 (140-295) | 119 | 120 |
| OspB (45-891) / p41 (122-234) / OspC (58-633) | 121 | 122 |
| OspA-Tro/OspA-Bo | 137 | 138 |
| OspA-PGau/OspA-Bo | 139 | 140 |
| OspA-B31/OspA-PGau/OspA-B31/ OspA-K48 | 143 | 144 |
| OspA-PGau/OspA-B31/OspA-K48 | 141 | 142 |

### C. Purification of Proteins Generated by Chimeric Nucleic Acid Sequences

The chimeric nucleic acid sequences described above, as well as chimeric nucleic acid sequences produced by the methods described above, are used to produce chimeric proteins encoded by the nucleic acid sequences. Standard methods, such as those described above in Example 3, concerning the expression of proteins from *Borrelia* genes, can be used to express the proteins in a compatible host organism. The chimeric proteins can then be isolated and purified using standard techniques.

If the chimeric protein is soluble, it can be purified on a Sepharose column. Insoluble proteins can be solubilized in guanidine and purified on a Ni²⁺ column; alternatively, they can be solubilized in 10 mM NaPO₄ with 0.1-1% TRIXON X 114, and subsequently purified over an S column (Pharmacia). Lipidated proteins were generally purified by the latter method. Solubility was determined by separating both soluble and insoluble fractions of cell lysate on a 12% PAGE gel, and checking for the localization of the protein by Coomasie staining, or by Western blotting with monoclonal antibodies directed to an antigenic polypeptide of the chimeric protein.

### Example 5. Generation of OspC/OspA Chimeric Nucleic Acids and Chimeric Proteins

### A. General Protocol for Creation of Chimeric Nucleic Acid Sequences

A large number of chimeric nucleic acid sequences encoding proteins comprising at least a first and a second polypeptide from *Borrelia burgdorferi* were generated. These chimeric nucleic acid sequences were produced such that the encoded chimeric protein comprised a *Borrelia burgdorferi* OspC polypeptide upstream of (or N-terminal to) a *Borrelia burgdorferi* OspA polypeptide. The chimeric nucleic acid sequences were also produced such that the nucleic acid encoding one polypeptide was in the same reading frame as the nucleic acid sequence encoding the next polypeptide in the chimeric protein.

The general cloning strategy used to construct the chimeric nucleic acid sequences. The desired fragment of OspC was amplified using a 5' primer containing a restriction site suitable for cloning the resultant product into a vector of interest and a 3' primer containing a restriction site suitable for ligating the OspC fragment to the OspA fragment. The OspC product is cloned into a suitable vector. For the OspA poriton of the chimeric nucleic acid, the desired OspA fragment was amplified using a 5' primer containing a restriction site for ligating the resultant OspA fragment to the OspC fragment and a 3' primer containing a restriction site suitable for cloning the resultant OspA product into the vector with the OspC product. The use of a restriction site to allow ligation of the OspC and OspA fragment results in the insertion of 0 to about 3 amino acids between the OspC and OspA fragments.

A specific example of such a construction follows. It is understood that other suitable restriction sites could be used with no more than routine experimentation. The resultant OspC/OspA chimers could have, therefore, the addition of 0 to about 3 amino acids between the OspC and OspA fragments, depending on the restriction site used.

For the OspC portions of the chimeric nucleic acids, desired fragments of OspC genes from various strains or genospecies were PCR amplified using a 5' primer containing an NdeI site and a 3' primer containing a NcoI and a BamHI site. The amplified OspC product was then cloned into the NdeI and BamHI sites of the T7 promoter driven expression vector, pET9c. For the OspA portion of the chimeric nucleic acid, desired fragments of OspA genes a strain of interest or genospecies of interest were PCR amplified using a 5' primer containing an NcoI site and a 3' primer containing a BamHI site. This OspA portion could then be directly cloned into the NcoI and BamHI sites of the pET9c vector containing the desired OspC sequence, thereby producing the desired OspC-OspA construct. By including the sequence for the NcoI restriction site in the primers, a nine nucleotide linker sequence encoding the amino acids Ser-Met-Ala was produced at the junction between the N-terminal OspC sequence and the C-terminal OspA sequence. The use of the NcoI restriction enzyme (CCATGG) in this cloning strategy was a suitable choice as *Borrelia* DNA is an AT-rich and therefore possesses only a few NcoI sites in its genome.

As an example, OspC-OspA chimeric nucleic acids which contain unlipidated OspC B31 were generated using the following primers:
(5'OspC-NdeI): 5'-GT CAT ATG GCT TGT AAT AAT TCA GGG AAA GA-3' (SEQ ID NO:181); and
(3'OspC-Ncol): 5'-T TTC CAT GGA AGG TTT TTT TGG ACT TTC TG-3' (SEQ ID NO: 182).

For OspC-OspA chimeric nucleic acids which contain unlipidated OspA B31, the following primers were used:
(5'OspA-NcoI:) 5'-TT TCC ATG GCC AAG CAA AAT GTT AGC AGC C-3' (SEQ ID NO:183); and
(3'OspA-BamHI): 5'-TAA GGA TCC TTA TTT TAA AGC GTT TTT-3' (SEQ ID NO:184).

Lipidated versions of the OspC/A chimeras can be constructed by cloning an expression vector that contains a leader sequence containing a lipidation site, such that the leader sequence is linked upstream of the OspC portion of the chimera and in frame with the chimera. The leader sequence comprising a lipidation signal can be, for example, from a gene encoding the OspA, B or C polypeptides.

Chimeric nucleic acid sequences, and the proteins that they encode, which were produced are summarized in table IV. Other additional chimeric nucleic acid sequences, and encoded proteins, are also depicted in Table IV. In further embodiments, chimeric OspC/OspA proteins are constructed wherein a first segment of OspA is from B31 and comprises base pairs from about 88 to about 450, and a second segment of OspA comprises base pairs from about 451 to about 537 of PKo. These chimeras can also comprise additional OspA segments such as the last two segments of SEQ ID NOs 167 or 165 or the last segment of SEQ ID NO: 163.

**Table IV. Chimeric OspC/OspA Nucleic Acid Sequences and Encoded Proteins**

| ¹Chimers Generated | SEQ ID NO. (nt) | SEQ ID NO. (protein) | FIG. NO. |
|---|---|---|---|
| OspC-B31 (bp55-633)/OspA-B31 (bp52-822) | 145 | | 55 |
| | | | |
| OspC-B31(aa19-211)/OspA-B31(aa18-273) | | 146 | 55 |
| OspC-B31 (bp55-624)/OspA-B31 (bp52-822) | 147 | | 56 |
| | | | |
| OspC-B31(aa19-208)/OspA-B31(aa18-273) | | 148 | 56 |
| OspC-C2(bp55-612)/OspA-B31(bp52-822) | 149 | | 57 |
| | | | |
| OspC-C2(aa19-204)/OspA-B31(aa18-273) | | 150 | 57 |
| OspC-B31 (bp55-633)/OspA-B31 (bp52-651)/OspA-K48(bp652-820) | 151 | | 58 |
| | | | |
| OspC-B31(aa19-211)/OspA-B31(aa18-216)/OspA-K48(aa217-273) | | 152 | 58 |
| OspC-C2(bp55-612)/OspA-B31(bp52-651)/OspA-K48(bp652-820) | 153 | | 59 |
| | | | |
| OspC-C2(aa19-204)/OspA-B31(aa18-216)/OspA-K48(aa217-273) | | 154 | 59 |
| OspC-B31 (bp55-633)/OspA-B31 (bp52-651)/OspA-PKo(bp652-820) | 155 | | 60 |
| | | | |
| OspC-B31(aa19-211)/OspA-B31(aa18-216)/OspA-PKo(aa217-273) | | 156 | 60 |
| OspC-C2(bp55-612)/OspA-B31(52-651)/OspA-PKo(bp652-820) | 157 | | 61 |
| | | | |
| OspC-C2(aa19-204)/OspA-B31(aa18-216)/OspA-PKo(aa217-273) | | 158 | 61 |
| OspC-B31 (bp55-633)/OspA-K48(bp52-654)/OspA-Tro(bp655-819) | 159 | | 62 |
| | | | |
| OspC-B31(aa19-211)/OspA-K48(aa18-217)/OspA-Tro(aa218-273) | | 160 | 62 |
| OspC-C2(bp55-612/OspA-K48(bp52-654)/OspA-Tro(bp655-819) | 161 | | 63 |
| | | | |
| OspC-C2(aa19-204)/OspA-K48(aa18-217)/OspA-Tro(aa218-273) | | 162 | 63 |
| OspC-C12(bp55-612)/OspA-B31(bp88-492)/OspA-PKo(bp493-537)/OspA-B31(bp538-822) | 163 | | 64- |
| | | | |
| OspC-C12(aa19-204)/OspA-B31(aa30-164)/OspA-PKo(aa165-179)/OspA-B31(aa180-273) | | 164 | 64 |
| OspC-PKo(bp55-639)/OspA-B31 (bp88-492)/OspA-PKo(bp493-537)/OspA-B31(bp538-651)/OspA-K48(bp652-825) | 165 | | 65 |
| | | | |
| OspC-PKo(aa19-213)/OspA-B31(aa30-164)/OspA-PKo(aa165-179)/OspA-B31(aa180-216)/OspA-K48(aa217-274) | | 166 | 65 |
| OspC-Tro(bp55-624)/OspA-B31 (bp88-492)/OspA-PKo(bp493-537)/OspA-B31(bp538-651)/OspA-PKo(bp652-822) | 167 | | 66 |
| | | | |
| OspC-Tro(aal9-208)/OspA-B3 l(aa30-164)/OspA-PKo(aal 65-179)/OspA-B31 (aal 80-216)/OspA-PKo(aa217-273) | | 168 | 66 |
| OspC-B31 (bp55-633)/OspA-B31 (bp394-820) | 169 | | 67 |
| | | | |
| OspC-B31(aa19-211)/OspA-B31(aa132-273) | | 170 | 67 |
| OspC-B31(bp55-631)/OspA-B31(bp394-651)/OspA-K48(652-820) | 171 | | 68 |
| | | | |
| OspC-B31(aa19-211)/OspA-B31(aa132-216)/OspA-K48(217-273) | | 172 | 68 |
| OspC-B31(bp55-633)/OspA-B31(bp394-651)/OspA-PKo(652-820) | 173 | | 69 |
| | | | |
| OspC-B31(aa19-211)/OspA-B31(aa132-216)/OspA-PKo(217-273) | | 174 | 69 |
| OspC-B31(bp55-633)/OspA-K48(bp394-654)/OspA-Tro(655-819) | 175 | | 70 |
| | | | |
| OspC-B31(aa19-211)/OspA-K48(aa132-217)/OspA-Tro(218-273) | | 176 | 70 |
| OspC-B31(bp55-633)/OspA-B31(bp88-492)/OspA-PKo(bp493-537)/OspA-B31(bp541-651)/OspA-PKo(bp652-822) | 177 | | 71 |
| | | | |
| OspC-B31(aa19-211)/OspA-B31(aa30-164)/OspA-PKo(aa165-179, aa164(K>G))/OspA-B31(aa180-216)(aa190(N-del))/OspA-PKo(aa217-273) | | 178 | 71 |
| OspC-C2(bp55-612)/OspA-B31(bp88-492)/OspA-PKo(bp493-537)/OspA-B31(bp541-651)/OspA-PKo(bp652-822) | 179 | | 72 |
| | | | |
| OspC-C2(aa19-204)/OspA-B31(aa30-164)/OspA-PKo(aa165-179, aa164(K>G))/OspA-B31(aa180-216)(aa190(N-del))/OspA-PKo(aa217-273) | | 180 | 72 |

| | | | |
|---|---|---|---|
| ¹Chimers Generated are listed as follows: Nucleotide or polypeptide fragment -strain (sequence in base pairs for the top listing and amino acids for the bottom listing) | | | |

Separate nucleotide or polypeptide fragments in the chimer are separated by a /

### B. Protein Expression

As described in the previous two examples, it is possible to express and purify *Borrelia* proteins such as OspA, OspC and chimeric OspC/OspA polypeptides. This is accomplished by incorporating the desired nucleic acid sequence, which encodes the protein of choice, into an expression plasmid, using standard techniques. This expression plasmid can then be transfected into a compatible host cell in order to express the desired protein.

The purified OspA, OspC or OspC/OspA chimeric proteins, that were used to immunize mice and in the ELISA tests described below, were generated and purified by cloning either OspA, OspC or OspC/OspA chimeric nucleic acid sequences, in frame, into the pET expression plasmid. The expression plasmid was then transfected into the compatible expression cell line *Escherichia coli* strain BL21 (DE3)/(pLysS) or strain B834 (DE3). The BL21 or B834 cells were then grown in 10 ml LB media (5 g/l NaCl, 10 g/l tryptone, 5 g/l yeast extract, 25 mg/l chloramphenicol and 50 mg/l ampicillin) at 37°C, with shaking. When the optical density at 600λ reached 0.3-0.4 units, recombinant protein expression was induced by adding IPTG (isopropyl B-D-thiogalactopyranoside) to a final concentration of 0.5 mM and the cells were grown for an additional three hours. The cultures were harvested by centrifugation at 3800xg for five minutes. The cells were resuspended in 20 mM NaPO₄, pH7.7 and stored at -20°C overnight. Once thawed, the crude extracts were incubated with DNase (2 µg/ml) in the presence of 2.5 mM MgCl₂ at room temperature for thirty minutes and then spun at 14,000 rpm (Eppendorf 5417C) for five minutes.

To purify the OspC proteins described below, the crude extracts from the OspC-expressing cells were loaded onto an anion exchange column (Q Sepharose Fast Flow, 2.2x10cm, Pharmacia) which had been pre-equilibrated with 20 mM Tris-Cl, pH 9.3. The column was washed in the same buffer (20 mM Tris-Cl, pH 9.3) which eluted the OspC protein. The wash fractions that contained OspC were concentrated using Amicon 10K and then were dialyzed with a solution containing 20 mM NaPO₄ pH 8.0 and 250 mM NaCl. The partially purified OspC was then passed over a Ni²⁺ metal affinity column (Chelating Sepharose Fast Flow 2.2x10 cm, Pharmacia) equilibrated with 20 mM NaPO₄ pH 8.0 and 250 mM NaCl. The column was washed using a decreasing pH gradient of 20 mM sodium acetic acid and 250 mM NaCl and the bound OspC eluted around pH 5.7. The OspC fractions were then concentrated by ultrafiltration and stored at -70°C.

For purification of OspA proteins, the same procedure was followed, except that the dialysis step, after the Amicon 10K cutoff, was done in 20 nM NaPO₄, pH 6.0. The partially purified OspA was then applied to a cation exchange column (S Sepharose Fast Flow 2.2x10 cm, Pharmacia) equilibrated with 20 nM NaPO₄, pH 6.0. The column was washed using an increasing NaCl gradient from 0 to 100 mM. The OspA-containing fractions were concentrated by ultrafiltration and stored at - 70°C.

As previously indicated, both lipidated and non-lipidated (truncated, i.e., without the first 17 amino acids) forms of OspC, OspA and OspC/OspA chimeric proteins were generated.

### C. Immunization of Mice and Serologic Characterization Using ELISA (Enzyme-Linked Immunosorbent Assay)

### Immunization of Mice

Mice, either C3H-J or ICR, were immunized with 3 ug of lipidated OspC/OspA chimeric protein or 6 ug of non-lipidated OspC/OspA chimers in 100 ul of aluminum hydroxide adjuvant (concentration of 1.8 mg(ml) by (SC) subcutaneous injection. As a negative control, mice were immunized with 100 ul of aluminum hydroxide adjuvant only. All mice received a total of three injections which were given at two week intervals. One week after the final immunization, blood was drawn from each mouse (including negative controls) and the serum was tested for IgG reacivity using the ELISA method described below, for the presence of anti-OspA antibodies to three different purified OspA proteins (*Borrelia burgdorferi* sensu stricto (B31), *Borrelia garinii* (K48) and *Borrelia afzelli* (PGau). The sera was tested at a dilution of 1: 1000.

Mice were immunized with the chimeric proteins described in Table V.

**Table V. Chimeric Proteins Used to Immunize Mice**

| Name | Description (amino aci d) | SEQ ID NO.: (nucleic acid) | SEQ ID NO.: (polypeptide) | Fig. No: |
|---|---|---|---|---|
| OspA | OspA-B31(18-273) | 6 | 7 | 47, 48 |
| OspC | OspC-B31 (19-211) | 29 | 30 | 47, 48 |
| OspC2-OspA | OspC-C2(19-204)/OspA-B31 (18-273) | 149 | 150 | 47, 48 |
| ¹lipOspAP/Bo | OspA-PGau(1-217)/OspA-Bo(218-273) | 139 | 140 | 49, 50 |
| ¹lipOspAB/P | OspA-B31(1-216)/OspA-PKo(217-273) | * | * | 49, 50 |
| OspC-OspAB/P | OspC-B31(19-211)/OspA-B31(18-216)/OspA-PKo(217-273) | 155 | 156 | 49, 50, 52, 53,54 |
| OspCB31-OspAB31 | OspC-B31(19-211)/OspA-B31 (18-273) | 145 | 146 | 51, 52, 53, 54 |
| OspC2-OspAB31 | OspC-C2(19-204)/OspA-B31(18-273) | 149 | 150 | 51,52 |
| ¹lip OspA K/T | OspA-K48(1-217)/OspA-Tro(218-273) | * | * | 52 |
| ¹lip OspC-B31 | OspC-B31(1-211) | 29 | 30 | 51 |
| OspCB31-OspABPBP | OspC-B31(19-211)/OspA-B31(30-150)/OspA-PKo(151-179)/OspA-B31(180-216) (190 N deletion)/OspA-PKo(217-273) B31/B31/PKo | 177 | 178 | 53, 54 |

| | | | | |
|---|---|---|---|---|
| ¹"lip" means the chimeric protein contains its native N-terminal lipidation signal Serologic Characterization Using ELISA (Enzyme-Linked Immunosorbent Assay) | | | | |

### Immobilization of antigen onto ELISA Plates

A solution of purified recombinant OspC or OspA protein from each of the *Borrelia burgdorferi* strains B31 *(Borrelia burgdorferi* sensu stricto), K48 *(Borrelia garinii)* and PGau *(Borrelia afzelli)* was added to sodium phosphate buffer, pH 9.0, and was used to coat a commercial microwell plate (MaxiSorp®, Nunc). The coating procedure was as follows: 100 µl of a solution containing the appropriate OspA or OspC protein (made up at a concentration of 250 ng/ml in the following coating buffer: 100 mM Bis-Tris propane, pH 9.7) was added to each well of a microtiter plate which was incubated for one hour at 37°C. The antigen solution was removed from the wells, the plate was washed three times with phosphate buffered saline (PBS) pH 9.0, and 300 µl of blocking buffer solution was added (3% dry milk, 0.1 % polyoxyethylenesorbitan (referred to herein as Tween 20^{™}), 0.02% NaN₃ in 100 nM Bis-Tris propane, pH 9.7). Following a one hour incubation at 37°C, the plates were washed four times with TBS-Tween 20™ wash buffer (20 mM Tris-Cl, pH 7.5, 136 mM NaCl, 0.1% Tween 20™ and 0.02% NaN₃) and then were allowed to dry. The plates were then wrapped in plastic and stored at 4°C until they were used.

### ELISA (Enzyme-Linked Immunosorbent Assay) Tests

The standard procedure for the ELISA tests was as follows: mouse serum was diluted 1:1000 in sample dilution buffer (1% dry milk, 136 mM NaCl, 0.1% Tween 20^{™}, 0.02% NaN₃ in 20 mM Tris-Cl, pH7.5) and 100 µl of the diluted serum was added to the ELISA microtiter plate wells that had been coated with antigen as described above. Following incubation for 1 hour at 37°C, the samples were removed and the plates were washed four times in TBS-Tween^{™} (20 mM Tris-Cl, pH 7.5; 136 mM NaCl; 0.1% Tween 20™ and 0.02% NaN₃). For the secondary antibody, goat anti-mouse antisera conjugated to alkaline phosphatase-specific for either IgM (Fc) or IgG (Fab), (Jackson Immuno Research Laboratories) was diluted 1:750 in sample dilution buffer (1% dry milk, 136 mM NaCl, 0.1 % Tween 20^{™}, 0.02% NaN₃ in 20 mM Tris-Cl, pH7.5) and 100 µl of the diluted secondary antibody was added to each well. Following incubation for thirty minutes at 37°C, the plates were washed three times with TBS-Tween^{™} (20 mM Tris-Cl, pH 7.5; 136 mM NaCl; 0.1% Tween 20™ and 0.02% NaN₃) and 100 µl of Phosphatase Substrate solution (5 mg ofp-nitrophenylphosphate tablets dissolved in 1X diethanolamine substrate buffer to yield a 2 mg/ml solution - Kirkegaard Perry Laboratory) was added to each well. The plates were incubated for thirty minutes at 37°C and 100 µl of stop solution (5 % EDTA) was added to each well. The absorbance at 405 nm was read on a microplate reader (Dynatech). A sample was considered positive if it produced an average absorbance greater than the mean of the negative controls plus three standard deviations.

Previous work has demonstrated that it is the carboxy-terminal region of OspA that contains the antigenic sites that provide the immunoprotective response. Thus, in addition to the ELISA test described above, a modified ELISA was performed (herein referred to as the Protective ELISA Test), wherein the purified N-terminal region of B31 OspA (amino acids 18-139) was used to block any antibodies present in the mouse serum that had specificity to this N-terminal OspA region. These protective ELISA tests were performed as above, except that 80 µg/ml of a purified B31 OspA fragment (amino acids 18-139) was added to the diluted mouse serum prior to adding the sera to the antigen-coated ELISA microtiter plate wells.

### Results of ELISA Tests

Using the above-described ELISA tests, it was demonstrated that mice immunized with a non-lipidated OspC/OspA chimeric protein (OspC2-OspA - composed of OspC (a.a.19-204 from strain C2)/OspA (a.a. 18-273 from strain B31) (SEQ ID NO.150) produced an immune response both to OspA and OspC that was comparable to the immune response generated to non-lipidated OspA (OspA - a.a. 18-273 from strain B31) and non-lipidated OspC (OspC - a.a. 19-211 from strain B31) control proteins (Fig. 47). As indicated in Fig. 47 and described above, mice were immunized with OspA, OspC or OspC2-OspA proteins and immune responses of the sera were measured against B31 OspA antigen (stippled bars) and B31 OspC antigen (solid bars).

Using the above-described Protective ELISA Test, it was also shown that mice immunized with the same non-lipidated OspC/OspA chimeric protein (OspC2-OspA - composed of OspC (a.a.19-204 from strain C2)/OspA (a.a. 18-273 from strain B31) (SEQ ID NO.150) produced an immune response to the C-terminal portion of OspA that was comparable to the immune response generated to the C-terminal portion of a non-lipidated OspA (OspA - a.a. 18-273 from strain B31) control protein (Fig. 48). As indicated in Fig. 48, mice were immunized with OspA, OspC or OspC2-OspA proteins and immune responses of the sera were measured against B31 OspA antigen. The protective antibody response to B31 OspA antigen is indicated in the stippled bars.

Thus, these results clearly demonstrate that non-lipidated chimeric OspC/OspA proteins are able to induce immune responses in mice that are comparable to the immune response generated against non-lipidated OspC and OspA control proteins.

It had been previously thought that the lipidation signals that are present on *Borrelia burgdorferi* outer surface proteins were required for immunogenicity and that OspC and OspA proteins that lacked this lipidation signal would be less or non-immunogenic. To test this idea, mice were immunized with a non-lipidated OspC/OspA chimeric protein (OspC-OspAB/P - composed of OspC (a.a. 19-211 from strain B31)/OspA (a.a. 18-216 from strain B31)/OspA (a.a. 217-273 from strain PKo)(SEQ ID NO: 156) as well as two lipidated OspA proteins, lipOspAP/Bo (composed of OspA (a.a. 1-217 from strain PGau)/OspA (a.a. 218-273 from strain Bo)) and lipOspAB/P (composed of OspA (a.a. 1-216 from strain B31)/OspA (a.a. 217-273 from strain PKo)) and were subjected ELISA tests. Mice immunized with the non-lipidated OspC/OspA chimeric protein (OspC-OspAB/P) produced an immune response to OspA from each of the *Borrelia burgdorferi* strains B31 *(Borrelia burgdorferi* sensu stricto), K48 *(Borrelia garinii)* and PGau *(Borrelia afzelli),* that was equivalent or greater than the immune response generated to the two lipidated OspA control proteins (lipOspAP/Bo and lipOspAb/P) (Fig. 49).

Similar results to these were obtained using the Protective ELISA Test described above. Mice immunized with the non-lipidated OspC/OspA chimeric protein (OspC-OspAB/P) produced an immune response to the C-terminal region of OspA from each of the *Borrelia burgdorferi* strains B31 *(Borrelia burgdorferi* sensu stricto), K48 *(Borrelia garinii)* and PGau *(Borrelia afzelli),* that was equivalent or greater than the immune response generated to the C-terminal region of OspA from the two lipidated OspA control proteins (lipOspAP/Bo and lipOspAb/P) (Fig. 50).

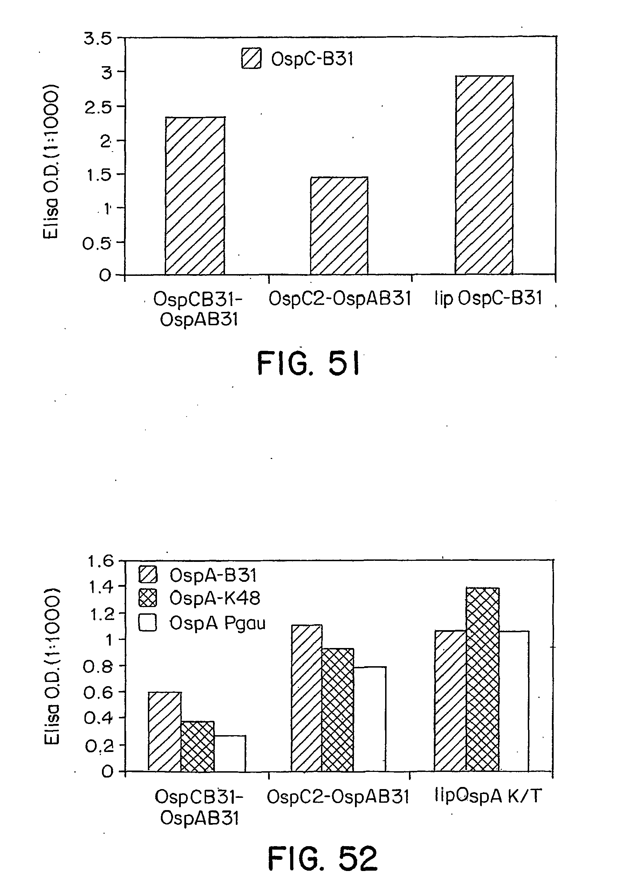

In addition to the comparisons between non-lipidated OspC/OspA chimeric proteins and lipidated OspA control proteins, experiments were also performed to compare non-lipidated OspC/OspA chimeric proteins with a lipidated OspC control protein (Fig. 51). Mice that were immunized with either the non-lipidated OspC/OspA chimeric protein OspCB31-OspAB31 (composed of OspC (a.a. 19-211 from strain B31)/OspA (a.a. 18-273 from strain B31) (SEQ ID NO:146) or the non-lipidated OspC/OspA chimeric protein OspC2-OspAB31 (composed of OspC (a.a. 19-204 from strain C2)/OspA (a.a. 18-273 from strain B31) (SEQ ID NO: 150) produced an immune response to OspC derived from the *Borrelia burgdorferi* strain B31 that was comparable to the immune response produced by a lipidated OspC control protein (lip OspC-B31 - composed of OspC (a.a. 1-211 from strain B31)) (Fig. 51).

Thus, these results clearly demonstrate that non-lipidated chimeric OspC/OspA proteins are able to induce immune responses against OspA and OspC that are comparable to the immune response generated against OspA and OspC using lipidated OspA or OspC control proteins. The use of unlipidated forms of these proteins as vaccine immunogens or diagnostic antigens is highly desirable because the product yield is much greater and the proteins are much easier to purify. For these reasons, the production of these proteins less expensive.

The OspC/OspA chimeric proteins of the present invention are also able to generate immune responses against OspA proteins that are derived from strains that are not represented in the chimeric protein. Mice immunized with the OspC/OspA chimeric proteins, OspCB31-OspAB31 (SEQ ID NO:146) and OspC2-OspAB31 (SEQ ID NO:150), are not only able to generate immune responses that recognize OspA derived from strain B31 *(Borrelia burgdorferi* sensu stricto), but also recognize OspA derived from strain K48 *(Borrelia garinii)* and strain PGau *(Borrelia afzelli)* (Fig. 52). For comparison, mice were also immunized with the lipidated OspA chimeric protein, Lip OspA K/T (composed of OspA (a.a. 1-217 from strain K48)/OspA (a.a. 218-273 from strain Tro)) (Fig. 52).

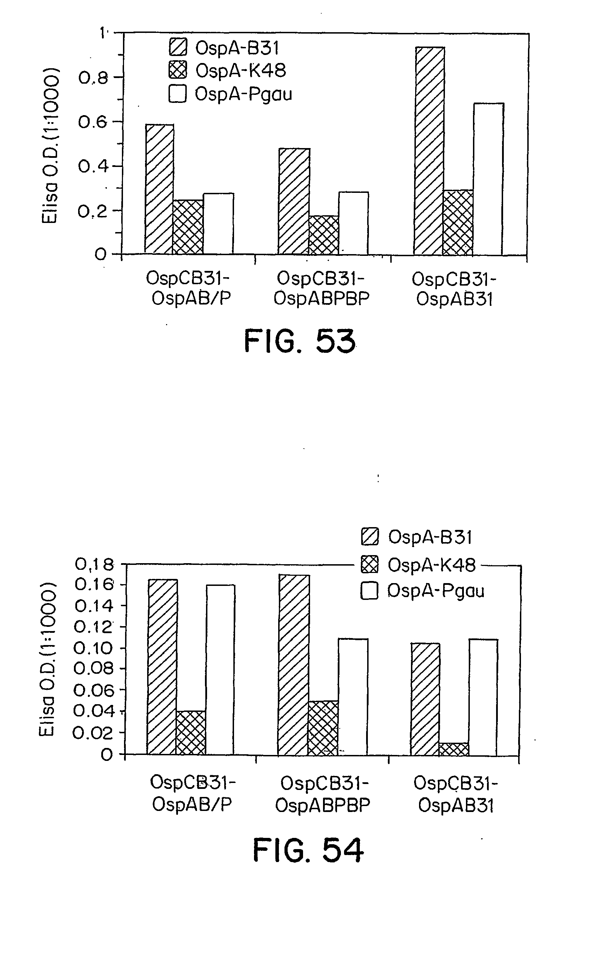

Additional antibody responses to OspA derived from strain B31 *(Borrelia burgdorferi* sensu stricto), strain K48 *(Borrelia garinii)* and strain PGau *(Borrelia afzelli)* are also presented for sera from mice immunized with other OspC/OspA chimeric proteins. Thus, Fig. 53 presents the ELISA results from mice immunized with either OspCB31-OspAB/P (SEQ ID NO:156), OspCB31-OspABPBP (SEQ ID NO:178) or OspCB31-OspAB31 (SEQ ID NO:146). In each case, sera from the immunized mice was tested against OspA derived from each of strain B31 *(Borrelia burgdorferi* sensu stricto), K48 *(Borrelia garinii)* and PGau *(Borrelia afzelli).* In all cases, a strong immune response was generated (Fig. 53). As with the previously described OspC/OspA chimeric proteins, the three OspC/OspA chimeric proteins used to immunize the mice in Fig. 52 also elicited a strong immune response to the C-terminal region of OspA when examined using the Protective ELISA Test described above (Fig. 54).

### Tick Challege of Immunized Mice

Mice, either C3H-J or JCR, that had been immunized as described above, were also challenged with either laboratory-infected nympha or field nympha. The immunized mice were placed in isolation cages and each mouse received 5-10 nymphs. All of the nymphs were collected at counted after 6 days. Four week after challenge, the mice were bled and sera was tested using commercially-available Western blot strips to *Borrelia burgdorferi* sensu stricto strain B31(MarDx strips) and/or *Borrelia garinii* (MRL strips). Eight weeks after challenge, the mice were bled, sera was tested again by Western blot and ear punch and bladder samples were cultured. As a positive control, mice which had been immunized with only aluminum hydroxide adjuvant, as described above, were subjected to the same challenge.

The results of the tick challenge studies (Table VI) demonstrate that while immunization with lipidated OspC protein was unable to protect the mice, as evidenced by a positive Western blot signal (in 4 out of 5 mice), immunization with two different OspC/OspA chimeric proteins (SEQ ID NO.146 and SEQ ID NO.150) did provide protection, as indicated by the absence of Western blot signal (in 0 out of 8 mice and 0 out of 3 mice) (Table VI). The sham positive control showed that the challenge by the ticks was successful in all cases, as evidenced by 100% positive signal in Western blots (Table VI).

**Table VI. Effect of Vaccination on Transmission of Borrelia from Ticks**

| Vaccine Candidate | Mouse | Tick-nymph | Seroconversion (Western Blots) Vaccinated | Seroconversion (Western Blots) Sham |
|---|---|---|---|---|
| OspCl-OspAB31 | C3H-J | Long Island | 0+/8 | 8+/8 |
| OspC2-OspAB31 | C3H-J | Long Island | 0+/3 | 4+/4 |
| Lip OspC12 | ICR | Long Island | 4+/5 | 5+/5 |

While this invention has been particularly shown and described with references to preferred embodiments thereof, it will be understood by those skilled in the art that various changes in form and details may be made therein without departing from the scope of the invention encompassed by the appended claims.

Embodiments of the invention may include the features of the following enumerated paragraphs ("paras").
1. A chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-tenninus of the protein.
2. A protein of para 1, wherein the OspC polypeptide is present in unlipidated form.
3. A protein of para 1, wherein the OspA polypeptide is present in unlipidated form.
4. A protein of para ; 1, wherein the OspA and OspC polypeptides are from the same strain *of Borrelia burgdorferi.*
5. A protein of para 1, wherein the OspA and OspC polypeptides are from the same genospecies *of Borrelia burgdorferi.*
6. A protein of para 1, wherein the OspA and OspC polypeptides are from different strains of *Borrelia burgdorferi.*
7. A protein of para 1, wherein the OspA and OspC polypeptides are from different genospecies of *Borrelia burgdorferi.*
8. A protein of para 1, wherein the OspA polypeptide comprises at least a first OspA amino acid sequence from a first strain *of Borrelia burgdorferi* and a second OspA amino acid sequence from a second strain *of Borrelia burgdorferi.*
9. A protein of para 8, wherein the first strain of *Borrelia burgdorferi* is from a first genospecies of *Borrelia burgdorferi* and wherein the second strain of *Borrelia burgdorferi* is from a second genospecies *of Borrelia burgdorferi.*
10. A protein of para 8, wherein the first OspA polypeptide fragment comprises a *Borrelia burgdorferi* OspA polypeptide from about amino acid residue 18 to about amino acid residue 216, and wherein the second OspA polypeptide fragment comprises a *Borrelia burgdorferi* OspA polypeptide from about amino acid residue 217 to about amino acid residue 273.
11. A protein of para 8, wherein the first OspA polypeptide fragment comprises a *Borrelia burgdorferi* OspA polypeptide from about amino acid residue 132 to about amino acid residue 217, and wherein the second OspA polypeptide fragment comprises a *Borrelia burgdorferi* OspA polypeptide from about amino acid residue 218 to about amino acid residue 273.
12. A protein of para 8, wherein the OspA polypeptide comprises at least four separate OspA polypeptide fragments.
13. A protein of para 12, wherein the first OspA polypeptide fragment comprises a *Borrelia burgdorferi* OspA polypeptide from about amino acid residue 30 to about amino acid residue 150, wherein the second OspA polypeptide fragment comprises a *Borrelia burgdorferi* OspA polypeptide from about amino acid residue 151 to about amino acid residue 179, wherein the third OspA polypeptide fragment comprises a *Borrelia burgdorferi* OspA polypeptide from about amino acid residue 180 to about amino acid residue 216, and wherein the fourth OspA polypeptide fragment comprises a *Borrelia burgdorferi* OspA polypeptide from about amino acid residue 217 to about amino acid residue 273.
14. A protein of para 1, wherein the first polypepetide comprises a *Borrelia burgdorferi* OspC polypeptide from about amino acid residue 19 to about amino acid residue 213.
15. A protein of para 1, wherein the first polypepetide comprises a *Borrelia burgdorferi* OspC polypeptide from about amino acid residue 19 to about amino acid residue 211.
16. A protein of para 1, wherein the first polypeptide comprises a *Borrelia burgdorferi* OspC polypeptide from about amino acid residue 19 to about amino acid residue 204.
17. A protein of para 1, wherein the second polypeptide comprises a *Borrelia burgdorferi* OspA polypeptide from about amino acid 18 to about amino acid 273.
18. A protein of para 1, wherein the second polypeptide comprises a *Borrelia burgdorferi* OspA polypeptide from about amino acid 132 to about amino acid 216.
19. A chimeric protein comprising an amino acid sequence selected from the group consisting of: SEQ ID NOs: 146, 148, 150, 152, 154, 156, 158, 160, 162, 164, 166, 168, 170, 172, 174, 176, 178 and 180.
20. A nucleic acid encoding a protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the encoded protein.
21. A nucleic acid encoding a protein comprising at least a first and a second polypeptide, wherein the first polypeptide comprises a *Borrelia burgdorferi* OspC polypeptide from about amino acid residue 19 to about amino acid residue 211, and wherein the second polypeptide comprises a *Borrelia burgdorferi* OspA polypeptide selected from the group consisting of:
   (a) a *Borrelia burgdorferi* OspA polypeptide from about amino acid residue 18 to about amino acid residue 273,
   (b) a *Borrelia burgdorferi* OspA polypeptide from about amino acid residue 132 to about amino acid residue 216,
   (c) a *Borrelia burgdorferi* polypeptide comprising at least two separate OspA polypeptide fragments, wherein the first OspA polypeptide fragment comprises an OspA polypeptide from about amino acid residue 18 to about amino acid residue 216 and, wherein the second OspA polypeptide comprises an OspA polypeptide from about amino acid residue 217 to about amino acid residue 273,
   (d) a *Borrelia burgdorferi* polypeptide comprising at least two separate OspA polypeptide fragments, wherein the first OspA polypeptide fragment comprises an OspA polypeptide from about amino acid residue 132 to about amino acid residue 216 and, wherein the second OspA polypeptide comprises an OspA polypeptide from about amino acid residue 217 to about amino acid residue 273, and
   (e) a *Borrelia burgdorferi* polypeptide comprising at least four separate OspA polypeptide fragments, wherein the first OspA polypeptide fragment comprises an OspA polypeptide from about amino acid residue 30 to about amino acid residue 150, wherein the second OspA polypeptide comprises an OspA polypeptide from about amino acid residue 151 to about amino acid residue 179, wherein the third OspA polypeptide comprises an OspA polypeptide from about amino acid residue 181 to about amino acid residue 216 and, wherein the fourth OspA polypeptide comprises an OspA polypeptide from about amino acid residue 216 to about amino acid residue 273.
22. A nucleic acid encoding a protein comprising at least a first and a second polypeptide, wherein the first polypeptide comprises a *Borrelia burgdorferi* OspC polypeptide from about amino acid residue 19 to about amino acid residue 204, and wherein the second polypeptide comprises a *Borrelia burgdorferi* OspA polypeptide selected from the group consisting of:
   (a) a *Borrelia burgdorferi* OspA polypeptide from about amino acid residue 18 to about amino acid residue 273,
   (b) a *Borrelia burgdorferi* OspA polypeptide from about amino acid residue 132 to about amino acid residue 216,
   (c) a *Borrelia burgdorferi* polypeptide comprising at least two separate OspA polypeptide fragments, wherein the first OspA polypeptide fragment comprises an OspA polypeptide from about amino acid residue 18 to about amino acid residue 216 and, wherein the second OspA polypeptide comprises an OspA polypeptide from about amino acid residue 217 to about amino acid residue 273,
   (d) a *Borrelia burgdorferi* polypeptide comprising at least two separate OspA polypeptide fragments, wherein the first OspA polypeptide fragment comprises an OspA polypeptide from about amino acid residue 132 to about amino acid residue 216 and, wherein the second OspA polypeptide comprises an OspA polypeptide from about amino acid residue 217 to about amino acid residue 273, and
   (e) a *Borrelia burgdorferi* polypeptide comprising at least four separate OspA polypeptide fragments, wherein the first OspA polypeptide fragment comprises an OspA polypeptide from about amino acid residue 30 to about amino acid residue 150, wherein the second OspA polypeptide comprises an OspA polypeptide from about amino acid residue 151 to about amino acid residue 179, wherein the third OspA polypeptide comprises an OspA polypeptide from about amino acid residue 181 to about amino acid residue 216 and, wherein the fourth OspA polypeptide comprises an OspA polypeptide from about amino acid residue 216 to about amino acid residue 273.
23. A nucleic acid of any one of paras 20, 21 or 22, wherein the first nucleic acid sequence is from a first strain *of Borrelia burgdorferi* and the second nucleic acid sequence is from a second strain *of Borrelia burgdorferi.*
24. A nucleic acid of any one of paras 20, 21 or 22, wherein the first nucleic acid sequence is from a first genospecies *of Borrelia burgdorferi* and the second nucleic acid sequence is from a second genospecies *of Borrelia burgdorferi.*
25. A nucleic acid of any one of paras 20, 21 or 22, wherein the first nucleic acid sequence is from a first strain *of Borrelia burgdorferi* and the second nucleic acid sequence is from a second strain *of Borrelia burgdorferi.*
26. A nucleic acid any one of paras 20, 21 or 22, wherein the first nucleic acid sequence is from a first genospecies *of Borrelia burgdorferi* and the second nucleic acid sequence is from a second genospecies of *Borrelia burgdorferi.*
27. A nucleic acid selected from the group consisting of: SEQ ID NOs: 145, 147, 149, 151, 153, 155, 157, 159, 161, 163, 165, 167, 169, 171, 173, 175, 177 and 179.
28. An expression vector comprising an isolated DNA having a nucleotide sequence selected from the group consisting of: SEQ ID NOs: 145, 147, 149, 151, 153, 155, 157, 159, 161, 163, 165, 167, 169, 171, 173, 175, 177 and 179.
29. An expression vector comprising an isolated DNA encoding a polypeptide having an amino acid sequence selected from the group consisting of: SEQ ID NOs: 146, 148, 150, 152, 154, 156, 158, 160, 162, 164, 166, 168, 170, 172, 174, 176, 178 and 180.
30. A host cell comprising a recombinant nucleic acid having a nucleotide sequence selected from the group consisting of: SEQ ID NOs: 145, 147, 149, 151, 153, 155, 157, 159, 161, 163, 165, 167, 169, 171, 173, 175, 177 and 179.
31. A host cell comprising a recombinant nucleic acid encoding a protein having an amino acid sequence selected from the group consisting of: SEQ ID NOs: 146, 148, 150, 152, 154, 156, 158, 160, 162, 164, 166, 168, 170, 172, 174, 176, 178 and 180.
32. A method of producing a chimeric *Borrelia burgdorferi* protein comprising;
   a) selecting a polynucleotide encoding OspC or an antigenic portion thereof;
   b) selecting a polynucleotide encoding OspA or an antigenic portion thereof; and
   c) ligating the polynucleotide of a) to the polynucleotide of b), to form a chimeric polynucleotide sequence encoding a chimeric OspC-OspA protein such that OspC is upstream of OspA.
33. The method of para 32, wherein the chimeric OspC-OspA protein has an amino acid sequence selected from the group consisting of: SEQ ID NOs: 146, 148, 150, 152, 154, 156, 158, 160, 162, 164, 166, 168, 170, 172, 174, 176, 178 and 180.
34. A method for producing a chimeric protein further comprising maintaining a host cell comprising the chimeric polynucleotide of para 32 under conditions suitable for expression of the polynucleotide.
35. A method of delivering a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, comprising administering the chimeric protein to an animal.
36. The method of para 35, wherein the chimeric protein is administered with a physiologically-acceptable vehicle or carrier.
37. The method of para 35, wherein the chimeric protein is encoded by a nucleic acid selected from the group consisting of: SEQ ID NOs: 145, 147, 149, 151, 153, 155, 157, 159, 161, 163, 165, 167, 169, 171, 173, 175, 177 and 179.
38. The method of para 35, wherein the chimeric protein comprises an amino acid sequence selected from the group consisting of: SEQ ID NOs: 146, 148, 150, 152, 154, 156, 158, 160, 162, 164, 166, 168, 170, 172, 174, 176, 178 and 180.
39. A method of detecting a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, comprising;
   a) contacting a host sample with the chimeric protein, under conditions wherein antibodies, if present, in the host sample bind to the chimeric protein forming antigen antibody complexes; and
   b) detecting the antigen antibody complexes.
40. The method of para 39, wherein the chimeric protein is encoded by a nucleic acid selected from the group consisting of: SEQ ID NOs: 145, 147; 149, 151, 153, 155, 157, 159, 161, 163, 165, 167, 169, 171, 173, 175, 177 and 179.
41. The method of para 39, wherein the chimeric protein comprises an amino acid sequence selected from the group consisting of SEQ ID NOs: 146, 148, 150, 152, 154, 156, 158, 160, 162, 164, 166, 168, 170, 172, 174, 176, 178 and 180.
42. A method of immunizing an animal or human against Lyme disease, comprising administering a composition comprising a chimeric protein of at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein.
43. The method of para 42, wherein the chimeric protein comprises an amino acid sequence selected from the group consisting of: SEQ ID NOs: 146, 148, 150, 152, 154, 156, 158, 160, 162, 164, 166, 168, 170, 172, 174, 176, 178 and 180.
44. A method of immunizing an animal against Lyme disease, comprising administering a composition comprising a nucleic acid that encodes a chimeric protein comprising a first and a second polypeptide, wherein the first polypeptide comprises *Borrelia burgdofferi* OspC and wherein the second polypeptide comprises *Borrelia burgdorferi* OspA, such that OspC comprises the N-terminus of the protein.
45. The method of para 44, wherein the nucleic acid encoding the chimeric protein is selected from the group consisting of: SEQ ID NOs: 145, 147, 149, 151, 153, 155, 157, 159, 161, 163, 165, 167, 169, 171, 173, 175, 177 and 179.
46. A physiological composition to vaccinate against and treat *Borrelia* infection in animals or humans, the composition comprising;
   a) a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, the amount being effective to ellicit an immune response to *Borrelia burgdorferi;*
   b) a physiologically-acceptable carrier or vehicle; and
   c) an adjuvant.
47. The composition of para 46, wherein the chimeric protein is selected from the group consisting of: SEQ ID NOs: 146, 148, 150, 152, 154, 156, 158, 160, 162, 164, 166, 168, 170, 172, 174, 176, 178 and 180.
48. A physiological composition to vaccinate against and treat *Borrelia* infection in animals or humans, the composition comprising;
   a) a nucleic acid that encodes a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, the amount being effective to elicit an immune response to *Borrelia burgdorferi;* and
   b) a physiologically-acceptable carrier or vehicle.
49. The composition of para 47, wherein the nucleic acid is selected from the group consisting of: SEQ ID NOs: 145, 147,149, 151, 153, 155, 157, 159, 161,163,165,167,169,171,173,175,177 and 179.
50. A kit comprising;
   a) a chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein; and
   b) reagents for detecting antibody antigen complexes formed between the chimeric protein and antibodies, if present, in a user-supplied host sample.
51. The kit of para 50, wherein the chimeric protein is selected from the group consisting of: SEQ ID NOs: 146, 148, 150, 152, 154, 156, 158, 160, 162, 164, 166, 168, 170, 172, 174, 176, 178 and 180.

SEQUENCE LISTING

<110> Research Foundation of the State University of New York
      Brookhaven Sciences Associates, LLC

<120> RECOMBINANT CONSTRUCTS OF BORRELIA BURDORFERI

<130> 15938EP div 1

<140> 07023008.1
<141> 2001-08-07

<150> US 60/226,484
<151> 2000-08-18

<150> US 09/666,017
<151> 2000-09-19

<160> 186

<170> FastSEQ for Windows Version 4.0

<210> 1
<211> 23
<212> DNA
<213> Artificial Sequence

<220>
<223> Oligonucleotide Primer

<400> 1
cttaatgact ctgacactag tgc                                              23

<210> 2
<211> 30
<212> DNA
<213> Artificial Sequence

<220>
<223> Oligonucleotide Primer

<400> 2
gctactaaaa aaaccgggaa atggaattca                                       30

<210> 3
<211> 33
<212> DNA
<213> Artificial Sequence

<220>
<223> Oligonucleotide Primer

<400> 3
gcagcttggg attcaaaaac atccacttta aca                                   33

<210> 4
<211> 18
<212> DNA
<213> Artificial Sequence

Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ala Gly Thr Ser Thr Leu Thr
    210                 215                 220
Ile Thr Val Asn Asn Lys Lys Thr Lys Ala Leu Val Phe Thr Lys Gln
225                 230                 235                 240
Asp Thr Ile Thr Ser Gln Lys Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Arg

## Claims

1. An unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, or the OspA polypeptide is either selected from the group consisting of: amino acid residue 18 to 273, and amino acid residue 132 to 273, wherein the amino acid numbering is based on the OspA polypeptide of SEQ ID NO: 7, or a chimeric polypeptide comprising OspA polypeptides from at least two different strains of *Borrelia burgdorferi.*

2. An unlipidated chimeric protein consisting of an OspC polypeptide and an OspA polypeptide from *Borrelia burgdorferi,* wherein the OspC polypeptide is linked to the N-terminus of the OspA polypeptide.

3. The chimeric protein of Claims 1 or 2, wherein:
(a) the OspA and OspC polypeptides are from the same strain of *Borrelia burgdorferi;* and/or
(b) the OspA and OspC polypeptides are from the same genospecies of *Borrelia burgdorferi;* and/or
(c) the OspA and OspC polypeptides are from different strains of *Borrelia burgdorferi;* and/or
(d) the OspA and OspC polypeptides are from different genospecies of *Borrelia burgdorferi;* and/or
(e) the chimeric OspA polypeptide comprises OspA polypeptides from different genospecies of *Borrelia burgdorferi.*

4. The chimeric protein of any one of Claims 1-3, wherein the OspA polypeptide comprises
(a) a *Borrelia burgdorferi* OspA polypeptide from amino acid residue 18 to amino acid residue 216 of a first strain of *Borrelia burgdorferi,* and from amino acid residue 217 to amino acid residue 273 of a second strain of *Borrelia burgdorferi;* or
(b) a *Borrelia burgdorferi* OspA polypeptide from amino acid residue 132 to amino acid residue 217 of a first strain of *Borrelia burgdorferi,* and a *Borrelia burgdorferi* OspA polypeptide from amino acid residue 218 to amino acid residue 273 of a second strain of *Borrelia burgdorferi,*
wherein the amino acid numbering is based on the OspA polypeptide sequence of SEQ ID NO: 7.

5. The chimeric protein of any one of Claims 1-4, wherein the OspA polypeptide comprises at least four separate OspA polypeptide fragments; and further optionally
wherein the first OspA polypeptide fragment comprises a *Borrelia burgdorferi* OspA polypeptide from amino acid residue 30 to amino acid residue 150, wherein the second OspA polypeptide fragment comprises a *Borrelia burgdorferi* OspA polypeptide from amino acid residue 151 to amino acid residue 179, wherein the third OspA polypeptide fragment comprises a *Borrelia burgdorferi* OspA polypeptide from amino acid residue 180 to amino acid residue 216, and wherein the fourth OspA polypeptide fragment comprises a *Borrelia burgdorferi* OspA polypeptide from amino acid residue 217 to amino acid residue 273, wherein the amino acid numbering is based on the OspA polypeptide sequence of SEQ ID NO: 7.

6. A chimeric protein comprising an amino acid sequence selected from the group consisting of: SEQ ID NOs: 146, 148, 150, 152, 154, 156, 158, 160, 162, 164, 166, 168, 170, 172, 174, 176, 178 and 180.

7. A nucleic acid encoding an unlipidated chimeric protein comprising:
at least a first and a second polypeptide, wherein the first polypeptide comprises *Borrelia burgdorferi* OspC and wherein the second polypeptide comprises *Borrelia burgdorferi* OspA, such that OspC comprises the N-terminus of the encoded protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, or
the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi.*

8. A nucleic acid encoding an unlipidated chimeric protein consisting of an OspC polypeptide and an OspA polypeptide from *Borrelia burgdorferi,* wherein the OspC polypeptide is linked to the N-terminus of the OspA polypeptide.

9. The nucleic acid of Claim 8, wherein the OspA polypeptide comprises amino acid residue 18 to amino acid residue 216 of a first strain of *Borrelia burgdorferi* and from amino acid residue 217 to amino acid residue 273 of a second strain of *Borrelia burgdorferi,* or from amino acid residue 132 to amino acid residue 216 of a first strain of *Borrelia burgdorferi* and amino acid residue 217 to amino acid residue 273 of a second strain of *Borrelia burgdorferi,* wherein the amino acid numbering is based on the OspA polypeptide sequence of SEQ ID NO: 7.

10. The nucleic acid of Claim 8 or 9, wherein the OspA polypeptide comprises at least four separate OspA polypeptide fragments, wherein the first OspA polypeptide fragment comprises a *Borrelia burgdorferi* OspA polypeptide from amino acid residue 30 to amino acid residue 150, wherein the second OspA polypeptide comprises a *Borrelia burgdorferi* OspA polypeptide from amino acid residue 151 to amino acid residue 179, wherein the third OspA polypeptide comprises a *Borrelia burgdorferi* OspA polypeptide from amino acid residue 181 to amino acid residue 216, and wherein the fourth OspA polypeptide comprises a *Borrelia burgdorferi* OspA polypeptide from amino acid residue 216 to amino acid residue 273, wherein the amino acid numbering is based on the OspA polypeptide sequence of SEQ ID NO: 7.

11. A nucleic acid of any one of Claims 8-10, wherein:
(a) the encoded OspC and OspA polypeptides are from different strains of *Borrelia burgdorferi* and/or the encoded OspC and OspA polypeptides are from different genospecies of *Borrelia burgdorferi;* and/or
(b) the encoded OspC and OspA polypeptides are from the same strains of *Borrelia burgdorferi;* and/or
(c) the encoded OspC and OspA polypeptides are from the same genospecies of *Borrelia burgdorferi.*

12. A nucleic acid selected from the group consisting of: SEQ ID NOs: 145, 147, 149, 151, 153, 155, 157, 159, 161, 163, 165, 167, 169, 171, 173, 175, 177 and 179.

13. An expression vector comprising either:
(a) an isolated DNA having a nucleotide sequence selected from the group consisting of: SEQ ID NOs: 145, 147, 149, 151, 153, 155, 157, 159, 161, 163, 165, 167, 169, 171, 173, 175, 177 and 179; or
(b) an isolated DNA encoding a polypeptide having an amino acid sequence selected from the group consisting of: SEQ ID NOs: 146, 148, 150, 152, 154, 156, 158, 160, 162, 164, 166, 168, 170, 172, 174, 176, 178 and 180.

14. A host cell comprising a recombinant nucleic acid either:
(a) having a nucleotide sequence selected from the group consisting of: SEQ ID NOs: 145, 147, 149, 151, 153, 155, 157, 159, 161, 163, 165, 167, 169, 171, 173, 175, 177 and 179; or
(b) encoding a protein having an amino acid sequence selected from the group consisting of: SEQ ID NOs: 146, 148, 150, 152, 154, 156, 158, 160, 162, 164, 166, 168, 170, 172, 174, 176, 178 and 180.

15. A method of producing an unlipidated chimeric *Borrelia burgdorferi* protein comprising;
(a) selecting a polynucleotide encoding OspC or an antigenic portion thereof;
(b) selecting a polynucleotide encoding OspA or an antigenic portion thereof; and
(c) ligating the polynucleotide of a) to the polynucleotide of b), to form a chimeric polynucleotide sequence encoding a chimeric OspC-OspA protein such that OspC is upstream of OspA, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, or the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi.*

16. The method of Claim 15, wherein:
(a) the chimeric OspC-OspA protein has an amino acid sequence selected from the group consisting of: SEQ ID NOs: 146, 148, 150, 152, 154, 156, 158, 160, 162, 164, 166, 168, 170, 172, 174, 176, 178 and 180; and/or
(b) the method for producing a chimeric protein further comprising maintaining a host cell comprising the chimeric polynucleotide of Claim 17 under conditions suitable for expression of the polynucleotide.

17. An unlipidated chimeric protein for use in immunizing an animal against Lyme's Disease, comprising at least a first and a second polypeptide, the first polypeptide comprises *Borrelia burgdorferi* OspC and wherein the second polypeptide comprises *Borrelia burgdorferi* OspA, such that OspC comprises the N-terminus of the protein, comprising administering the chimeric protein to an animal, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, or
the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi.*

18. The chimeric protein of Claim 17, wherein:
(a) the chimeric protein is administered with a physiologically-acceptable vehicle or carrier;
(b) the chimeric protein is encoded by a nucleic acid selected from the group consisting of: SEQ ID NOs: 145, 147, 149, 151, 153, 155, 157, 159, 161, 163, 165, 167, 169, 171, 173, 175, 177 and 179; or
(c) the chimeric protein comprises an amino acid sequence selected from the group consisting of: SEQ ID NOs: 146, 148, 150, 152, 154, 156, 158, 160, 162, 164, 166, 168, 170, 172, 174, 176, 178 and 180.

19. A method of detecting an unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, comprising;
(a) contacting a host sample with the chimeric protein, under conditions wherein antibodies, if present, in the host sample bind to the chimeric protein forming antigen antibody complexes; and
(b) detecting the antigen antibody complexes,
wherein the OspC polypeptide is selected from the group consisting of:
amino acid residue 19 to amino acid residue 211, amino acid residue 19 to 204, and amino acid residue 19 to 213, wherein the amino acid numbering is based on the OspC polypeptide of SEQ ID NO: 30, or
the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi.*

20. The method of Claim 19, wherein:
(a) the chimeric protein is encoded by a nucleic acid selected from the group consisting of: SEQ ID NOs: 145, 147, 149, 151, 153, 155, 157, 159, 161, 163, 165, 167, 169, 171, 173, 175, 177 and 179; or
(b) the chimeric protein comprises an amino acid sequence selected from the group consisting of: SEQ ID NOs: 146, 148, 150, 152, 154, 156, 158, 160, 162, 164, 166, 168, 170, 172, 174, 176, 178 and 180.

21. A nucleic acid that encodes an unlipidated chimeric protein comprising a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, for use in immunizing an animal against Lyme Disease,
wherein the OspC polypeptide is selected from the group consisting of:
amino acid residue 19 to amino acid residue 211, amino acid residue 19 to 204, and amino acid residue 19 to 213, wherein the amino acid numbering is based on the OspC polypeptide of SEQ ID NO: 30, or
the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi* and optionally
wherein the nucleic acid encoding the chimeric protein is selected from the group consisting of: SEQ ID NOs: 145, 147, 149, 151, 153, 155, 157, 159, 161, 163, 165, 167, 169, 171, 173, 175, 177 and 179.

22. A physiological composition to vaccinate against and treat *Borrelia* infection in animals or humans, the composition comprising;
(a) either (i) an unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, or
the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi,* the amount being effective to elicit an immun Type your question here and then click Searche response to *Borrelia burgdorferi;* or (ii) a nucleic acid encoding said chimeric protein;
(b) a physiologically-acceptable carrier or vehicle; and optionally
(c) an adjuvant.

23. The composition of Claim 24, wherein either
(a) the chimeric protein is selected from the group consisting of: SEQ ID NOs: 146, 148, 150, 152, 154, 156, 158, 160, 162, 164, 166, 168, 170, 172, 174, 176, 178 and 180; and/or
(b) the nucleic acid is selected from the group consisting of: SEQ ID NOs: 145, 147, 149, 151, 153, 155, 157, 159, 161, 163, 165, 167, 169, 171, 173, 175, 177 and 179.

24. A kit comprising;
(a) an unlipidated chimeric protein comprising at least a first and a second polypeptide, wherein the first polypeptide comprises *Borrelia burgdorferi* OspC and wherein the second polypeptide comprises *Borrelia burgdorferi* OspA, such that OspC comprises the N-terminus of the protein, wherein the OspC polypeptide is selected from the group consisting of: amino acid residue 19 to amino acid residue 211, amino acid residue 19 to 204, and amino acid residue 19 to 213, wherein the amino acid numbering is based on the OspC polypeptide of SEQ ID NO: 30, or
the OspA polypeptide is selected from the group consisting of: amino acid residue 18 to 273, and amino acid residue 132 to 273, wherein the amino acid numbering is based on the OspA polypeptide of SEQ ID NO: 7, or
the OspA polypeptide is a chimeric polypeptide comprising OspA polypeptides from at least two different strains of *Borrelia burgdorferi,* and
(b) reagents for detecting antibody antigen complexes formed between the chimeric protein and antibodies, if present, in a user-supplied host sample.

25. The kit of Claim 24, wherein the chimeric protein is selected from the group consisting of: SEQ ID NOs: 146, 148, 150, 152, 154, 156, 158, 160, 162, 164, 166, 168, 170, 172, 174, 176, 178 and 180.
